# EUROPEAN PATENT SPECIFICATION

(11) **EP 4 600 325 B1**
(45) Date of publication and mention of the grant of the patent: **29.04.2026**
(21) Application number: 24793970.5
(22) Date of filing: 04.03.2024
(51) Int. Cl.: C09K 11/06, H10K 50/16, H10K 85/60, C07D 401/04, C07D 251/24

(54) **ORGANIC LIGHT-EMITTING COMPOUND, AND ORGANIC ELECTROLUMINESCENT DEVICE COMPRISING SAME**
ORGANISCHE LICHTEMITTIERENDE VERBINDUNG UND ORGANISCHE ELEKTROLUMINESZENZVORRICHTUNG DAMIT
COMPOSÉ ÉLECTROLUMINESCENT ORGANIQUE ET DISPOSITIF ÉLECTROLUMINESCENT ORGANIQUE LE COMPRENANT

(30) Priority: 20.12.2023 KR 20230186800
(43) Date of publication of application: 13.08.2025
(73) Proprietor: Solus Advanced Materials Co., Ltd., Iksan-si, Jeollabuk-do 54584 (KR)
(72) Inventor: LEE, Ui Geon, Gyeonggi-do 13636 (KR); SON, Hyo Suk, Gyeonggi-do 13636 (KR); SIM, Sang Eun, Gyeonggi-do 13636 (KR); YANG, Seung Gyu, Gyeonggi-do 13636 (KR); YU, Eun Soo, Gyeonggi-do 13636 (KR); LEE, Yong Hwan, Gyeonggi-do 13636 (KR); HYEONG, Do Seong, Gyeonggi-do 13636 (KR); SIM, Jae Yi, Gyeonggi-do 13636 (KR)
(74) Representative: Nordic Patent Service A/S
(86) International application number: PCT/KR2024/002729
(87) International publication number: WO 2025/135308

(56) References cited:
- EP-A1- 4 046 992
- WO-A1-2021/075775
- WO-A1-2021/075775
- WO-A1-2021/256871
- CN-A- 112 094 226
- CN-A- 112 094 226
- CN-A- 114 763 341
- KR-A- 20200 142 220
- KR-A- 20220 008 636
- KR-A- 20220 008 636

## Description

### [Technical Field

### CROSS-REFERENCE TO THE RELATED APPLICATION

The present application claims priority to and the benefit of Korean Patent Application No. 10-2023-0186800, filed on December 20, 2023, in the Korean Intellectual Property Office.

### Technical Field

Embodiments of the present disclosure relate to a novel organic light-emitting compound and an organic electroluminescent device containing the same.

### [Background Art]

Since luminescence from an organic film was first identified by Bernanose in the 1950s, research was conducted on organic electroluminescent (EL) devices based on blue electroluminescence using anthracene single crystals in 1965 and an organic electroluminescent device with a stack structure divided into functional layers including a hole layer and a light-emitting layer was suggested by Tang in 1987. Since then, organic electroluminescent devices including respective characteristic organic layers in order to impart high efficiency and long lifespan to the devices and specific materials used for the devices have been developed. For example CN114763341A and KR20220008636A disclose the use of organic light-emitting compounds in OLEDs.

When a voltage is applied between two electrodes in an organic electroluminescent device, holes are injected from an anode and electrons are injected from the cathode into an organic layer. When the injected holes combine with electrons, excitons are formed. When the excitons fall to the ground state, light is emitted. In this case, the material used for the organic material layer may be divided into a light-emitting material, a hole injection material, a hole transport material, an electron transport material, an electron injection material, and the like, depending on the function thereof.

Light-emitting materials for organic electroluminescent devices may be classified into blue, green, and red light-emitting materials depending on the color of the light. In addition, yellow and orange light-emitting materials are also used as light-emitting materials to realize better natural colors. In addition, a host/dopant system may be used as a light-emitting material in order to increase color purity and luminous efficacy through energy transfer.

Dopant materials may be divided into fluorescent dopants using organic materials and phosphorescent dopants using metal complex compounds containing heavy atoms such as Ir and Pt. Since such phosphorescent materials can theoretically improve luminous efficacy by up to four times of fluorescent materials, a great deal of research is being conducted on phosphorescent host materials as well as phosphorescent dopant materials.

NPB, BCP, Alq₃, and the like are widely known to date as materials for a hole injection layer, a hole transport layer, a hole blocking layer and an electron transport layer, and anthracene derivatives are reported as light-emitting layer materials. In particular, among light-emitting layer materials, metal complex compounds containing Ir such as FIrpic, Ir(ppy)₃, and (acac)Ir(btp)₂, which have an advantage of improving efficiency, are used as blue, green, and red phosphorescent dopant materials, and 4,4-dicarbazolybiphenyl (CBP) is used as a phosphorescent host material.

As such, conventional organic layer materials are advantageous in terms of luminescence characteristics, but are unsatisfactory to improve lifespan of organic electroluminescent devices due to low glass transition temperature and thus very poor thermal stability.

Therefore, there is a need for development of organic layer materials with excellent performance.

### Prior Art Literature

Korean Patent Laid-open Publication No. 10-2015-0069346

### [Disclosure]

### [Technical Problem]

Embodiments of the present disclosure provide novel compounds that may be used as materials for organic layers of organic electroluminescent devices with excellent thermal stability, electron injection and transport ability, and luminescence performance, specifically as materials for an electron transport layer and an electron transport auxiliary layer, and uses thereof.

Embodiments of the present disclosure provide organic electroluminescent devices with significantly improved luminous performance, driving voltage, lifespan, and efficiency including the novel organic light-emitting compounds.

It should be noted that objects of the present disclosure are not limited to the object as mentioned above, and other unmentioned objects of the present disclosure will be clearly understood by those skilled in the art from the following description.

### [Technical Solution]

Embodiments of the present disclosure provide an organic light-emitting compound represented by the following Formula 1:

The organic light-emitting compound represented by the following Formula 1:

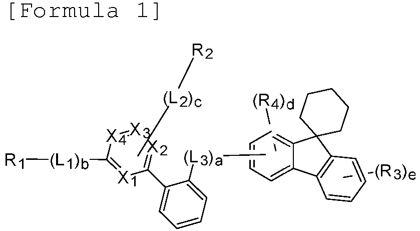

wherein
one of X₁ to X₄ is C-(L₂)_{c}-R₂, the others are each independently N or CR, and at least two of X₁ to X₄ are N, wherein R is hydrogen, an alkyl group containing 1 to 40 carbon atoms, an aryl group containing 6 to 60 carbon atoms, or a heteroaryl group containing 2 to 60 carbon atoms, each of which is unsubstituted or substituted,
R₁ and R₂ are each independently hydrogen, an alkenyl group containing 2 to 40 carbon atoms, an alkynyl group containing 2 to 40 carbon atoms, a cycloalkyl group containing 3 to 40 carbon atoms, a heterocycloalkyl group containing 1 to 40 carbon atoms, an alkyl group containing 1 to 40 carbon atoms, an aryl group containing 6 to 60 carbon atoms, a heteroaryl group containing 2 to 60 carbon atoms, an alkylsilyl group containing 1 to 40 carbon atoms, an arylsilyl group containing 6 to 60 carbon atoms, an alkyl boron group containing 1 to 40 carbon atoms, an aryl boron group containing 6 to 60 carbon atoms, an arylphosphine group containing 6 to 60 carbon atoms, an alkylphosphine oxide group containing 1 to 40 carbon atoms, an arylphosphine oxide group containing 6 to 60 carbon atoms, an alkylamine group containing 1 to 40 carbon atoms, or an arylamine group containing 6 to 60 carbon atoms, each of which is unsubstituted or substituted,
L₁ to L₃ are each independently a single bond, an arylene group containing 6 to 60 carbon atoms, or a heteroarylene group containing 2 to 60 carbon atoms, each of which is unsubstituted or substituted,
R₃ and R₄ are each independently an aryl group containing 6 to 60 carbon atoms substituted with a cyano group, an aryl group containing 6 to 60 carbon atoms substituted with a nitrogen-containing heteroaryl group containing 2 to 60 carbon atoms, a nitrogen-containing heteroaryl group containing 2 to 60 carbon atoms, deuterium or a cyano group, each of which is unsubstituted or substituted,
a to c are each independently an integer from 0 to 5, and
d is an integer of 0 to 3, and e is an integer of 0 to 4, with the proviso of d+e ≥ 1.

Embodiments of the present disclosure provide an organic electroluminescent device containing the organic light-emitting compound.

Embodiments of the present disclosure provide the use of the organic light-emitting compound for an organic electroluminescent device.

### [Advantageous Effects]

The organic light-emitting compound according to the present disclosure contains an azine group, which is a strong electron acceptor in the molecular structure thereof, improves the ability to transport electrons to the light-emitting layer due to excellent electron injection and transport ability, and thus imparts excellent driving voltage to the device.

In addition, the organic light-emitting compound according to the present disclosure exhibits excellent thermal stability because it has an aromatic hydrocarbon group and a condensed structure rather than a simple aliphatic ring group, and is effective in improving the processability as well as lifespan of the device because it does not have a crystallization temperature (Tc).

In addition, the organic light-emitting compound according to the present disclosure has a structure in which a cyclohexylfluorene group substituted with a cyano group or a pyridine group is linked to an azine moiety at the ortho position through a linker, and thus improves stability and the dipole moment. As a result, the organic light-emitting compound can increase interaction with the cathode, thus improving electron generation in the device and effectively improving the driving voltage characteristics.

In addition, the organic electroluminescent device containing the organic light-emitting compound according to the present disclosure can achieve a low driving voltage and greatly improve luminous performance, lifespan, and efficiency, thereby being more effectively applied to full-color display panels and the like.

The effects of the present disclosure are not limited to those described above, and other unmentioned technical effects will be apparent to those skilled in the art from the following description.

### [Best Mode]

The advantages and features of the present disclosure and methods of accomplishing the same will be clearly understood from the following embodiments described in detail with reference to the attached drawings. However, the present disclosure is not limited to the embodiments and may be embodied in different forms. The embodiments are suggested only to offer a thorough and complete understanding of the disclosure and to sufficiently inform those skilled in the art of the technical concept of the present disclosure and the present disclosure is defined only by the scope of claims.

The terms used herein are provided only to illustrate the exemplary embodiments and should not be construed as limiting the scope of the disclosure. Singular forms are intended to include plural forms as well, unless the context clearly indicates otherwise. It will be further understood that the terms "comprises" and/or "comprising" used herein do not preclude the presence or addition of one or more other components in addition to the mentioned components.

Unless defined otherwise, all terms used herein (including technical or scientific terms) have the same meanings as generally understood by those skilled in the art to which the present disclosure pertains. In addition, terms identical to those defined in generally used dictionaries should be interpreted as having meanings identical to contextual meanings of the related art, and are not interpreted as having ideal or excessively formal meanings unless they are definitely defined in the present disclosure.

Hereinafter, embodiments of the present disclosure will be described in detail.

Prior to the description, the meanings of terms used herein will be briefly described. However, the explanation of terms is provided for better understanding of the present disclosure and the terms should not be construed as limiting the technical idea of the present disclosure unless the context clearly indicates the terms are used to limit the scope of the present disclosure.

As used herein, the term "aryl group" refers to a monovalent functional group derived from an aromatic hydrocarbon. The aryl group, for example, means a phenyl group, a naphthyl group, an anthracenyl group, a naphthacenyl group, a pyrenyl group, a tolyl group, a biphenyl group, a terphenyl group, a triphenylenyl group, a chrysenyl group, a spirobifluorenyl group, a fluoranthenyl group, a fluorenyl group, a perylenyl group, an indenyl group, an azulenyl group, a heptalenyl group, a phenalenyl group, a phenanthrenyl group, or the like, but is not limited thereto. In addition, the term "arylene group" refers to a divalent functional group derived from an aromatic hydrocarbon.

As used herein, the term "heteroaryl group" may refer to a monovalent functional group derived from an aromatic heterocycle having a monocyclic or condensed cyclic structure, and the heteroaryl group may include, as a heteroatom, at least one of nitrogen (N), sulfur (S), oxygen (O), phosphorus (P), selenium (Se), or silicon (Si), in addition to carbon atoms. Specific examples of the heteroaryl group include: a nitrogen-containing heteroaryl group including a pyrrolyl group, a pyridyl group, a pyridazinyl group, a triazinyl group, a pyrimidinyl group, a pyrazinyl group, a naphthyridyl group, an acenaphthopyridyl group, a triazolyl group, a tetrazolyl group, a benzotriazolyl group, a pyrazolyl group, an imidazolyl group, a benzimidazolyl group, an indolyl group, an isoindolyl group, an indolizinyl group, a purinyl group, an indazolyl group, a quinolyl group, a benzoquinolyl group, an isoquinolinyl group, a quinolizinyl group, a phthalazinyl group, a naphthylidinyl group, a quinoxalinyl group, a quinazolinyl group, a cinnolinyl group, a pteridinyl group, an imidazotriazinyl group, an acridinyl group, a phenanthridyl group, a carbazolyl group, a phenanthrolinyl group, a phenazinyl group, an imidazopyridyl group, an imidazopyrimidinyl group, a pyrazolopyridyl group, a heptaazaphenalenyl group or the like; a sulfur-containing heteroaryl group including a thienyl group, a benzothiophenyl group, a dibenzothiophenyl group, a benzonaphthothiophenyl group or the like; an oxygen-containing heteroaryl group including a furyl group, a pyranyl group, a benzofuranyl group, an isobenzofuranyl group, a dibenzofuranyl group, a benzonaphthofuranyl group, an oxanthrenyl group, a xanthenyl group, a benzoxanthenyl group or the like; a heteroaryl group containing a combination of oxygen and sulfur, including a thiadiazolyl group, an oxadiazolyl group, a phenoxathiinyl group, a benzothienopyrimidinyl group, a benzofuropyridyl group or the like. In addition, the term "heteroarylene group" refers to a divalent functional group derived from an aromatic hydrocarbon containing at least one of nitrogen (N), sulfur (S), oxygen (O), phosphorus (P), selenium (Se), or silicon (Si) as a heteroatom. The heteroaryl group and heteroarylene group may be defined using the number of nuclear atoms, instead of the number of carbon atoms. Here, the number of nuclear atoms may mean the number of atoms including one or more heteroatoms selected from the group consisting of nitrogen (N), sulfur (S), oxygen (O), phosphorus (P), selenium (Se), and silicon (Si), in addition to carbon (C). For example, the number of nuclear atoms in each of the heteroaryl group and the heteroarylene group may be 5 to 60, 5 to 30, or 5 to 20.

As used herein, the term "alkyl group" refers to a monovalent functional group derived from a saturated hydrocarbon having a linear or branched structure. The alkyl group is, for example, a methyl group, an ethyl group, an n-propyl group, an isopropyl group, an n-butyl group, a sec-butyl group, a tert-butyl group, an n-pentyl group, a 1,1-dimethylpropyl group, a 1,2-dimethylpropyl group, a 2,2-dimethylpropyl group, a 1-ethylpropyl group, a 2-ethylpropyl group, an n-hexyl group, a 1-methyl-2-ethylpropyl group, a 1-ethyl-2-methylpropyl group, a 1,1,2-trimethylpropyl group, a 1-propylpropyl group, a 1-methylbutyl group, a 2-methylbutyl group, a 1,1-dimethylbutyl group, a 1,2-dimethylbutyl group, a 2,2-dimethylbutyl group a 1,3-dimethylbutyl group, a 2,3-dimethylbutyl group, a 2-ethylbutyl group, a 2-methylpentyl group, a 3-methylpentyl group or the like, but is not limited thereto.

As used herein, the term "cycloalkyl group" refers to a monovalent functional group derived from a saturated hydrocarbon with a ring structure. The cycloalkyl group is, for example, a cyclopropyl group, a cyclobutyl group, a cyclopentyl group, a cyclohexyl group, a cycloheptyl group, a cyclooctyl group, a bicyclononyl group, an adamantyl group or the like, but is not limited thereto.

As used herein, the term "heterocycloalkyl group" may refer to a monovalent functional group derived from a saturated hydrocarbon having a cyclic structure, and the heterocycloalkyl group may include, as a heteroatom, at least one of nitrogen (N), sulfur (S), oxygen (O), phosphorus (P), selenium (Se), or silicon (Si), in addition to carbon atoms. The heterocycloalkyl group may be defined using the number of nuclear atoms, instead of the number of carbon atoms. Here, the number of nuclear atoms may mean the number of atoms including one or more heteroatoms selected from the group consisting of nitrogen (N), sulfur (S), oxygen (O), phosphorus (P), selenium (Se), and silicon (Si) in addition to carbon (C) atoms. For example, the number of nuclear atoms in the heterocycloalkyl group may be 5 to 60, 5 to 30, or 5 to 20.

As used herein, the term "alkenyl group" refers to a monovalent functional group derived from a hydrocarbon containing one or more carbon double bonds at the center or end of the alkyl group.

As used herein, the term "alkynyl group" refers to a monovalent functional group derived from a hydrocarbon containing one or more carbon triple bonds at the middle or end of the alkyl group.

As used herein, the terms "alkylsilyl group" and "arylsilyl group" refer to monovalent functional groups derived from compounds in which at least one hydrogen of silane is substituted with the above-described alkyl group or aryl group, respectively.

As used herein, the terms "alkylamine group" and "arylamine group" refer to monovalent functional groups derived from compounds in which at least one hydrogen of the amine is substituted with the above-described alkyl group or aryl group, respectively.

As used herein, the terms "alkyl boron group" and "aryl boron group" refer to monovalent functional groups derived from compounds in which at least one hydrogen of borane is substituted with the above-described alkyl group or aryl group, respectively.

As used herein, the term "arylphosphine group" refers to a monovalent functional group derived from a compound in which phosphine is replaced with the above-described aryl group.

As used herein, the terms "alkylphosphine oxide group" and "arylphosphine oxide group" refer to monovalent functional groups derived from compounds in which phosphine oxide is substituted with the above-described alkyl group or aryl group, respectively.

As used herein, the term "substitution" refers to substitution with at least one substituent selected from the group consisting of deuterium, an alkenyl group containing 2 to 20 carbon atoms, an alkynyl group containing 2 to 20 carbon atoms, a cycloalkyl group containing 3 to 20 carbon atoms, a heterocycloalkyl group containing 1 to 20 carbon atoms, a heterocycloalkyl group containing 3 to 20 nuclear atoms, an alkyl group containing 1 to 20 carbon atoms, an aryl group containing 6 to 30 carbon atoms, a heteroaryl group containing 2 to 30 carbon atoms, a heteroaryl group containing 5 to 30 nuclear atoms, an alkylsilyl group containing 1 to 20 carbon atoms, an arylsilyl group containing 6 to 30 carbon atoms, an alkyl boron group having 1 to 20 carbon atoms, an aryl boron group having 6 to 30 carbon atoms, an arylphosphine group having 6 to 30 carbon atoms, an alkylphosphine oxide group having 1 to 20 carbon atoms, an arylphosphine oxide group having 6 to 30 carbon atoms, an alkylamine group having 1 to 20 carbon atoms, or an arylamine group having 6 to 30 carbon atoms. In substitution with a plurality of substituents, the substituents may be the same as or different from each other.

### <Organic light-emitting compound>

The present disclosure provides a novel organic light-emitting compound. The organic light-emitting compound is represented by the following formula (1). 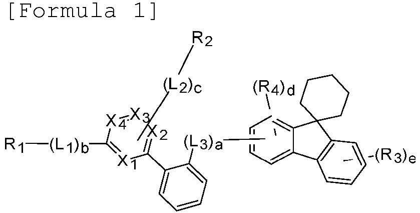 wherein
one of X₁ to X₄ is C-(L₂)_{c}-R₂, the others are each independently N or CR, and at least two of X₁ to X₄ are N, wherein R is hydrogen, an alkyl group containing 1 to 40 carbon atoms, an aryl group containing 6 to 60 carbon atoms, a heteroaryl group containing 2 to 60 carbon atoms, or a heteroaryl group containing 5 to 60 nuclear atoms, each of which is unsubstituted or substituted,
R₁ and R₂ are each independently hydrogen, an alkenyl group containing 2 to 40 carbon atoms, an alkynyl group containing 2 to 40 carbon atoms, a cycloalkyl group containing 3 to 40 carbon atoms, a heterocycloalkyl group containing 1 to 40 carbon atoms, a heterocycloalkyl group containing 3 to 40 nuclear atoms, an alkyl group containing 1 to 40 carbon atoms, an aryl group containing 6 to 60 carbon atoms, a heteroaryl group containing 2 to 60 carbon atoms, a heteroaryl group containing 5 to 60 nuclear atoms, an alkylsilyl group containing 1 to 40 carbon atoms, an arylsilyl group containing 6 to 60 carbon atoms, an alkyl boron group containing 1 to 40 carbon atoms, an aryl boron group containing 6 to 60 carbon atoms, an arylphosphine group containing 6 to 60 carbon atoms, an alkylphosphine oxide group containing 1 to 40 carbon atoms, an arylphosphine oxide group containing 6 to 60 carbon atoms, an alkylamine group containing 1 to 40 carbon atoms, or an arylamine group containing 6 to 60 carbon atoms, each of which is unsubstituted or substituted,
L₁ to L₃ are each independently a single bond, an arylene group containing 6 to 60 carbon atoms, a heteroarylene group containing 2 to 60 carbon atoms, or a heteroarylene group containing 5 to 60 nuclear atoms, each of which is unsubstituted or substituted,
R₃ and R₄ are each independently an aryl group containing 6 to 60 carbon atoms substituted with a cyano group, an aryl group containing 6 to 60 carbon atoms substituted with a nitrogen-containing heteroaryl group containing 2 to 60 carbon atoms, an aryl group containing 6 to 60 carbon atoms substituted with a nitrogen-containing heteroaryl group containing 5 to 60 nuclear atoms, a nitrogen-containing heteroaryl group containing 2 to 60 carbon atoms, a nitrogen-containing heteroaryl group containing 5 to 60 nuclear atoms, deuterium or a cyano group, each of which is unsubstituted or substituted,
a to c are each independently an integer from 0 to 5, and
d is an integer of 0 to 3, and e is an integer of 0 to 4, with the proviso of d+e ≥ 1.

In Formula 1, when a, b or c is 0, it means a single bond.

In Formula 1, when d is 0, the hydrogen present in the benzene ring that may be substituted with R₄ is not substituted with R₄, and when e is 0, the hydrogen present in the benzene ring that may be substituted with R₃ is not substituted with R₃.

Specifically, at least one of hydrogen, an alkenyl group, an alkynyl group, an alkyl group, a cycloalkyl group, a heterocycloalkyl group, an aryl group, a heteroaryl group, an alkylsilyl group, an arylsilyl group, an alkyl boron group, an aryl boron group, an alkylamine group, an arylamine group, an arylphosphine group, an alkylphosphine oxide group, or an arylphosphine oxide group that may be present as R, R₁ to R₄ and L₁ to L₃ is each independently not substituted, or substituted with at least one substituent selected from the group consisting of deuterium, an alkenyl group containing 2 to 20 carbon atoms, an alkynyl group containing 2 to 20 carbon atoms, a cycloalkyl group containing 3 to 20 carbon atoms, a heterocycloalkyl group containing 1 to 20 carbon atoms, a heterocycloalkyl group containing 3 to 20 nuclear atoms, an alkyl group containing 1 to 20 carbon atoms, an aryl group containing 6 to 30 carbon atoms, a heteroaryl group containing 2 to 30 carbon atoms, a heteroaryl group containing 5 to 30 nuclear atoms, an alkylsilyl group containing 1 to 20 carbon atoms, an arylsilyl group containing 6 to 30 carbon atoms, an alkyl boron group having 1 to 20 carbon atoms, an aryl boron group having 6 to 30 carbon atoms, an arylphosphine group having 6 to 30 carbon atoms, an alkylphosphine oxide group having 1 to 20 carbon atoms, an arylphosphine oxide group having 6 to 30 carbon atoms, an alkylamine group having 1 to 20 carbon atoms, or an arylamine group having 6 to 30 carbon atoms. In substitution with a plurality of substituents, the substituents may be the same as or different from each other.

In an embodiment, in Formula 1,
one of X₁ to X₄ is C-(L₂)_{c}-R₂, the others are each independently N or CR, and at least two of X₁ to X₄ are N, wherein R is hydrogen, an alkyl group containing 1 to 20 carbon atoms, an aryl group containing 6 to 30 carbon atoms, a heteroaryl group containing 2 to 30 carbon atoms, or a heteroaryl group containing 5 to 30 nuclear atoms,
R₁ and R₂ are each independently an alkenyl group containing 2 to 40 carbon atoms, an alkynyl group containing 2 to 20 carbon atoms, a cycloalkyl group containing 3 to 20 carbon atoms, a heterocycloalkyl group containing 1 to 20 carbon atoms, a heterocycloalkyl group containing 3 to 20 nuclear atoms, an alkyl group containing 1 to 20 carbon atoms, an aryl group containing 6 to 30 carbon atoms, a heteroaryl group containing 2 to 30 carbon atoms, a heteroaryl group containing 5 to 30 nuclear atoms, an alkylsilyl group containing 1 to 20 carbon atoms, an arylsilyl group containing 6 to 30 carbon atoms, an alkyl boron group containing 1 to 20 carbon atoms, an aryl boron group containing 6 to 30 carbon atoms, an arylphosphine group containing 6 to 30 carbon atoms, an arylphosphine oxide group containing 1 to 20 carbon atoms, an arylphosphine oxide group containing 6 to 30 carbon atoms, an alkylamine group containing 1 to 20 carbon atoms, or an arylamine group containing 6 to 30 carbon atoms, each of which is unsubstituted or substituted with deuterium, an alkenyl group containing 2 to 20 carbon atoms, an alkynyl group containing 2 to 20 carbon atoms, a cycloalkyl group containing 3 to 20 carbon atoms, a heterocycloalkyl group containing 1 to 20 carbon atoms, a heterocycloalkyl group containing 3 to 20 nuclear atoms, an alkyl group containing 1 to 20 carbon atoms, an aryl group containing 6 to 30 carbon atoms, a heteroaryl group containing 2 to 30 carbon atoms, a heteroaryl group containing 5 to 30 nuclear atoms, an alkylsilyl group containing 1 to 20 carbon atoms, an arylsilyl group containing 6 to 30 carbon atoms, an alkyl boron group having 1 to 20 carbon atoms, an aryl boron group having 6 to 30 carbon atoms, an arylphosphine group having 6 to 30 carbon atoms, an alkylphosphine oxide group having 1 to 20 carbon atoms, an arylphosphine oxide group having 6 to 30 carbon atoms, an alkylamine group having 1 to 20 carbon atoms, or an arylamine group having 6 to 30 carbon atoms,
L₁ to L₃ are each independently a single bond, an arylene group containing 6 to 30 carbon atoms, a heteroarylene group containing 2 to 30 carbon atoms, or a heteroarylene group containing 5 to 30 nuclear atoms,
R₃ and R₄ are each independently an aryl group containing 6 to 30 carbon atoms substituted with a cyano group, an aryl group containing 6 to 30 carbon atoms substituted with a nitrogen-containing heteroaryl group containing 2 to 30 carbon atoms, an aryl group containing 6 to 30 carbon atoms substituted with a nitrogen-containing heteroaryl group containing 5 to 30 nuclear atoms, a nitrogen-containing heteroaryl group containing 2 to 30 carbon atoms, a nitrogen-containing heteroaryl group containing 5 to 30 nuclear atoms, deuterium or a cyano group, each of which is unsubstituted or substituted,
a to c are each independently an integer from 0 to 2, and
d is an integer of 0 to 2 and e is an integer of 0 to 2, with the proviso of d+e ≥ 1.

In Formula 1, when a, b or c is 0, it means a single bond.

In Formula 1, when d is 0, the hydrogen present in the benzene ring that may be substituted with R₄ is not substituted with R₄, and when e is 0, the hydrogen present in the benzene ring that may be substituted with R₃ is not substituted with R₃.

In an embodiment, in Formula 1,
one of X₁ to X₄ is C-(L₂)_{c}-R₂, the others are each independently N or CH, and at least two of X₁ to X₄ are N,
R₁ and R₂ are each independently an aryl group containing 6 to 30 carbon atoms, a heteroaryl group containing 2 to 30 carbon atoms, or a heteroaryl group containing 5 to 30 nuclear atoms, each of which is unsubstituted or substituted with a cycloalkyl group containing 3 to 20 carbon atoms, an alkyl group containing 1 to 20 carbon atoms, an aryl group containing 6 to 30 carbon atoms, a heteroaryl group containing 2 to 30 carbon atoms, a heteroaryl group containing 5 to 30 nuclear atoms, an alkylsilyl group containing 1 to 20 carbon atoms, an arylsilyl group containing 6 to 30 carbon atoms, an alkylphosphine oxide group containing 1 to 20 carbon atoms, or an arylphosphine oxide group containing 6 to 30 carbon atoms,
L₁ to L₃ are each independently a single bond, an arylene group containing 6 to 30 carbon atoms, a heteroarylene group containing 2 to 30 carbon atoms, or a heteroarylene group containing 5 to 30 nuclear atoms,
R₃ and R₄ are each independently an aryl group containing 6 to 30 carbon atoms substituted with a cyano group, a nitrogen-containing heteroaryl group containing 2 to 30 carbon atoms, a nitrogen-containing heteroaryl group containing 5 to 30 nuclear atoms, or a cyano group,
a to c are each independently an integer from 0 to 2, and
d is an integer of 0 to 2 and e is an integer of 0 to 2, with the proviso of d+e ≥ 1.

In one embodiment, in Formula 1, a and b may be 0 or 1, c may be 0, d may be 1, and e may be 0.

In one embodiment, in Formula 1, a and b may be 0 or 1, c may be 0, d may be 0, and e may be 1.

In Formula 1, when a, b or c is 0, it means a single bond.

In Formula 1, when d is 0, the hydrogen present in the benzene ring that may be substituted with R₄ is not substituted with R₄, and when e is 0, the hydrogen present in the benzene ring that may be substituted with R₃ is not substituted with R₃.

In one embodiment, Formula 1 may be represented by any one selected from Formulas 2 to 9 below. 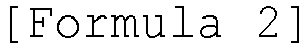 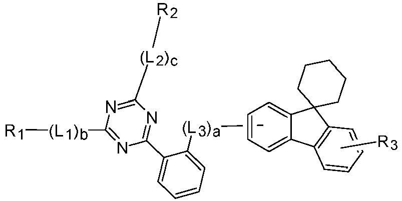 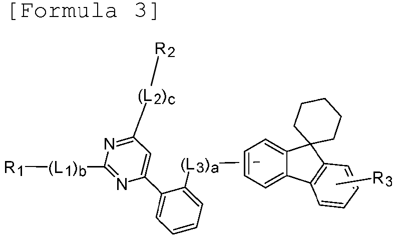 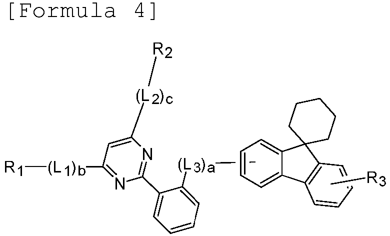 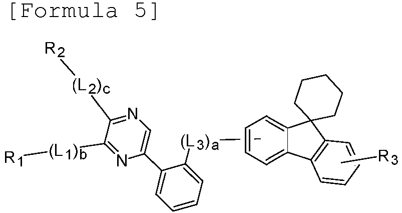 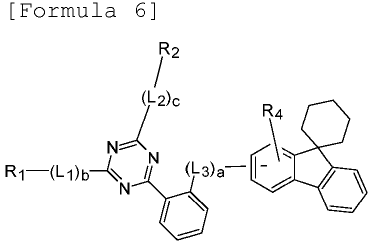 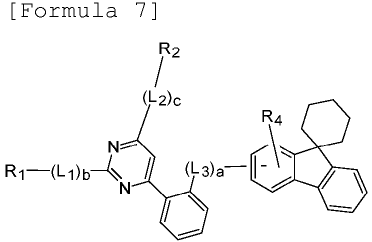 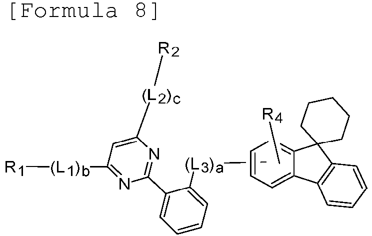 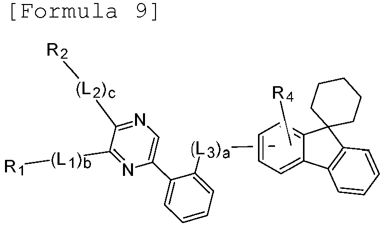 wherein in each of formulas 2 to 9,
R₁ and R₂ are each independently hydrogen, an aryl group containing 6 to 30 carbon atoms, a heteroaryl group containing 2 to 30 carbon atoms, a heteroaryl group containing 5 to 30 nuclear atoms, each of which is unsubstituted or substituted with a cycloalkyl group containing 3 to 20 carbon atoms, an alkyl group containing 1 to 20 carbon atoms, an aryl group containing 6 to 30 carbon atoms, a heteroaryl group containing 2 to 30 carbon atoms, a heteroaryl group containing 5 to 30 nuclear atoms, an alkylsilyl group containing 1 to 20 carbon atoms, an arylsilyl group containing 6 to 30 carbon atoms, an alkylphosphine oxide group containing 1 to 20 carbon atoms, or an arylphosphine oxide group containing 6 to 30 carbon atoms,
L₁ to L₃ are each independently a single bond, an arylene group containing 6 to 30 carbon atoms, a heteroarylene group containing 2 to 30 carbon atoms, or a heteroarylene group containing 5 to 30 nuclear atoms,
R₃ and R₄ are each independently an aryl group containing 6 to 30 carbon atoms substituted with a cyano group, a nitrogen-containing heteroaryl group containing 2 to 30 carbon atoms, a nitrogen-containing heteroaryl group containing 5 to 30 nuclear atoms, or a cyano group, and
a to c are each independently an integer from 0 to 2.

In each of Formulas 2 to 9, when a, b or c is 0, it means a single bond.

In one embodiment, Formula 1 may be represented by any one selected from Formulas 2 to 7 below. 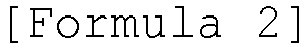 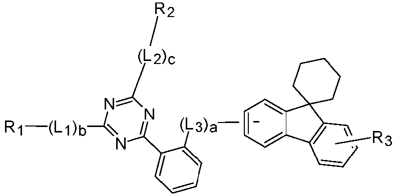 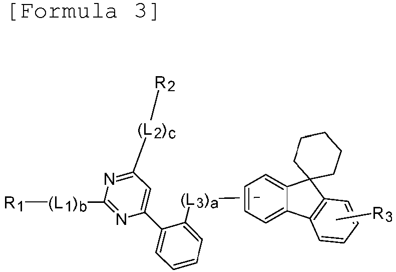 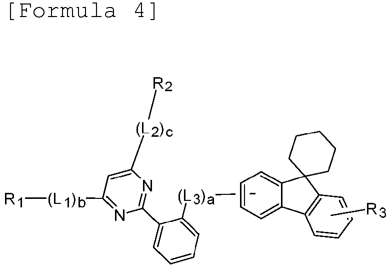 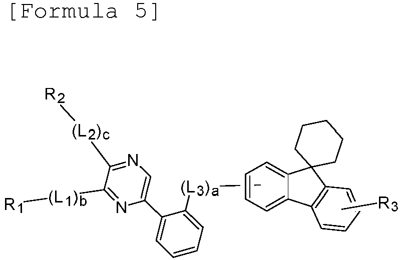 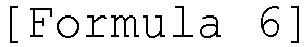 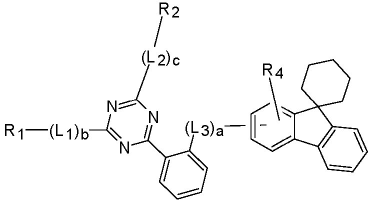 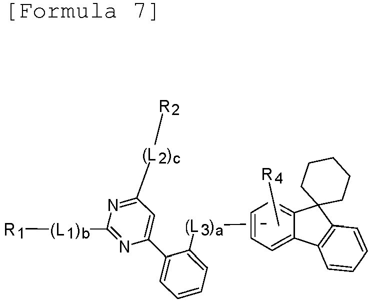 wherein in each of the formulas 2 to 9,
R₁ and R₂ are each independently hydrogen, a phenyl group, a biphenyl group, a naphthyl group, a terphenyl group, a fluorenyl group, a pyridyl group, a carbazolyl group, a dibenzothiophenyl group, or a dibenzofuranyl group, each of which is unsubstituted or substituted with a methyl group, a phenyl group, a cyclohexyl group, an adamantyl group, a pyridyl group, a carbazolyl group, a dibenzothiophenyl group, a dibenzofuranyl group, a trimethylsilyl group, a triphenylsilyl group, a dimethylphosphine oxide group, or a diphenylphosphine oxide group,
L₁ to L₃ are each independently a single bond, a phenylene group, a biphenylene group, a naphthylene group, a terphenylene group, a dibenzofuranylene group, or a dibenzothiophenylene group,
R₃ and R₄ are each independently a phenyl group substituted with a cyano group, a pyridyl group, or a cyano group, and
a to c are each independently be an integer of 0 to 2.

In each of Formulas 2 to 7, when a, b, or c is 0, it means a single bond.

In one embodiment, the organic light-emitting compound represented by Formula 1 may be any one selected from the following Compounds 001 to 774.

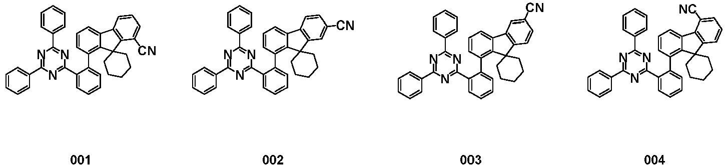

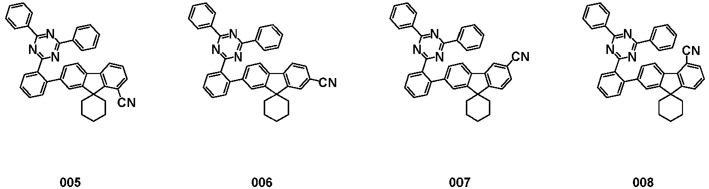

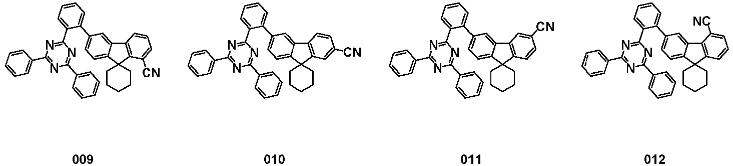

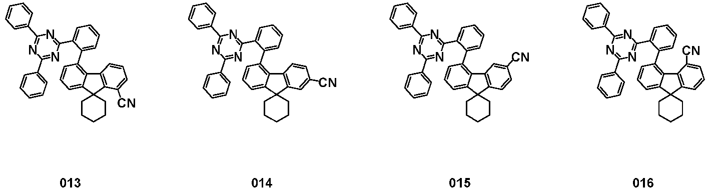

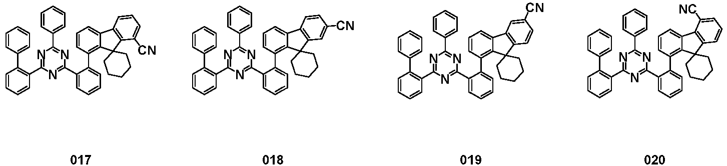

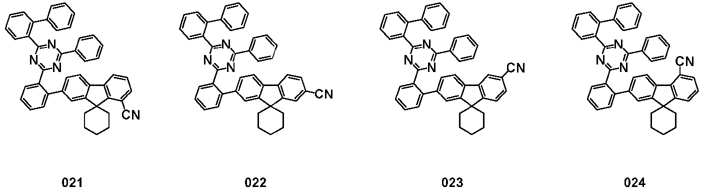

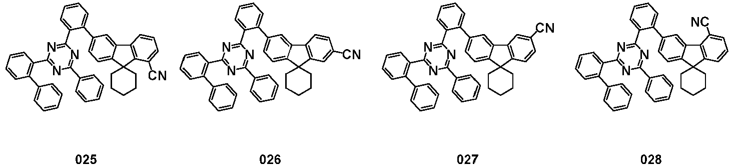

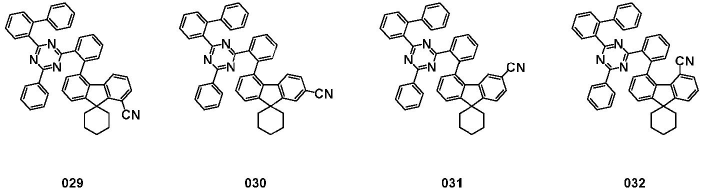

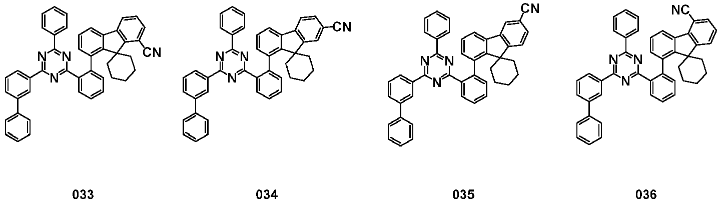

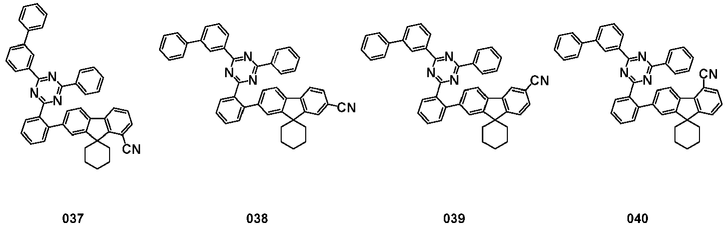

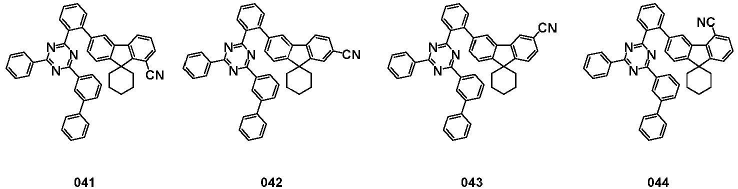

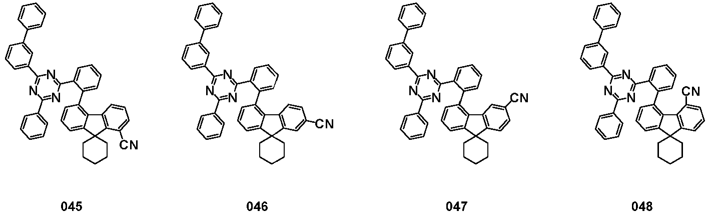

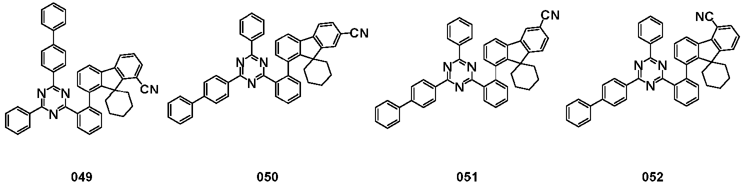

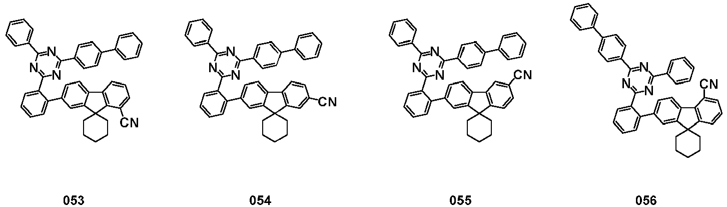

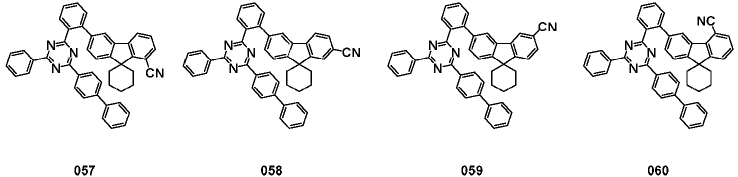

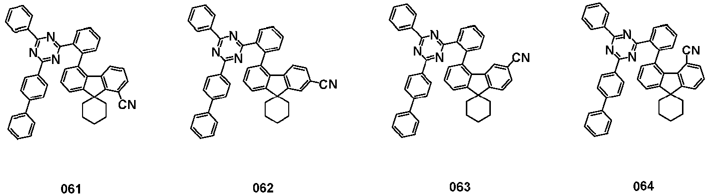

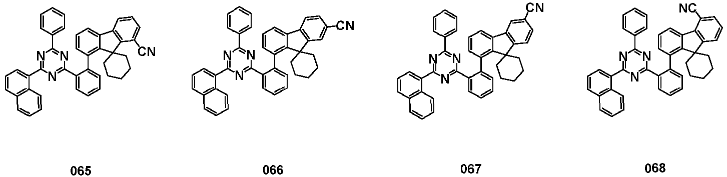

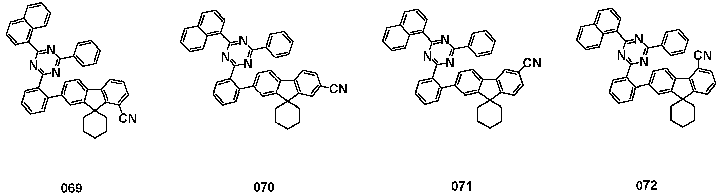

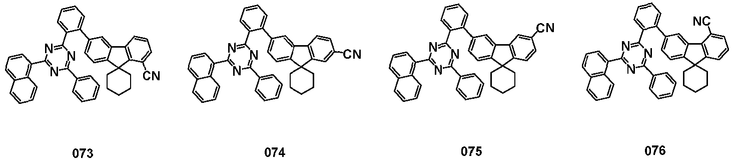

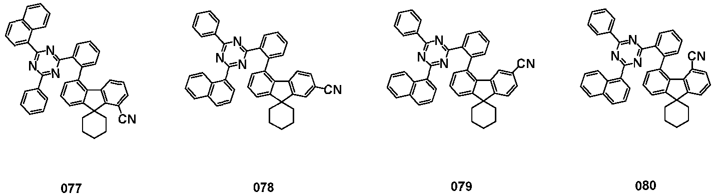

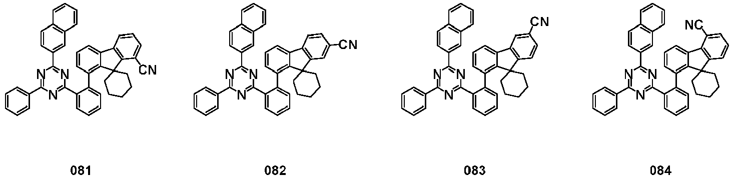

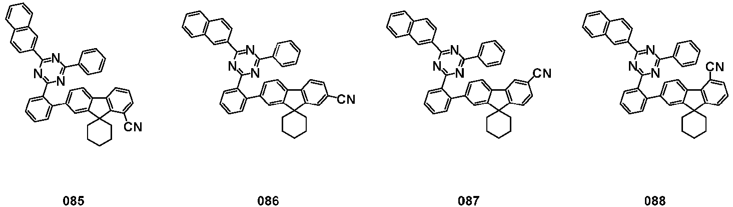

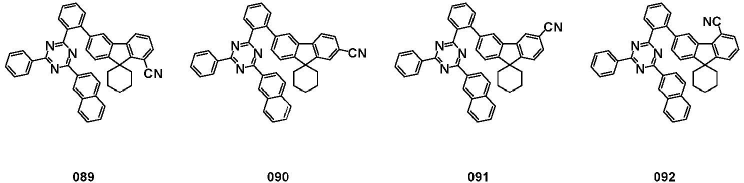

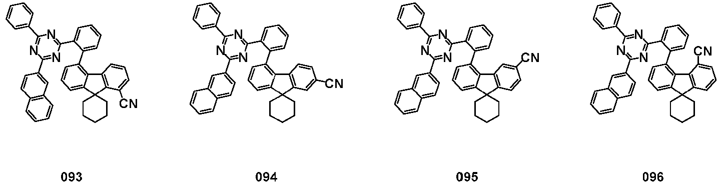

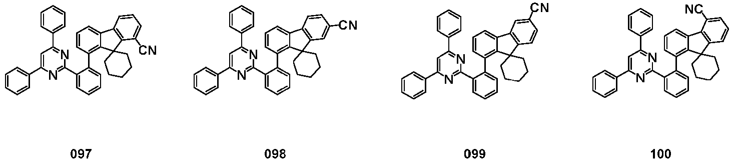

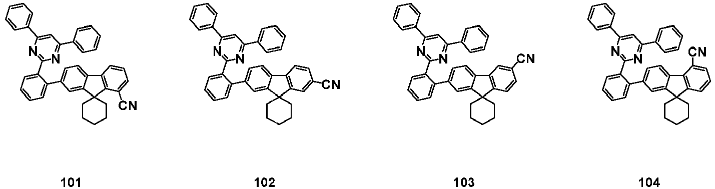

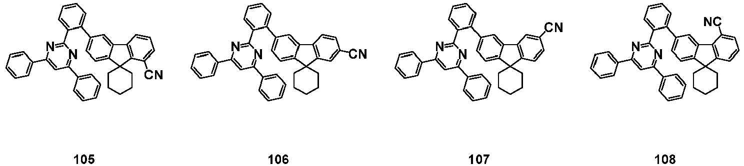

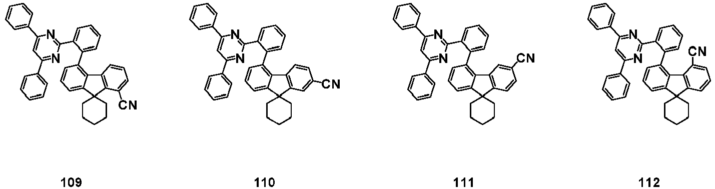

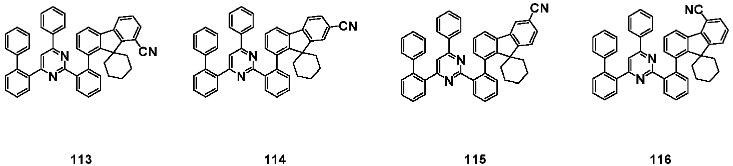

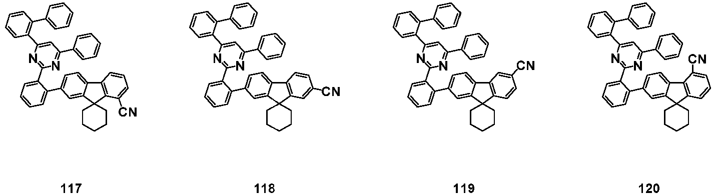

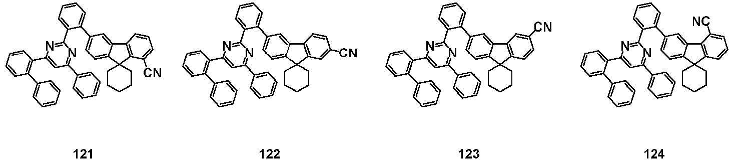

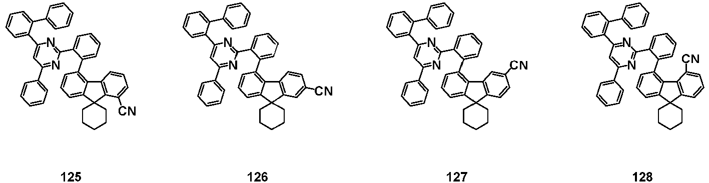

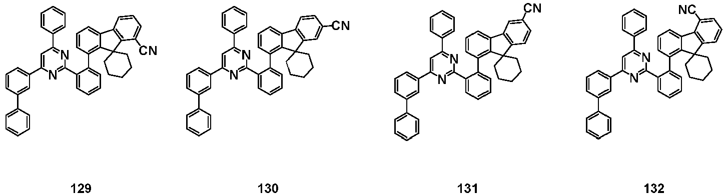

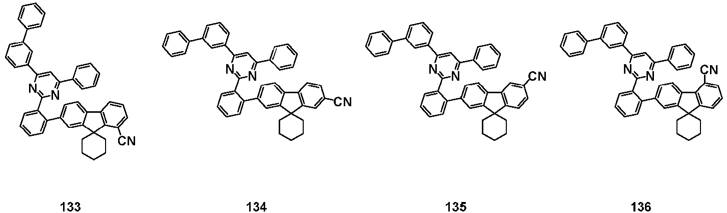

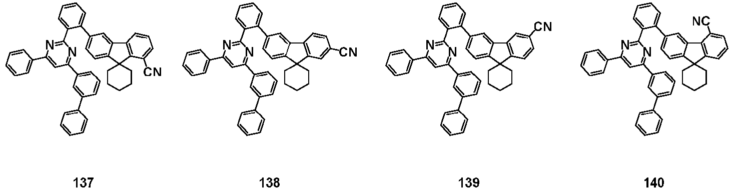

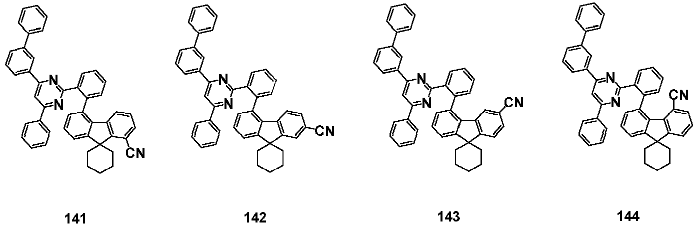

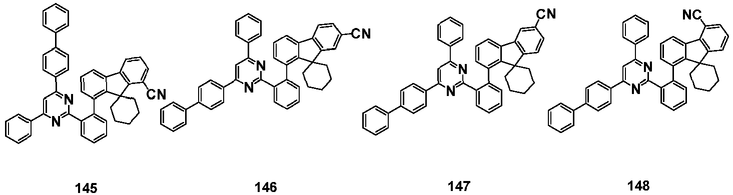

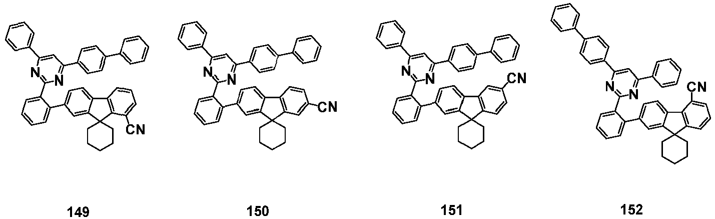

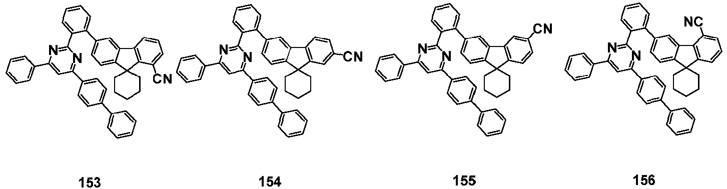

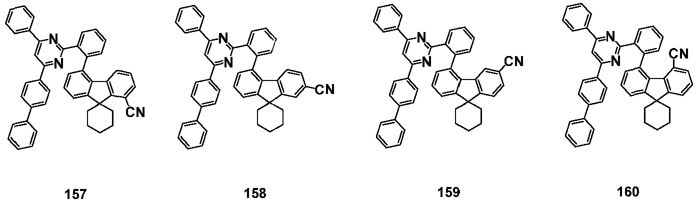

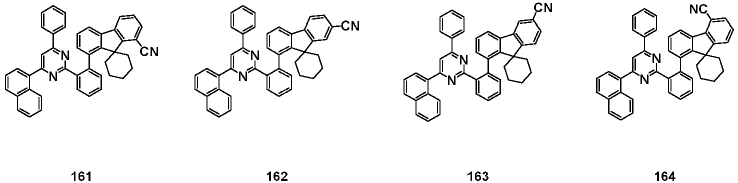

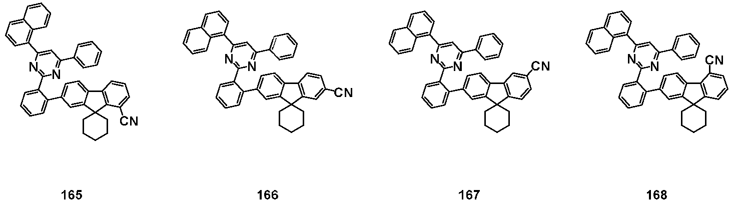

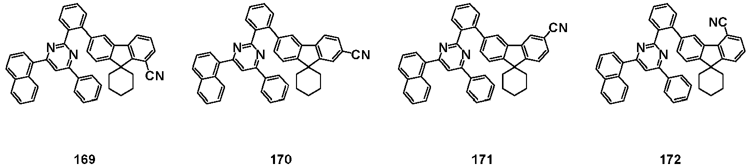

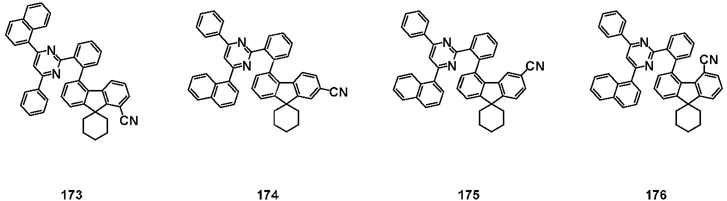

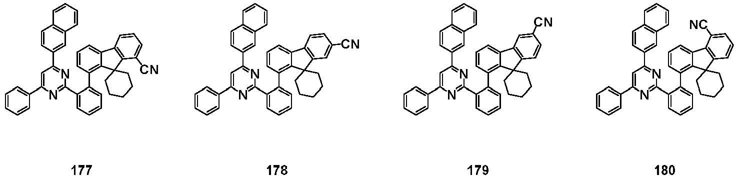

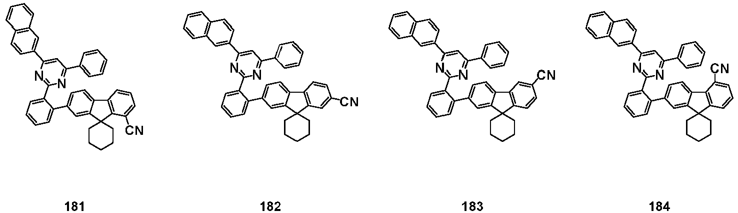

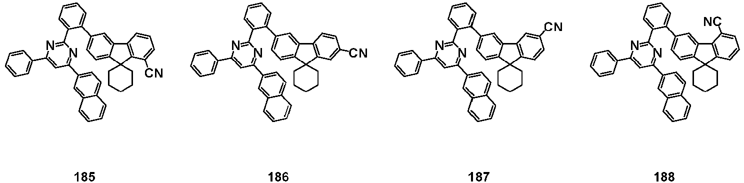

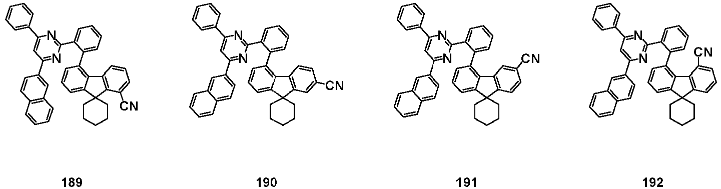

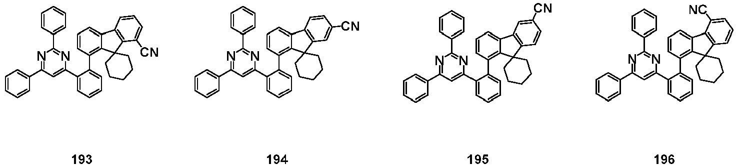

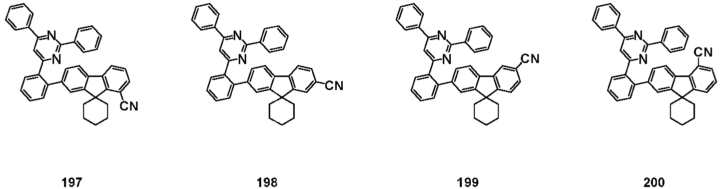

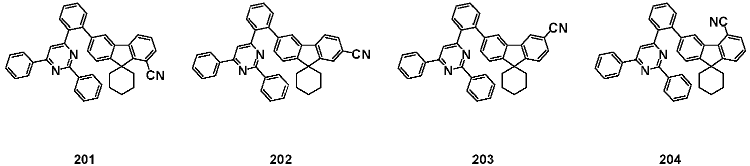

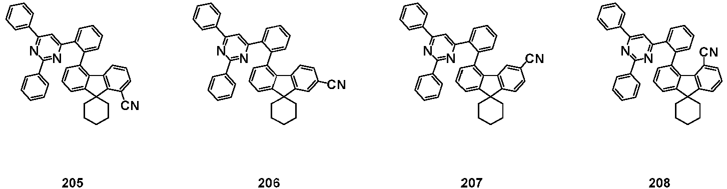

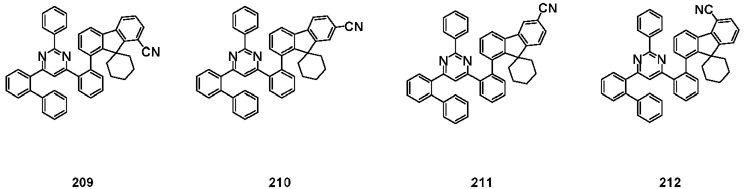

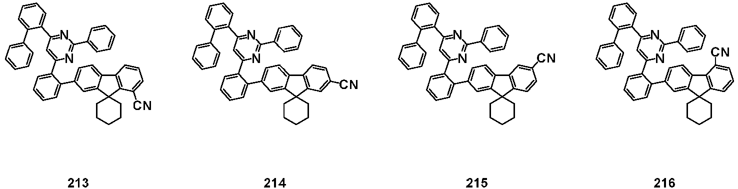

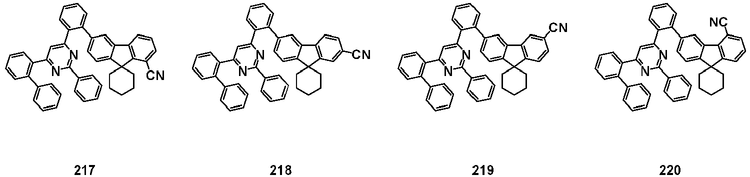

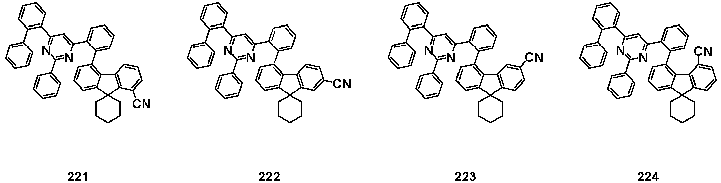

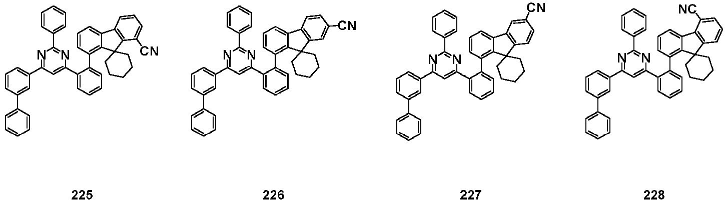

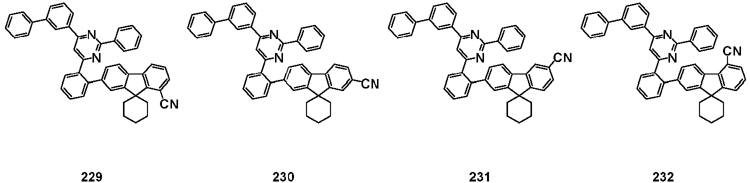

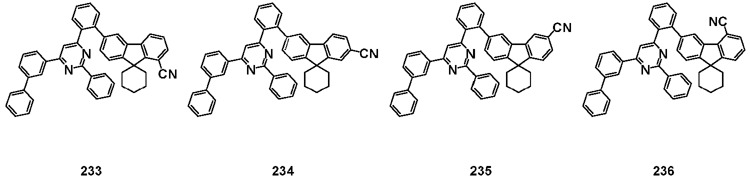

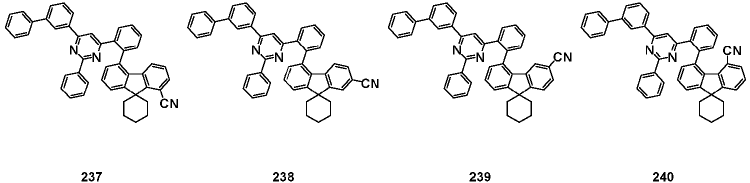

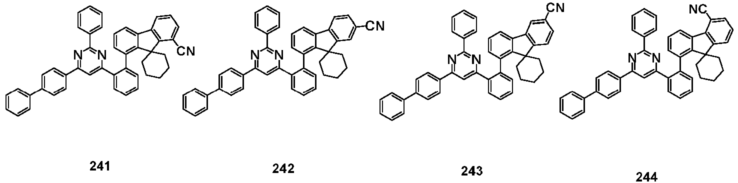

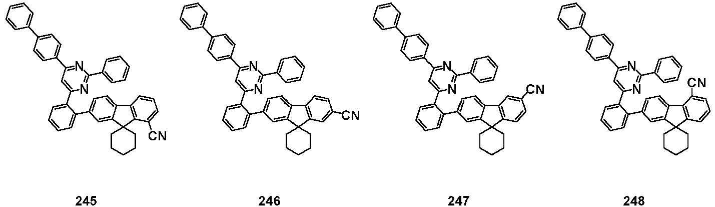

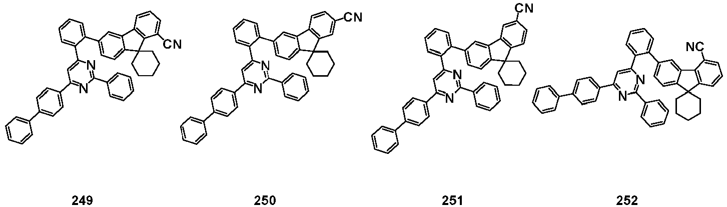

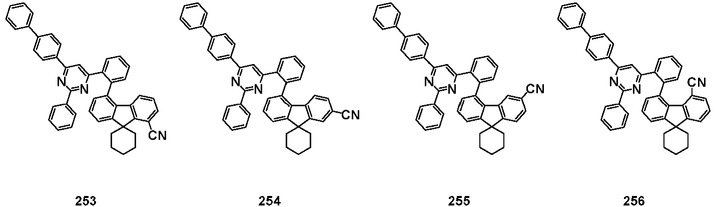

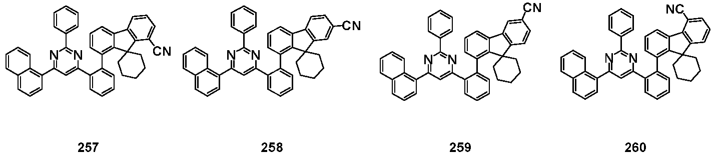

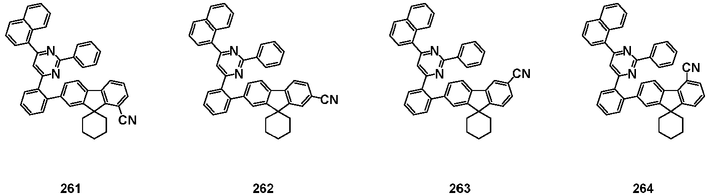

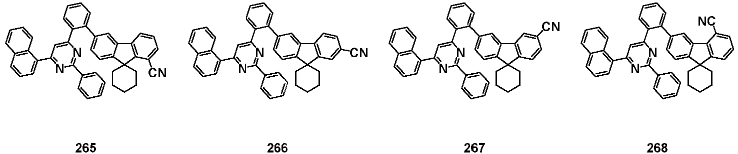

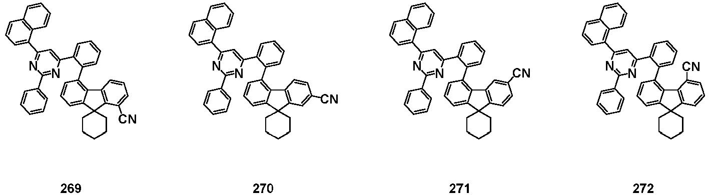

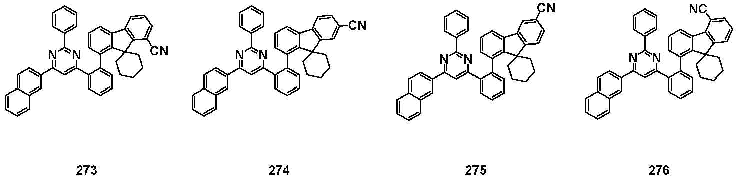

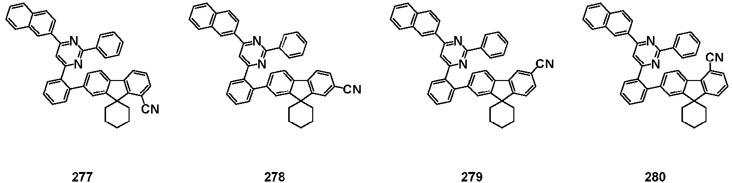

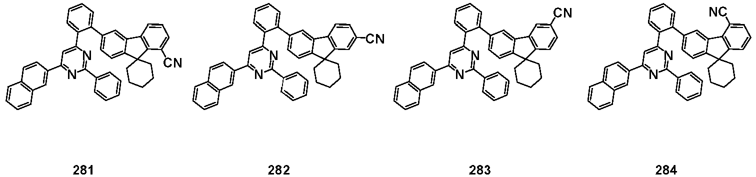

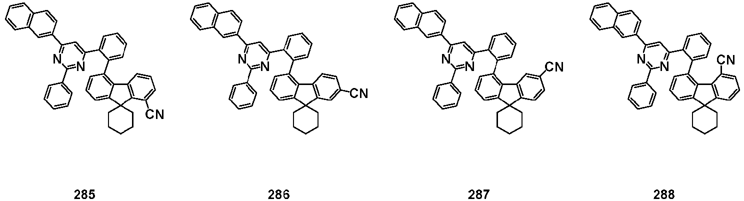

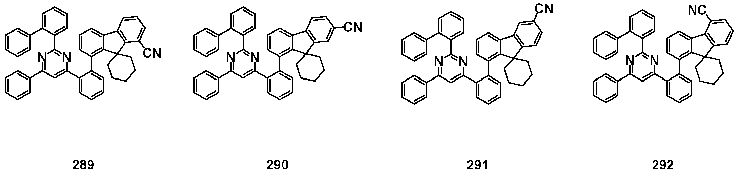

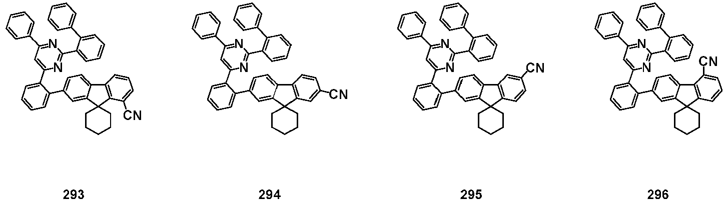

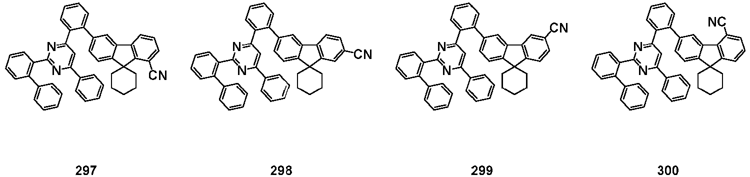

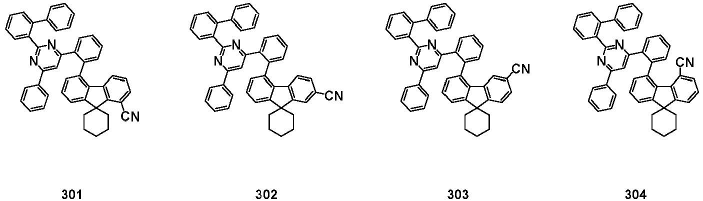

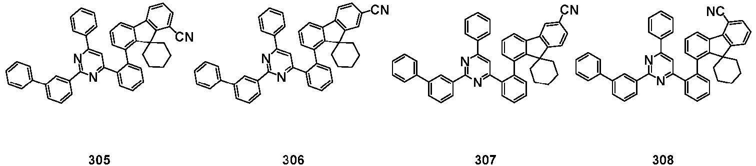

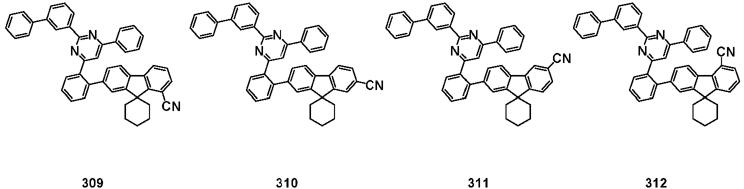

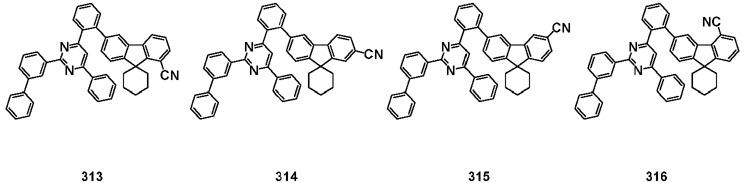

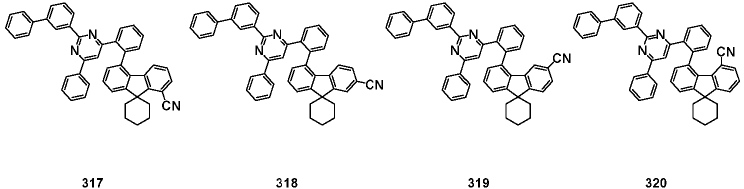

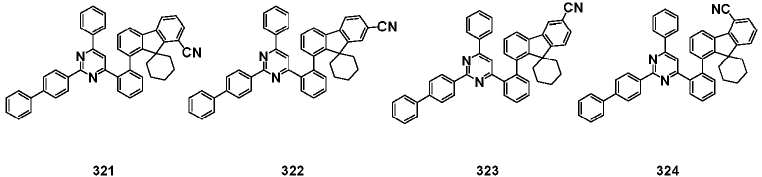

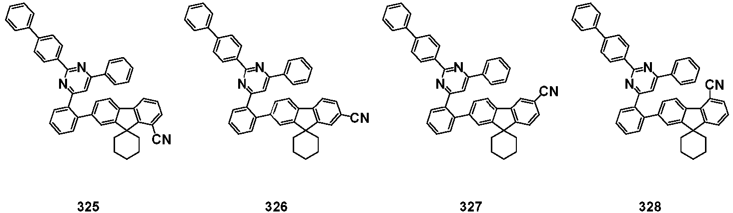

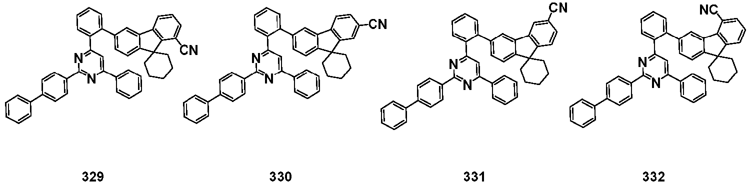

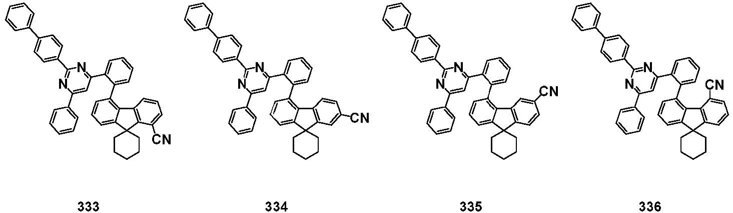

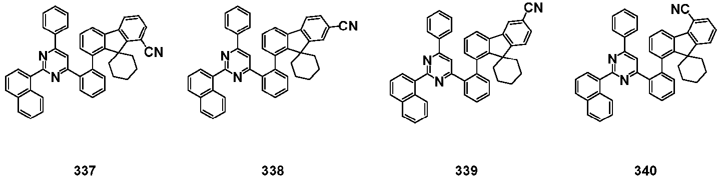

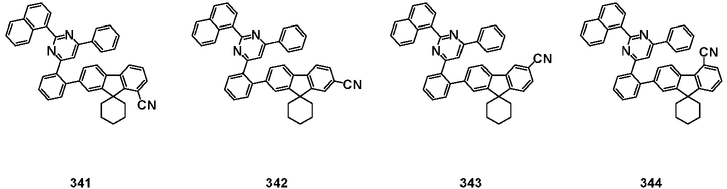

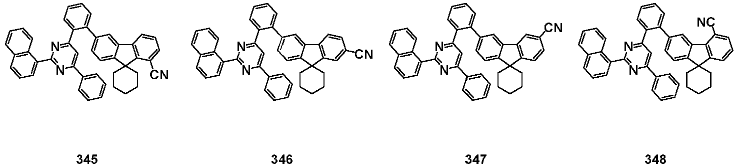

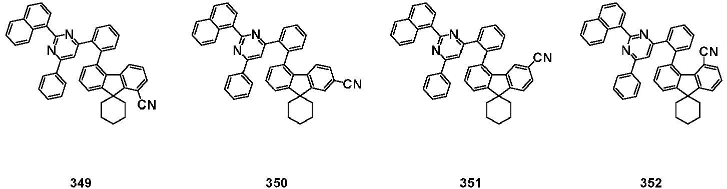

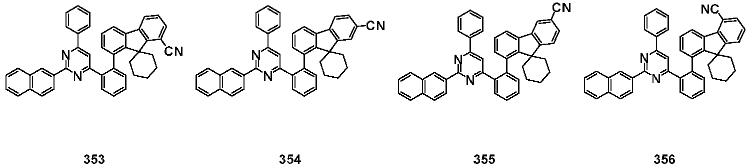

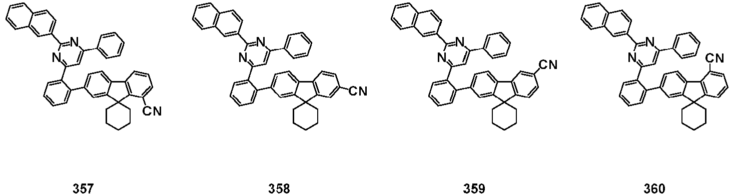

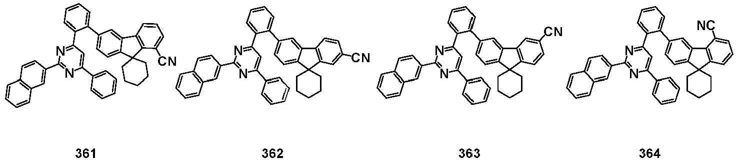

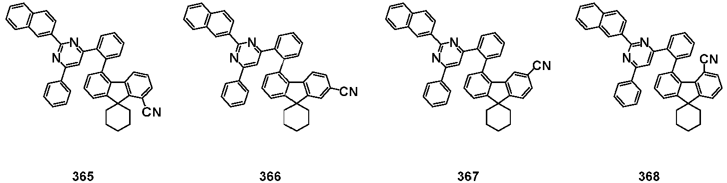

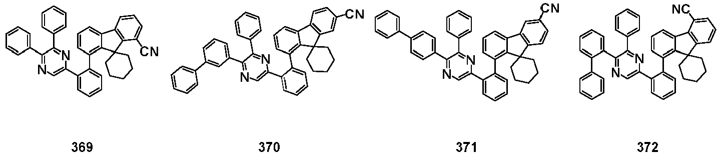

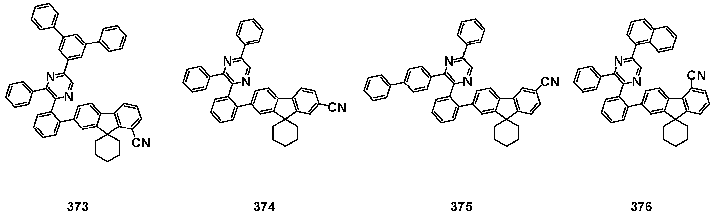

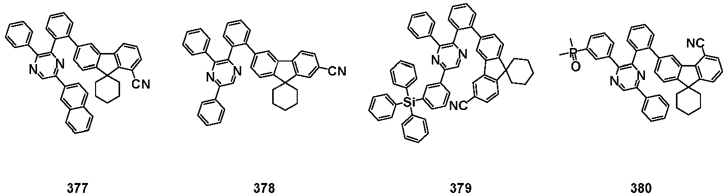

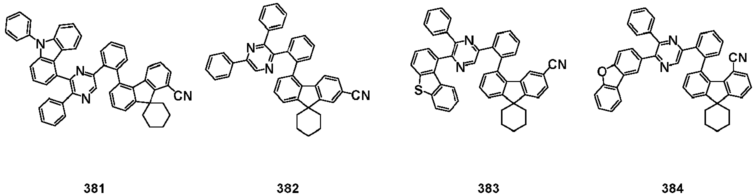

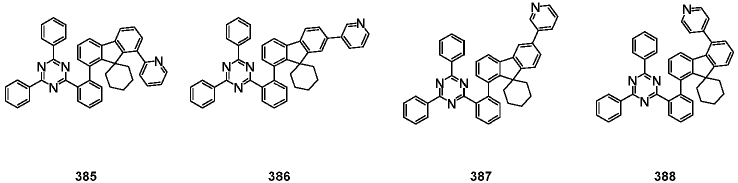

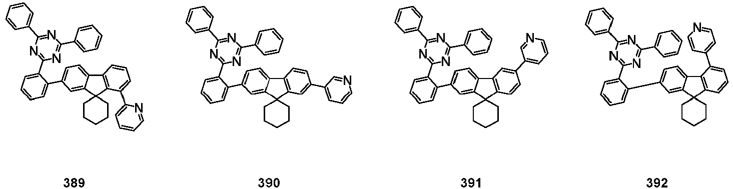

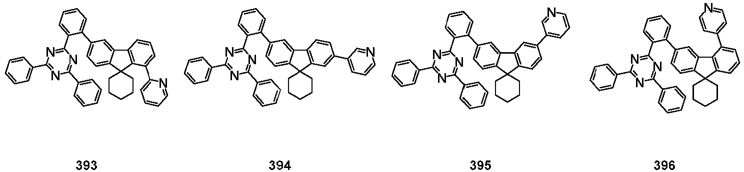

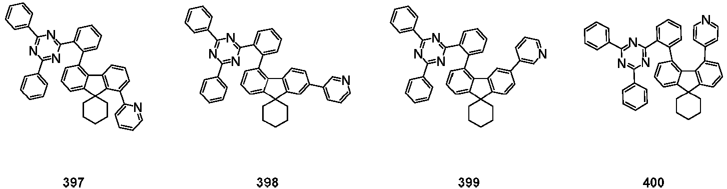

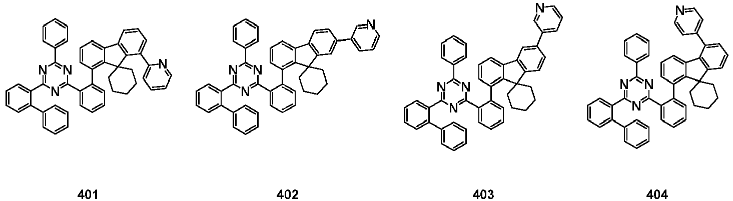

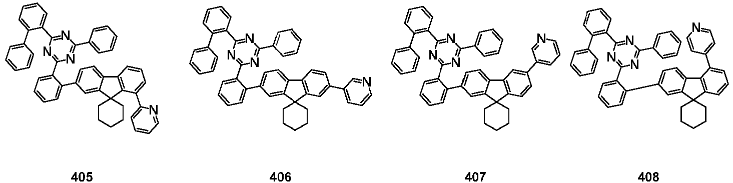

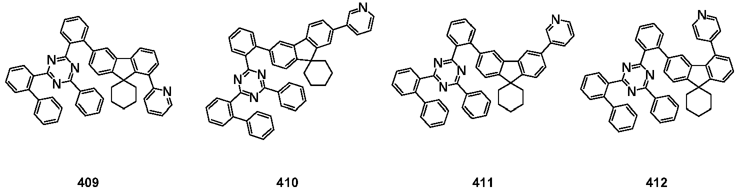

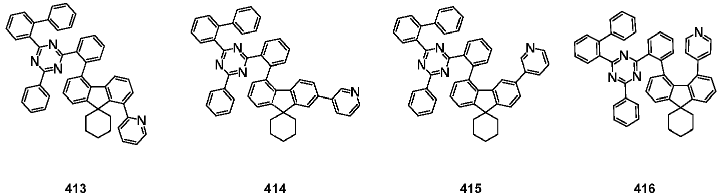

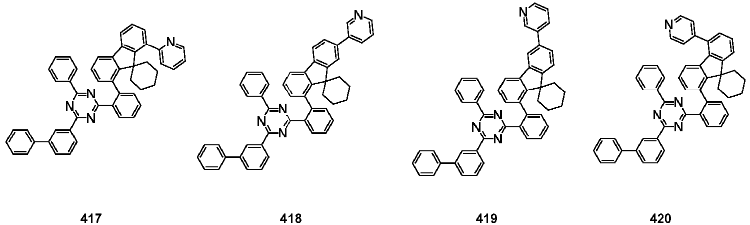

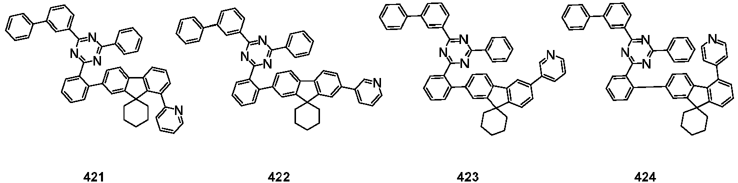

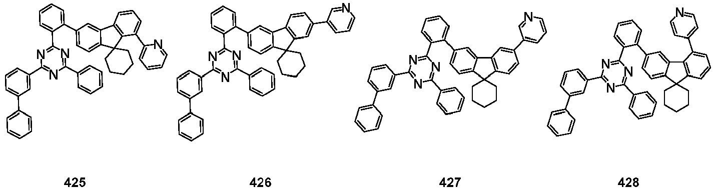

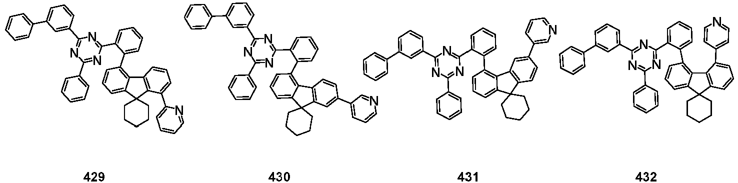

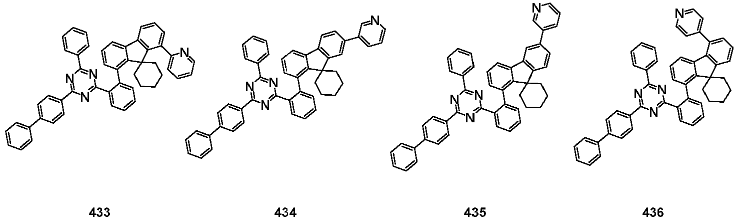

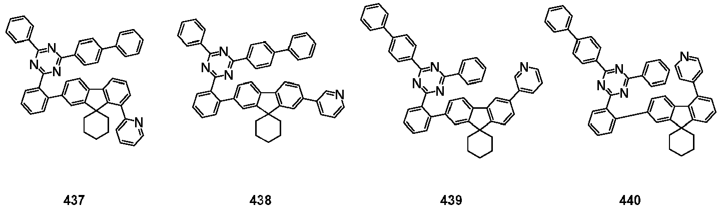

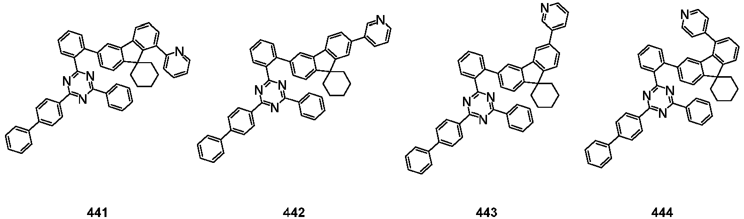

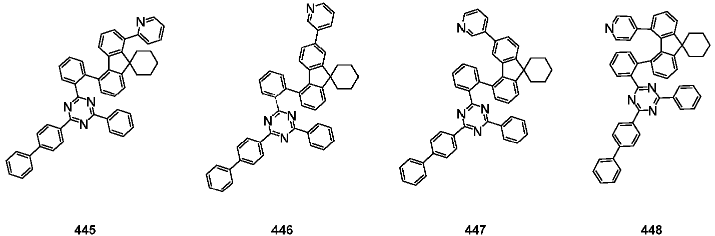

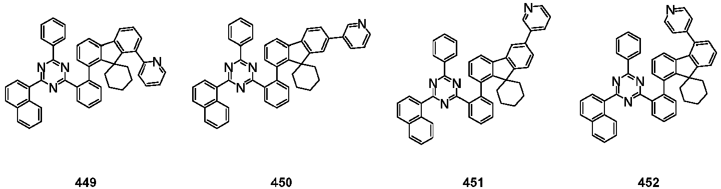

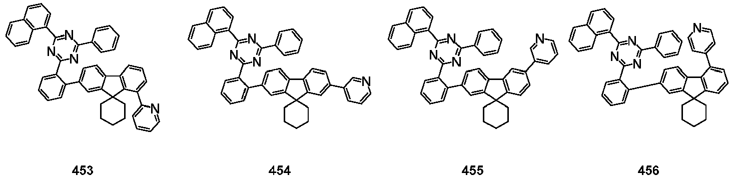

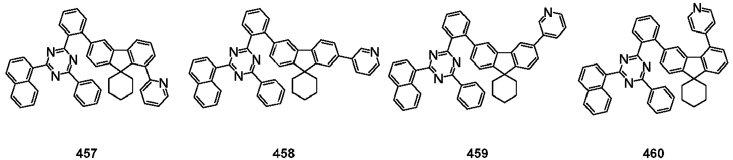

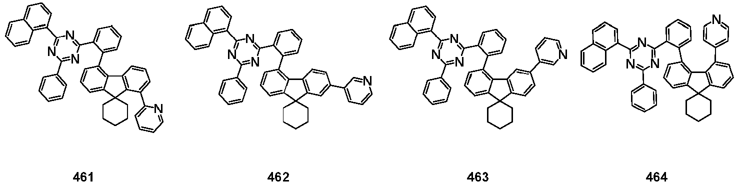

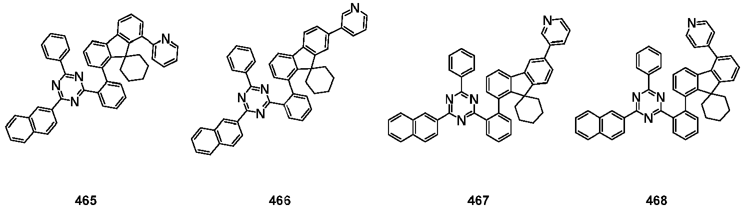

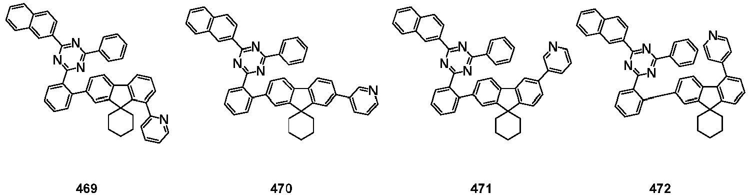

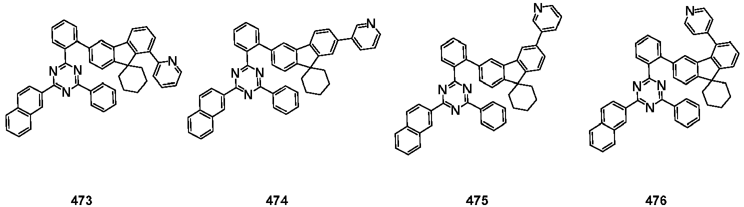

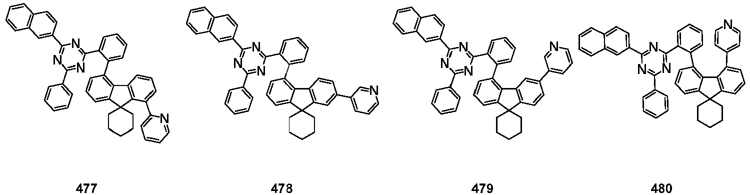

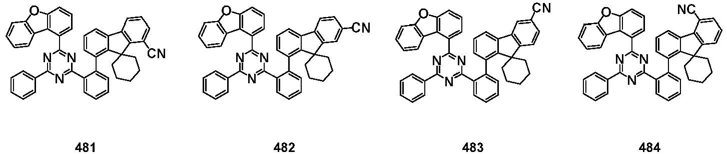

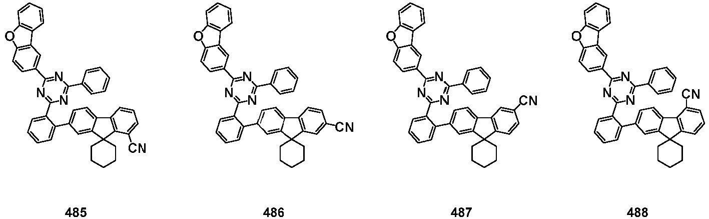

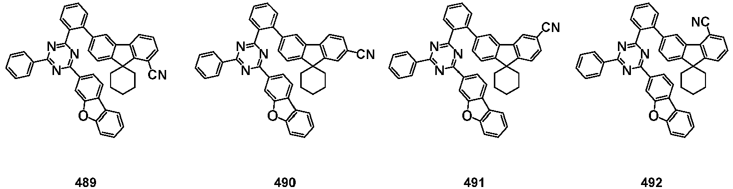

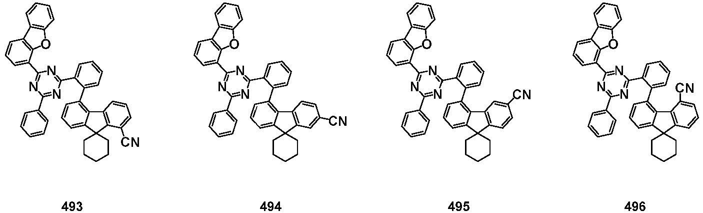

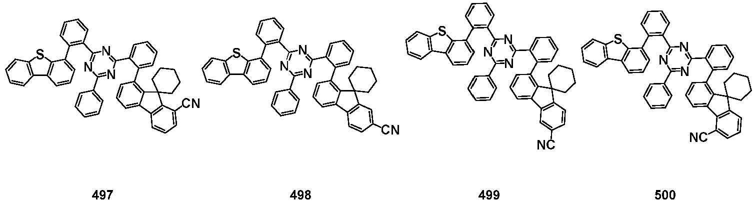

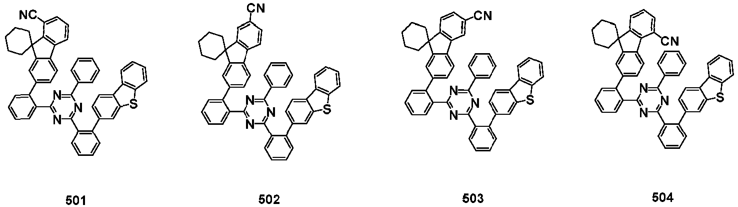

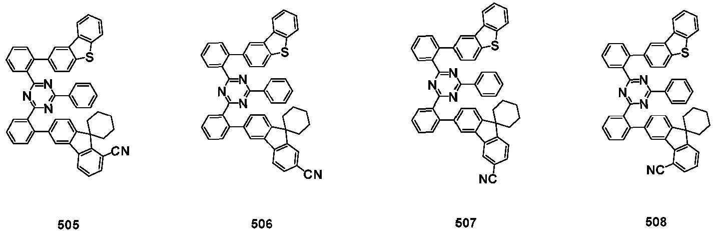

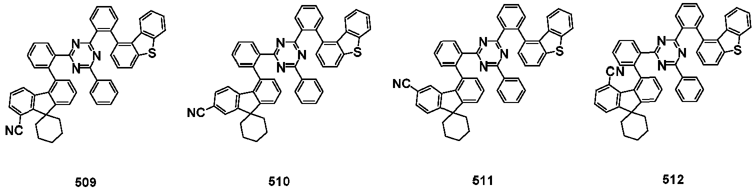

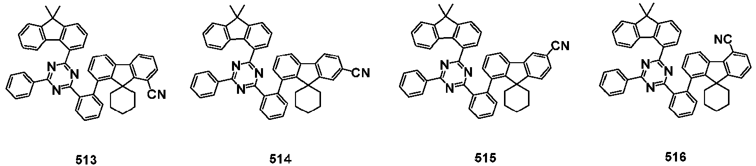

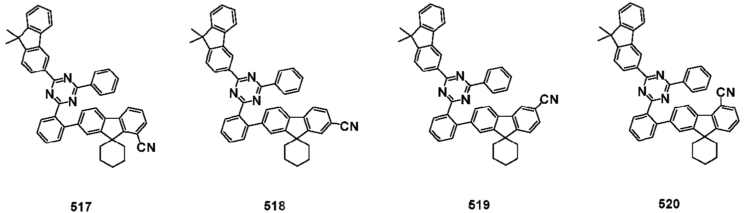

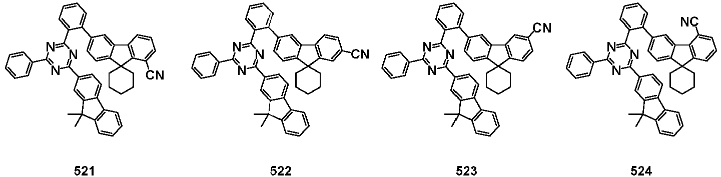

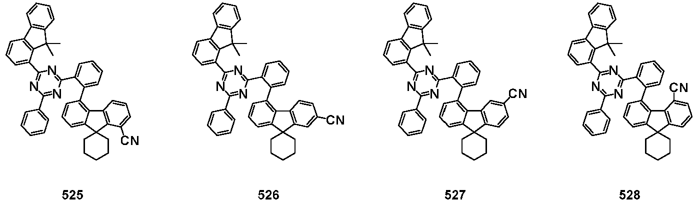

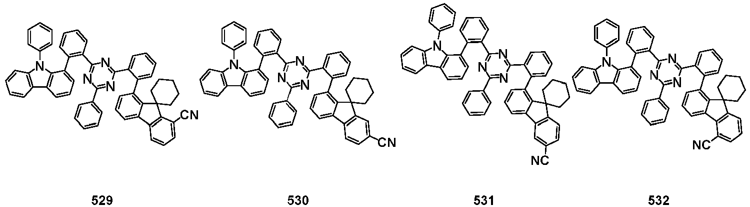

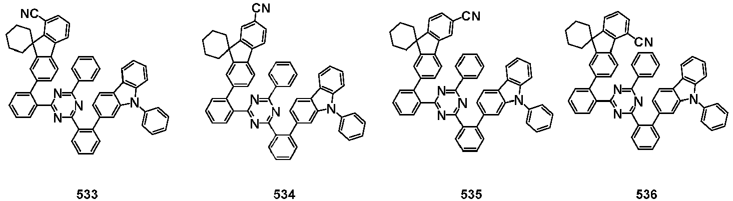

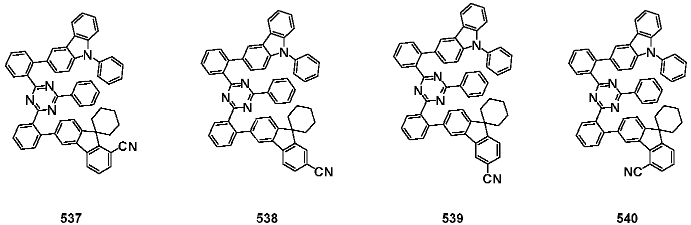

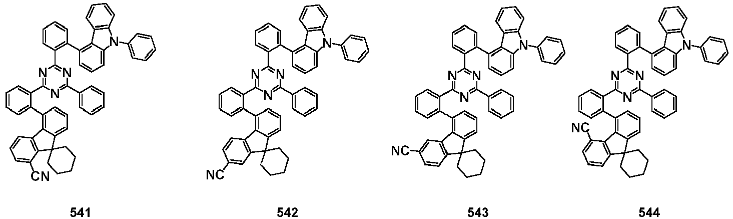

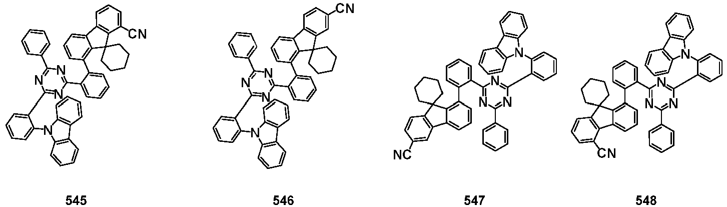

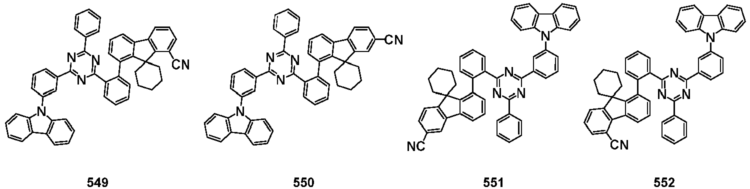

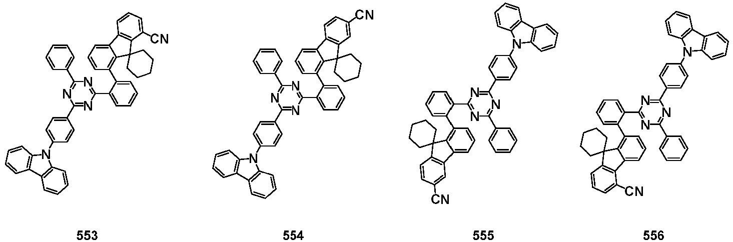

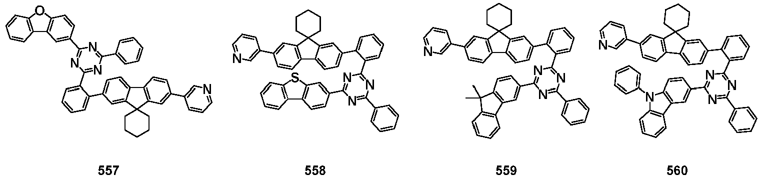

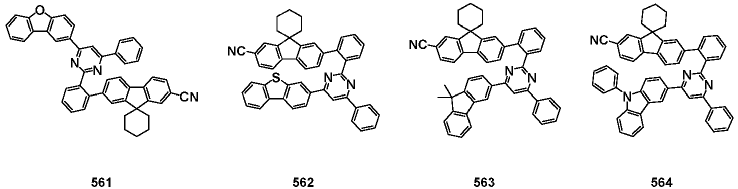

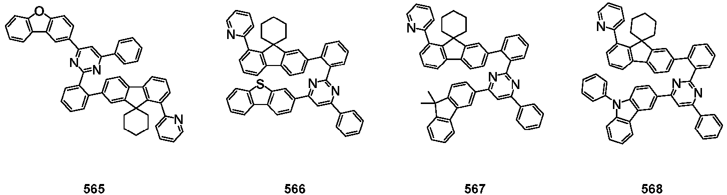

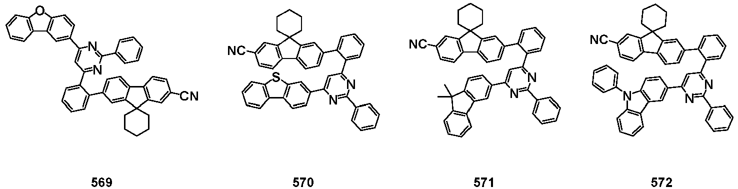

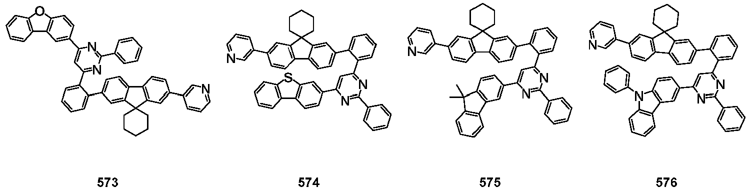

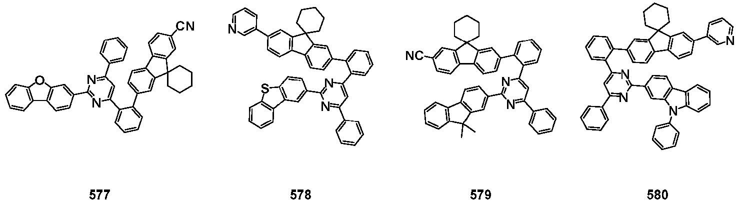

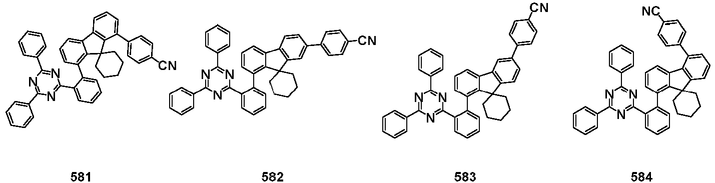

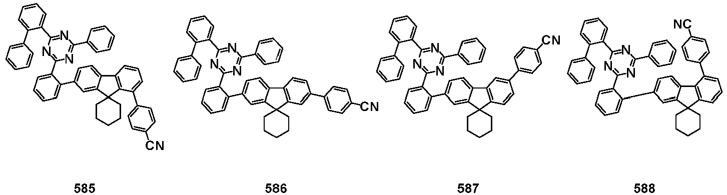

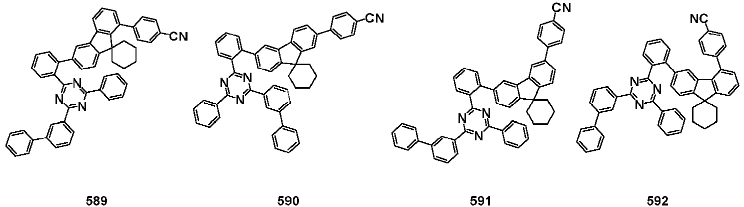

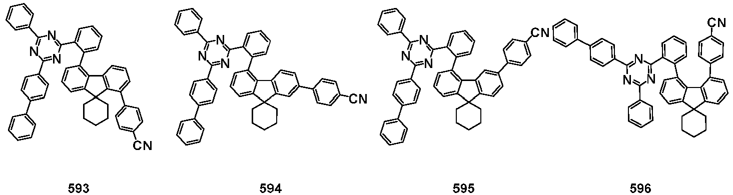

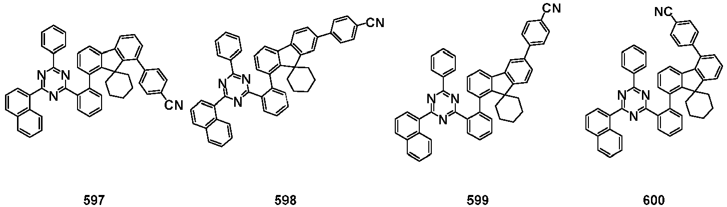

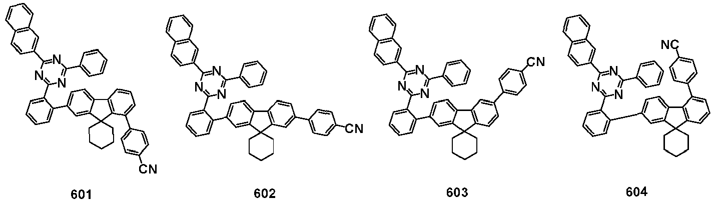

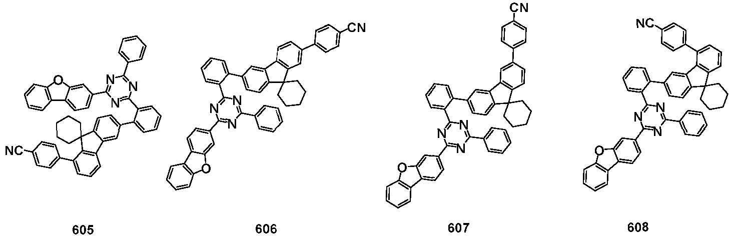

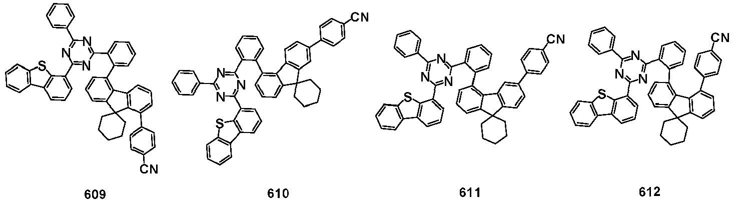

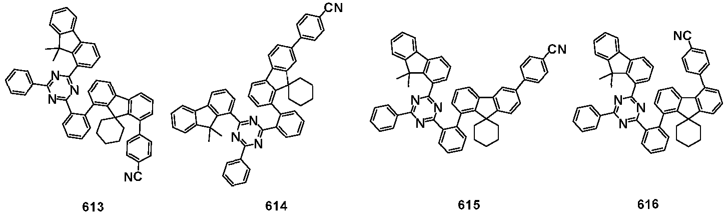

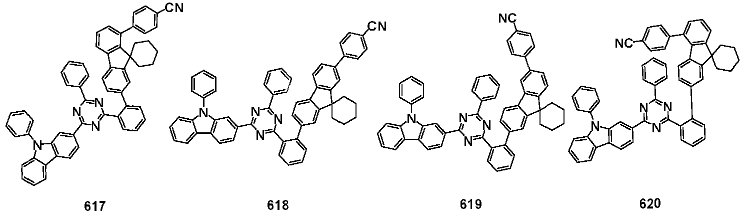

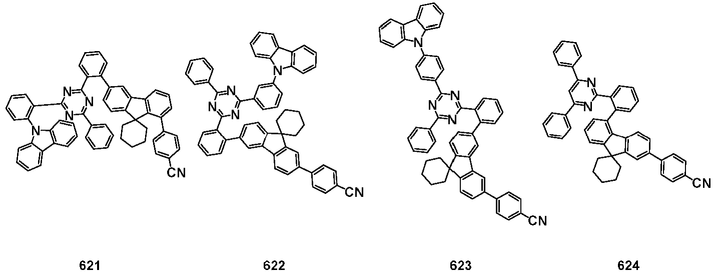

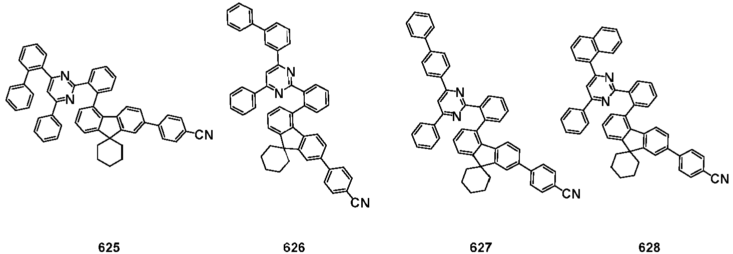

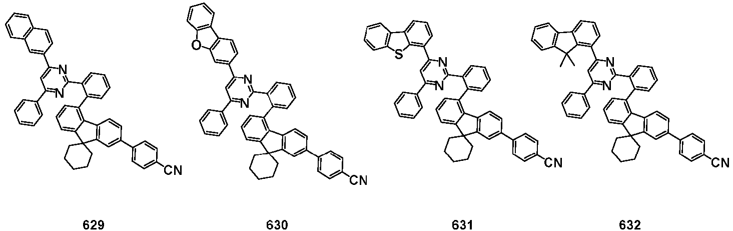

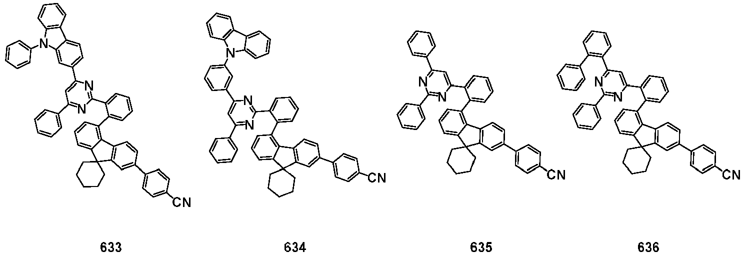

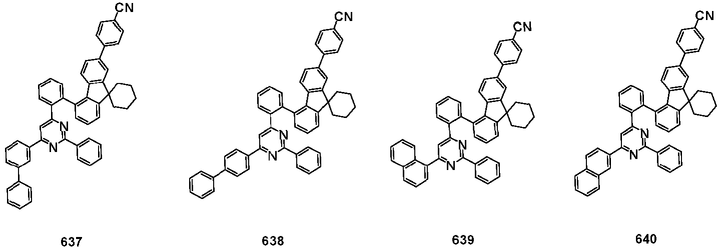

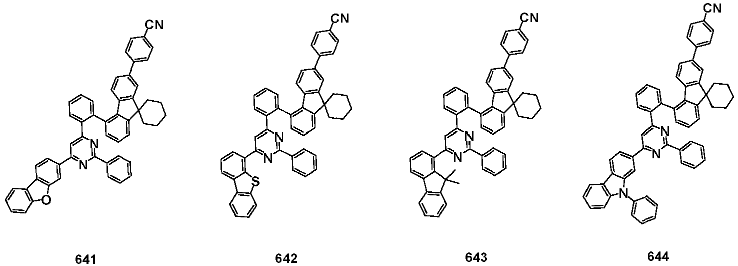

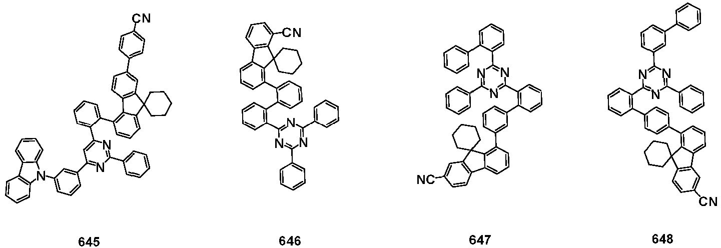

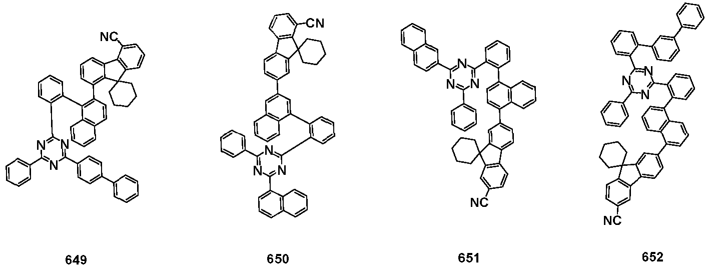

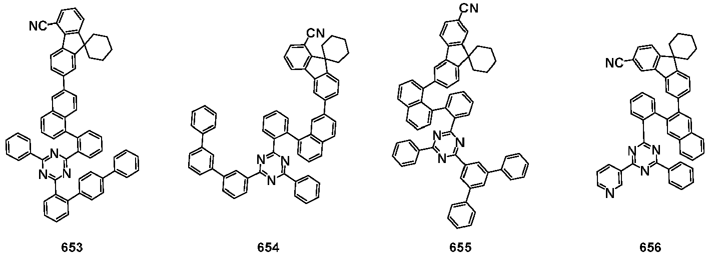

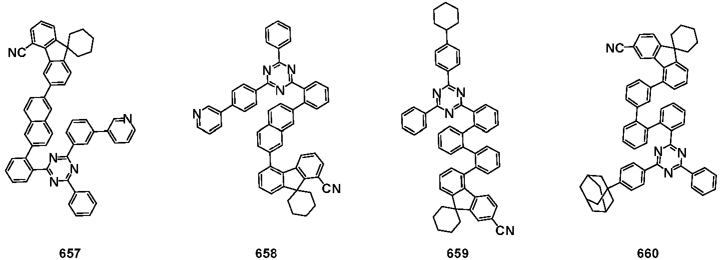

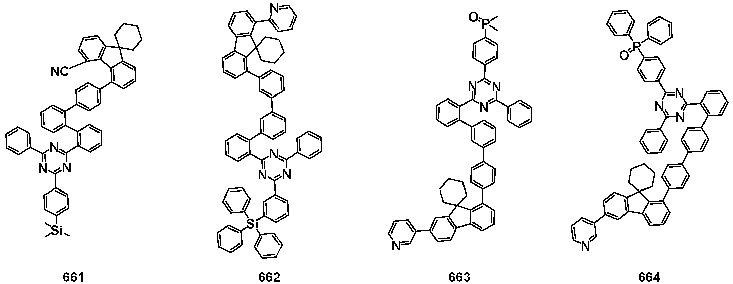

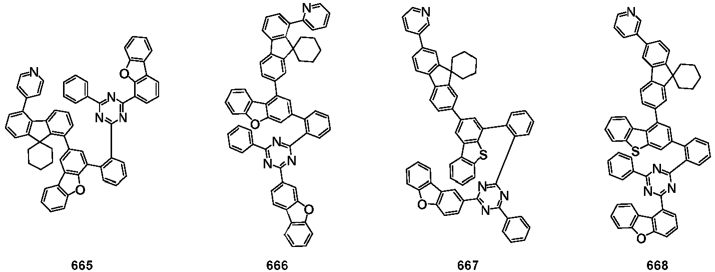

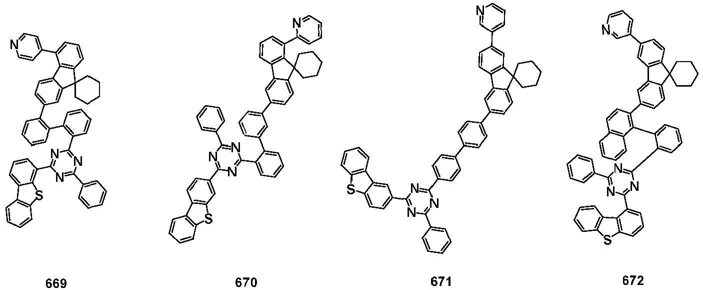

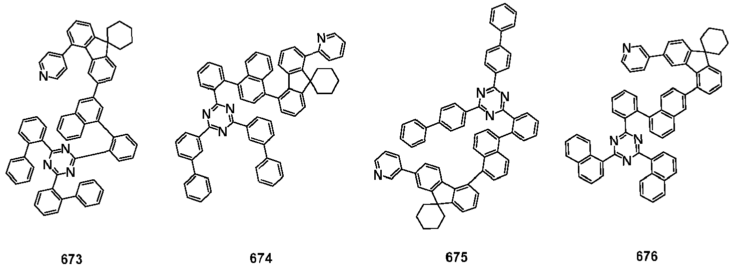

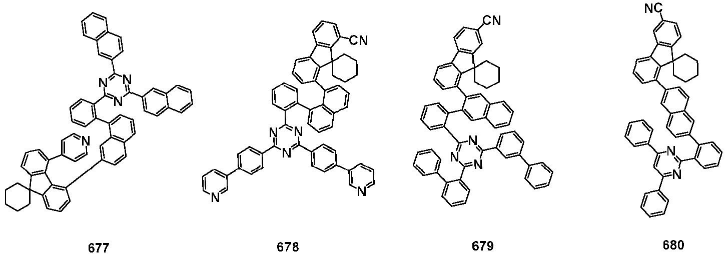

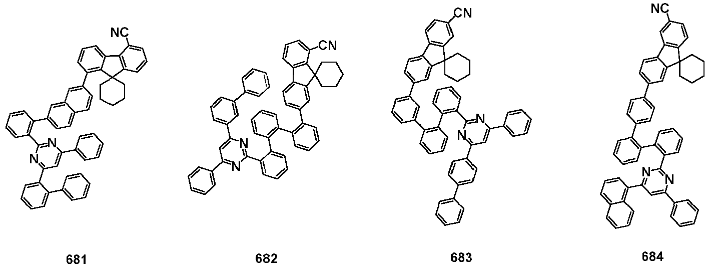

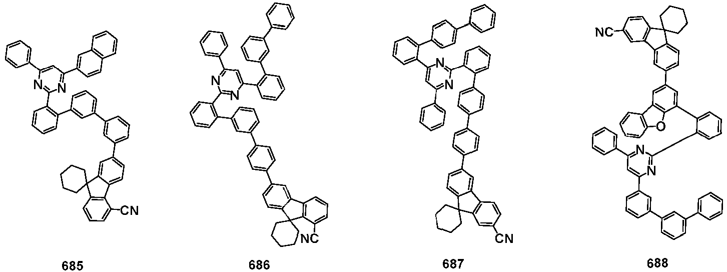

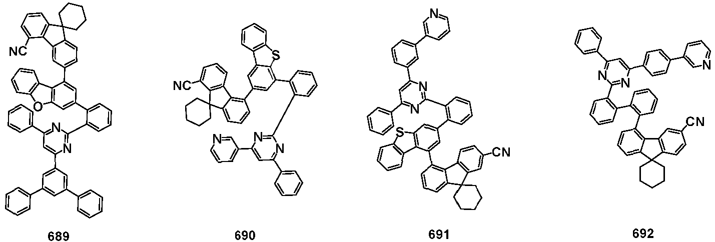

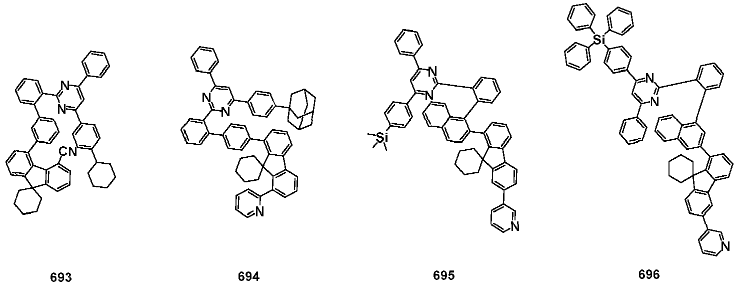

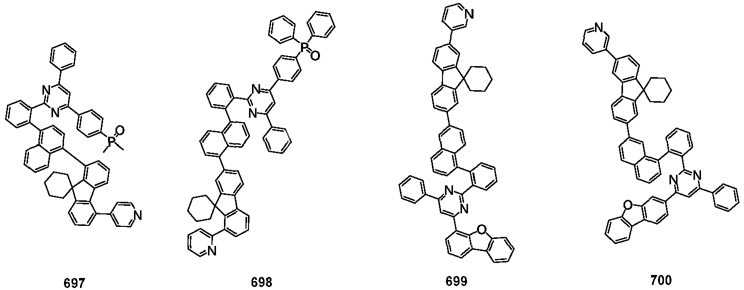

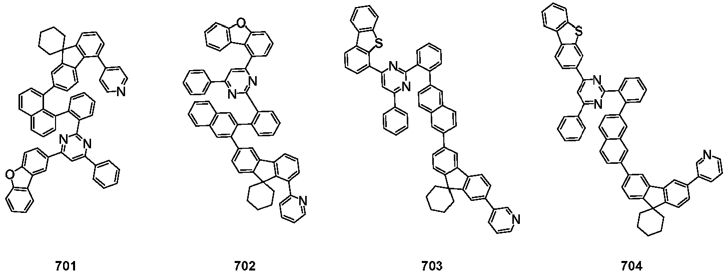

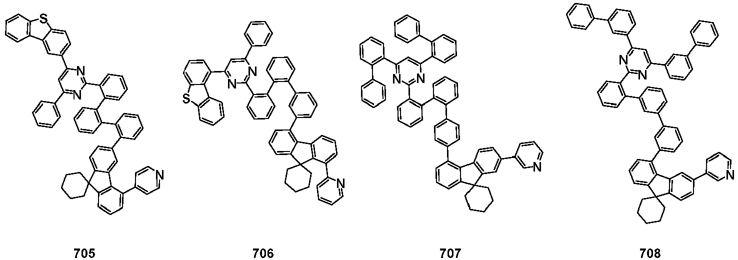

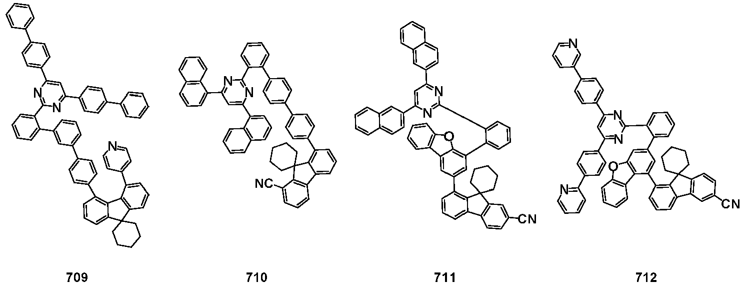

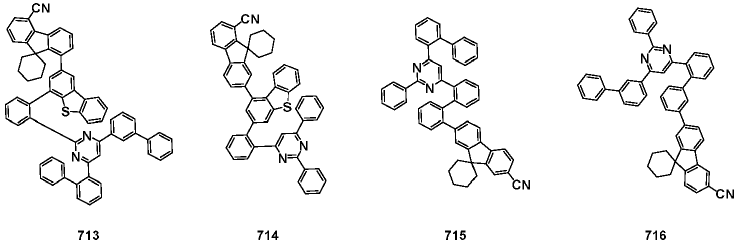

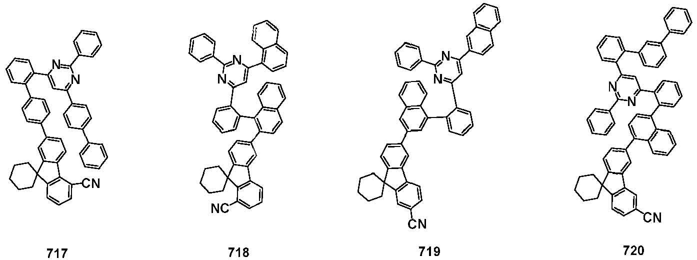

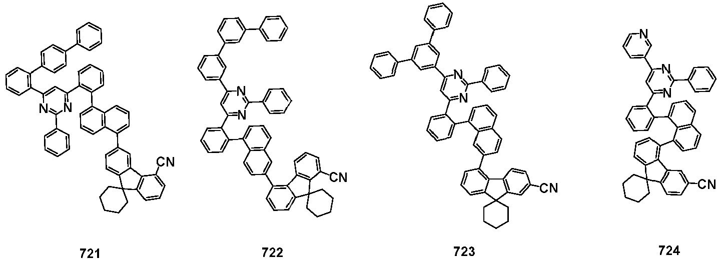

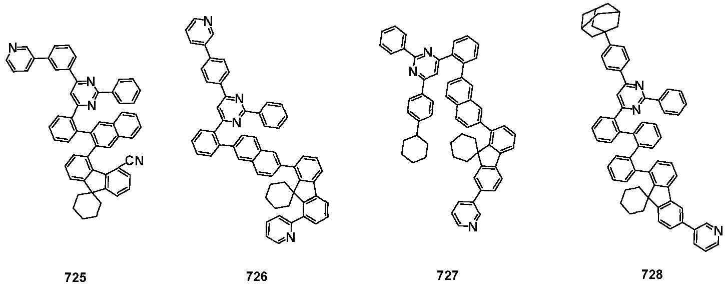

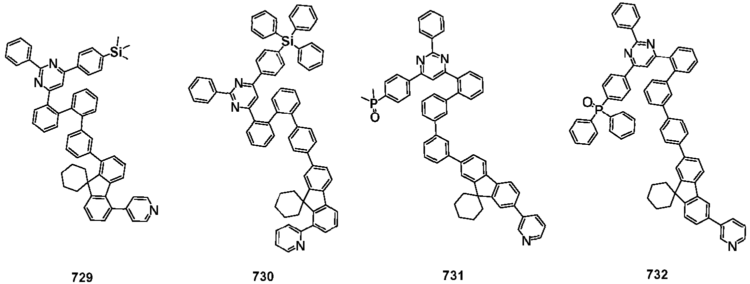

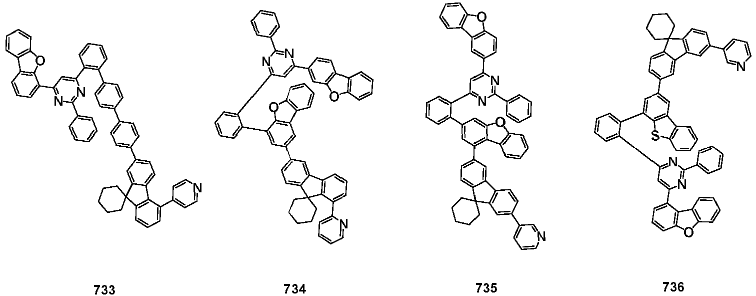

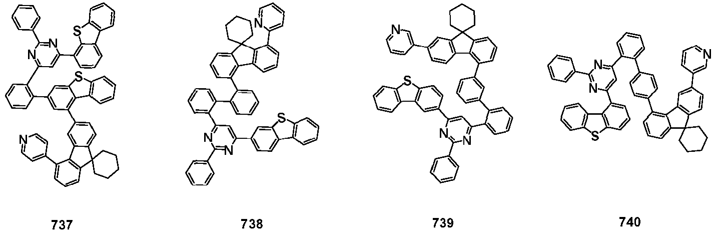

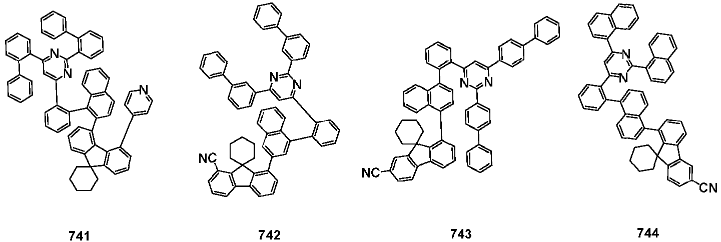

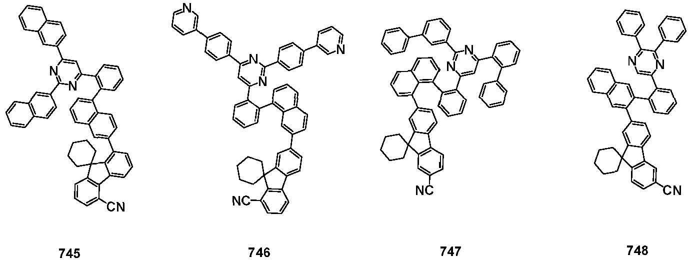

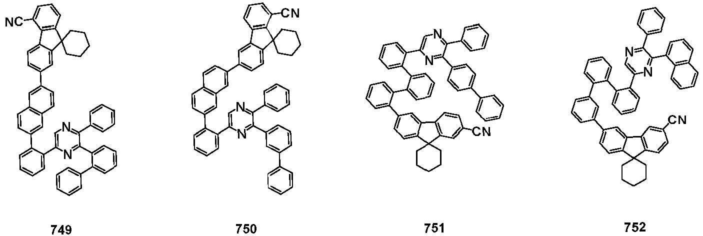

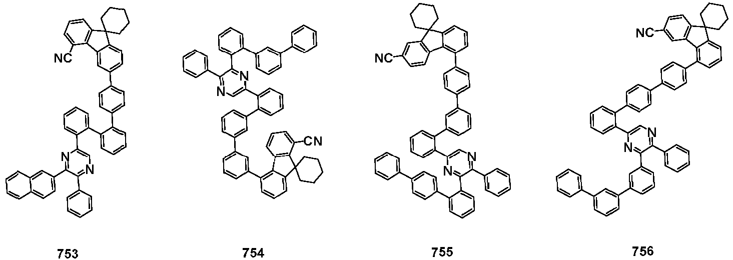

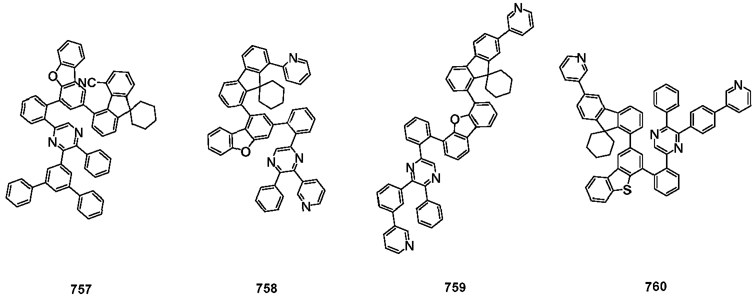

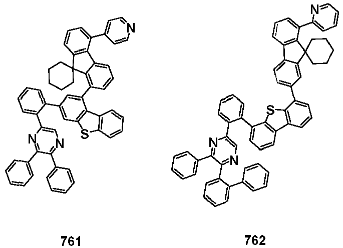

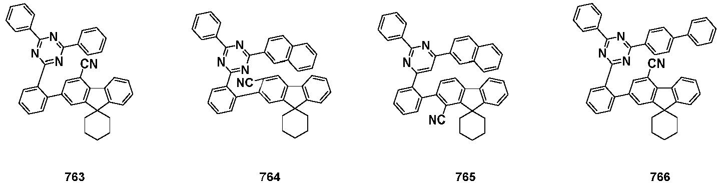

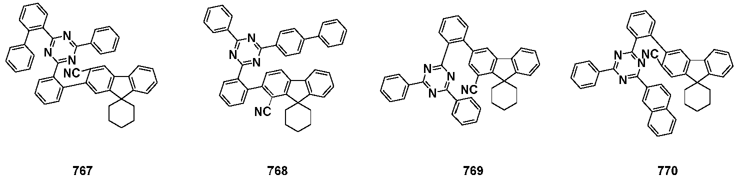

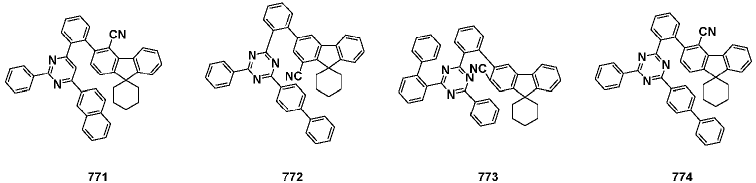

As a specific example, the compound represented by Formula 1 may be any one selected from compounds 006, 010, 014, 022, 026, 030, 038, 042, 046, 054, 058, 062, 070, 074, 078, 086, 090, 094, 102, 106, 110, 118, 122, 126, 134, 138, 142, 150, 154, 158, 166, 170, 174, 182, 186, 190, 198, 202, 206, 214, 218, 222, 230, 234, 238, 246, 250, 254, 262, 266, 270, 278, 282, 286, 294, 298, 302, 310, 314, 318, 326, 330, 334, 342, 346, 350, 358, 362, 366, 374, 378, 382, 390, 394, 398, 406, 410, 414, 422, 426, 430, 438, 442, 446, 454, 458, 462, 470, 474, 478, 486, 502, 518, 534, 550, 561, 570, 624, 635, 646, 714, 761 and 763.

The organic light-emitting compound according to the present disclosure contains an azine group, which is a strong electron acceptor, in the molecular structure thereof, and thus has excellent electron injection and transfer ability, thus improving the electron transfer ability to the light-emitting layer and exhibiting excellent driving voltage of the device.

In addition, the organic light-emitting compound according to the present disclosure has excellent thermal stability because it contains an aromatic hydrocarbon group and a condensed structure rather than a simple aliphatic ring group, and is effective in improving the processability and lifespan of the device because it has no crystallization temperature (Tc).

In addition, the organic light-emitting compound according to the present disclosure has a structure in which a cyclohexylfluorene group substituted with a cyano group or a pyridine group is linked to an azine moiety at the ortho position through a linker, and thus improves stability and the dipole moment. As a result, the organic light-emitting compound can increase interaction with the cathode, thus improving electron generation within the device and effectively improving the driving voltage characteristics.

In addition, by using the organic light-emitting compound according to the present disclosure as a material for an electron transport layer, excellent performance in terms of driving voltage, EL peak, and current efficiency can be obtained.

### <Organic electroluminescent device>

The present disclosure provides an organic electroluminescent device containing the novel organic light-emitting compound described above. The organic light-emitting compound according to the present disclosure may be incorporated in at least one of organic material layers disposed between the cathode and the anode of the organic electroluminescent device.

In one embodiment, the organic electroluminescent device includes an anode, a cathode, a light-emitting layer disposed between the cathode and the anode, and an electron transport region disposed between the cathode and the light-emitting layer, wherein the electron transport region contains the organic light-emitting compound according to the present disclosure.

### Anode

The organic electroluminescent device of the present disclosure includes an anode. The anode serves to inject holes into the organic layer. Here, the organic layer may mean at least one layer formed between the anode and the cathode.

The type of the anode material is not particularly limited and may be prepared in accordance with a conventional method known in the art. The anode material may, for example, include: a metal such as vanadium, chromium, copper, zinc, and gold, or an alloy thereof; a metal oxide such as zinc oxide, indium oxide, indium tin oxide (ITO), or indium zinc oxide (IZO); a composite of a metal and an oxide such as ZnO:Al and SnO₂:Sb; a conductive polymer such as polythiophene, poly(3-methylthiophene), poly[3,4-(ethylene-1,2-dioxy)thiophene] (PEDT), polypyrrole, or polyaniline; or carbon black. This compound may be used alone or as a combination of two or more thereof.

The method of preparing the anode is also not particularly limited and may be prepared in accordance with a conventional method known in the art. For example, the anode may be formed by coating a substrate, such as, a silicon wafer, quartz, a glass plate, a metal plate, or a plastic film with an anode material.

### Cathode

The organic electroluminescent device of the present disclosure includes a cathode. The cathode serves to inject electrons into the organic layer.

The type of cathode material constituting the cathode is not particularly limited and may be prepared in accordance with a conventional method known in the art. The cathode material, for example, includes: a metal such as magnesium, calcium, sodium, potassium, titanium, indium, yttrium, lithium, gadolinium, aluminum, silver, tin or lead, or an alloy thereof; or a multilayer structure material such as LiF/Al or LiO₂/Al.

### Light-emitting layer

The organic electroluminescent device of the present disclosure includes a light-emitting layer disposed between the cathode and the anode. The light-emitting layer is a layer where holes combine with electrons to form excitons, and the color of light emitted by the organic electroluminescent device may vary depending on the material constituting the light-emitting layer.

The light-emitting material constituting the light-emitting layer may be selected from various commercially available materials without particular limitation, depending on the desired emission wavelength of light.

In one embodiment, the light-emitting material may be classified into blue, green, and red light-emitting materials, and the like, depending on the color of emitted light. The light-emitting layer may be formed as a combination of a host and a dopant as the light-emitting material to prevent problems such as deterioration in color purity or reduction in device efficiency due to the light emission attenuation effect. The luminous efficacy of the organic electroluminescent device may be improved using a host material, which is the main material constituting the light-emitting layer, and a small amount of a dopant having a smaller energy band gap than the host material.

### Electron transport region

The organic electroluminescent device of the present disclosure includes an electron transport region disposed between the light-emitting layer and the cathode.

The electron transport region serves to move electrons injected from the cathode to the light-emitting layer. Such an electron transport region may include at least one selected from the group consisting of an electron injection layer or an electron transport layer. In this case, the organic electroluminescent device preferably includes both the electron transport layer and the electron injection layer described above in consideration of the characteristics of the organic electroluminescent device.

In the electron transport region, the electron injection layer may be formed of any electron injection material that facilitates injection of electrons from the cathode and has high electron mobility. Non-limiting examples of useful electron injection materials include the above-described amphiphilic compounds, anthracene derivatives, heteroaromatic compounds, alkali metal complexes and the like. As a specific example, the electron injection material includes at least one selected from the group consisting of: lanthanide metals such as LiF, Li₂O, BaO, NaCl, CsF and Yb; and halogenated metals such as RbCl and RbI.

The electron transport layer may include the organic light-emitting compound according to the present disclosure described above. Additionally, by using the novel organic light-emitting compound according to the present disclosure as an electron transport layer material, excellent performance can be achieved in terms of driving voltage, EL peak, and current efficiency.

The electron transport layer may be formed by mixing the organic light-emitting compound according to the present disclosure with lithium quinolate (Liq). Liq has a conduction band of 5.58 eV and a balance band of 3.153 eV, which has the effect of lowering the potential barrier.

The electron transport region may be prepared in accordance with a conventional method known in the art. The method for forming the electron transport region may, for example, include vacuum deposition, spin coating, casting, LB (Langmuir-Blodgett), inkjet printing, laser printing, and laser induced thermal imaging (LITI), but is not limited thereto.

### Electron transport auxiliary layer

The organic electric light-emitting element of the present disclosure may include an electron transport auxiliary layer disposed between the light-emitting layer and the electron transport region. The electron transport auxiliary layer may prevent the excitons or holes generated in the light-emitting layer from diffusing into the electron transport region.

The electron transport auxiliary layer may include an organic light-emitting compound according to the present disclosure described above. In addition, by using the novel organic light-emitting compound according to the present disclosure as a material for an electron transport auxiliary layer, excellent performance can be realized in terms of driving voltage, EL peak, and current efficiency.

The electron transport auxiliary layer may be formed in accordance with a conventional method known in the art, such as vacuum deposition, spin coating, casting, LB (Langmuir-Blodgett), inkjet printing, laser printing, or laser induced thermal imaging (LITI), but is not limited thereto.

### Hole transport region

The organic electroluminescent device of the present disclosure includes a hole transport region disposed between the anode and the light-emitting layer. The hole transport region serves to move holes injected from the anode to the light-emitting layer.

The hole transport region may include at least one selected from the group consisting of a hole injection layer or a hole transport layer. In this case, the organic electroluminescent device preferably includes both the hole transport layer and the hole injection layer described above in consideration of the characteristics of the organic electroluminescent device.

Any material may be used as the material for the hole injection layer and the hole transport layer without particular limitation as long as it has low hole injection barrier and high hole mobility, and may be selected from hole injection and hole transport materials used in the art without limitation. The material constituting the hole injection layer and the material constituting the hole transport layer may be the same as or different from each other.

The hole injection material may be selected from hole injection materials known in the art without limitation. Non-limiting examples of useful hole injection materials include phthalocyanine compounds such as copper phthalocyanine; DNTPD (N,N'-diphenyl-N,N'-bis-[4-(phenyl-m-tolyl-amino)-phenyl]-biphenyl-4,4'-diamine), m-MTDATA (4,4',4"-tris(3-methylphenylphenylamino) triphenylamine), TDATA (4,4'4"-tris (N,N diphenylamino) triphenylamine), 2TNATA (4,4',4"-tris{N,-(2-naphthyl)-N-phenylamino}-triphenylamine), PEDOT/PSS (poly(3,4-ethylenedioxythiophene)/poly(4-styrenesulfonate)), PANI/DBSA (polyaniline/dodecylbenzenesulfonic acid), PANI/CSA (polyaniline/camphor sulfonic acid), PANI/PSS ((polyaniline)/poly(4-styrenesulfonate)) and the like. This compound may be used alone or as a combination of two or more thereof.

In addition, the hole transport material may be selected from hole transport materials known in the art without limitation. Non-limitation examples of useful hole transport materials include: carbazol derivatives such as phenylcarbazole and polyvinylcarbazole; fluorene-based derivatives; triphenylamine derivatives such as TPD (N,N'-bis(3-methylphenyl)-N,N'-diphenyl-[1,1-biphenyl]-4,4'-diamine), and TCTA (4,4',4"-tris (N-carbazolyl)triphenylamine); NPB (N,N'-di(1-naphthyl)-N,N'-diphenylbenzidine), TAPC (4,4'-cyclohexylidene bis[N,N-bis (4-methylphenyl)benzenamine]) and the like. This compound may be used alone or as a combination of two or more thereof.

The hole transport layer may be formed in accordance with a conventional method known in the art, such as vacuum deposition, spin coating, casting, LB (Langmuir-Blodgett), inkjet printing, laser printing, or laser induced thermal imaging (LITI), but is not limited thereto.

### Hole transport auxiliary layer

The organic electroluminescent device according to the present disclosure may further include a hole transport auxiliary layer disposed between the hole transport region and the light-emitting layer. The hole transport auxiliary layer serves to control the thickness of the organic layer while transporting the holes from the hole transport region to the light-emitting layer. The hole transport auxiliary layer prevents electrons from moving to the hole transport layer based on high LUMO thereof and prevents excitons from moving from the light-emitting layer to the hole transport layer based on the high triplet (T1) energy.

The hole transport auxiliary layer may include a hole transport material and may be formed of the same material as in the hole transport region. In addition, the hole transport auxiliary layers of red, green and blue organic electroluminescent devices may be formed of the same material as each other.

The material for the hole transport auxiliary layer is not particularly limited and is, for example, a carbazole derivative, an arylamine derivative, or a carbazol-arylamine derivative. In addition, the hole transport auxiliary layer may selectively include a p-type dopant in addition to the above-described material. The p-type dopant may be any p-type dopant used in the art.

### Capping layer

The organic electroluminescent device of the present disclosure may further include a capping layer disposed on the cathode. The capping layer serves to protect the electroluminescent device and facilitate efficient emission of the light generated in the organic layer to the outside.

The capping material constituting the capping layer may, for example, include at least one selected from the group consisting of tris-8-hydroxyquinoline aluminum (Alq3), ZnSe, 2,5-bis(6'-(2',2"-bipyridyl))-1,1-dimethyl-3,4-diphenylsilole, 4'-bis[N-(1-naphthyl)-N-phenyl-amino]biphenyl (α-NPD), N,N'-diphenyl-N,N'-bis (3-methylphenyl)-1,1'-biphenyl-4,4'-diamine (TPD), and 1,1'-bis (di-4-tolylaminophenyl) cyclohexane (TAPC), but is not limited thereto.

The capping layer may be a single layer or may include two or more layers having different refractive indexes to gradually change refractive index of light that passes through the two or more layers.

The capping layer may be formed in accordance with a conventional method known in the art and the method may be selected from a variety of methods such as vacuum deposition, spin-coating, casting, and LB (Langmuir-Blodgett).

The present disclosure provides the use of the organic light-emitting compound described above for the organic electroluminescent device. The organic light-emitting compound imparts significantly improved light-emitting performance, driving voltage, lifespan and efficiency to the organic electroluminescent device.

In one embodiment, the organic light-emitting compound may be used as an electron transport material in the organic electroluminescent device.

In one embodiment, when the organic light-emitting compound is used as an electron transport material in the organic electroluminescent device, it may be used as a material for the electron transport region.

In one embodiment, the organic light-emitting compound may be used as a material for the electron transport layer and/or electron transport auxiliary layer in the organic electroluminescent device.

Hereinafter, the present disclosure will be described with reference to specific examples. However, the following examples are only provided to illustrate the present disclosure in detail and should not be construed as limiting the scope of the present disclosure.

### [Preparation Example]

### [Preparation Example 1]: Synthesis of Compound S01

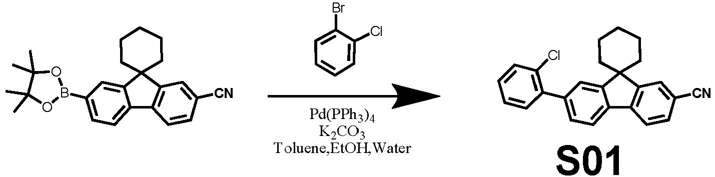

7'-(4,4,5,5-tetramethyl-1,3,2-dioxaborolan-2-yl)spiro[cyclohexane-1,9'-fluorene]-2'-carbonitrile (25.0 g, 64.9 mmol), 1-bromo-2-chlorobenzene (12.4 g, 64.9 mmol), Pd(PPh₃)₄ (2.2 g, 1.9 mmol) and K₂CO₃ (26.9 g, 194.6 mmol) were reacted under reflux in the presence of a mixed solvent of 250 ml of toluene, 37.5 ml of ethanol and 37.5 ml of water for 2 hours. After completion of the reaction, the reaction product was extracted with methylene chloride, and the extracted organic layer was dehydrated with magnesium sulfate, concentrated, and purified by column chromatography to obtain Compound S01 (2'-(2-chlorophenyl)spiro[cyclohexane-1,9'-fluorene]-7'-carbonitrile) (20.4 g, 55.2 mmol, yield 85 %).

Mass: [(M+H)⁺] :371

### [Preparation Example 2]: Synthesis of Compound S02

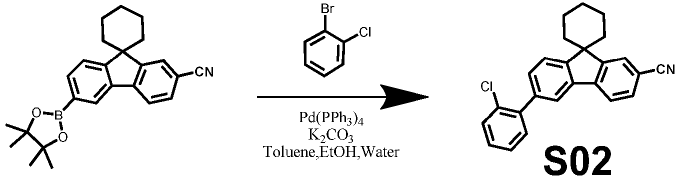

6'-(4,4,5,5-tetramethyl-1,3,2-dioxaborolan-2-yl)spiro[cyclohexane-1,9'-fluorene]-2'-carbonitrile (25.0 g, 64.9 mmol), 1-bromo-2-chlorobenzene (12.4 g, 64.9 mmol), Pd(PPh₃)₄ (2.2 g, 1.9 mmol) and K₂CO₃ (26.9 g, 194.6 mmol) were reacted under reflux in the presence of a mixed solvent of 250 ml of toluene, 37.5 ml of ethanol and 37.5 ml of water for 2 hours. After completion of the reaction, the reaction product was extracted with methylene chloride, and the extracted organic layer was dehydrated with magnesium sulfate, concentrated, and purified by column chromatography to obtain Compound S02 (2'-(2-chlorophenyl)spiro[cyclohexane-1,9'-fluorene]-7'-carbonitrile) (20.6 g, 55.8 mmol, yield 86%).

Mass: [(M+H)⁺] :371

### [Preparation Example 3]: Synthesis of Compound S03

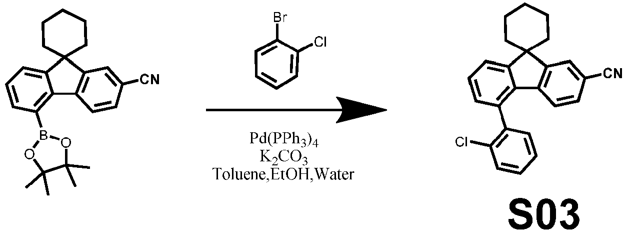

5'-(4,4,5,5-tetramethyl-1,3,2-dioxaborolan-2-yl)spiro[cyclohexane-1,9'-fluorene]-2'-carbonitrile (25.0 g, 64.9 mmol), 1-bromo-2-chlorobenzene (12.4 g, 64.9 mmol), Pd(PPh₃)₄ (2.2 g, 1.9 mmol) and K₂CO₃ (26.9 g, 194.6 mmol) were reacted under reflux in the presence of a mixed solvent of 250 ml of toluene, 37.5 ml of ethanol and 37.5 ml of water for 2 hours. After completion of the reaction, the reaction product was extracted with methylene chloride, and the extracted organic layer was dehydrated with magnesium sulfate, concentrated, and purified by column chromatography to obtain Compound S03 (5'-(2-chlorophenyl)spiro[cyclohexane-1,9'-fluorene]-2'-carbonitrile) (20.9 g, 56.4 mmol, yield 87%).

Mass: [(M+H)⁺] :371

### [Preparation Example 4]: Synthesis of Compound S04

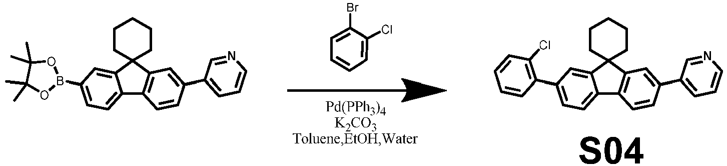

3-(2'-(4,4,5,5-tetramethyl-1,3,2-dioxaborolan-2-yl)spiro[cyclohexane-1,9'-fluoren]-7'-yl)pyridine (25.0 g, 57.2 mmol), 1-bromo-2-chlorobenzene (10.9 g, 57.2 mmol), Pd(PPh₃)₄ (2.0 g, 1.7 mmol) and K₂CO₃ (23.7 g, 171.5 mmol) were reacted under reflux in the presence of a mixed solvent of 250 ml of toluene, 37.5 ml of ethanol and 37.5 ml of water for 2 hours. After completion of the reaction, the reaction product was extracted with methylene chloride, and the extracted organic layer was dehydrated with magnesium sulfate, concentrated, and purified by column chromatography to obtain Compound S04 (3-(2'-(2-chlorophenyl)spiro[cyclohexane-1,9'-fluoren]-7'-yl)pyridine) (21.0 g, 49.7 mmol, yield 87%).

Mass: [(M+H)⁺] :423

### [Preparation Example 5]: Synthesis of Compound S05

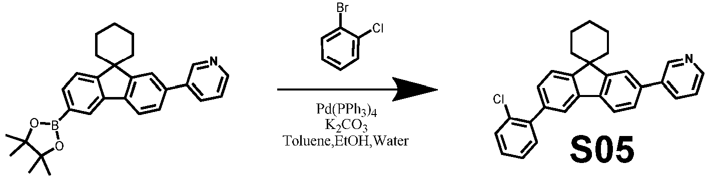

3-(3'-(4,4,5,5-tetramethyl-1,3,2-dioxaborolan-2-yl)spiro[cyclohexane-1,9'-fluoren]-7'-yl)pyridine (25.0 g, 57.2 mmol), 1-bromo-2-chlorobenzene (10.9 g, 57.2 mmol), Pd(PPh₃)₄ (2.0 g, 1.7 mmol) and K₂CO₃ (23.7 g, 171.5 mmol) were reacted under reflux in the presence of a mixed solvent of 250 ml of toluene, 37.5 ml of ethanol and 37.5 ml of water for 2 hours. After completion of the reaction, the reaction product was extracted with methylene chloride, and the extracted organic layer was dehydrated with magnesium sulfate, concentrated, and purified by column chromatography to obtain Compound S05 (3-(3'-(2-chlorophenyl)spiro[cyclohexane-1,9'-fluoren]-7'-yl)pyridine) (20.7 g, 49.2 mmol, yield 86%).

Mass: [(M+H)⁺]: 423

### [Preparation Example 6]: Synthesis of Compound S06

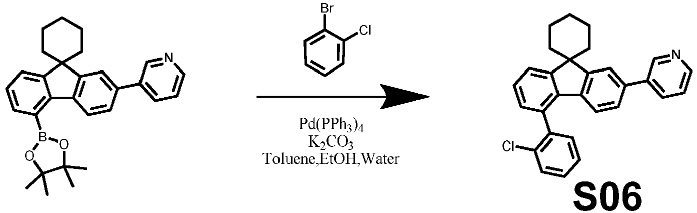

3-(4'-(4,4,5,5-tetramethyl-1,3,2-dioxaborolan-2-yl)spiro[cyclohexane-1,9'-fluoren]-7'-yl)pyridine (25.0 g, 57.2 mmol), 1-bromo-2-chlorobenzene (10.9 g, 57.2 mmol), Pd(PPh₃)₄ (2.0 g, 1.7 mmol) and K₂CO₃ (23.7 g, 171.5 mmol) were reacted under reflux in the presence of a mixed solvent of 250 ml of toluene, 37.5 ml of ethanol and 37.5 ml of water for 2 hours. After completion of the reaction, the reaction product was extracted with methylene chloride, and the extracted organic layer was dehydrated with magnesium sulfate, concentrated, and purified by column chromatography to obtain Compound S06 (3-(3'-(2-chlorophenyl)spiro[cyclohexane-1,9'-fluoren]-7'-yl)pyridine) (20.5 g, 48.6 mmol, yield 85%).

Mass: [(M+H)⁺] :423

### [Preparation Example 7]: Synthesis of Compound S07

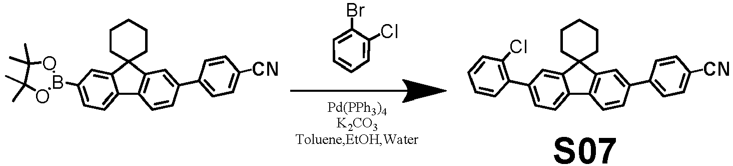

4-(2'-(4,4,5,5-tetramethyl-1,3,2-dioxaborolan-2-yl)spiro[cyclohexane-1,9'-fluoren]-7'-yl)benzonitrile (25.0 g, 54.2 mmol), 1-bromo-2-chlorobenzene (10.4 g, 54.2 mmol), Pd(PPh₃)₄ (1.9 g, 1.6 mmol) and K₂CO₃ (22.5 g, 162.5 mmol) were reacted under reflux in the presence of a mixed solvent of 250 ml of toluene, 37.5 ml of ethanol and 37.5 ml of water for 2 hours. After completion of the reaction, the reaction product was extracted with methylene chloride, and the extracted organic layer was dehydrated with magnesium sulfate, concentrated, and purified by column chromatography to obtain Compound S07 (4-(2'-(2-chlorophenyl)spiro[cyclohexane-1,9'-fluoren]-7'-yl)benzonitrile) (20.5 g, 46.1 mmol, yield 85%).

Mass: [(M+H)⁺] :447

### [Preparation Example 8]: Synthesis of Compound A01

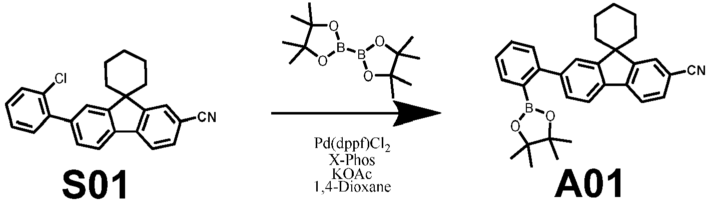

Compound S01 (20.0 g, 54.1 mmol) synthesized by the method of Preparation Example 1, bis(pinacolato)diboron (17.8 g, 70.3 mmol), Pd(dppf)Cl₂ (1.2 g, 1.6 mmol), X-Phos (1.5 g, 3.2 mmol) and KOAc (10.6 g, 108.1 mmol) were reacted under reflux in the presence of 200 ml of 1,4-dioxane for 6 hours. After the reaction was completed, the reaction product was filtered, concentrated to remove the solvent, and purified by column chromatography to obtain Compound A01 (7'-(2-(4,4,5,5-tetramethyl-1,3,2-dioxaborolan-2-yl)phenyl)spiro[cyclohexane-1,9'-fluorene]-2'-carbonitrile) (20.5 g, 44.3 mmol, yield 82%).

Mass: [(M+H)⁺] : 462

### [Preparation Example 9]: Synthesis of Compound A02

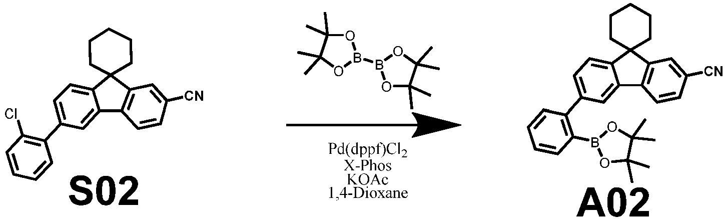

Compound S02 (20.0 g, 54.1 mmol) synthesized by the method of Preparation Example 2, bis(pinacolato)diboron (17.8 g, 70.3 mmol), Pd(dppf)Cl₂ (1.2 g, 1.6 mmol), X-Phos (1.5 g, 3.2 mmol) and KOAc (10.6 g, 108.1 mmol) were reacted under reflux in the presence of 200 ml of 1,4-dioxane for 6 hours. After the reaction was completed, the reaction product was filtered, concentrated to remove the solvent, and purified by column chromatography to obtain Compound A02 (6'-(2-(4,4,5,5-tetramethyl-1,3,2-dioxaborolan-2-yl)phenyl)spiro[cyclohexane-1,9'-fluorene]-2'-carbonitrile) (20.7 g, 44.9 mmol, yield 83%).

Mass: [(M+H)⁺] :462

### [Preparation Example 10]: Synthesis of Compound A03

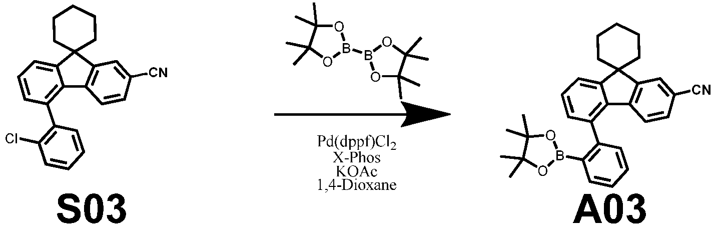

Compound S03 (20.0 g, 54.1 mmol) synthesized by the method of Preparation Example 3, bis(pinacolato)diboron (17.8 g, 70.3 mmol), Pd(dppf)Cl₂ (1.2 g, 1.6 mmol), X-Phos (1.5 g, 3.2 mmol) and KOAc (10.6 g, 108.1 mmol) were reacted under reflux in the presence of 200 ml of 1,4-dioxane for 6 hours. After the reaction was completed, the reaction product was filtered, concentrated to remove the solvent, and purified by column chromatography to obtain Compound A03 (5'-(2-(4,4,5,5-tetramethyl-1,3,2-dioxaborolan-2-yl)phenyl)spiro[cyclohexane-1,9'-fluorene]-2'-carbonitrile) (20.5 g, 44.3 mmol, yield 82%).

Mass: [(M+H)⁺] :462

### [Preparation Example 11]: Synthesis of Compound A04

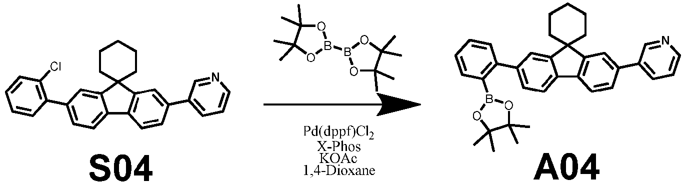

Compound S04 (20.0 g, 47.4 mmol) synthesized by the method of Preparation Example 4, bis(pinacolato)diboron (15.6 g, 61.6 mmol), Pd(dppf)Cl₂ (1.0 g, 1.4 mmol), X-Phos (0.3 g, 0.7 mmol) and KOAc (2.1 g, 21.9 mmol) were reacted under reflux in the presence of 200 ml of 1,4-dioxane for 6 hours. After the reaction was completed, the reaction product was filtered, concentrated to remove the solvent, and then purified by column chromatography to obtain Compound A04 (3-(2'-(2-(4,4,5,5-tetramethyl-1,3,2-dioxaborolan-2-yl)phenyl)spiro[cyclohexane-1,9'-fluoren]-7'-yl)pyridine) (18.4 g, 39.8 mmol, yield 84%).

Mass: [(M+H)⁺] :462

### [Preparation Example 12]: Synthesis of Compound A05

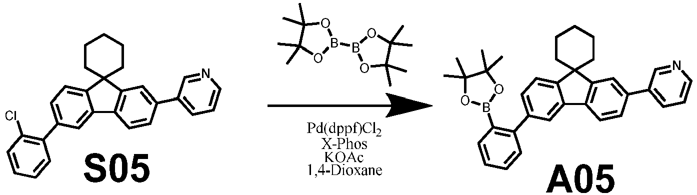

Compound S05 (20.0 g, 47.4 mmol) synthesized by the method of Preparation Example 5, bis(pinacolato)diboron (15.6 g, 61.6 mmol), Pd(dppf)Cl₂ (1.0 g, 1.4 mmol), X-Phos (0.3 g, 0.7 mmol) and KOAc (2.1 g, 21.9 mmol) were reacted under reflux in the presence of 200 ml of 1,4-dioxane for 6 hours. After the reaction was completed, the reaction product was filtered, concentrated to remove the solvent, and purified by column chromatography to obtain Compound A05 (3-(3'-(2-(4,4,5,5-tetramethyl-1,3,2-dioxaborolan-2-yl)phenyl)spiro[cyclohexane-1,9'-fluoren]-7'-yl)pyridine)(17.9 g, 38.9 mmol, yield 82%).

Mass: [(M+H)⁺] :462

### [Preparation Example 13]: Synthesis of Compound A06

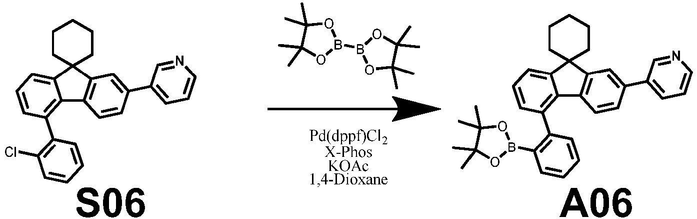

Compound S06 (20.0 g, 47.4 mmol) synthesized by the method of Preparation Example 6, bis(pinacolato)diboron (15.6 g, 61.6 mmol), Pd(dppf)Cl₂ (1.0 g, 1.4 mmol), X-Phos (0.3 g, 0.7 mmol) and KOAc (2.1 g, 21.9 mmol) were reacted under reflux in the presence of 200 ml of 1,4-dioxane for 6 hours. After the reaction was completed, the reaction product was filtered, concentrated to remove the solvent, and purified by column chromatography to obtain Compound A06 (3-(4'-(2-(4,4,5,5-tetramethyl-1,3,2-dioxaborolan-2-yl)phenyl)spiro[cyclohexane-1,9'-fluoren]-7'-yl)pyridine)(18.2 g, 39.3 mmol, yield 83%).

Mass: [(M+H)⁺] :462

### [Preparation Example 14]: Synthesis of Compound A07

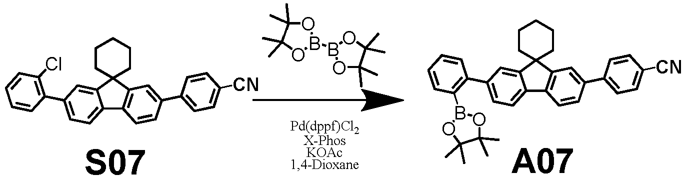

S07 (20.0 g, 44.8 mmol) synthesized by the method of Preparation Example 7, bis(pinacolato)diboron (14.8 g, 58.3 mmol), Pd(dppf)Cl₂ (1.0 g, 1.3 mmol), X-Phos (0.3 g, 0.7 mmol) and KOAc (2.1 g, 21.9 mmol) were reacted under reflux in the presence of 200 ml of 1,4-dioxane for 6 hours. After the reaction was completed, the reaction product was filtered, concentrated to remove the solvent, and purified by column chromatography to obtain Compound A07 (4-(2'-(2-(4,4,5,5-tetramethyl-1,3,2-dioxaborolan-2-yl)phenyl)spiro[cyclohexane-1,9'-fluoren]-7'-yl)benzonitrile) (20.0 g, 37.2 mmol, yield 83%).

Mass: [(M+H)⁺] :539

### [Preparation Example 15]: Synthesis of Compound B01

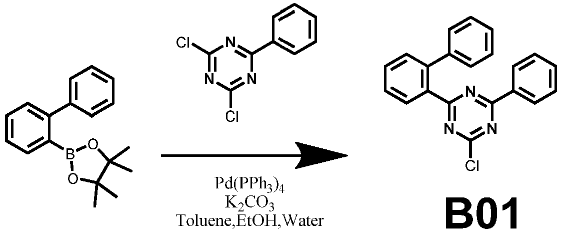

2-([1,1'-biphenyl]-2-yl)-4,4,5,5-tetramethyl-1,3,2-dioxaborolane (15.0 g, 53.5 mmol), 2,4-dichloro-6-phenyl-1,3,5-triazine (24.2 g, 107.1 mmol), Pd(PPh₃)₄ (1.9 g, 1.6 mmol) and K₂CO₃ (22.2 g, 160.6 mmol) were reacted under reflux in the presence of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B01 (2-([1,1'-biphenyl]-2-yl)-4-chloro-6-phenyl-1,3,5-triazine)(15.5 g, 45.0 mmol, yield 84%).

Mass: [(M+H)⁺] :345

### [Preparation Example 16]: Synthesis of Compound B02

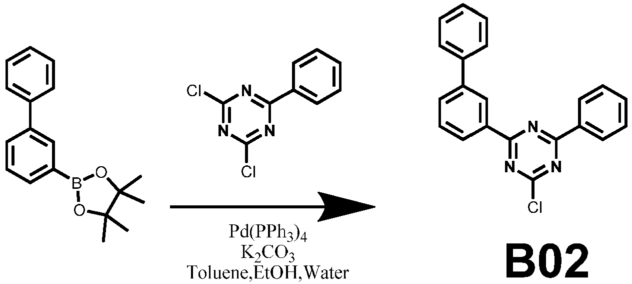

2-([1,1'-biphenyl]-3-yl)-4,4,5,5-tetramethyl-1,3,2-dioxaborolane (15.0 g, 53.5 mmol), 2,4-dichloro-6-phenyl-1,3,5-triazine (24.2 g, 107.1 mmol), Pd(PPh₃)₄ (1.9 g, 1.6 mmol) and K₂CO₃ (22.2 g, 160.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B02 (2-([1,1'-biphenyl]-3-yl)-4-chloro-6-phenyl-1,3,5-triazine) (15.3 g, 44.4 mmol, yield 83%).

Mass: [(M+H)⁺] :345

### [Preparation Example 17]: Synthesis of Compound B03

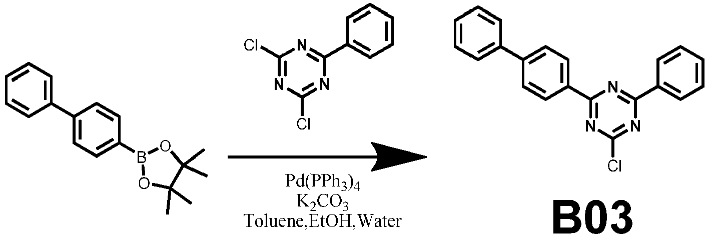

2-([1,1'-biphenyl]-3-yl)-4,4,5,5-tetramethyl-1,3,2-dioxaborolane (15.0 g, 53.5 mmol), 2,4-dichloro-6-phenyl-1,3,5-triazine (24.2 g, 107.1 mmol), Pd(PPh₃)₄ (1.9 g, 1.6 mmol) and K₂CO₃ (22.2 g, 160.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B03 (2-([1,1'-biphenyl]-4-yl)-4-chloro-6-phenyl-1,3,5-triazine) (15.1 g, 43.9 mmol, yield 82%).

Mass: [(M+H)⁺] :345

### [Preparation Example 18]: Synthesis of Compound B04

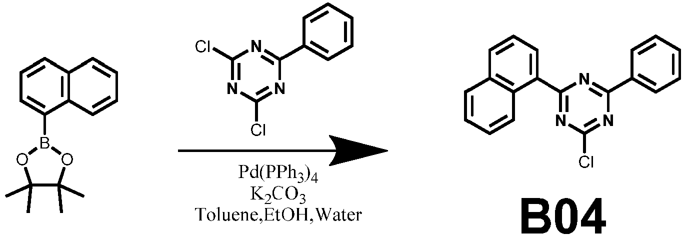

4,4,5,5-tetramethyl-2-(naphthalen-1-yl)-1,3,2-dioxaborolane (15.0 g, 59.0 mmol), 2,4-dichloro-6-phenyl-1,3,5-triazine (26.7 g, 118.0 mmol), Pd(PPh₃)₄ (2.0 g, 1.8 mmol) and K₂CO₃ (24.5 g, 177.1 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B04 (2-chloro-4-(naphthalen-1-yl)-6-phenyl-1,3,5-triazine) (15.9 g, 50.2 mmol, yield 85%).

Mass: [(M+H)⁺] :319

### [Preparation Example 19]: Synthesis of Compound B05

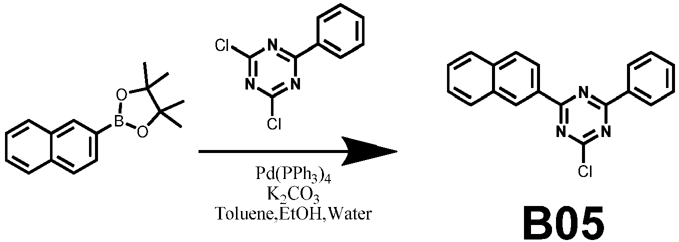

4,4,5,5-tetramethyl-2-(naphthalen-2-yl)-1,3,2-dioxaborolane (15.0 g, 59.0 mmol), 2,4-dichloro-6-phenyl-1,3,5-triazine (26.7 g, 118.0 mmol), Pd(PPh₃)₄ (2.0 g, 1.8 mmol) and K₂CO₃ (24.5 g, 177.1 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B05 (2-chloro-4-(naphthalen-2-yl)-6-phenyl-1,3,5-triazine) (15.8 g, 49.6 mmol, yield 84%).

Mass: [(M+H)⁺] :319

### [Preparation Example 20]: Synthesis of Compound B06

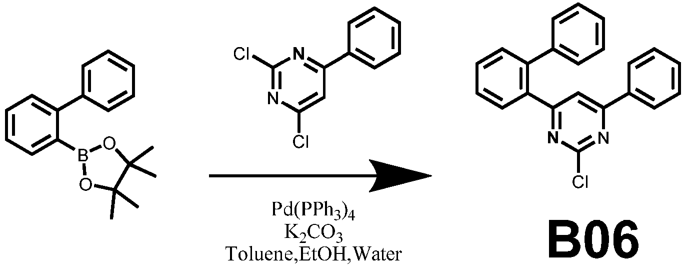

2-([1,1'-biphenyl]-2-yl)-4,4,5,5-tetramethyl-1,3,2-dioxaborolane (15.0 g, 53.5 mmol), 2,4-dichloro-6-phenylpyrimidine (24.1 g, 107.1 mmol), Pd(PPh₃)₄ (1.9 g, 1.6 mmol) and K₂CO₃ (22.2 g, 160.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B06 (4-([1,1'-biphenyl]-2-yl)-2-chloro-6-phenylpyrimidine)(15.2 g, 44.4 mmol, yield 83%).

Mass: [(M+H)⁺] :344

### [Preparation Example 21]: Synthesis of Compound B07

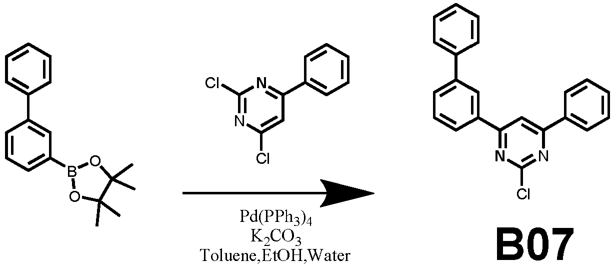

2-([1,1'-biphenyl]-3-yl)-4,4,5,5-tetramethyl-1,3,2-dioxaborolane (15.0 g, 53.5 mmol), 2,4-dichloro-6-phenylpyrimidine (24.1 g, 107.1 mmol), Pd(PPh₃)₄ (1.9 g, 1.6 mmol) and K₂CO₃ (22.2 g, 160.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B07 (4-([1,1'-biphenyl]-3-yl)-2-chloro-6-phenylpyrimidine)(15.4 g, 45.0 mmol, yield 84%).

Mass: [(M+H)⁺] :344

### [Preparation Example 22]: Synthesis of Compound B08

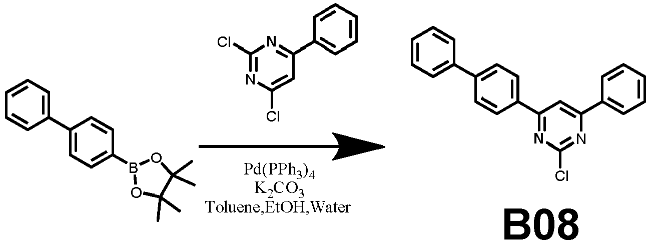

2-([1,1'-biphenyl]-3-yl)-4,4,5,5-tetramethyl-1,3,2-dioxaborolane (15.0 g, 53.5 mmol), 2,4-dichloro-6-phenylpyrimidine (24.1 g, 107.1 mmol), Pd(PPh₃)₄ (1.9 g, 1.6 mmol) and K₂CO₃ (22.2 g, 160.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B08 (4-([1,1'-biphenyl]-4-yl)-2-chloro-6-phenylpyrimidine)(15.2 g, 44.4 mmol, yield 83%).

Mass: [(M+H)⁺] :344

### [Preparation Example 23]: Synthesis of Compound B09

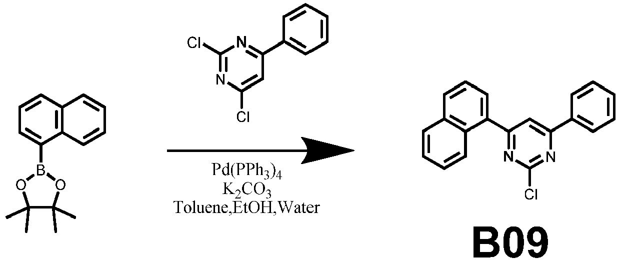

4,4,5,5-tetramethyl-2-(naphthalen-1-yl)-1,3,2-dioxaborolane (15.0 g, 59.0 mmol), 2,4-dichloro-6-phenylpyrimidine (26.6 g, 118.0 mmol), Pd(PPh₃)₄ (2.0 g, 1.8 mmol) and K₂CO₃ (24.5 g, 177.1 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B09 (2-chloro-4-(naphthalen-1-yl)-6-phenylpyrimidine) (15.7 g, 49.6 mmol, yield 84%).

Mass: [(M+H)⁺] :318

### [Preparation Example 24]: Synthesis of Compound B10

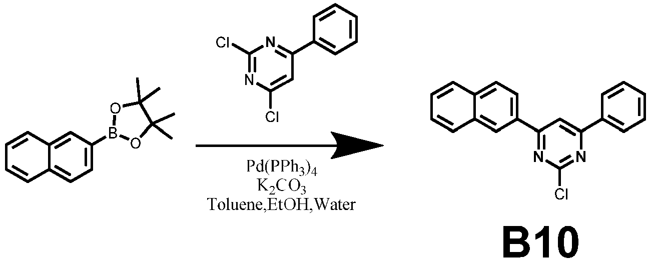

4,4,5,5-tetramethyl-2-(naphthalen-2-yl)-1,3,2-dioxaborolane (15.0 g, 59.0 mmol), 2,4-dichloro-6-phenylpyrimidine (26.6 g, 118.0 mmol), Pd(PPh₃)₄ (2.0 g, 1.8 mmol) and K₂CO₃ (24.5 g, 177.1 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B10 (2-chloro-4-(naphthalen-2-yl)-6-phenylpyrimidine) (15.5 g, 49.0 mmol, yield 83%).

Mass: [(M+H)⁺] :318

### [Preparation Example 25]: Synthesis of Compound B11

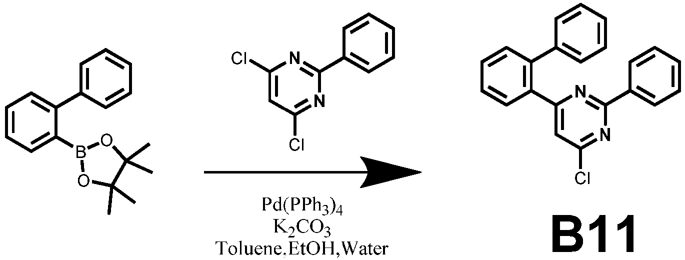

2-([1,1'-biphenyl]-2-yl)-4,4,5,5-tetramethyl-1,3,2-dioxaborolane (15.0 g, 53.5 mmol), 4,6-dichloro-2-phenylpyrimidine (24.1 g, 107.1 mmol), Pd(PPh₃)₄ (1.9 g, 1.6 mmol) and K₂CO₃ (22.2 g, 160.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B11 (4-([1,1'-biphenyl]-2-yl)-2-chloro-6-phenylpyrimidine) (15.6 g, 45.5 mmol, yield 85%).

Mass: [(M+H)⁺] :344

### [Preparation Example 26]: Synthesis of Compound B12

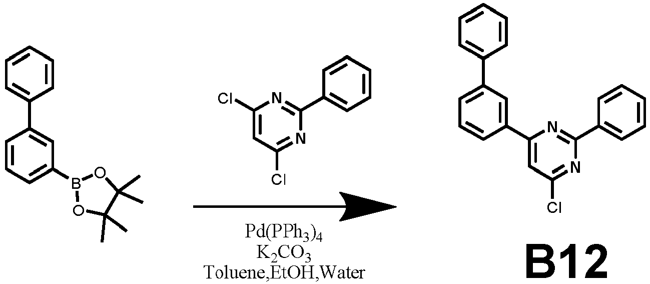

2-([1,1'-biphenyl]-3-yl)-4,4,5,5-tetramethyl-1,3,2-dioxaborolane (15.0 g, 53.5 mmol), 4,6-dichloro-2-phenylpyrimidine (24.1 g, 107.1 mmol), Pd(PPh₃)₄ (1.9 g, 1.6 mmol) and K₂CO₃ (22.2 g, 160.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B12 (4-([1,1'-biphenyl]-3-yl)-2-chloro-6-phenylpyrimidine) (15.6 g, 45.5 mmol, yield 85%).

Mass: [(M+H)⁺] :344

### [Preparation Example 27]: Synthesis of Compound B13

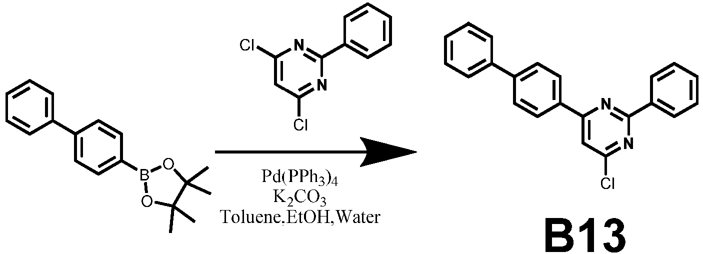

2-([1,1'-biphenyl]-3-yl)-4,4,5,5-tetramethyl-1,3,2-dioxaborolane (15.0 g, 53.5 mmol), 4,6-dichloro-2-phenylpyrimidine (24.1 g, 107.1 mmol), Pd(PPh₃)₄ (1.9 g, 1.6 mmol) and K₂CO₃ (22.2 g, 160.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B13 (4-([1,1'-biphenyl]-4-yl)-6-chloro-2-phenylpyrimidine)(15.4 g, 45.0 mmol, yield 84%).

Mass: [(M+H)⁺] :344

### [Preparation Example 28]: Synthesis of Compound B14

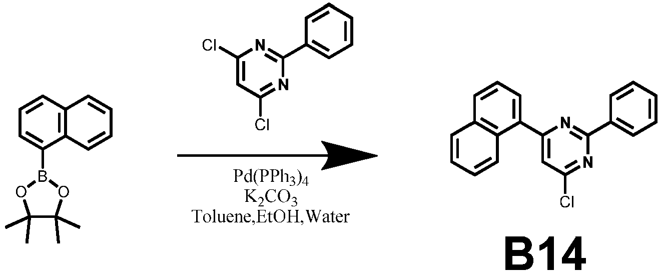

4,4,5,5-tetramethyl-2-(naphthalen-1-yl)-1,3,2-dioxaborolane (15.0 g, 59.0 mmol), 4,6-dichloro-2-phenylpyrimidine (26.6 g, 118.0 mmol), Pd(PPh₃)₄ (2.0 g, 1.8 mmol) and K₂CO₃ (24.5 g, 177.1 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B14 (4-chloro-6-(naphthalen-1-yl)-2-phenylpyrimidine) (15.5 g, 49.0 mmol, yield 83%).

Mass: [(M+H)⁺] :318

### [Preparation Example 29]: Synthesis of Compound B15

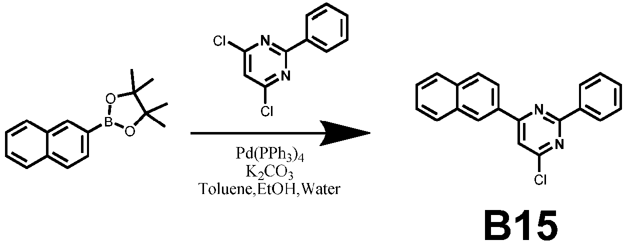

4,4,5,5-tetramethyl-2-(naphthalen-2-yl)-1,3,2-dioxaborolane (15.0 g, 59.0 mmol), 4,6-dichloro-2-phenylpyrimidine (26.6 g, 118.0 mmol), Pd(PPh₃)₄ (2.0 g, 1.8 mmol) and K₂CO₃ (24.5 g, 177.1 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B15 (4-chloro-6-(naphthalen-2-yl)-2-phenylpyrimidine) (15.5 g, 49.0 mmol, yield 83%).

Mass: [(M+H)⁺] :318

### [Preparation Example 30]: Synthesis of Compound B16

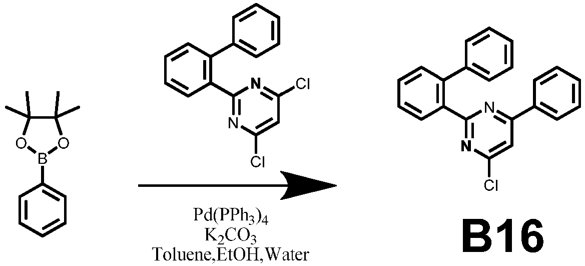

4,4,5,5-tetramethyl-2-phenyl-1,3,2-dioxaborolane (15.0 g, 73.5 mmol), 2-([1,1'-biphenyl]-2-yl)-4,6-dichloropyrimidine (44.3 g, 147.0 mmol), Pd(PPh₃)₄ (2.5 g, 2.2 mmol) and K₂CO₃ (30.5 g, 220.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B16 (2-([1,1'-biphenyl]-2-yl)-4-chloro-6-phenylpyrimidine) (21.4 g, 62.5 mmol, yield 85%).

Mass: [(M+H)⁺] :344

### [Preparation Example 31]: Synthesis of Compound B17

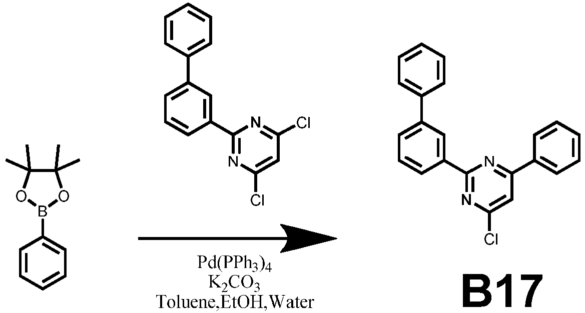

4,4,5,5-tetramethyl-2-phenyl-1,3,2-dioxaborolane (15.0 g, 73.5 mmol), 2-([1,1'-biphenyl]-2-yl)-4,6-dichloropyrimidine (44.3 g, 147.0 mmol), Pd(PPh₃)₄ (2.5 g, 2.2 mmol) and K₂CO₃ (30.5 g, 220.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B17 (2-([1,1'-biphenyl]-3-yl)-4-chloro-6-phenylpyrimidine)(21.2 g, 61.7 mmol, yield 84%).

Mass: [(M+H)⁺] :344

### [Preparation Example 32]: Synthesis of Compound B18

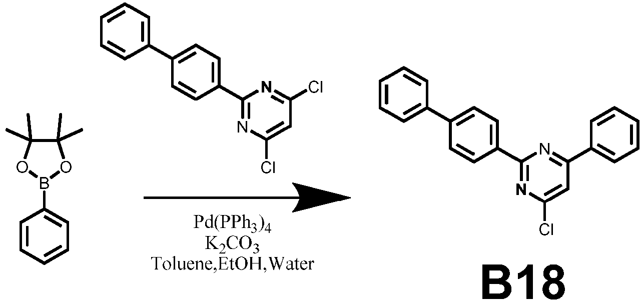

4,4,5,5-tetramethyl-2-phenyl-1,3,2-dioxaborolane (15.0 g, 73.5 mmol), 2-([1,1'-biphenyl]-2-yl)-4,6-dichloropyrimidine (44.3 g, 147.0 mmol), Pd(PPh₃)₄ (2.5 g, 2.2 mmol) and K₂CO₃ (30.5 g, 220.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B18 (2-([1,1'-biphenyl]-4-yl)-4-chloro-6-phenylpyrimidine)(20.9 g, 61.0 mmol, yield 83%).

Mass: [(M+H)⁺] :344

### [Preparation Example 33]: Synthesis of Compound B19

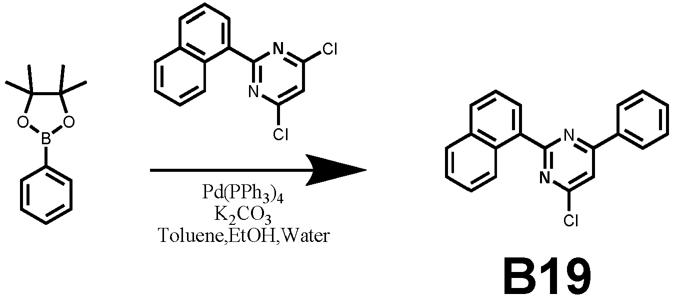

4,4,5,5-tetramethyl-2-phenyl-1,3,2-dioxaborolane (15.0 g, 73.5 mmol), 2-([1,1'-biphenyl]-2-yl)-4,6-dichloropyrimidine (44.3 g, 147.0 mmol), Pd(PPh₃)₄ (2.5 g, 2.2 mmol) and K₂CO₃ (30.5 g, 220.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B19 (4-chloro-2-(naphthalen-1-yl)-6-phenylpyrimidine) (19.1 g, 60.3 mmol, yield 82%).

Mass: [(M+H)⁺] :318

### [Preparation Example 34]: Synthesis of Compound B20

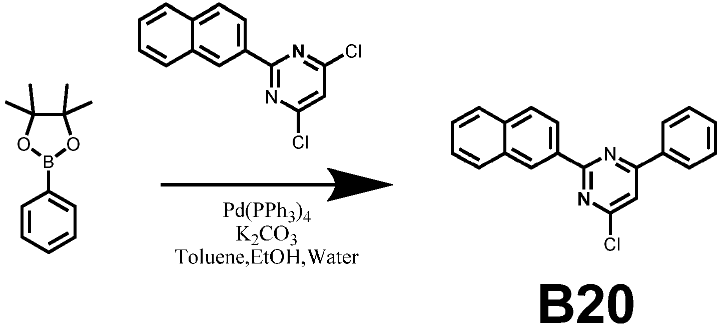

4,4,5,5-tetramethyl-2-phenyl-1,3,2-dioxaborolane (15.0 g, 73.5 mmol), 2-([1,1'-biphenyl]-2-yl)-4,6-dichloropyrimidine (44.3 g, 147.0 mmol), Pd(PPh₃)₄ (2.5 g, 2.2 mmol) and K₂CO₃ (30.5 g, 220.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B20 (4-chloro-2-(naphthalen-2-yl)-6-phenylpyrimidine) (19.3 g, 61.0 mmol, yield 83%).

Mass: [(M+H)⁺] :318

### [Preparation Example 35]: Synthesis of Compound B21

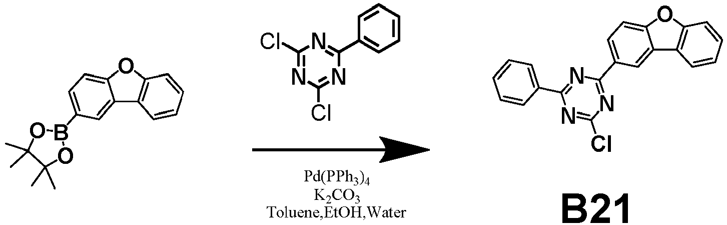

2-(dibenzo[b,d]furan-2-yl)-4,4,5,5-tetramethyl-1,3,2-dioxaborolane (15.0 g, 51.0 mmol), 2,4-dichloro-6-phenyl-1,3,5-triazine (23.1 g, 102.0 mmol), Pd(PPh₃)₄ (1.8 g, 1.5 mmol) and K₂CO₃ (21.1 g, 153.0 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B21 (2-chloro-4-(dibenzo[b,d]furan-2-yl)-6-phenyl-1,3,5-triazine) (15.3 g, 42.8 mmol, yield 84%).

Mass: [(M+H)⁺] :339

### [Preparation Example 36]: Synthesis of Compound B22

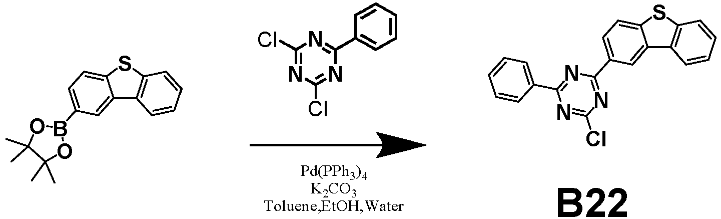

2-(dibenzo[b,d]thiophen-2-yl)-4,4,5,5-tetramethyl-1,3,2-dioxaborolane (15.0 g, 48.4 mmol), 2,4-dichloro-6-phenyl-1,3,5-triazine (21.9 g, 96.7 mmol), Pd(PPh₃)₄ (1.7 g, 1.5 mmol) and K₂CO₃ (20.0 g, 145.1 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B22 (2-chloro-4-(dibenzo[b,d]thiophen-2-yl)-6-phenyl-1,3,5-triazine) (15.2 g, 40.6 mmol, yield 84%).

Mass: [(M+H)⁺] :375

### [Preparation Example 37]: Synthesis of Compound B23

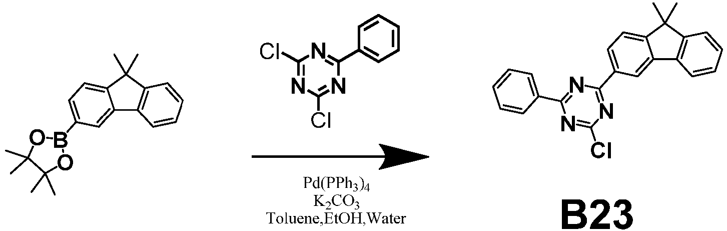

2-(9,9-dimethyl-9H-fluoren-3-yl)-4,4,5,5-tetramethyl-1,3,2-dioxaborolane (15.0 g, 46.8 mmol), 2,4-dichloro-6-phenyl-1,3,5-triazine (21.2 g, 93.7 mmol), Pd(PPh₃)₄ (1.6 g, 1.4 mmol) and K₂CO₃ (19.4 g, 140.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B23 (2-chloro-4-(9,9-dimethyl-9H-fluoren-3-yl)-6-phenyl-1,3,5-triazine) (15.1 g, 39.3 mmol, yield 84%).

Mass: [(M+H)⁺] :385

### [Preparation Example 38]: Synthesis of Compound B24

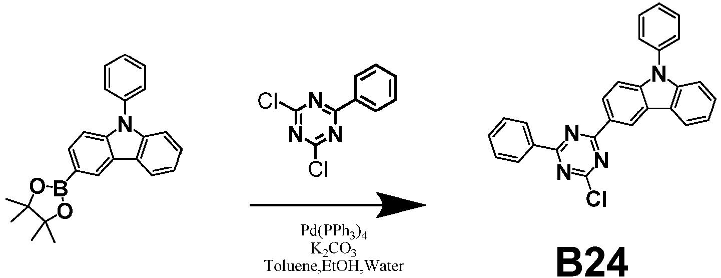

9-phenyl-3-(4,4,5,5-tetramethyl-1,3,2-dioxaborolan-2-yl)-9H-carbazole (15.0 g, 40.6 mmol), 2,4-dichloro-6-phenyl-1,3,5-triazine (18.4 g, 81.2 mmol), Pd(PPh₃)₄ (1.4 g, 1.2 mmol) and K₂CO₃ (16.8 g, 121.9 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B24 (3-(4-chloro-6-phenyl-1,3,5-triazin-2-yl)-9-phenyl-9H-carbazole) (14.8 g, 34.1 mmol, yield 84%).

Mass: [(M+H)⁺] :434

### [Preparation Example 39]: Synthesis of Compound B25

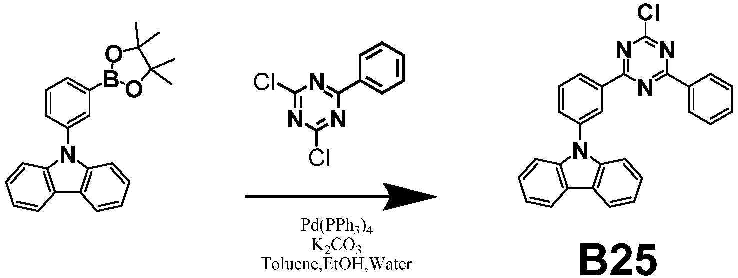

9-(3-(4,4,5,5-tetramethyl-1,3,2-dioxaborolan-2-yl)phenyl)-9H-carbazole (15.0 g, 40.6 mmol), 2,4-dichloro-6-phenyl-1,3,5-triazine (18.4 g, 81.2 mmol), Pd(PPh₃)₄ (1.4 g, 1.2 mmol) and K₂CO₃ (16.8 g, 121.9 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B25 (9-(3-(4-chloro-6-phenyl-1,3,5-triazin-2-yl)phenyl)-9H-carbazole) (14.8 g, 34.1 mmol, yield 84%).

Mass: [(M+H)⁺] :434

### [Preparation Example 40]: Synthesis of Compound B26

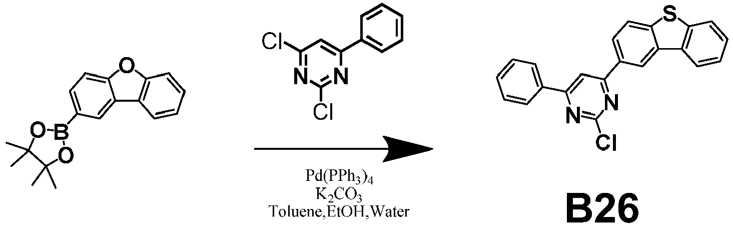

2-(dibenzo[b,d]furan-2-yl)-4,4,5,5-tetramethyl-1,3,2-dioxaborolane (15.0 g, 51.0 mmol), 2,4-dichloro-6-phenylpyrimidine (23.0 g, 102.0 mmol), Pd(PPh₃)₄ (1.8 g, 1.5 mmol) and K₂CO₃ (21.1 g, 153.0 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B26 (2-chloro-4-(dibenzo[b,d]thiophen-2-yl)-6-phenylpyrimidine) (15.8 g, 42.3 mmol, yield 83%).

Mass: [(M+H)⁺] :374

### [Preparation Example 41]: Synthesis of Compound B27

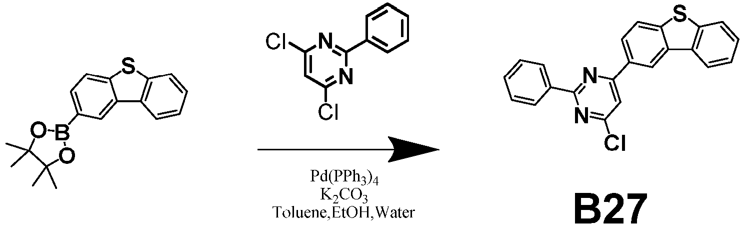

2-(dibenzo[b,d]thiophen-2-yl)-4,4,5,5-tetramethyl-1,3,2-dioxaborolane (15.0 g, 48.4 mmol), 4,6-dichloro-2-phenylpyrimidine (21.8 g, 96.7 mmol), Pd(PPh₃)₄ (1.7 g, 1.5 mmol) and K₂CO₃ (20.0 g, 145.1 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B27 (4-chloro-6-(dibenzo[b,d]thiophen-2-yl)-2-phenylpyrimidine) (15.1 g, 40.6 mmol, yield 84%).

Mass: [(M+H)⁺] :374

### [Preparation Example 42]: Synthesis of Compound B28

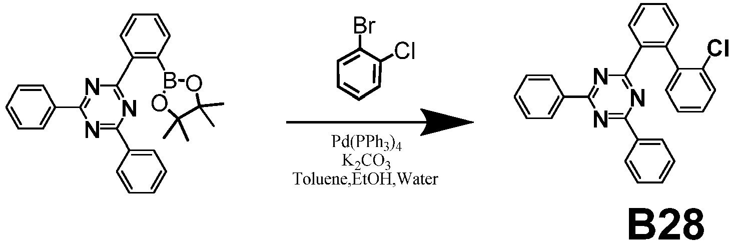

2,4-diphenyl-6-(2-(4,4,5,5-tetramethyl-1,3,2-dioxaborolan-2-yl)phenyl)-1,3,5-triazine (25.0 g, 57.4 mmol), 1-bromo-2-chlorobenzene (11.0 g, 57.4 mmol), Pd(PPh₃)₄ (2.0 g, 1.7 mmol) and K₂CO₃ (23.8 g, 172.3 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B28 (2-(2'-chloro-[1,1'-biphenyl]-2-yl)-4,6-diphenyl-1,3,5-triazine) (20.5 g, 48.8 mmol, yield 85%).

Mass: [(M+H)⁺] :421

### [Preparation Example 43]: Synthesis of Compound B29

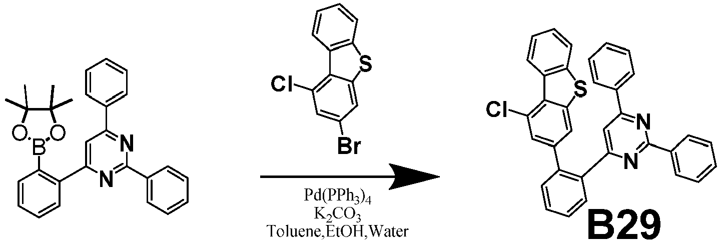

2,4-diphenyl-6-(2-(4,4,5,5-tetramethyl-1,3,2-dioxaborolan-2-yl)phenyl)pyrimidine (25.0 g, 57.6 mmol), 3-bromo-1-chlorodibenzo[b,d]thiophene (17.1 g, 57.6 mmol), Pd(PPh₃)₄ (2.0 g, 1.7 mmol) and K₂CO₃ (23.9 g, 172.7 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B29 (4-(2-(1-chlorodibenzo[b,d]thiophen-3-yl)phenyl)-2,6-diphenylpyrimidine) (20.6 g, 48.9 mmol, yield 85%) .

Mass: [(M+H)⁺] :423

### [Preparation Example 44]: Synthesis of Compound B30

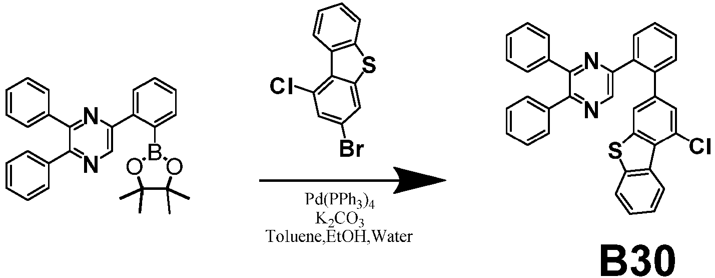

2,3-diphenyl-5-(2-(4,4,5,5-tetramethyl-1,3,2-dioxaborolan-2-yl)phenyl)pyrazine (25.0 g, 57.6 mmol), 3-bromo-1-chlorodibenzo[b,d]thiophene (17.1 g, 57.6 mmol), Pd(PPh₃)₄ (2.0 g, 1.7 mmol) and K₂CO₃ (23.9 g, 172.7 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound B30 (5-(2-(1-chlorodibenzo[b,d]thiophen-3-yl)phenyl)-2,3-diphenylpyrazine)(25.7 g, 48.9 mmol, yield 85%) .

Mass: [(M+H)⁺] :526

### [Synthesis Example]

### [Synthesis Example 1]: Synthesis of Compound 006

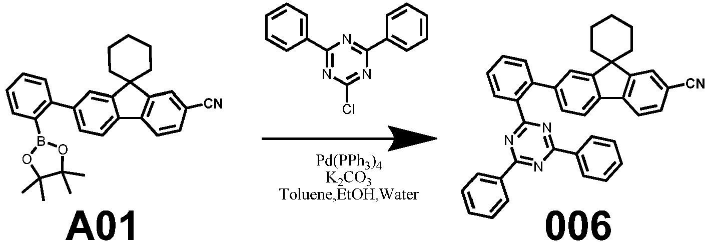

Compound A01 (15.0 g, 32.5 mmol) synthesized by the method of Preparation Example 8, 2-chloro-4,6-diphenyl-1,3,5-triazine (8.7 g, 32.5 mmol), Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 006 (7'-(2-(4,6-diphenyl-1,3,5-triazin-2-yl)phenyl)spiro[cyclohexane-1,9'-fluorene]-2'-carbonitrile) (13.3 g, 23.4 mmol, yield 72%).

Mass: [(M+H)⁺] :568

### [Synthesis Example 2]: Synthesis of Compound 010

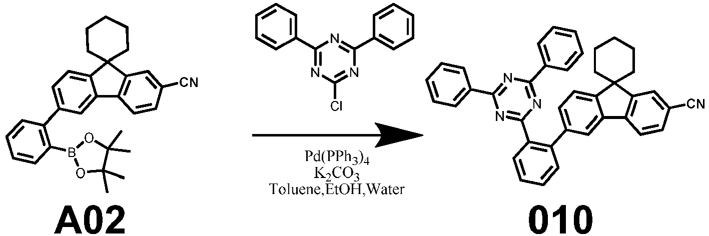

Compound A02 (15.0 g, 32.5 mmol) synthesized by the method of Preparation Example 9, 2-chloro-4,6-diphenyl-1,3,5-triazine (8.7 g, 32.5 mmol), Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 010 (6'-(2-(4,6-diphenyl-1,3,5-triazin-2-yl)phenyl)spiro[cyclohexane-1,9'-fluorene]-2'-carbonitrile) (13.8 g, 24.4 mmol, yield 75%).

Mass: [(M+H)⁺] :568

### [Synthesis Example 3]: Synthesis of Compound 014

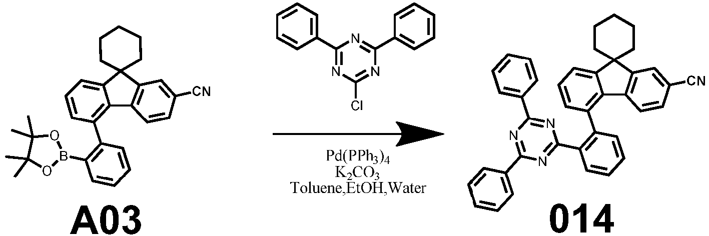

Compound A03 (15.0 g, 32.5 mmol) synthesized by the method of Preparation Example 10, 2-chloro-4,6-diphenyl-1,3,5-triazine (8.7 g, 32.5 mmol), Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 014 (5'-(2-(4,6-diphenyl-1,3,5-triazin-2-yl)phenyl)spiro[cyclohexane-1,9'-fluorene]-2'-carbonitrile) (13.4 g, 23.7 mmol, yield 73%).

Mass: [(M+H)⁺] :568

### [Synthesis Example 4]: Synthesis of Compound 022

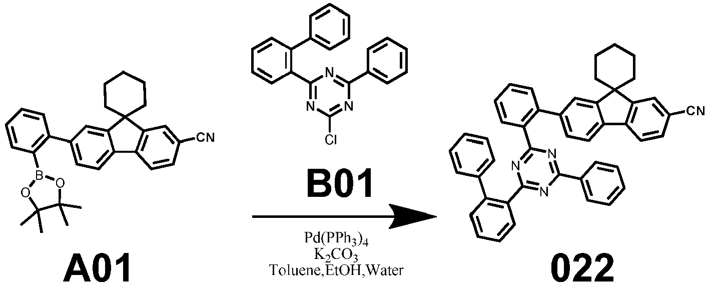

Compound A01 (15.0 g, 32.5 mmol) synthesized by the method of Preparation Example 8, Compound B01 (11.2 g, 32.5 mmol) synthesized by the method of Preparation Example 15, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 022 (2'-(2-(4-([1,1'-biphenyl]-2-yl)-6-phenyl-1,3,5-triazin-2-yl)phenyl)spiro[cyclohexane-1,9'-fluorene]-7'-carbonitrile) (15.7 g, 23.7 mmol, yield 75%).

Mass: [(M+H)⁺] :644

### [Synthesis Example 5]: Synthesis of Compound 026

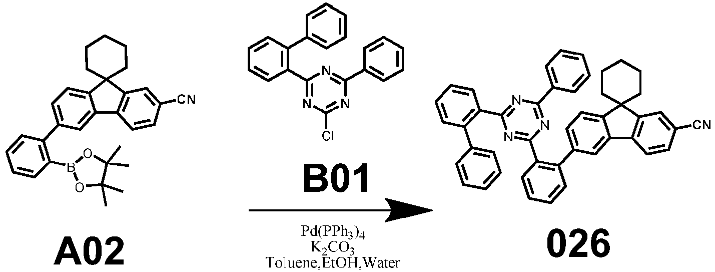

Compound A02 (15.0 g, 32.5 mmol) synthesized by the method of Preparation Example 9, Compound B01 (11.2 g, 32.5 mmol) synthesized by the method of Preparation Example 15, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 026 (6'-(2-(4-([1,1'-biphenyl]-2-yl)-6-phenyl-1,3,5-triazin-2-yl)phenyl)spiro[cyclohexane-1,9'-fluorene]-2'-carbonitrile) (15.5 g, 24.7 mmol, yield 74%).

Mass: [(M+H)⁺] :644

### [Synthesis Example 6]: Synthesis of Compound 030

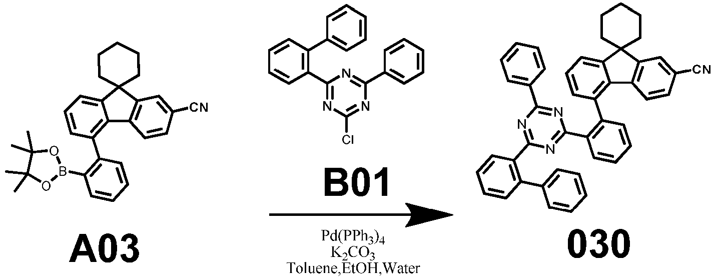

Compound A03 (15.0 g, 32.5 mmol) synthesized by the method of Preparation Example 10, Compound B01 (11.2 g, 32.5 mmol) synthesized by the method of Preparation Example 15, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 030 (5'-(2-(4-([1,1'-biphenyl]-2-yl)-6-phenyl-1,3,5-triazin-2-yl)phenyl)spiro[cyclohexane-1,9'-fluorene]-2'-carbonitrile) (15.0 g, 23.4 mmol, yield 72%).

Mass: [(M+H)⁺] :644

### [Synthesis Example 7]: Synthesis of Compound 038

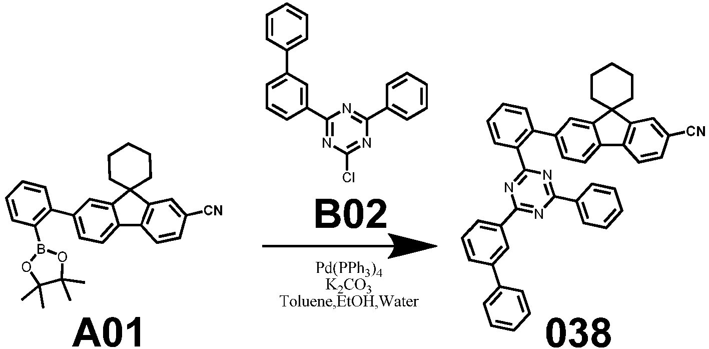

Compound A01 (15.0 g, 32.5 mmol) synthesized by the method of Preparation Example 8, Compound B02 (11.2 g, 32.5 mmol) synthesized by the method of Preparation Example 16, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 038 (2'-(2-(4-([1,1'-biphenyl]-3-yl)-6-phenyl-1,3,5-triazin-2-yl)phenyl)spiro[cyclohexane-1,9'-fluorene]-7'-carbonitrile) (16.4 g, 23.7 mmol, yield 73%).

Mass: [(M+H)⁺] :644

### [Synthesis Example 8]: Synthesis of Compound 042

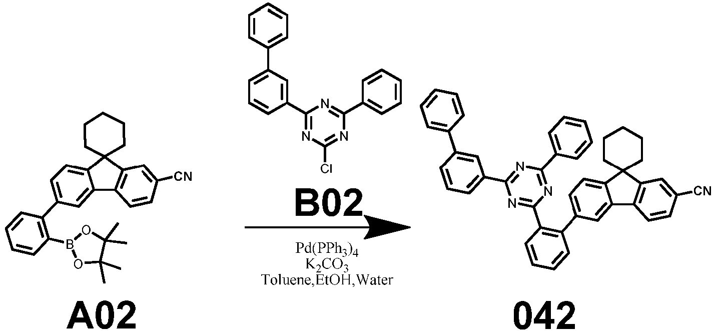

Compound A02 (15.0 g, 32.5 mmol) synthesized by the method of Preparation Example 9, Compound B02 (11.2 g, 32.5 mmol) synthesized by the method of Preparation Example 16, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 042 (6'-(2-(4-([1,1'-biphenyl]-3-yl)-6-phenyl-1,3,5-triazin-2-yl)phenyl)spiro[cyclohexane-1,9'-fluorene]-2'-carbonitrile) (16.7 g, 24.1 mmol, yield 74%).

Mass: [(M+H)⁺] :644

### [Synthesis Example 9]: Synthesis of Compound 046

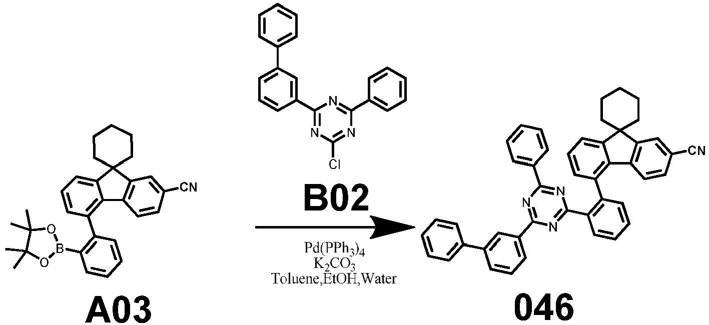

Compound A03 (15.0 g, 32.5 mmol) synthesized by the method of Preparation Example 10, Compound B02 (11.2 g, 32.5 mmol) synthesized by the method of Preparation Example 16, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 046 (5'-(2-(4-([1,1'-biphenyl]-3-yl)-6-phenyl-1,3,5-triazin-2-yl)phenyl)spiro[cyclohexane-1,9'-fluorene]-2'-carbonitrile) (16.4 g, 23.7 mmol, yield 73%).

Mass: [(M+H)⁺] :644

### [Synthesis Example 10]: Synthesis of Compound 054

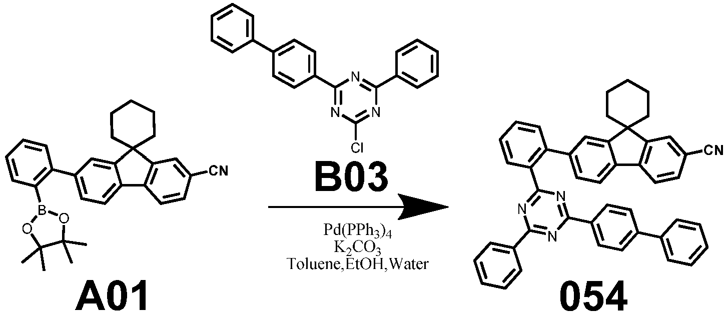

Compound A01 (15.0 g, 32.5 mmol) synthesized by the method of Preparation Example 8, Compound B03 (11.2 g, 32.5 mmol) synthesized by the method of Preparation Example 17, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 054 (2'-(2-(4-([1,1'-biphenyl]-4-yl)-6-phenyl-1,3,5-triazin-2-yl)phenyl)spiro[cyclohexane-1,9'-fluorene]-7'-carbonitrile) (16.2 g, 23.4 mmol, yield 72%).

Mass: [(M+H)⁺] :644

### [Synthesis Example 11]: Synthesis of Compound 058

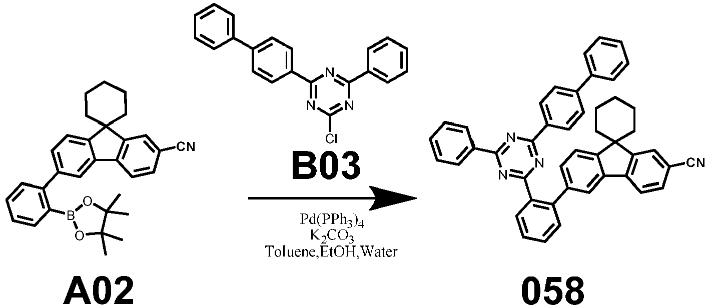

Compound A02 (15.0 g, 32.5 mmol) synthesized by the method of Preparation Example 9, Compound B03 (11.2 g, 32.5 mmol) synthesized by the method of Preparation Example 17, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 058 (6'-(2-(4-([1,1'-biphenyl]-4-yl)-6-phenyl-1,3,5-triazin-2-yl)phenyl)spiro[cyclohexane-1,9'-fluorene]-2'-carbonitrile) (16.7 g, 23.1 mmol, yield 74%).

Mass: [(M+H)⁺] :644

### [Synthesis Example 12]: Synthesis of Compound 062

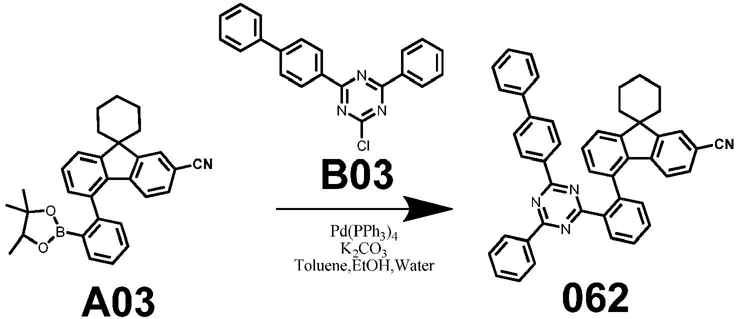

Compound A03 (15.0 g, 32.5 mmol) synthesized by the method of Preparation Example 10, Compound B03 (11.2 g, 32.5 mmol) synthesized by the method of Preparation Example 17, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 062 (5'-(2-(4-([1,1'-biphenyl]-4-yl)-6-phenyl-1,3,5-triazin-2-yl)phenyl)spiro[cyclohexane-1,9'-fluorene]-2'-carbonitrile) (17.1 g, 24.7 mmol, yield 76%).

Mass: [(M+H)⁺] :644

### [Synthesis Example 13]: Synthesis of Compound 070

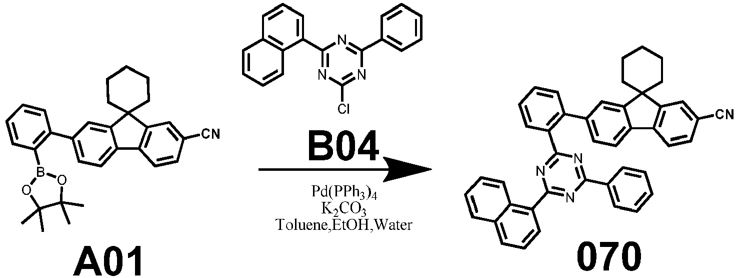

Compound A01 (15.0 g, 32.5 mmol) synthesized by the method of Preparation Example 8, Compound B02 (10.3 g, 32.5 mmol) synthesized by the method of Preparation Example 16, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 070 (7'-(2-(4-(naphthalen-1-yl)-6-phenyl-1,3,5-triazin-2-yl)phenyl)spiro[cyclohexane-1,9'-fluorene]-2'-carbonitrile) (16.9 g, 24.4 mmol, yield 75%).

Mass: [(M+H)⁺] :618

### [Synthesis Example 14]: Synthesis of Compound 074

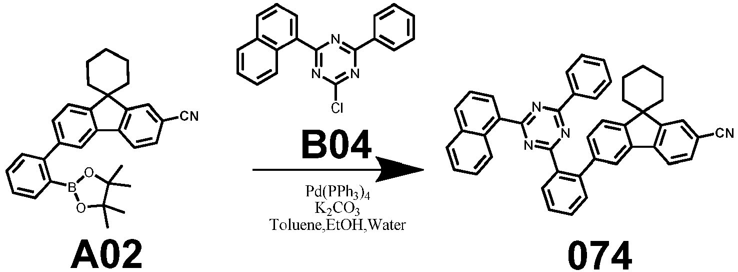

Compound A02 (15.0 g, 32.5 mmol) synthesized by the method of Preparation Example 9, B04 (10.3 g, 32.5 mmol) synthesized by the method of Preparation Example 18, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 074 (6'-(2-(4-(naphthalen-1-yl)-6-phenyl-1,3,5-triazin-2-yl)phenyl)spiro[cyclohexane-1,9'-fluorene]-2'-carbonitrile) (17.3 g, 25.0 mmol, yield 77%).

Mass: [(M+H)⁺] :618

### [Synthesis Example 15]: Synthesis of Compound 078

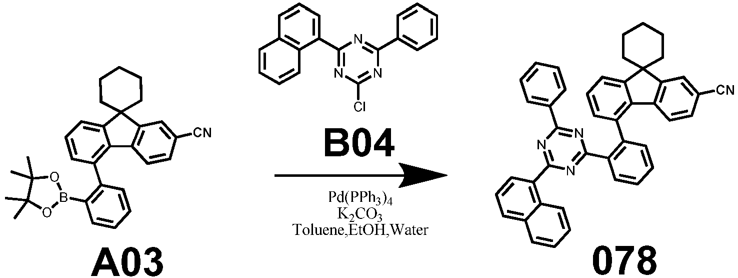

Compound A03 (15.0 g, 32.5 mmol) synthesized by the method of Preparation Example 10, Compound B04 (10.3 g, 32.5 mmol) synthesized by the method of Preparation Example 18, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 078 (5'-(2-(4-(naphthalen-1-yl)-6-phenyl-1,3,5-triazin-2-yl)phenyl)spiro[cyclohexane-1,9'-fluorene]-2'-carbonitrile) (16.7 g, 24.1 mmol, yield 74%).

Mass: [(M+H)⁺] :618

### [Synthesis Example 16]: Synthesis of Compound 086

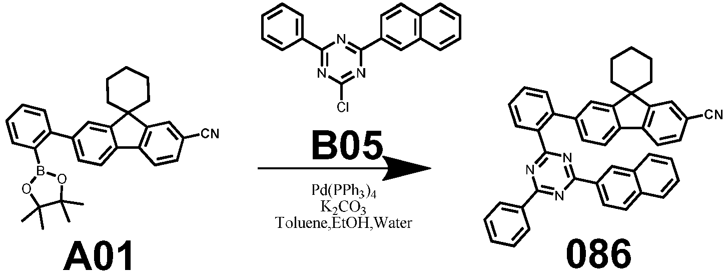

Compound A01 (15.0 g, 32.5 mmol) synthesized by the method of Preparation Example 8, Compound B05 (10.3 g, 32.5 mmol) synthesized by the method of Preparation Example 19, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 086 (7'-(2-(4-(naphthalen-2-yl)-6-phenyl-1,3,5-triazin-2-yl)phenyl)spiro[cyclohexane-1,9'-fluorene]-2'-carbonitrile) (16.2 g, 23.4 mmol, yield 72%).

Mass: [(M+H)⁺] :618

### [Synthesis Example 17]: Synthesis of Compound 090

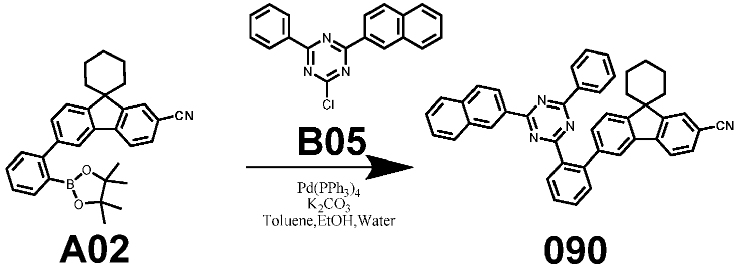

Compound A02 (15.0 g, 32.5 mmol) synthesized by the method of Preparation Example 9, Compound B05 (10.3 g, 32.5 mmol) synthesized by the method of Preparation Example 19, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 090 (6'-(2-(4-(naphthalen-2-yl)-6-phenyl-1,3,5-triazin-2-yl)phenyl)spiro[cyclohexane-1,9'-fluorene]-2'-carbonitrile) (16.2 g, 23.4 mmol, yield 72%).

Mass: [(M+H)⁺] :618

### [Synthesis Example 18]: Synthesis of Compound 094

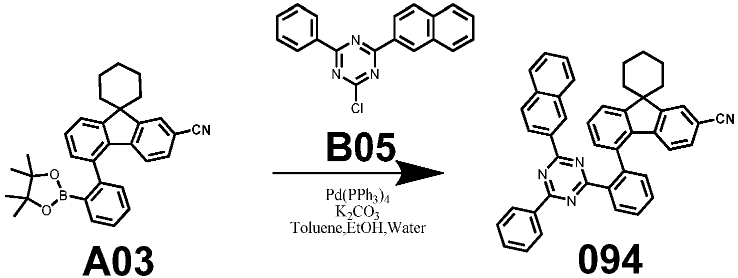

Compound A03 (15.0 g, 32.5 mmol) synthesized by the method of Preparation Example 10, B05 (10.3 g, 32.5 mmol) synthesized by the method of Preparation Example 19, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 094 (5'-(2-(4-(naphthalen-2-yl)-6-phenyl-1,3,5-triazin-2-yl)phenyl)spiro[cyclohexane-1,9'-fluorene]-2'-carbonitrile) (16.2 g, 23.4 mmol, yield 72%).

Mass: [(M+H)⁺] :618

### [Synthesis Example 19]: Synthesis of Compound 102

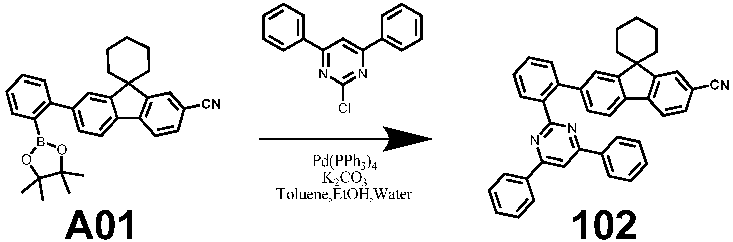

Compound A01 (15.0 g, 32.5 mmol) synthesized by the method of Preparation Example 8, 2-chloro-4,6-diphenylpyrimidine (8.7 g, 32.5 mmol), Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 102 (7'-(2-(4,6-diphenylpyrimidin-2-yl)phenyl)spiro[cyclohexane-1,9'-fluorene]-2'-carbonitrile) (16.4 g, 23.7 mmol, yield 73%).

Mass: [(M+H)⁺] :567

### [Synthesis Example 20]: Synthesis of Compound 106

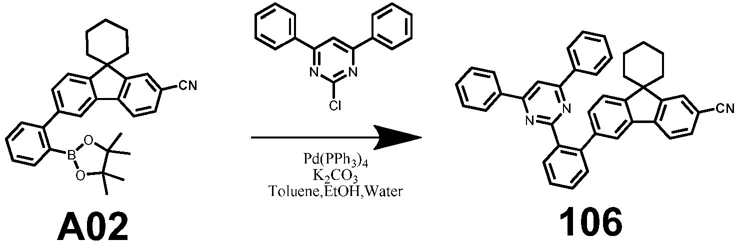

Compound A02 (15.0 g, 32.5 mmol) synthesized by the method of Preparation Example 9, 2-chloro-4,6-diphenylpyrimidine (8.7 g, 32.5 mmol), Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 106 (6'-(2-(4,6-diphenylpyrimidin-2-yl)phenyl)spiro[cyclohexane-1,9'-fluorene]-2'-carbonitrile) (16.4 g, 23.7 mmol, yield 73%).

Mass: [(M+H)⁺] :567

### [Synthesis Example 21]: Synthesis of Compound 110

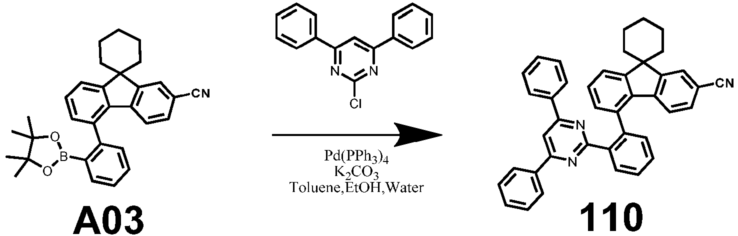

Compound A03 (15.0 g, 32.5 mmol) synthesized by the method of Preparation Example 10, 2-chloro-4,6-diphenylpyrimidine (8.7 g, 32.5 mmol), Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 110 (5'-(2-(4,6-diphenylpyrimidin-2-yl)phenyl)spiro[cyclohexane-1,9'-fluorene]-2'-carbonitrile) (16.7 g, 24.1 mmol, yield 74%).

Mass: [(M+H)⁺] :567

### [Synthesis Example 22]: Synthesis of Compound 118

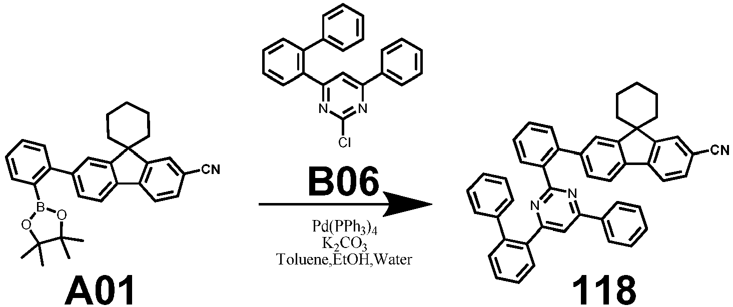

Compound A01 (15.0 g, 32.5 mmol) synthesized by the method of Preparation Example 8, Compound B06 (11.1 g, 32.5 mmol) synthesized by the method of Preparation Example 20, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 118 (2'-(2-(4-([1,1'-biphenyl]-2-yl)-6-phenylpyrimidin-2-yl)phenyl)spiro[cyclohexane-1,9'-fluorene]-7'-carbonitrile) (16.2 g, 23.4 mmol, yield 72%).

Mass: [(M+H)⁺] :643

### [Synthesis Example 23]: Synthesis of Compound 122

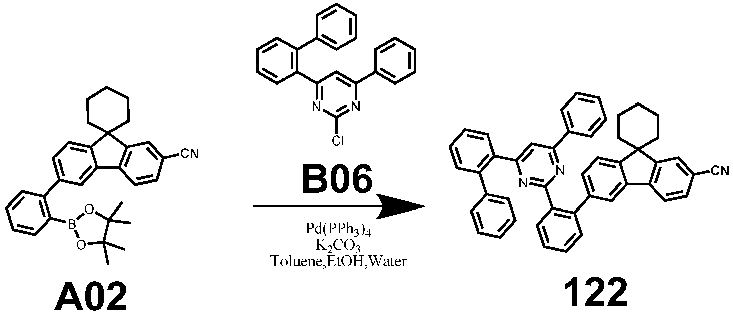

Compound A02 (15.0 g, 32.5 mmol) synthesized by the method of Preparation Example 9, Compound B06 (11.1 g, 32.5 mmol) synthesized by the method of Preparation Example 20, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 122 (6'-(2-(4-([1,1'-biphenyl]-2-yl)-6-phenylpyrimidin-2-yl)phenyl)spiro[cyclohexane-1,9'-fluorene]-2'-carbonitrile) (16.9 g, 24.4 mmol, yield 75%).

Mass: [(M+H)⁺] :643

### [Synthesis Example 24]: Synthesis of Compound 126

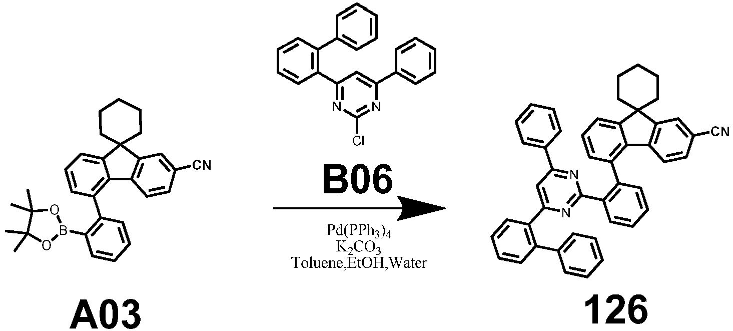

Compound A03 (15.0 g, 32.5 mmol) synthesized by the method of Preparation Example 10, Compound B06 (11.1 g, 32.5 mmol) synthesized by the method of Preparation Example 20, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 126 (5'-(2-(4-([1,1'-biphenyl]-2-yl)-6-phenylpyrimidin-2-yl)phenyl)spiro[cyclohexane-1,9'-fluorene]-2'-carbonitrile) (16.2 g, 23.4 mmol, yield 72%).

Mass: [(M+H)⁺] :643

### [Synthesis Example 25]: Synthesis of Compound 134

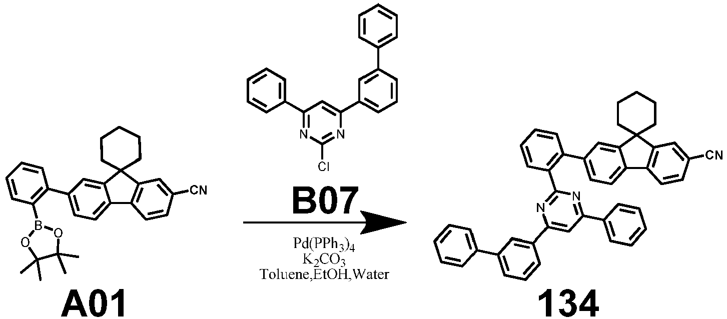

Compound A01 (15.0 g, 32.5 mmol) synthesized by the method of Preparation Example 8, Compound B07 (11.1 g, 32.5 mmol) synthesized by the method of Preparation Example 21, Pd(PPh₃)₄ (1.1 g, 1.0 mmol), and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 134 (2'-(2-(4-([1,1'-biphenyl]-3-yl)-6-phenylpyrimidin-2-yl)phenyl)spiro[cyclohexane-1,9'-fluorene]-7'-carbonitrile) (16.4 g, 23.7 mmol, yield 73%).

Mass: [(M+H)⁺] :643

### [Synthesis Example 26]: Synthesis of Compound 138

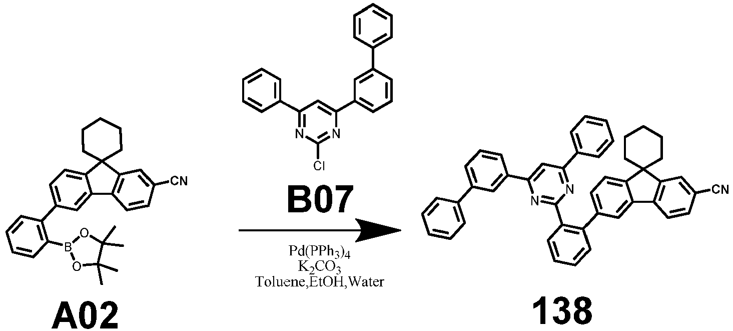

Compound A02 (15.0 g, 32.5 mmol) synthesized by the method of Preparation Example 9, Compound B07 (11.1 g, 32.5 mmol) synthesized by the method of Preparation Example 21, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 138 (6'-(2-(4-([1,1'-biphenyl]-3-yl)-6-phenylpyrimidin-2-yl)phenyl)spiro[cyclohexane-1,9'-fluorene]-2'-carbonitrile) (16.2 g, 23.4 mmol, yield 72%).

Mass: [(M+H)⁺] :643

### [Synthesis Example 27]: Synthesis of Compound 142

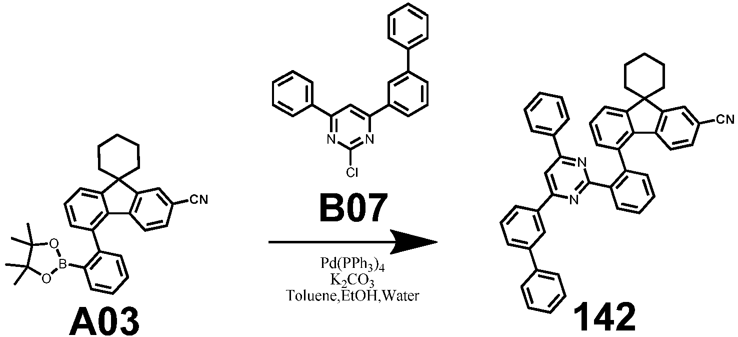

Compound A03 (15.0 g, 32.5 mmol) synthesized by the method of Preparation Example 10, B07 (11.1 g, 32.5 mmol) synthesized by the method of Preparation Example 21, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 142 (5'-(2-(4-([1,1'-biphenyl]-3-yl)-6-phenylpyrimidin-2-yl)phenyl)spiro[cyclohexane-1,9'-fluorene]-2'-carbonitrile) (16.4 g, 23.7 mmol, yield 73%).

Mass: [(M+H)⁺] :643

### [Synthesis Example 28]: Synthesis of Compound 150

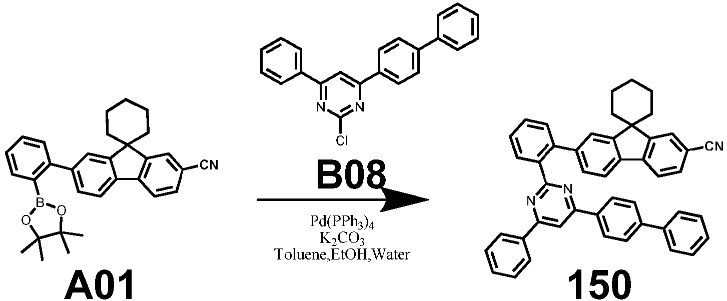

Compound A01 (15.0 g, 32.5 mmol) synthesized by the method of Preparation Example 8, Compound B08 (11.1 g, 32.5 mmol) synthesized by the method of Preparation Example 22, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 150 (2'-(2-(4-([1,1'-biphenyl]-4-yl)-6-phenylpyrimidin-2-yl)phenyl)spiro[cyclohexane-1,9'-fluorene]-7'-carbonitrile) (16.2 g, 23.4 mmol, yield 72%).

Mass: [(M+H)⁺] :643

### [Synthesis Example 29]: Synthesis of Compound 154

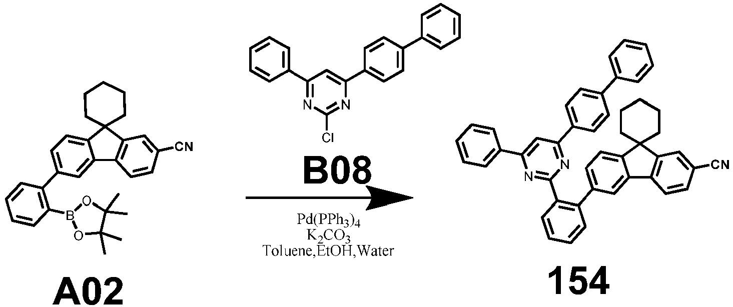

Compound A02 (15.0 g, 32.5 mmol) synthesized by the method of Preparation Example 9, Compound B08 (11.1 g, 32.5 mmol) synthesized by the method of Preparation Example 22, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 154 (6'-(2-(4-([1,1'-biphenyl]-4-yl)-6-phenylpyrimidin-2-yl)phenyl)spiro[cyclohexane-1,9'-fluorene]-2'-carbonitrile) (16.9 g, 24.4 mmol, yield 75%).

Mass: [(M+H)⁺] :643

### [Synthesis Example 30]: Synthesis of Compound 158

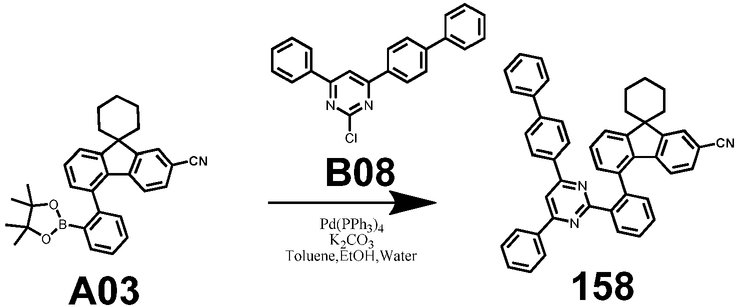

Compound A03 (15.0 g, 32.5 mmol) synthesized by the method of Preparation Example 10, Compound B08 (11.1 g, 32.5 mmol) synthesized by the method of Preparation Example 22, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 158 (5'-(2-(4-([1,1'-biphenyl]-4-yl)-6-phenylpyrimidin-2-yl)phenyl)spiro[cyclohexane-1,9'-fluorene]-2'-carbonitrile) (16.4 g, 23.7 mmol, yield 73%).

Mass: [(M+H)⁺] :643

### [Synthesis Example 31]: Synthesis of Compound 166

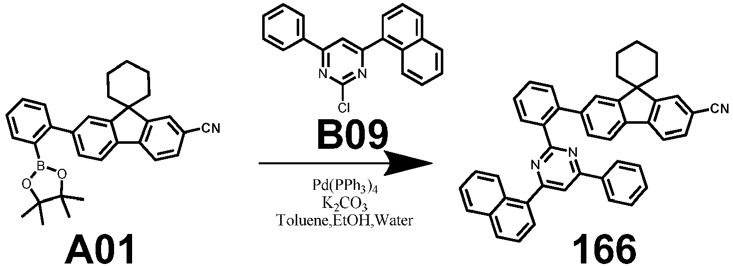

Compound A01 (15.0 g, 32.5 mmol) synthesized by the method of Preparation Example 8, Compound B09 (10.3 g, 32.5 mmol) synthesized by the method of Preparation Example 23, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 166 (7'-(2-(4-(naphthalen-1-yl)-6-phenylpyrimidin-2-yl)phenyl)spiro[cyclohexane-1,9'-fluorene]-2'-carbonitrile) (14.4 g, 23.4 mmol, yield 72%).

Mass: [(M+H)⁺] :617

### [Synthesis Example 32]: Synthesis of Compound 170

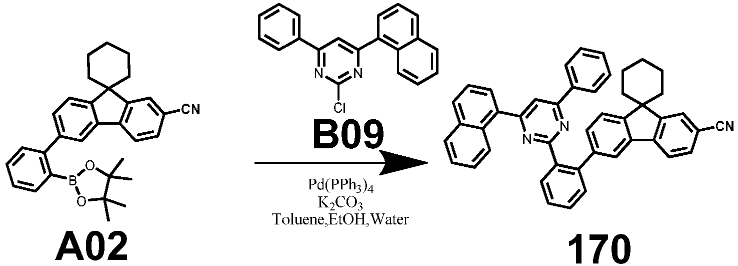

Compound A02 (15.0 g, 32.5 mmol) synthesized by the method of Preparation Example 9, Compound B09 (10.3 g, 32.5 mmol) synthesized by the method of Preparation Example 23, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 170 (6'-(2-(4-(naphthalen-1-yl)-6-phenylpyrimidin-2-yl)phenyl)spiro[cyclohexane-1,9'-fluorene]-2'-carbonitrile) (15.0 g, 24.4 mmol, yield 75%).

Mass: [(M+H)⁺] :617

### [Synthesis Example 33]: Synthesis of Compound 174

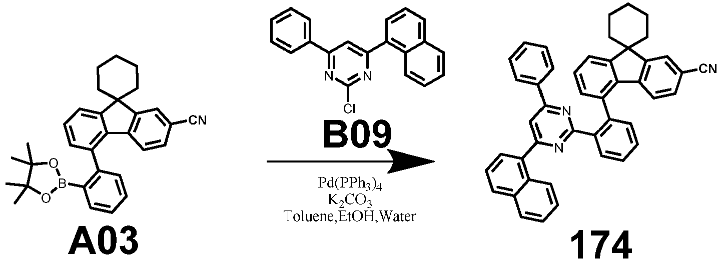

Compound A03 (15.0 g, 32.5 mmol) synthesized by the method of Preparation Example 10, Compound B09 (10.3 g, 32.5 mmol) synthesized by the method of Preparation Example 23, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 174 (5'-(2-(4-(naphthalen-1-yl)-6-phenylpyrimidin-2-yl)phenyl)spiro[cyclohexane-1,9'-fluorene]-2'-carbonitrile) (14.6 g, 23.7 mmol, yield 73%).

Mass: [(M+H)⁺] :617

### [Synthesis Example 34]: Synthesis of Compound 182

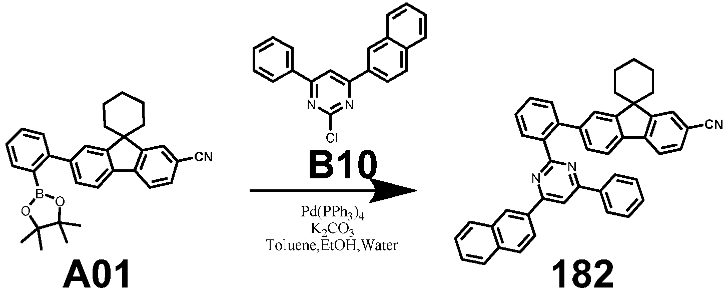

Compound A01 (15.0 g, 32.5 mmol) synthesized by the method of Preparation Example 8, Compound B10 (10.3 g, 32.5 mmol) synthesized by the method of Preparation Example 24, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 182 (7'-(2-(4-(naphthalen-2-yl)-6-phenylpyrimidin-2-yl)phenyl)spiro[cyclohexane-1,9'-fluorene]-2'-carbonitrile) (15.0 g, 24.4 mmol, yield 75%).

Mass: [(M+H)⁺] :617

### [Synthesis Example 35]: Synthesis of Compound 186

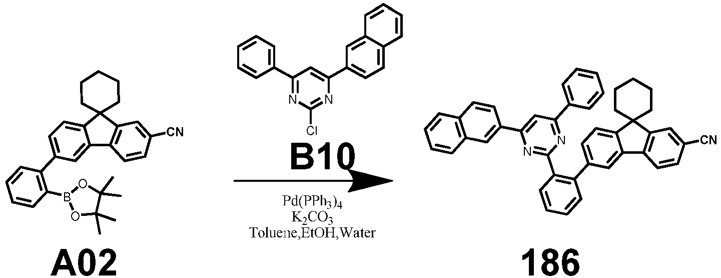

Compound A02 (15.0 g, 32.5 mmol) synthesized by the method of Preparation Example 9, Compound B10 (10.3 g, 32.5 mmol) synthesized by the method of Preparation Example 24, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 186 (6'-(2-(4-(naphthalen-2-yl)-6-phenylpyrimidin-2-yl)phenyl)spiro[cyclohexane-1,9'-fluorene]-2'-carbonitrile) (14.8 g, 24.1 mmol, yield 74%).

Mass: [(M+H)⁺] :617

### [Synthesis Example 36]: Synthesis of Compound 190

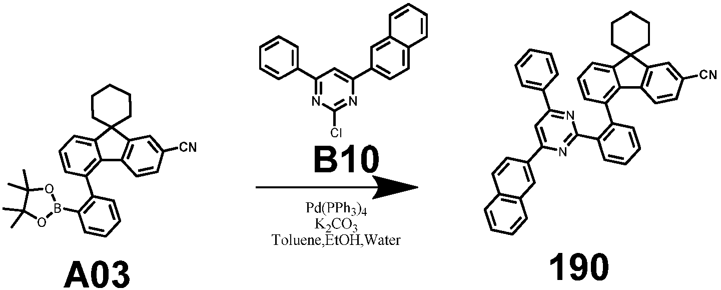

Compound A03 (15.0 g, 32.5 mmol) synthesized by the method of Preparation Example 10, Compound B10 (10.3 g, 32.5 mmol) synthesized by the method of Preparation Example 24, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 190 (5'-(2-(4-(naphthalen-2-yl)-6-phenylpyrimidin-2-yl)phenyl)spiro[cyclohexane-1,9'-fluorene]-2'-carbonitrile) (14.4 g, 23.4 mmol, yield 72%).

Mass: [(M+H)⁺] :617

### [Synthesis Example 37]: Synthesis of Compound 198

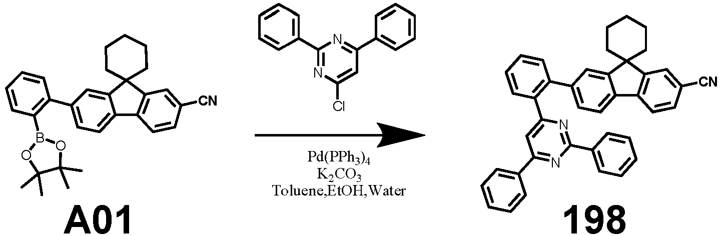

Compound A01 (15.0 g, 32.5 mmol) synthesized by the method of Preparation Example 8, 4-chloro-2,6-diphenylpyrimidine (8.7 g, 32.5 mmol), Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 198 (7'-(2-(2,6-diphenylpyrimidin-4-yl)phenyl)spiro[cyclohexane-1,9'-fluorene]-2'-carbonitrile) (13.4 g, 23.7 mmol, yield 73%).

Mass: [(M+H)⁺] :567

### [Synthesis Example 38]: Synthesis of Compound 202

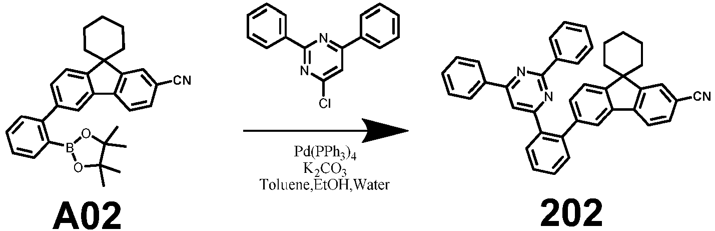

Compound A02 (15.0 g, 32.5 mmol) synthesized by the method of Preparation Example 9, 4-chloro-2,6-diphenylpyrimidine (8.7 g, 32.5 mmol), Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 202 (6'-(2-(2,6-diphenylpyrimidin-4-yl)phenyl)spiro[cyclohexane-1,9'-fluorene]-2'-carbonitrile) (13.6 g, 24.1 mmol, yield 74%).

Mass: [(M+H)⁺] :567

### [Synthesis Example 39]: Synthesis of Compound 206

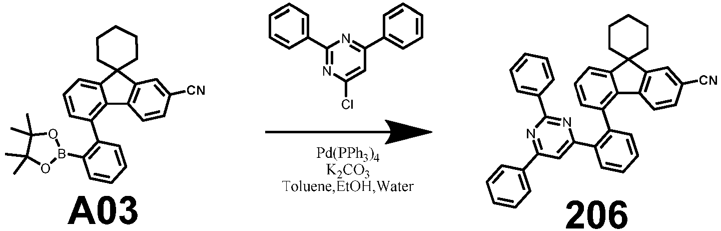

Compound A03 (15.0 g, 32.5 mmol) synthesized by the method of Preparation Example 102, 4-chloro-2,6-diphenylpyrimidine (8.7 g, 32.5 mmol) (10.3 g, 32.5 mmol), Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 206 (5'-(2-(2,6-diphenylpyrimidin-4-yl)phenyl)spiro[cyclohexane-1,9'-fluorene]-2'-carbonitrile) (13.4 g, 23.7 mmol, yield 73%).

Mass: [(M+H)⁺] :567

### [Synthesis Example 40]: Synthesis of Compound 214

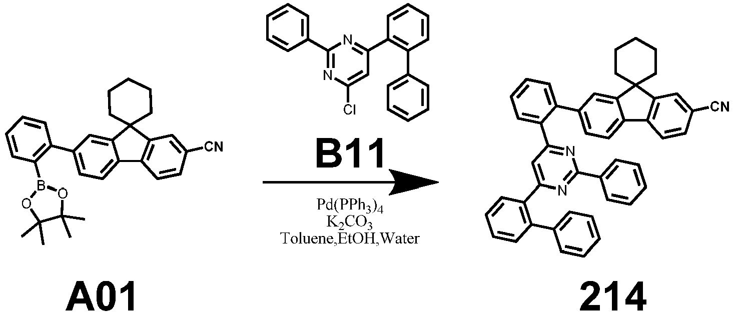

Compound A01 (15.0 g, 32.5 mmol) synthesized by the method of Preparation Example 8, Compound B11 (11.1 g, 32.5 mmol) synthesized by the method of Preparation Example 25, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 214 (2'-(2-(6-([1,1'-biphenyl]-2-yl)-2-phenylpyrimidin-4-yl)phenyl)spiro[cyclohexane-1,9'-fluorene]-7'-carbonitrile) (15.0 g, 23.4 mmol, yield 72%).

Mass: [(M+H)⁺] :643

### [Synthesis Example 41]: Synthesis of Compound 218

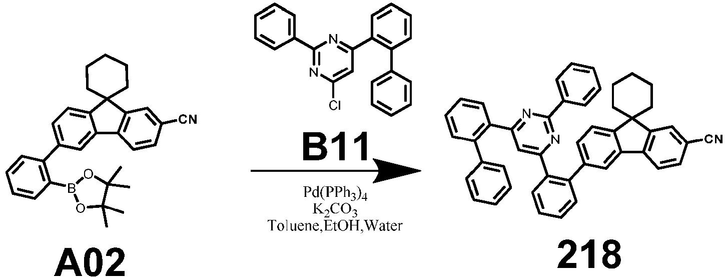

Compound A02 (15.0 g, 32.5 mmol) synthesized by the method of Preparation Example 9, Compound B11 (11.1 g, 32.5 mmol) synthesized by the method of Preparation Example 25, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 218 (6'-(2-(6-([1,1'-biphenyl]-2-yl)-2-phenylpyrimidin-4-yl)phenyl)spiro[cyclohexane-1,9'-fluorene]-2'-carbonitrile) (15.4 g, 24.1 mmol, yield 74%).

Mass: [(M+H)⁺] :643

### [Synthesis Example 42]: Synthesis of Compound 222

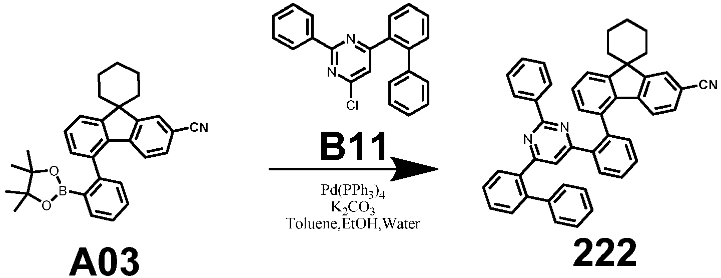

Compound A03 (15.0 g, 32.5 mmol) synthesized by the method of Preparation Example 9, Compound B11 (11.1 g, 32.5 mmol) (10.3 g, 32.5 mmol) synthesized by the method of Preparation Example 25, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 222 (5'-(2-(6-([1,1'-biphenyl]-2-yl)-2-phenylpyrimidin-4-yl)phenyl)spiro[cyclohexane-1,9'-fluorene]-2'-carbonitrile) (15.9 g, 24.7 mmol, yield 76%).

Mass: [(M+H)⁺] :643

### [Synthesis Example 43]: Synthesis of Compound 230

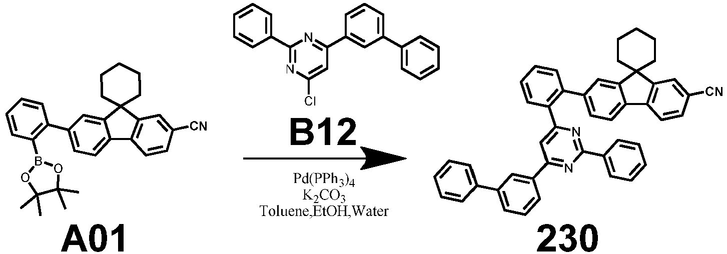

Compound A01 (15.0 g, 32.5 mmol) synthesized by the method of Preparation Example 8, Compound B12 (11.1 g, 32.5 mmol) synthesized by the method of Preparation Example 262, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 230 (2'-(2-(6-([1,1'-biphenyl]-3-yl)-2-phenylpyrimidin-4-yl)phenyl)spiro[cyclohexane-1,9'-fluorene]-7'-carbonitrile) (15.6 g, 24.4 mmol, yield 75%).

Mass: [(M+H)⁺] :643

### [Synthesis Example 44]: Synthesis of Compound 234

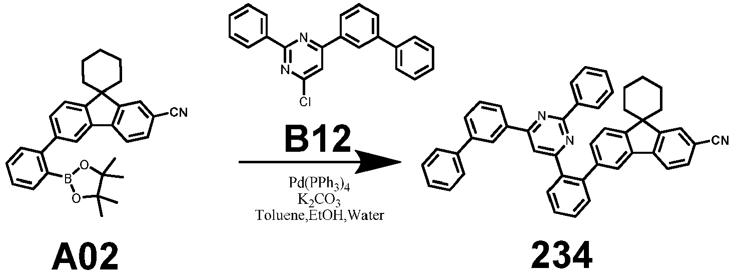

Compound A02 (15.0 g, 32.5 mmol) synthesized by the method of Preparation Example 9, Compound B12 (11.1 g, 32.5 mmol) synthesized by the method of Preparation Example 26, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 234 (6'-(2-(6-([1,1'-biphenyl]-3-yl)-2-phenylpyrimidin-4-yl)phenyl)spiro[cyclohexane-1,9'-fluorene]-2'-carbonitrile) (16.1 g, 25.0 mmol, yield 77%).

Mass: [(M+H)⁺] :643

### [Synthesis Example 45]: Synthesis of Compound 238

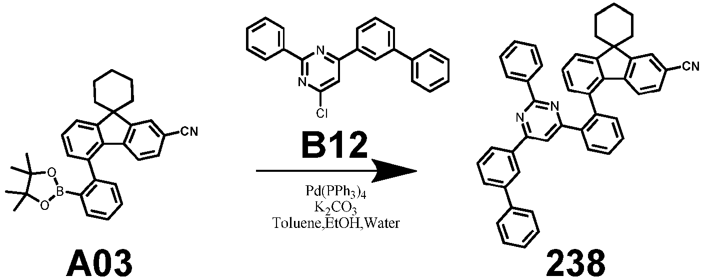

Compound A03 (15.0 g, 32.5 mmol) synthesized by the method of Preparation Example 10, Compound B12 (11.1 g, 32.5 mmol) (10.3 g, 32.5 mmol) synthesized by the method of Preparation Example 26, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 238 (5'-(2-(6-([1,1'-biphenyl]-3-yl)-2-phenylpyrimidin-4-yl)phenyl)spiro[cyclohexane-1,9'-fluorene]-2'-carbonitrile) (15.4 g, 24.1 mmol, yield 74%).

Mass: [(M+H)⁺] :643

### [Synthesis Example 46]: Synthesis of Compound 246

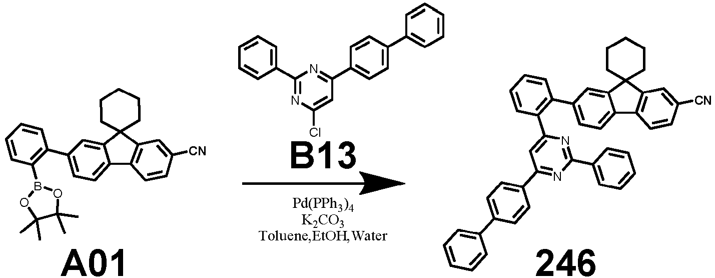

Compound A01 (15.0 g, 32.5 mmol) synthesized by the method of Preparation Example 8, Compound B13 (11.1 g, 32.5 mmol) synthesized by the method of Preparation Example 27, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 246 (2'-(2-(6-([1,1'-biphenyl]-4-yl)-2-phenylpyrimidin-4-yl)phenyl)spiro[cyclohexane-1,9'-fluorene]-7'-carbonitrile) (15.0 g, 23.4 mmol, yield 72%).

Mass: [(M+H)⁺] :643

### [Synthesis Example 47]: Synthesis of Compound 250

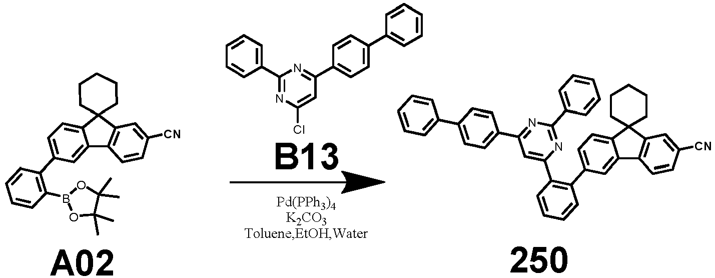

Compound A02 (15.0 g, 32.5 mmol) synthesized by the method of Preparation Example 9, Compound B11 (11.1 g, 32.5 mmol) synthesized by the method of Preparation Example 25, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 250 (6'-(2-(6-([1,1'-biphenyl]-4-yl)-2-phenylpyrimidin-4-yl)phenyl)spiro[cyclohexane-1,9'-fluorene]-2'-carbonitrile) (15.0 g, 23.4 mmol, yield 72%).

Mass: [(M+H)⁺] :643

### [Synthesis Example 48]: Synthesis of Compound 254

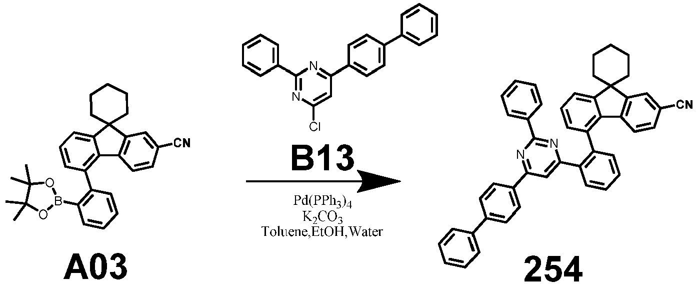

Compound A03 (15.0 g, 32.5 mmol synthesized by the method of Preparation Example 10, Compound B13 (11.1 g, 32.5 mmol) (10.3 g, 32.5 mmol) synthesized by the method of Preparation Example 27, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 254 (5'-(2-(6-([1,1'-biphenyl]-4-yl)-2-phenylpyrimidin-4-yl)phenyl)spiro[cyclohexane-1,9'-fluorene]-2'-carbonitrile) (15.0 g, 23.4 mmol, yield 72%).

Mass: [(M+H)⁺] :643

### [Synthesis Example 49]: Synthesis of Compound 262

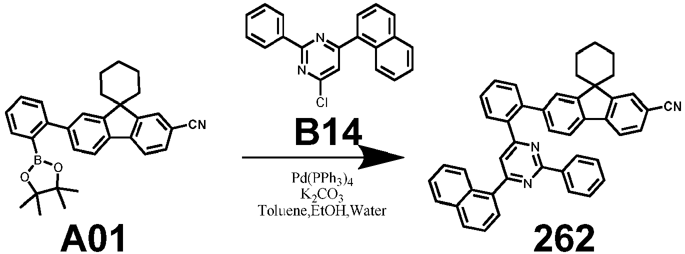

Compound A01 (15.0 g, 32.5 mmol) synthesized by the method of Preparation Example 8, Compound B14 (10.3 g, 32.5 mmol) synthesized by the method of Preparation Example 28, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 262 (7'-(2-(6-(naphthalen-1-yl)-2-phenylpyrimidin-4-yl)phenyl)spiro[cyclohexane-1,9'-fluorene]-2'-carbonitrile) (14.6 g, 23.7 mmol, yield 73%).

Mass: [(M+H)⁺]: 617

### [Synthesis Example 50]: Synthesis of Compound 266

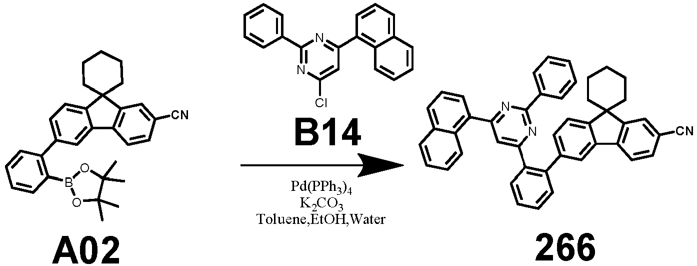

Compound A02 (15.0 g, 32.5 mmol) synthesized by the method of Preparation Example 8, Compound B14 (10.3 g, 32.5 mmol) synthesized by the method of Preparation Example 28, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 266 (6'-(2-(6-(naphthalen-1-yl)-2-phenylpyrimidin-4-yl)phenyl)spiro[cyclohexane-1,9'-fluorene]-2'-carbonitrile) (15.0 g, 24.4 mmol, yield 75%).

Mass: [(M+H)⁺] :617

### [Synthesis Example 51]: Synthesis of Compound 270

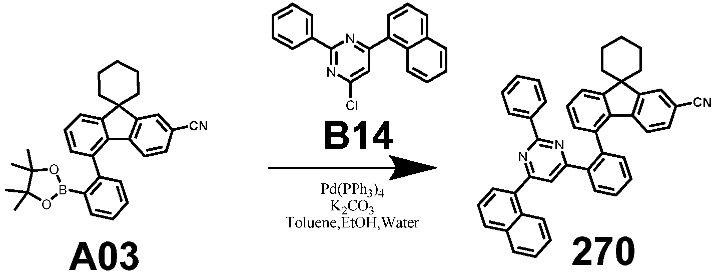

Compound A03 (15.0 g, 32.5 mmol) synthesized by the method of Preparation Example 10, Compound B14 (10.3 g, 32.5 mmol) synthesized by the method of Preparation Example 28, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 270 (5'-(2-(6-(naphthalen-1-yl)-2-phenylpyrimidin-4-yl)phenyl)spiro[cyclohexane-1,9'-fluorene]-2'-carbonitrile) (14.8 g, 24.1 mmol, yield 74%).

Mass: [(M+H)⁺] :617

### [Synthesis Example 52]: Synthesis of Compound 278

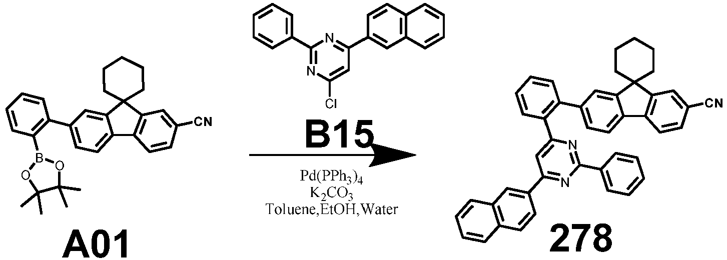

Compound A01 (15.0 g, 32.5 mmol) synthesized by the method of Preparation Example 8, Compound B15 (10.3 g, 32.5 mmol) synthesized by the method of Preparation Example 29, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 278 (7'-(2-(6-(naphthalen-2-yl)-2-phenylpyrimidin-4-yl)phenyl)spiro[cyclohexane-1,9'-fluorene]-2'-carbonitrile) (14.4 g, 23.4 mmol, yield 72%).

Mass: [(M+H)⁺] :617

### [Synthesis Example 53]: Synthesis of Compound 282

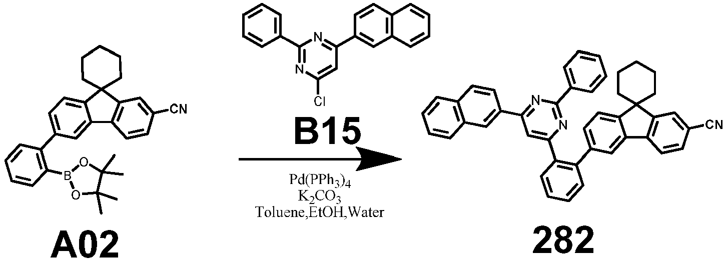

Compound A02 (15.0 g, 32.5 mmol) synthesized by the method of Preparation Example 9, Compound B15 (10.3 g, 32.5 mmol) synthesized by the method of Preparation Example 29, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 282 (6'-(2-(6-(naphthalen-2-yl)-2-phenylpyrimidin-4-yl)phenyl)spiro[cyclohexane-1,9'-fluorene]-2'-carbonitrile) (15.0 g, 24.4 mmol, yield 75%).

Mass: [(M+H)⁺] :617

### [Synthesis Example 54]: Synthesis of Compound 286

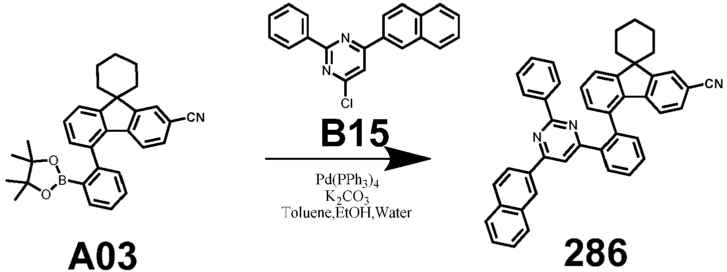

Compound A03 (15.0 g, 32.5 mmol) synthesized by the method of Preparation Example 10, Compound B15 (10.3 g, 32.5 mmol) synthesized by the method of Preparation Example 29, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 286 (5'-(2-(6-(naphthalen-1-yl)-2-phenylpyrimidin-4-yl)phenyl)spiro[cyclohexane-1,9'-fluorene]-2'-carbonitrile) (14.4 g, 23.4 mmol, yield 72%).

Mass: [(M+H)⁺] :617

### [Synthesis Example 55]: Synthesis of Compound 294

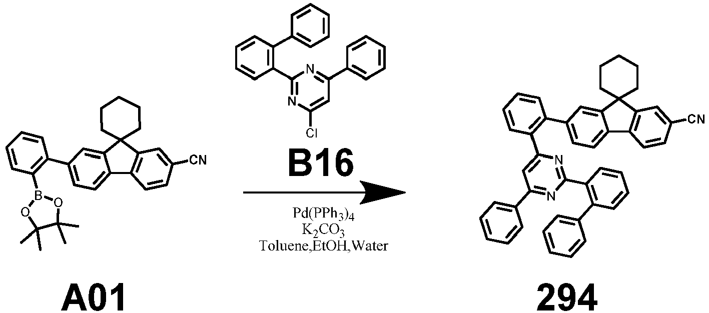

Compound A01 (15.0 g, 32.5 mmol) synthesized by the method of Preparation Example 8, Compound B16 (11.1 g, 32.5 mmol) synthesized by the method of Preparation Example 30, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 294 (2'-(2-(2-([1,1'-biphenyl]-2-yl)-6-phenylpyrimidin-4-yl)phenyl)spiro[cyclohexane-1,9'-fluorene]-7'-carbonitrile) (15.0 g, 23.4 mmol, yield 72%).

Mass: [(M+H)⁺] :643

### [Synthesis Example 56]: Synthesis of Compound 298

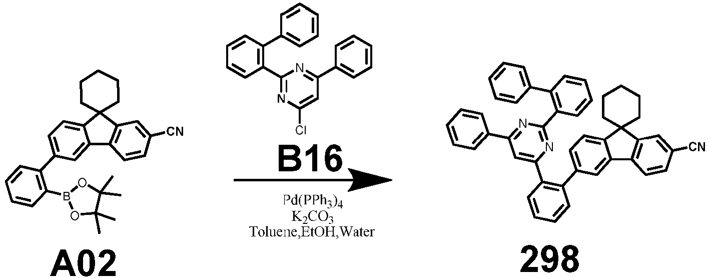

Compound A02 (15.0 g, 32.5 mmol) synthesized by the method of Preparation Example 9, Compound B16 (11.1 g, 32.5 mmol) synthesized by the method of Preparation Example 30, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 298 (6'-(2-(2-([1,1'-biphenyl]-2-yl)-6-phenylpyrimidin-4-yl)phenyl)spiro[cyclohexane-1,9'-fluorene]-2'-carbonitrile) (15.6 g, 24.4 mmol, yield 75%).

Mass: [(M+H)⁺] :643

### [Synthesis Example 57]: Synthesis of Compound 302

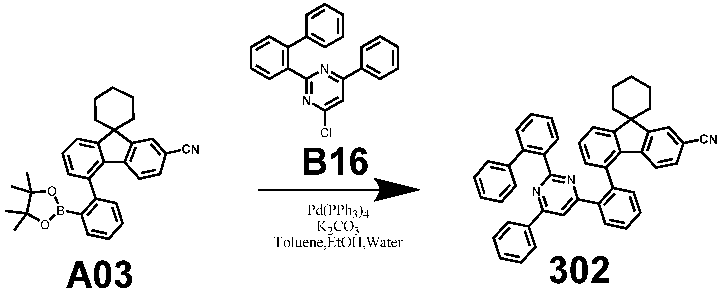

Compound A03 (15.0 g, 32.5 mmol) synthesized by the method of Preparation Example 10, Compound B16 (11.1 g, 32.5 mmol) synthesized by the method of Preparation Example 30, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 302 (5'-(2-(2-([1,1'-biphenyl]-2-yl)-6-phenylpyrimidin-4-yl)phenyl)spiro[cyclohexane-1,9'-fluorene]-2'-carbonitrile) (15.2 g, 23.7 mmol, yield 73%).

Mass: [(M+H)⁺] :643

### [Synthesis Example 58]: Synthesis of Compound 310

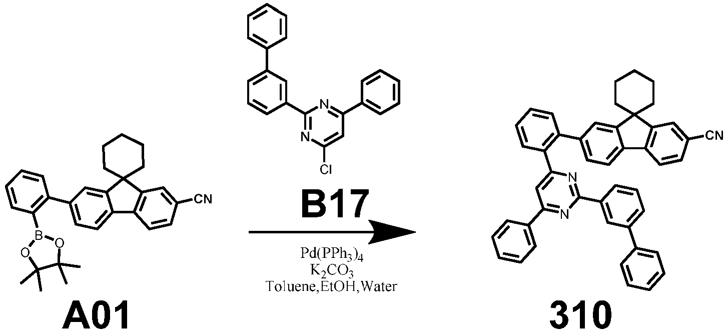

A01 (15.0 g, 32.5 mmol) synthesized by the method of Preparation Example 8, B17 (11.1 g, 32.5 mmol) synthesized by the method of Preparation Example 31, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 310 (2'-(2-(2-([1,1'-biphenyl]-3-yl)-6-phenylpyrimidin-4-yl)phenyl)spiro[cyclohexane-1,9'-fluorene]-7'-carbonitrile) (15.0 g, 23.4 mmol, yield 72%).

Mass: [(M+H)⁺] : 643

### [Synthesis Example 59]: Synthesis of Compound 314

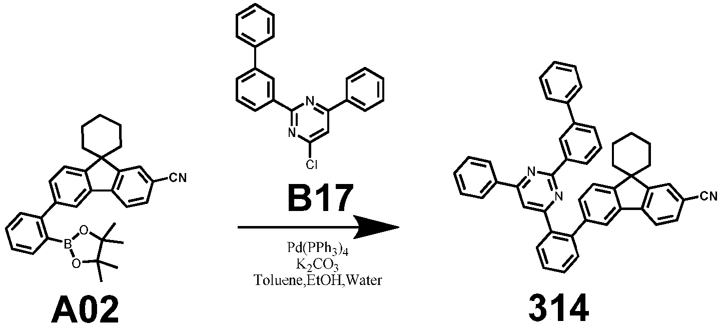

A02 (15.0 g, 32.5 mmol) synthesized by the method of Preparation Example 9, Compound B17 (11.1 g, 32.5 mmol) synthesized by the method of Preparation Example 31, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 314 (6'-(2-(2-([1,1'-biphenyl]-3-yl)-6-phenylpyrimidin-4-yl)phenyl)spiro[cyclohexane-1,9'-fluorene]-2'-carbonitrile) (15.6 g, 24.4 mmol, yield 75%).

Mass: [(M+H)⁺] : 643

### [Synthesis Example 60]: Synthesis of Compound 318

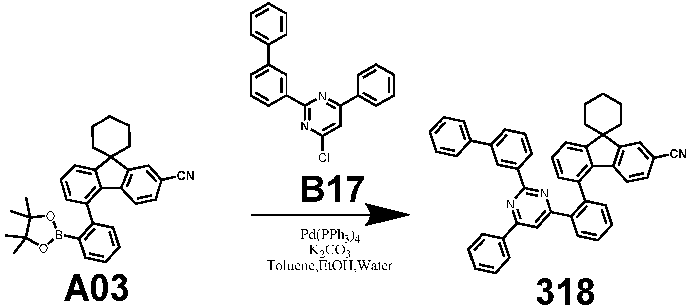

A03 (15.0 g, 32.5 mmol) synthesized by the method of Preparation Example 10, Compound B17 (11.1 g, 32.5 mmol) synthesized by the method of Preparation Example 31, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 318 (5'-(2-(2-([1,1'-biphenyl]-3-yl)-6-phenylpyrimidin-4-yl)phenyl)spiro[cyclohexane-1,9'-fluorene]-2'-carbonitrile) (15.2 g, 23.7 mmol, yield 73%).

Mass: [(M+H)⁺] : 643

### [Synthesis Example 61]: Synthesis of Compound 326

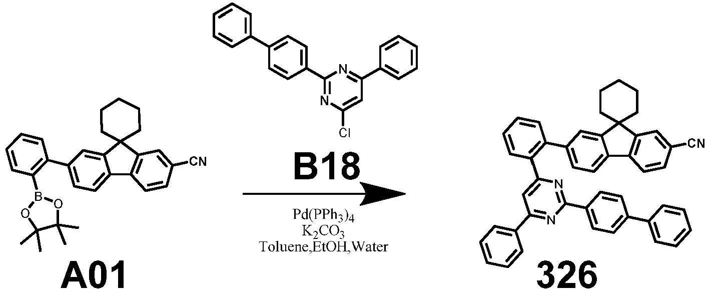

A01 (15.0 g, 32.5 mmol) synthesized by the method of Preparation Example 8, B18 (11.1 g, 32.5 mmol) synthesized by the method of Preparation Example 32, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 326 (2'-(2-(2-([1,1'-biphenyl]-4-yl)-6-phenylpyrimidin-4-yl)phenyl)spiro[cyclohexane-1,9'-fluorene]-7'-carbonitrile) (15.6 g, 24.4 mmol, yield 75%).

Mass: [(M+H)⁺] : 643

### [Synthesis Example 62]: Synthesis of Compound 330

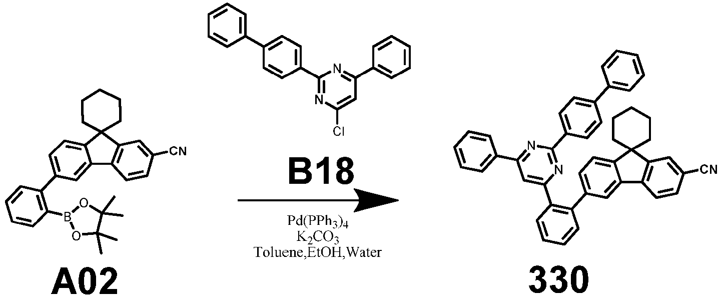

A02 (15.0 g, 32.5 mmol) synthesized by the method of Preparation Example 9, B18 (11.1 g, 32.5 mmol) synthesized by the method of Preparation Example 32, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 330 (6'-(2-(2-([1,1'-biphenyl]-4-yl)-6-phenylpyrimidin-4-yl)phenyl)spiro[cyclohexane-1,9'-fluorene]-2'-carbonitrile) (15.4 g, 24.1 mmol, yield 74%).

Mass: [(M+H)⁺] : 643

### [Synthesis Example 63]: Synthesis of Compound 334

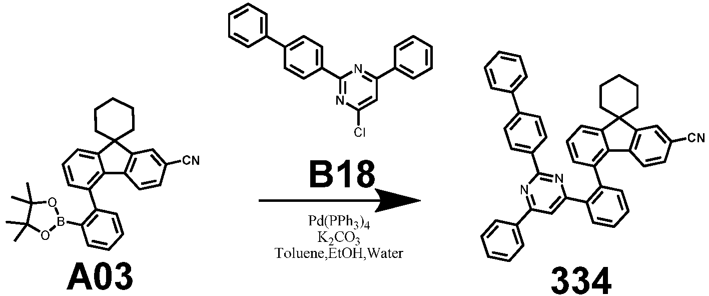

A03 (15.0 g, 32.5 mmol) synthesized by the method of Preparation Example 10, B18 (11.1 g, 32.5 mmol) synthesized by the method of Preparation Example 32, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 334 (5'-(2-(2-([1,1'-biphenyl]-4-yl)-6-phenylpyrimidin-4-yl)phenyl)spiro[cyclohexane-1,9'-fluorene]-2'-carbonitrile) (15.0 g, 23.4 mmol, yield 72%).

Mass: [(M+H)⁺] : 643

### [Synthesis Example 64]: Synthesis of Compound 342

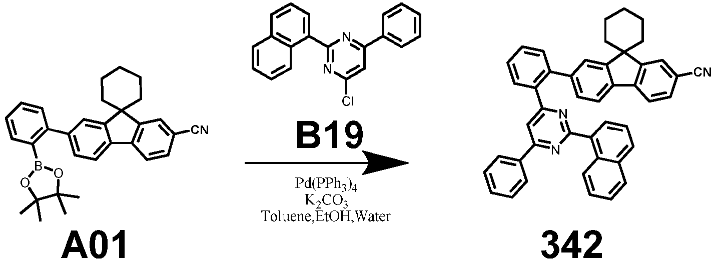

A01 (15.0 g, 32.5 mmol) synthesized by the method of Preparation Example 8, B19 (10.3 g, 32.5 mmol) synthesized by the method of Preparation Example 33, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 342 (7'-(2-(2-(naphthalen-1-yl)-6-phenylpyrimidin-4-yl)phenyl)spiro[cyclohexane-1,9'-fluorene]-2'-carbonitrile) (14.6 g, 23.7 mmol, yield 73%).

Mass: [(M+H)⁺] : 617

### [Synthesis Example 65]: Synthesis of Compound 346

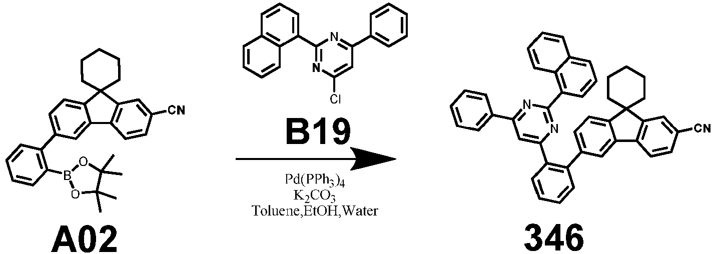

A02 (15.0 g, 32.5 mmol) synthesized by the method of Preparation Example 9, B19 (10.3 g, 32.5 mmol) synthesized by the method of Preparation Example 33, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 346 (6'-(2-(2-(naphthalen-1-yl)-6-phenylpyrimidin-4-yl)phenyl)spiro[cyclohexane-1,9'-fluorene]-2'-carbonitrile) (14.6 g, 23.7 mmol, yield 73%).

Mass: [(M+H)⁺] : 617

### [Synthesis Example 66]: Synthesis of Compound 350

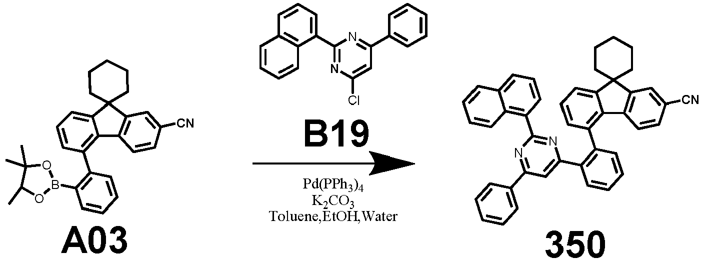

A03 (15.0 g, 32.5 mmol) synthesized by the method of Preparation Example 10, B19 (10.3 g, 32.5 mmol) synthesized by the method of Preparation Example 33, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 350 (5'-(2-(2-(naphthalen-1-yl)-6-phenylpyrimidin-4-yl)phenyl)spiro[cyclohexane-1,9'-fluorene]-2'-carbonitrile) (14.6 g, 23.7 mmol, yield 73%).

Mass: [(M+H)⁺] : 617

### [Synthesis Example 67]: Synthesis of Compound 358

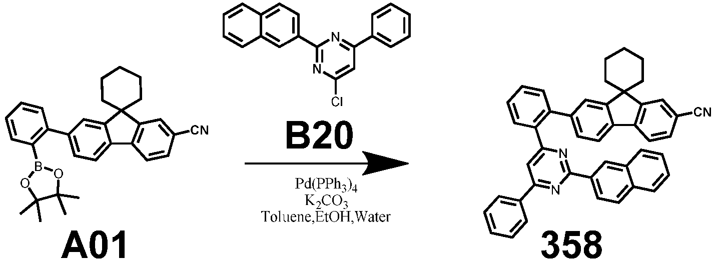

Compound A01 (15.0 g, 32.5 mmol) synthesized by the method of Preparation Example 8, B20 (10.3 g, 32.5 mmol) synthesized by the method of Preparation Example 34, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 358 (7'-(2-(2-(naphthalen-2-yl)-6-phenylpyrimidin-4-yl)phenyl)spiro[cyclohexane-1,9'-fluorene]-2'-carbonitrile) (14.4 g, 23.4 mmol, yield 72%).

Mass: [(M+H)⁺] : 617

### [Synthesis Example 68]: Synthesis of Compound 362

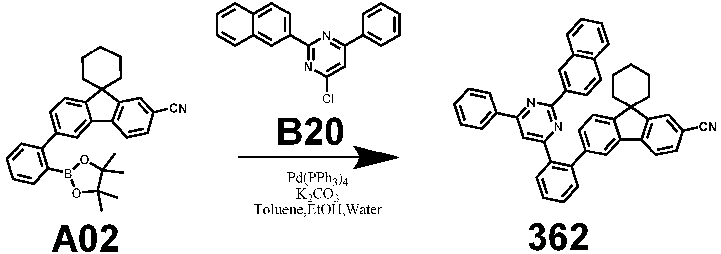

A02 (15.0 g, 32.5 mmol) synthesized by the method of Preparation Example 9, B20 (10.3 g, 32.5 mmol) synthesized by the method of Preparation Example 34, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 362 (6'-(2-(2-(naphthalen-2-yl)-6-phenylpyrimidin-4-yl)phenyl)spiro[cyclohexane-1,9'-fluorene]-2'-carbonitrile) (14.8 g, 24.1 mmol, yield 74%).

Mass: [(M+H)⁺] : 617

### [Synthesis Example 69]: Synthesis of Compound 366

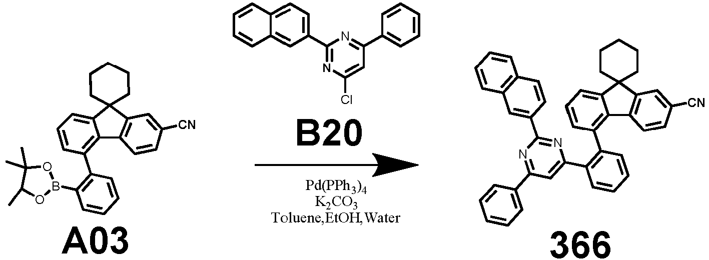

A03 (15.0 g, 32.5 mmol) synthesized by the method of Preparation Example 10, B20 (10.3 g, 32.5 mmol) synthesized by the method of Preparation Example 34, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 366 (5'-(2-(2-(naphthalen-2-yl)-6-phenylpyrimidin-4-yl)phenyl)spiro[cyclohexane-1,9'-fluorene]-2'-carbonitrile) (15.2 g, 24.7 mmol, yield 76%).

Mass: [(M+H)⁺] : 617

### [Synthesis Example 70]: Synthesis of Compound 374

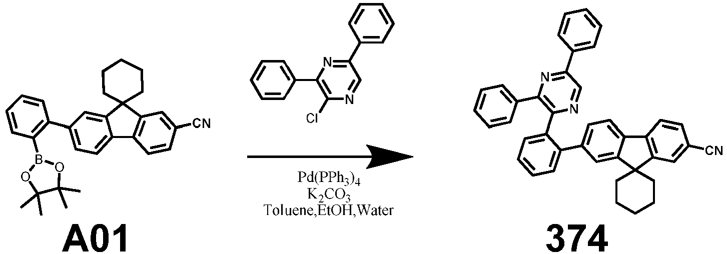

Compound A01 (15.0 g, 32.5 mmol) synthesized by the method of Preparation Example 8, 2-chloro-3,5-diphenylpyrazine (8.7 g, 32.5 mmol), Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 374 (7'-(2-(3,5-diphenylpyrazin-2-yl)phenyl)spiro[cyclohexane-1,9'-fluorene]-2'-carbonitrile) (13.4 g, 23.7 mmol, yield 73%).

Mass: [(M+H)⁺] :567

### [Synthesis Example 71]: Synthesis of Compound 378

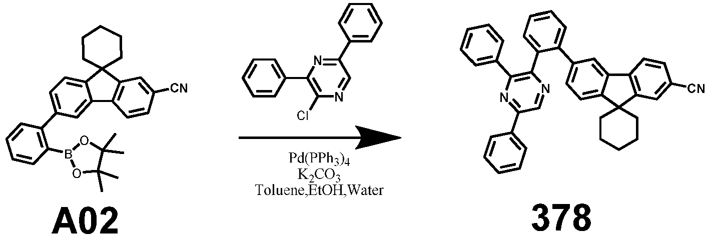

Compound A02 (15.0 g, 32.5 mmol) synthesized by the method of Preparation Example 9, 2-chloro-3,5-diphenylpyrazine (8.7 g, 32.5 mmol), Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 378 (6'-(2-(3,5-diphenylpyrazin-2-yl)phenyl)spiro[cyclohexane-1,9'-fluorene]-2'-carbonitrile) (13.2 g, 23.4 mmol, yield 72%).

Mass: [(M+H)⁺] :567

### [Synthesis Example 72]: Synthesis of Compound 382

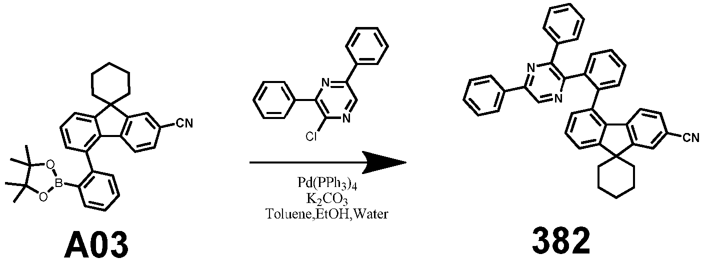

Compound A03 (15.0 g, 32.5 mmol) synthesized by the method of Preparation Example 10, 2-chloro-3,5-diphenylpyrazine (8.7 g, 32.5 mmol), Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 382 (5'-(2-(3,5-diphenylpyrazin-2-yl)phenyl)spiro[cyclohexane-1,9'-fluorene]-2'-carbonitrile) (13.4 g, 23.7 mmol, yield 73%).

Mass: [(M+H)⁺] :567

### [Synthesis Example 73]: Synthesis of Compound 390

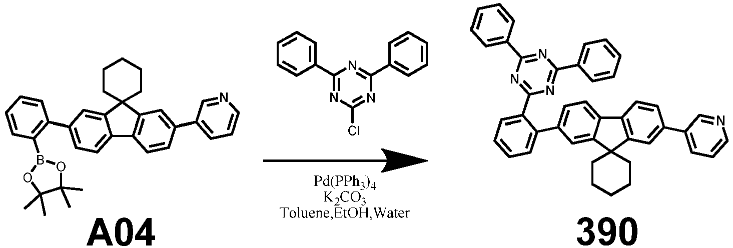

A04 (15.0 g, 29.2 mmol) synthesized by the method of Preparation Example 11, 2-chloro-4,6-diphenyl-1,3,5-triazine (7.8 g, 29.2 mmol), Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 390 (2,4-diphenyl-6-(2-(2'-(pyridin-3-yl)spiro[cyclohexane-1,9'-fluoren]-7'-yl)phenyl)-1,3,5-triazine) (13.6 g, 21.9 mmol, yield 75%).

Mass: [(M+H)⁺] : 620

### [Synthesis Example 74]: Synthesis of Compound 394

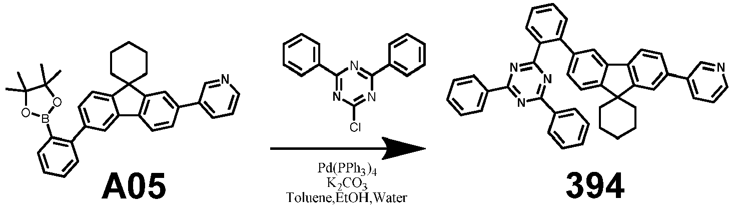

A05 (15.0 g, 29.2 mmol) synthesized by the method of Preparation Example 12, 2-chloro-4,6-diphenyl-1,3,5-triazine (7.8 g, 29.2 mmol), Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 394 (2,4-diphenyl-6-(2-(2'-(pyridin-3-yl)spiro[cyclohexane-1,9'-fluoren]-6'-yl)phenyl)-1,3,5-triazine) (13.9 g, 22.5 mmol, yield 77%).

Mass: [(M+H)⁺] : 620

### [Synthesis Example 75]: Synthesis of Compound 398

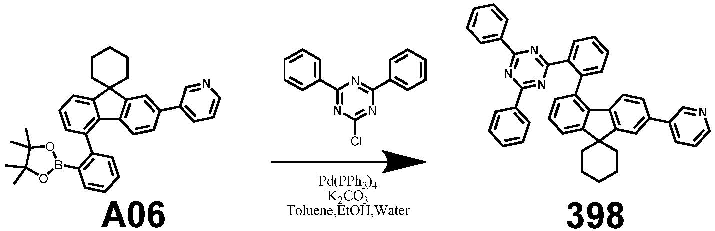

A06 (15.0 g, 29.2 mmol) synthesized by the method of Preparation Example 13, 2-chloro-4,6-diphenyl-1,3,5-triazine (7.8 g, 29.2 mmol), Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 398 (2,4-diphenyl-6-(2-(2'-(pyridin-3-yl)spiro[cyclohexane-1,9'-fluoren]-5'-yl)phenyl)-1,3,5-triazine) (13.4 g, 21.6 mmol, yield 74%).

Mass: [(M+H)⁺] : 620

### [Synthesis Example 76]: Synthesis of Compound 406

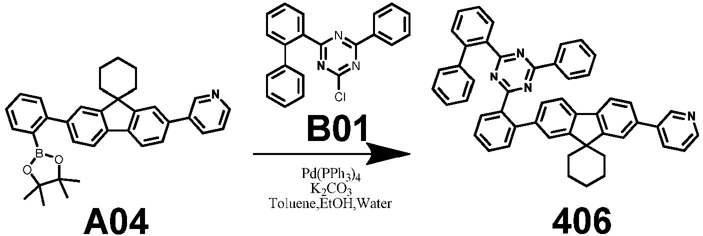

A04 (15.0 g, 29.2 mmol) synthesized by the method of Preparation Example 11, B01 (10.0 g, 29.2 mmol) synthesized by the method of Preparation Example 15, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 406 (2-([1,1'-biphenyl]-2-yl)-4-phenyl-6-(2-(2'-(pyridin-3-yl)spiro[cyclohexane-1,9'-fluoren]-7'-yl)phenyl)-1,3,5-triazine) (14.6 g, 21.0 mmol, yield 72%).

Mass: [(M+H)⁺] : 696

### [Synthesis Example 77]: Synthesis of Compound 410

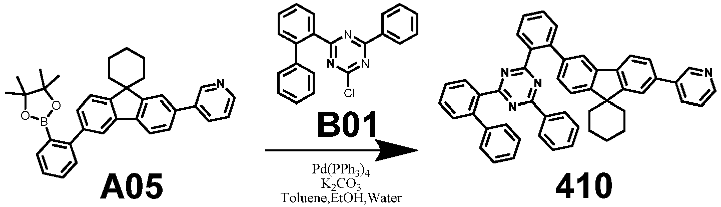

Compound A05 (15.0 g, 29.2 mmol) synthesized by the method of Preparation Example 12, Compound B01 (10.0 g, 29.2 mmol) synthesized by the method of Preparation Example 15, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 410 (2-([1,1'-biphenyl]-2-yl)-4-phenyl-6-(2-(2'-(pyridin-3-yl)spiro[cyclohexane-1,9'-fluoren]-6'-yl)phenyl)-1,3,5-triazine) (14.6 g, 21.0 mmol, yield 72%).

Mass: [(M+H)⁺] : 696

### [Synthesis Example 78]: Synthesis of Compound 414

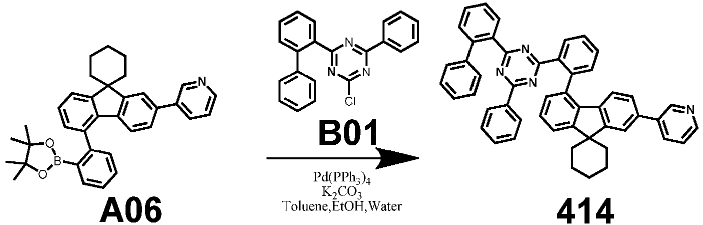

Compound A06 (15.0 g, 29.2 mmol) synthesized by the method of Preparation Example 13, Compound B01 (10.0 g, 29.2 mmol) synthesized by the method of Preparation Example 15, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 414 (2-([1,1'-biphenyl]-2-yl)-4-phenyl-6-(2-(2'-(pyridin-3-yl)spiro[cyclohexane-1,9'-fluoren]-5'-yl)phenyl)-1,3,5-triazine) (14.6 g, 21.0 mmol, yield 72%).

Mass: [(M+H)⁺] : 696

### [Synthesis Example 79]: Synthesis of Compound 422

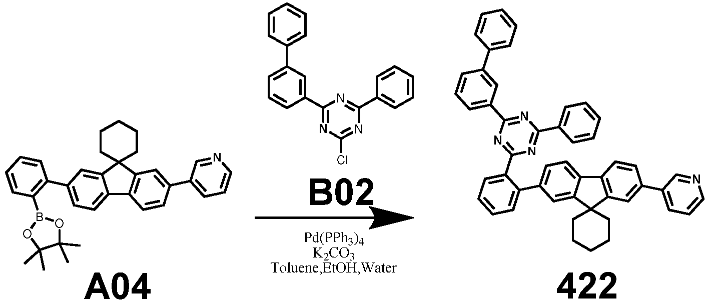

Compound A04 (15.0 g, 29.2 mmol) synthesized by the method of Preparation Example 11, Compound B02 (10.0 g, 29.2 mmol) synthesized by the method of Preparation Example 16, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 422 (2-([1,1'-biphenyl]-3-yl)-4-phenyl-6-(2-(2'-(pyridin-3-yl)spiro[cyclohexane-1,9'-fluoren]-7'-yl)phenyl)-1,3,5-triazine) (14.8 g, 21.3 mmol, yield 73%).

Mass: [(M+H)⁺] : 696

### [Synthesis Example 80]: Synthesis of Compound 426

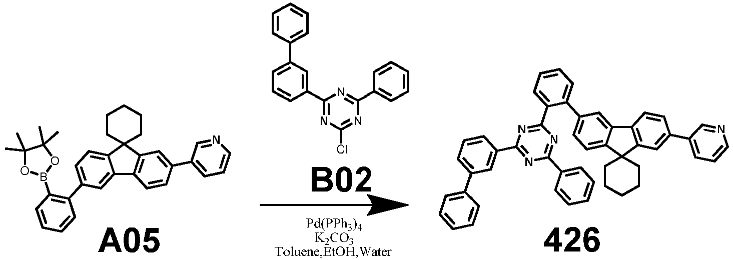

Compound A05 (15.0 g, 29.2 mmol) synthesized by the method of Preparation Example 12, Compound B02 (10.0 g, 29.2 mmol) synthesized by the method of Preparation Example 16, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 426 (2-([1,1'-biphenyl]-3-yl)-4-phenyl-6-(2-(2'-(pyridin-3-yl)spiro[cyclohexane-1,9'-fluoren]-6'-yl)phenyl)-1,3,5-triazine) (15.2 g, 21.9 mmol, yield 75%).

Mass: [(M+H)⁺] : 696

### [Synthesis Example 81]: Synthesis of Compound 430

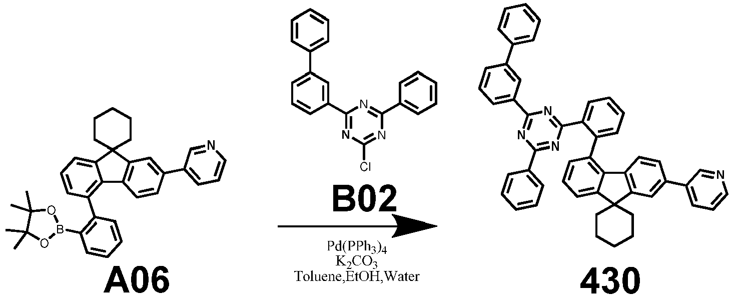

Compound A06 (15.0 g, 29.2 mmol) synthesized by the method of Preparation Example 13, Compound B02 (10.0 g, 29.2 mmol) synthesized by the method of Preparation Example 16, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 430 (2-([1,1'-biphenyl]-3-yl)-4-phenyl-6-(2-(2'-(pyridin-3-yl)spiro[cyclohexane-1,9'-fluoren]-5'-yl)phenyl)-1,3,5-triazine) (15.0 g, 21.6 mmol, yield 74%).

Mass: [(M+H)⁺] : 696

### [Synthesis Example 82]: Synthesis of Compound 438

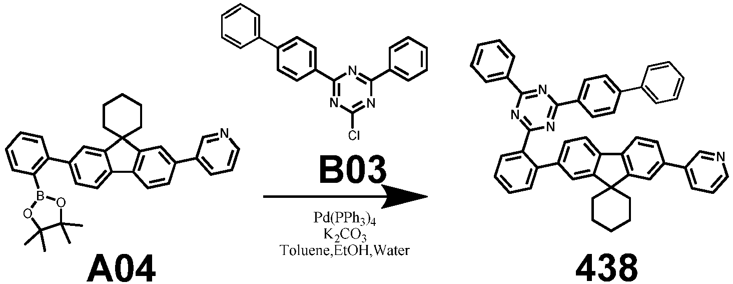

Compound A04 (15.0 g, 29.2 mmol) synthesized by the method of Preparation Example 11, Compound B03 (10.0 g, 29.2 mmol) synthesized by the method of Preparation Example 17, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 438 (2-([1,1'-biphenyl]-4-yl)-4-phenyl-6-(2-(2'-(pyridin-3-yl)spiro[cyclohexane-1,9'-fluoren]-7'-yl)phenyl)-1,3,5-triazine) (14.6 g, 21.0 mmol, yield 72%).

Mass: [(M+H)⁺] : 696

### [Synthesis Example 83]: Synthesis of Compound 442

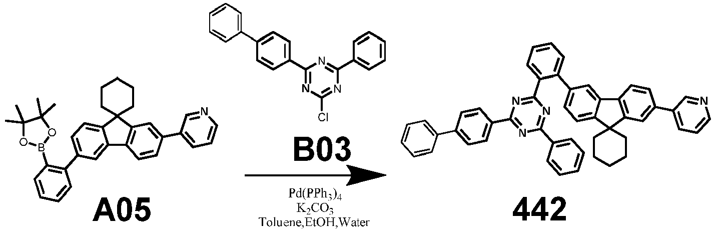

Compound A05 (15.0 g, 29.2 mmol) synthesized by the method of Preparation Example 12, Compound B03 (10.0 g, 29.2 mmol) synthesized by the method of Preparation Example 17, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 442 (2-([1,1'-biphenyl]-4-yl)-4-phenyl-6-(2-(2'-(pyridin-3-yl)spiro[cyclohexane-1,9'-fluoren]-6'-yl)phenyl)-1,3,5-triazine) (15.2 g, 21.9 mmol, yield 75%).

Mass: [(M+H)⁺] : 696

### [Synthesis Example 84]: Synthesis of Compound 446

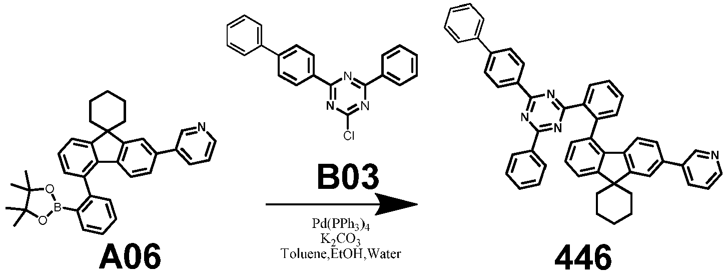

Compound A06 (15.0 g, 29.2 mmol) synthesized by the method of Preparation Example 13, Compound B03 (10.0 g, 29.2 mmol) synthesized by the method of Preparation Example 17, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 446 (2-([1,1'-biphenyl]-4-yl)-4-phenyl-6-(2-(2'-(pyridin-3-yl)spiro[cyclohexane-1,9'-fluoren]-5'-yl)phenyl)-1,3,5-triazine) (14.6 g, 21.0 mmol, yield 72%).

Mass: [(M+H)⁺] : 696

### [Synthesis Example 85]: Synthesis of Compound 454

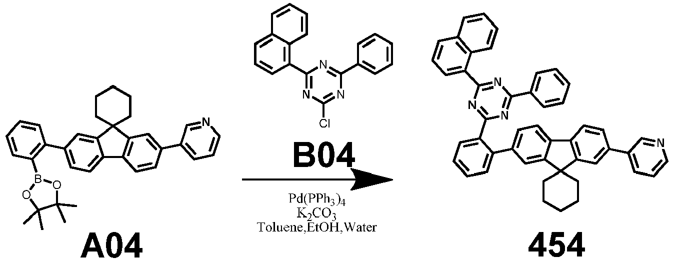

Compound A04 (15.0 g, 29.2 mmol) synthesized by the method of Preparation Example 11, Compound B04 (9.3 g, 29.2 mmol) synthesized by the method of Preparation Example 18, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 454 (2-(naphthalen-1-yl)-4-phenyl-6-(2-(2'-(pyridin-3-yl)spiro[cyclohexane-1,9'-fluoren]-7'-yl)phenyl)-1,3,5-triazine) (14.3 g, 21.3 mmol, yield 73%).

Mass: [(M+H)⁺] : 670

### [Synthesis Example 86]: Synthesis of Compound 458

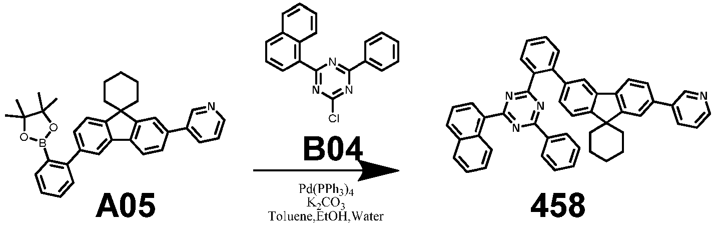

Compound A05 (15.0 g, 29.2 mmol) synthesized by the method of Preparation Example 12, Compound B04 (9.3 g, 29.2 mmol) synthesized by the method of Preparation Example 18, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 458 (2-(naphthalen-1-yl)-4-phenyl-6-(2-(2'-(pyridin-3-yl)spiro[cyclohexane-1,9'-fluoren]-6'-yl)phenyl)-1,3,5-triazine) (14.1 g, 21.0 mmol, yield 72%).

Mass: [(M+H)⁺] : 670

### [Synthesis Example 87]: Synthesis of Compound 462

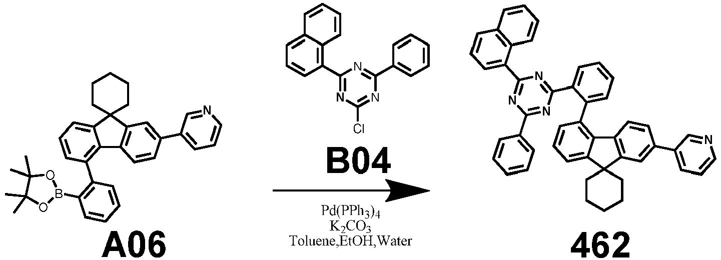

Compound A06 (15.0 g, 29.2 mmol) synthesized by the method of Preparation Example 13, Compound B04 (9.3 g, 29.2 mmol) synthesized by the method of Preparation Example 18, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 462 (2-(naphthalen-1-yl)-4-phenyl-6-(2-(2'-(pyridin-3-yl)spiro[cyclohexane-1,9'-fluoren]-5'-yl)phenyl)-1,3,5-triazine) (14.3 g, 21.3 mmol, yield 73%).

Mass: [(M+H)⁺] : 670

### [Synthesis Example 88]: Synthesis of Compound 470

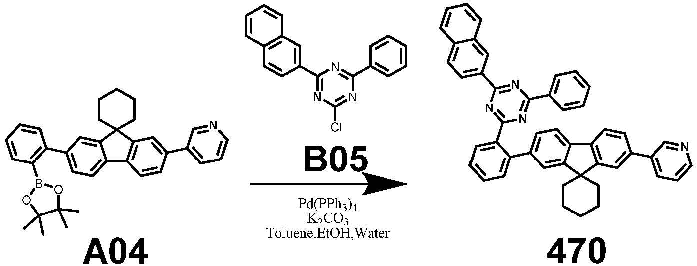

Compound A04 (15.0 g, 29.2 mmol) synthesized by the method of Preparation Example 11, Compound B05 (9.3 g, 29.2 mmol) synthesized by the method of Preparation Example 19, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 470 (2-(naphthalen-2-yl)-4-phenyl-6-(2-(2'-(pyridin-3-yl)spiro[cyclohexane-1,9'-fluoren]-7'-yl)phenyl)-1,3,5-triazine) (14.1 g, 21.0 mmol, yield 72%).

Mass: [(M+H)⁺] : 670

### [Synthesis Example 89]: Synthesis of Compound 474

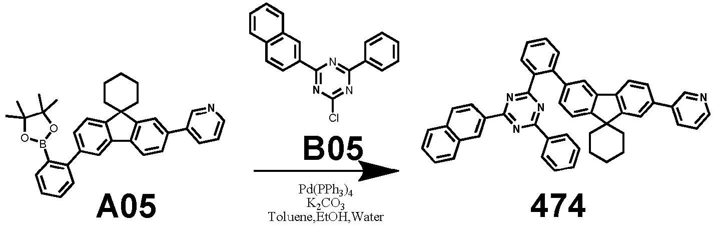

Compound A05 (15.0 g, 29.2 mmol) synthesized by the method of Preparation Example 12, Compound B05 (9.3 g, 29.2 mmol) synthesized by the method of Preparation Example 19, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 474 (2-(naphthalen-2-yl)-4-phenyl-6-(2-(2'-(pyridin-3-yl)spiro[cyclohexane-1,9'-fluoren]-6'-yl)phenyl)-1,3,5-triazine) (14.7 g, 21.9 mmol, yield 75%).

Mass: [(M+H)⁺] : 670

### [Synthesis Example 90]: Synthesis of Compound 478

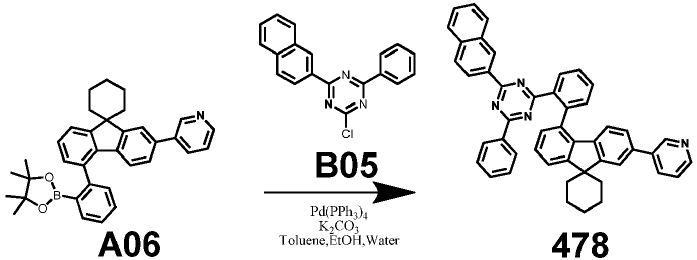

Compound A06 (15.0 g, 29.2 mmol) synthesized by the method of Preparation Example 13, Compound B05 (9.3 g, 29.2 mmol) synthesized by the method of Preparation Example 19, Pd(PPh₃)₄ (1.0 g, 0.9 mmol) and K₂CO₃ (12.1 g, 87.6 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 478 (2-(naphthalen-2-yl)-4-phenyl-6-(2-(2'-(pyridin-3-yl)spiro[cyclohexane-1,9'-fluoren]-5'-yl)phenyl)-1,3,5-triazine) (14.3 g, 21.3 mmol, yield 73%).

Mass: [(M+H)⁺] : 670

### [Synthesis Example 91]: Synthesis of Compound 486

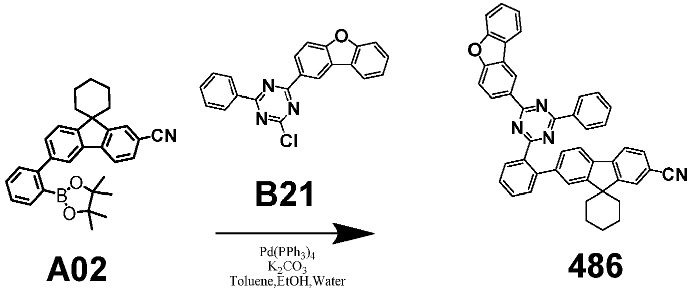

Compound A02 (15.0 g, 32.5 mmol) synthesized by the method of Preparation Example 9, Compound B21 (11.6 g, 32.5 mmol) synthesized by the method of Preparation Example 35, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 486 (2-(naphthalen-2-yl)-4-phenyl-6-(2-(2'-(pyridin-3-yl)spiro[cyclohexane-1,9'-fluoren]-5'-yl)phenyl)-1,3,5-triazine) (16.0 g, 24.4 mmol, yield 75%).

Mass: [(M+H)⁺] : 658

### [Synthesis Example 92]: Synthesis of Compound 502

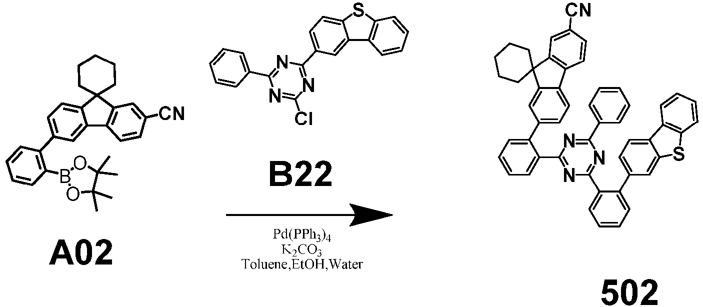

Compound A02 (15.0 g, 32.5 mmol) synthesized by the method of Preparation Example 9, Compound B22 (12.2 g, 32.5 mmol) synthesized by the method of Preparation Example 36, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 502 (7'-(2-(4-(2-(dibenzo[b,d]thiophen-3-yl)phenyl)-6-phenyl-1,3,5-triazin-2-yl)phenyl)spiro[cyclohexane-1,9'-fluorene]-2'-carbonitrile) (18.3 g, 24.4 mmol, yield 75%).

Mass: [(M+H)⁺] : 750

### [Synthesis Example 93]: Synthesis of Compound 518

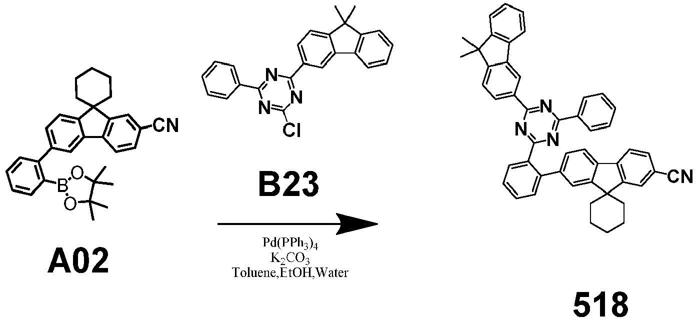

Compound A02 (15.0 g, 32.5 mmol) synthesized by the method of Preparation Example 9, Compound B23 (12.2 g, 32.5 mmol) synthesized by the method of Preparation Example 37, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 518 (7'-(2-(4-(9,9-dimethyl-9H-fluoren-3-yl)-6-phenyl-1,3,5-triazin-2-yl)phenyl) spiro[cyclohexane-1,9'-fluorene]-2'-carbonitrile) (16.6 g, 24.4 mmol, yield 75%).

Mass: [(M+H)⁺] : 684

### [Synthesis Example 94]: Synthesis of Compound 534

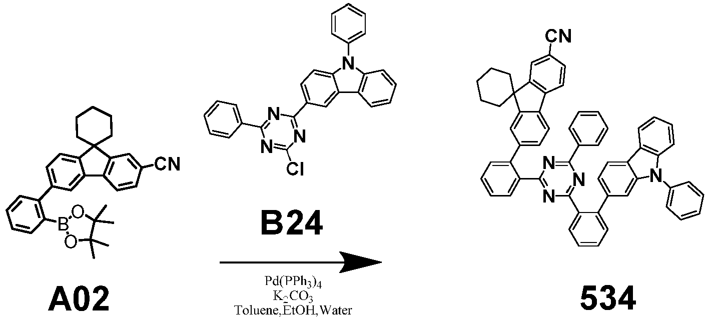

Compound A02 (15.0 g, 32.5 mmol) synthesized by the method of Preparation Example 9, Compound B24 (14.1 g, 32.5 mmol) synthesized by the method of Preparation Example 38, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 534 (7'-(2-(4-phenyl-6-(2-(9-phenyl-9H-carbazol-2-yl)phenyl)-1,3,5-triazin-2-yl)phenyl)spiro[cyclohexane-1,9'-fluorene]-2'-carbonitrile) (19.7 g, 24.4 mmol, yield 75%) .

Mass: [(M+H)⁺] : 809

### [Synthesis Example 95]: Synthesis of Compound 550

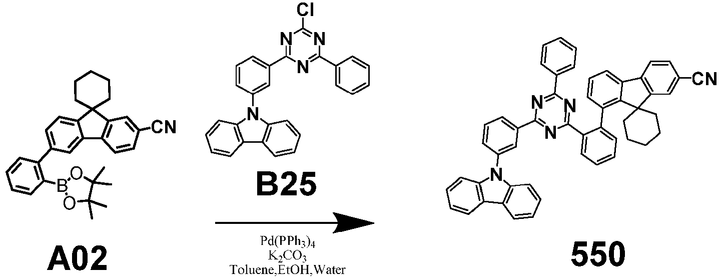

Compound A02 (15.0 g, 32.5 mmol) synthesized by the method of Preparation Example 9, Compound B25 (14.1 g, 32.5 mmol) synthesized by the method of Preparation Example 39, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 550 (1'-(2-(4-(3-(9H-carbazol-9-yl)phenyl)-6-phenyl-1,3,5-triazin-2-yl)phenyl)spiro[cyclohexane-1,9'-fluorene]-7'-carbonitrile) (17.8 g, 24.4 mmol, yield 75%).

Mass: [(M+H)⁺] : 733

### [Synthesis Example 96]: Synthesis of Compound 561

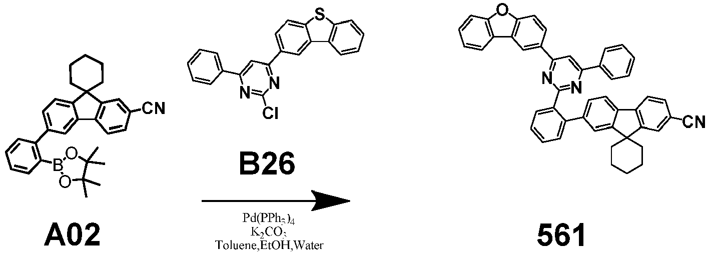

Compound A02 (15.0 g, 32.5 mmol) synthesized by the method of Preparation Example 9, Compound B26 (12.1 g, 32.5 mmol) synthesized by the method of Preparation Example 40, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 561 (7'-(2-(4-(dibenzo[b,d]furan-2-yl)-6-phenylpyrimidin-2-yl)phenyl)spiro[cyclohexane-1,9'-fluorene]-2'-carbonitrile) (17.8 g, 24.4 mmol, yield 75%).

Mass: [(M+H)⁺] : 733

### [Synthesis Example 97]: Synthesis of Compound 570

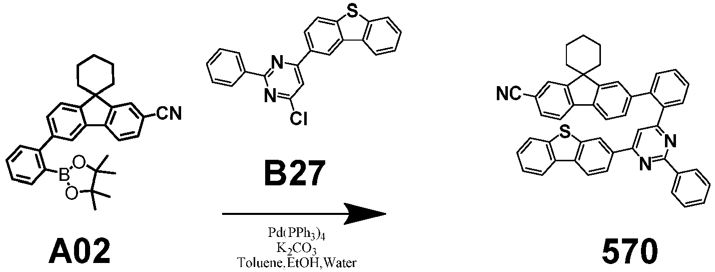

Compound A02 (15.0 g, 32.5 mmol) synthesized by the method of Preparation Example 9, Compound B27 (12.1 g, 32.5 mmol) synthesized by the method of Preparation Example 41, Pd(PPh₃)₄ (1.1 g, 1.0 mmol) and K₂CO₃ (13.5 g, 97.5 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 570 (7'-(2-(6-(dibenzo[b,d]thiophen-3-yl)-2-phenylpyrimidin-4-yl)phenyl)spiro[cyclohexane-1,9'-fluorene]-2'-carbonitrile) (16.4 g, 24.4 mmol, yield 75%).

Mass: [(M+H)⁺] : 733

### [Synthesis Example 98]: Synthesis of Compound 624

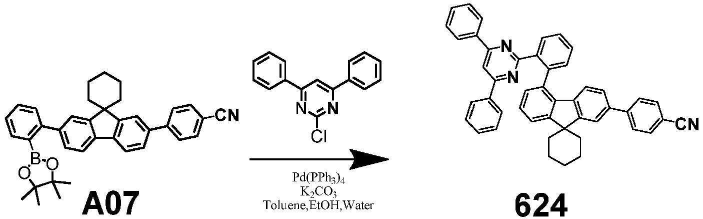

Compound A07 (15.0 g, 27.9 mmol) synthesized by the method of Preparation Example 14, 2-chloro-4,6-diphenylpyrimidine (7.4 g, 27.9 mmol), Pd(PPh₃)₄ (1.0 g, 0.8 mmol) and K₂CO₃ (11.6 g, 83.7 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 624 (4-(4'-(2-(4,6-diphenylpyrimidin-2-yl)phenyl)spiro[cyclohexane-1,9'-fluoren]-7'-yl)benzonitrile) (13.4 g, 20.9 mmol, yield 75%).

Mass: [(M+H)⁺] : 643

### [Synthesis Example 99]: Synthesis of Compound 635

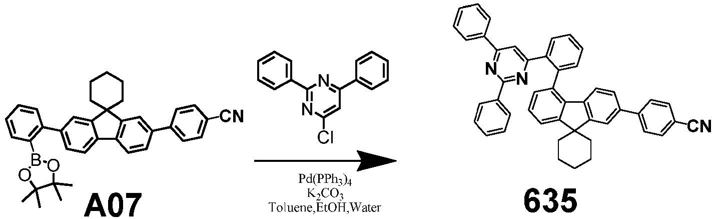

Compound A07 (15.0 g, 27.9 mmol) synthesized by the method of Preparation Example 14, 4-chloro-2,6-diphenylpyrimidine (7.4 g, 27.9 mmol), Pd(PPh₃)₄ (1.0 g, 0.8 mmol) and K₂CO₃ (11.6 g, 83.7 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 635 (4-(4'-(2-(2,6-diphenylpyrimidin-4-yl)phenyl)spiro[cyclohexane-1,9'-fluoren]-7'-yl)benzonitrile) (13.4 g, 20.9 mmol, yield 75%).

Mass: [(M+H)⁺] : 643

### [Synthesis Example 100]: Synthesis of Compound 646

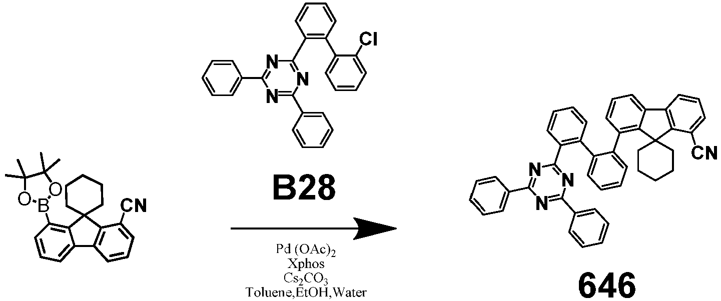

8'-(4,4,5,5-tetramethyl-1,3,2-dioxaborolan-2-yl)spiro[cyclohexane-1,9'-fluorene]-1'-carbonitrile (15.0 g, 31.6 mmol) and Compound B28 (13.3 g, 31.6 mmol) synthesized by the method of Preparation Example 42, Pd(OAc)₂ (0.2 g, 0.9 mmol), Xphos (0.9 g, 1.9 mmol) and Cs₂CO₃ (20.6 g, 63.2 mmol) were reacted under reflux in the presence of a mixed solvent of 180 ml of toluene, 30 ml of EtOH and 30 ml of water for 4 hours. After completion of the reaction, the reaction product was cooled to room temperature and extracted with methylene chloride, and the extracted organic layer was dehydrated with magnesium sulfate, concentrated, and purified by column chromatography to obtain Compound 646 (8'-(2'-(4,6-diphenyl-1,3,5-triazin-2-yl)-[1,1'-biphenyl]-2-yl)spiro[cyclohexane-1,9'-fluorene]-1'-carbonitrile)(21.0 g, 25.6 mmol, yield 81%).

Mass: [(M+H)⁺] : 643

### [Synthesis Example 101]: Compound 714

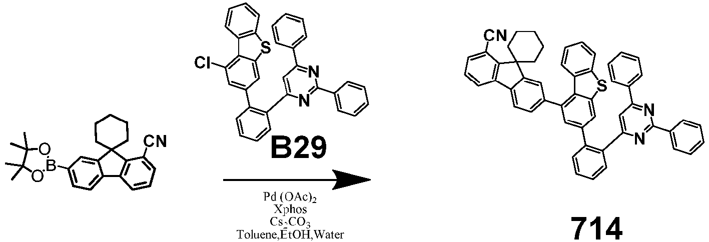

7'-(4,4,5,5-tetramethyl-1,3,2-dioxaborolan-2-yl)spiro[cyclohexane-1,9'-fluorene]-1'-carbonitrile (15.0 g, 31.6 mmol) and Compound B29 (16.6 g, 31.6 mmol) synthesized by the method of Preparation Example 43, Pd(OAc)₂ (0.2 g, 0.9 mmol), Xphos (0.9 g, 1.9 mmol) and Cs₂CO₃ (20.6 g, 63.2 mmol) were reacted under reflux in the presence of a mixed solvent of 180 ml of toluene, 30 ml of EtOH and 30 ml of water for 4 hours. After completion of the reaction, the reaction product was cooled to room temperature and extracted with methylene chloride, and the extracted organic layer was dehydrated with magnesium sulfate, concentrated, and purified by column chromatography to obtain Compound 714 (7'-(3-(2-(2,6-diphenylpyrimidin-4-yl)phenyl)dibenzo[b,d]thiophen-1-yl)spiro[cyclohexane-1,9'-fluorene]-1'-carbonitrile)(21.1 g, 25.6 mmol, yield 81%).

Mass: [(M+H)⁺] : 749

### [Synthesis Example 102]: Compound 761

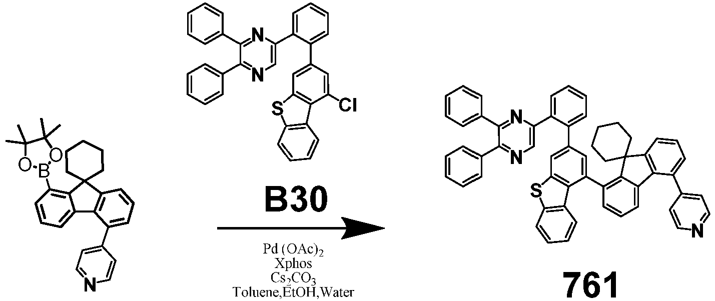

4-(1'-(4,4,5,5-tetramethyl-1,3,2-dioxaborolan-2-yl)spiro[cyclohexane-1,9'-fluoren]-5'-yl)pyridine (15.0 g, 31.6 mmol) and Compound B30 (16.6 g, 31.6 mmol) synthesized by the method of Preparation Example 44, Pd(OAc)₂ (0.2 g, 0.9 mmol), Xphos (0.9 g, 1.9 mmol) and Cs₂CO₃ (20.6 g, 63.2 mmol) were reacted under reflux in the presence of a mixed solvent of 180 ml of toluene, 30 ml of EtOH and 30 ml of water for 4 hours. After completion of the reaction, the reaction product was cooled to room temperature and extracted with methylene chloride, and the extracted organic layer was dehydrated with magnesium sulfate, concentrated, and purified by column chromatography to obtain Compound 761 (2,3-diphenyl-5-(2-(1-(4'-(pyridin-4-yl)spiro[cyclohexane-1,9'-fluoren]-8'-yl)dibenzo[b,d]thiophen-3-yl)phenyl)pyrazine)(21.1 g, 25.6 mmol, yield 81%).

Mass: [(M+H)⁺] : 801

### [Synthesis Example 103]: Compound 763

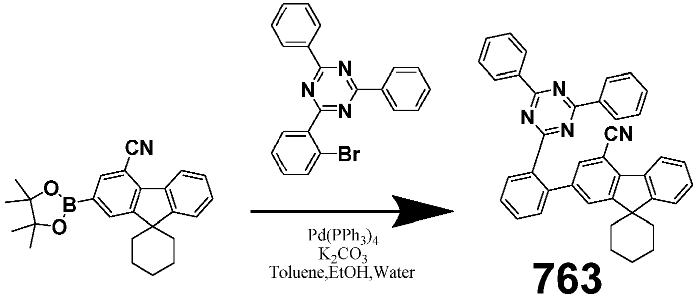

2'-(4,4,5,5-tetramethyl-1,3,2-dioxaborolan-2-yl)spiro[cyclohexane-1,9'-fluorene]-4'-carbonitrile (15.0 g, 38.9 mmol), 2-(2-bromophenyl)-4,6-diphenyl-1,3,5-triazine (15.1 g, 38.9 mmol), and Pd(PPh₃)₄ (1.3 g, 1.2 mmol) and K₂CO₃ (16.1 g, 116.8 mmol) were reacted under reflux in the presence of a mixed solvent of 150 ml of toluene, 22.5 ml of ethanol and 22.5 ml of water for 2 hours. After completion of the reaction, the reaction product was extracted with methylene chloride, and the extracted organic layer was dehydrated with magnesium sulfate, concentrated, and purified by column chromatography to obtain Compound 763 (2'-(2-(4-([1,1'-biphenyl]-4-yl)-6-phenylpyrimidin-2-yl)phenyl)spiro[cyclohexane-1,9'-fluorene]-7'-carbonitrile) (17.4 g, 30.8 mmol, yield 79%).

Mass: [(M+H)⁺] :568

### [Examples and Comparative Examples] - 1

### [Examples 1 to 103 and Comparative Examples 1 to 7]: Fabrication of blue organic electroluminescent devices

The compounds synthesized in Synthesis Examples were purified by sublimation to high purity using a commonly known method and then blue organic electroluminescent devices were fabricated in accordance with the following process.

First, a glass substrate coated to a thickness of 1,200 Å with indium tin oxide (ITO) was subjected to ultrasonic cleaning with distilled water. After cleaning with distilled water, the glass substrate was subjected to ultrasonic cleaning with a solvent such as isopropyl alcohol, acetone, or methanol, followed by drying and cleaning with UV using a UV OZONE cleaner (Power sonic 405, Hwashin Tech) for 5 minutes to manufacture a substrate with an ITO transparent electrode. The manufactured substrate was transferred to a vacuum evaporator.

A hole injection layer, a hole transport layer, a hole transport auxiliary layer, a light-emitting layer, an electron transport auxiliary layer, an electron transport layer, an electron injection layer, and a cathode were laminated in that order on the ITO transparent electrode (anode) of the substrate prepared as above, to fabricate an organic electroluminescent device. Specifically, the hole injection layer was formed by co-depositing Compound A and Compound B at a weight ratio of 98:2 to a thickness of 100 Å on the anode, the hole transport layer was formed by depositing Compound A to a thickness of 1,400 Å on the hole injection layer, the hole transport auxiliary layer was formed by depositing Compound C to a thickness of 50 Å on the hole transport layer, the light-emitting layer was formed by co-depositing Compound D and Compound E at a weight ratio of 98:2 to a thickness of 200 Å on the hole transport auxiliary layer, the electron transport auxiliary layer was formed by depositing Compound F to a thickness of 50 Å on the light- emitting layer, the electron transport layer was formed by co-depositing a material for an electron transport layer and Compound H at a weight ratio of 1:1 to a thickness of 300 Å on the electron transport auxiliary layer, the electron injection layer was formed by depositing LiF to a thickness of 10 Å on the electron transport layer, and the cathode was formed by depositing Al to a thickness of 1,000 Å on the electron injection layer. The structures of Compounds A to H are shown in Table 1 below and the material for the electron transport layer is shown in Table 2 below.

**[Table 1]**

| | |
|---|---|
| Compound A | 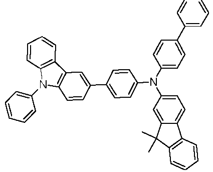 |
| Compound B | 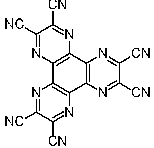 |
| Compound C | 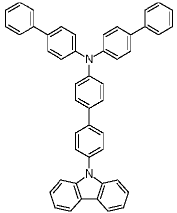 |
| Compound D | 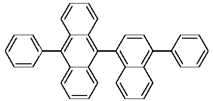 |
| Compound E | 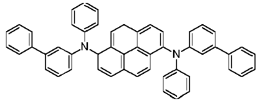 |
| Compound F | 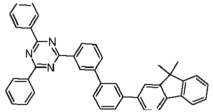 |
| Compound G | 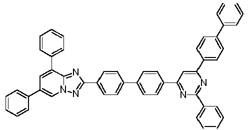 |
| Compound H | 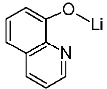 |

**[Table 2]**

| | Material for electron transport layer | |
|---|---|---|
| Example 1 | Compound 006 | |
| Example 2 | Compound 010 | |
| Example 3 | Compound 014 | |
| Example 4 | Compound 022 | |
| Example 5 | Compound 026 | |
| Example 6 | Compound 030 | |
| Example 7 | Compound 038 | |
| Example 8 | Compound 042 | |
| Example 9 | Compound 046 | |
| Example 10 | Compound 054 | |
| Example 11 | Compound 058 | |
| Example 12 | Compound 062 | |
| Example 13 | Compound 070 | |
| Example 14 | Compound 074 | |
| Example 15 | Compound 078 | |
| Example 16 | Compound 086 | |
| Example 17 | Compound 090 | |
| Example 18 | Compound 094 | |
| Example 19 | Compound 102 | |
| Example 20 | Compound 106 | |
| Example 21 | Compound 110 | |
| Example 22 | Compound 118 | |
| Example 23 | Compound 122 | |
| Example 24 | Compound 126 | |
| Example 25 | Compound 134 | |
| Example 26 | Compound 138 | |
| Example 27 | Compound 142 | |
| Example 28 | Compound 150 | |
| Example 29 | Compound 154 | |
| Example 30 | Compound 158 | |
| Example 31 | Compound 166 | |
| Example 32 | Compound 170 | |
| Example 33 | Compound 174 | |
| Example 34 | Compound 182 | |
| Example 35 | Compound 186 | |
| Example 36 | Compound 190 | |
| Example 37 | Compound 198 | |
| Example 38 | Compound 202 | |
| Example 39 | Compound 206 | |
| Example 40 | Compound 214 | |
| Example 41 | Compound 218 | |
| Example 42 | Compound 222 | |
| Example 43 | Compound 230 | |
| Example 44 | Compound 234 | |
| Example 45 | Compound 238 | |
| Example 46 | Compound 246 | |
| Example 47 | Compound 250 | |
| Example 48 | Compound 254 | |
| Example 49 | Compound 262 | |
| Example 50 | Compound 266 | |
| Example 51 | Compound 270 | |
| Example 52 | Compound 278 | |
| Example 53 | Compound 282 | |
| Example 54 | Compound 286 | |
| Example 55 | Compound 294 | |
| Example 56 | Compound 298 | |
| Example 57 | Compound 302 | |
| Example 58 | Compound 310 | |
| Example 59 | Compound 314 | |
| Example 60 | Compound 318 | |
| Example 61 | Compound 326 | |
| Example 62 | Compound 330 | |
| Example 63 | Compound 334 | |
| Example 64 | Compound 342 | |
| Example 65 | Compound 346 | |
| Example 66 | Compound 350 | |
| Example 67 | Compound 358 | |
| Example 68 | Compound 362 | |
| Example 69 | Compound 366 | |
| Example 70 | Compound 374 | |
| Example 71 | Compound 378 | |
| Example 72 | Compound 382 | |
| Example 73 | Compound 390 | |
| Example 74 | Compound 394 | |
| Example 75 | Compound 398 | |
| Example 76 | Compound 406 | |
| Example 77 | Compound 410 | |
| Example 78 | Compound 414 | |
| Example 79 | Compound 422 | |
| Example 80 | Compound 426 | |
| Example 81 | Compound 430 | |
| Example 82 | Compound 438 | |
| Example 83 | Compound 442 | |
| Example 84 | Compound 446 | |
| Example 85 | Compound 454 | |
| Example 86 | Compound 458 | |
| Example 87 | Compound 462 | |
| Example 88 | Compound 470 | |
| Example 89 | Compound 474 | |
| Example 90 | Compound 478 | |
| Example 91 | Compound 486 | |
| Example 92 | Compound 502 | |
| Example 93 | Compound 518 | |
| Example 94 | Compound 534 | |
| Example 95 | Compound 550 | |
| Example 96 | Compound 561 | |
| Example 97 | Compound 570 | |
| Example 98 | Compound 624 | |
| Example 99 | Compound 635 | |
| Example 100 | Compound 646 | |
| Example 101 | Compound 714 | |
| Example 102 | Compound 761 | |
| Example 103 | Compound 763 | |
| Comparative Example 1 | Compound I | 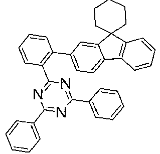 |
| Comparative Example 2 | Compound J | 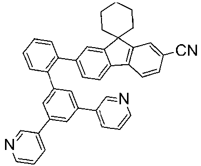 |
| Comparative Example 3 | Compound K | 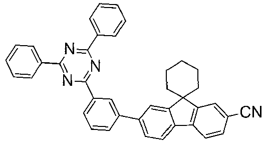 |
| Comparative Example 4 | Compound L | 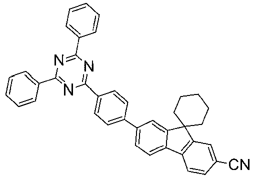 |
| Comparative Example 5 | Compound M | 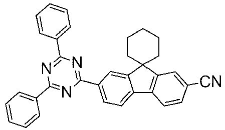 |
| Comparative Example 6 | Compound N | 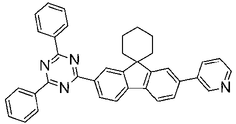 |
| Comparative Example 7 | Compound O | 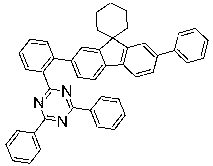 |

### [Experimental Example 1]: Evaluation of performance of blue organic electroluminescent devices of Examples 1 to 103 and Comparative Examples 1 to 7

The driving voltage, EL peak, and current efficiency of the organic electroluminescent devices fabricated in Examples 1 to 103 and Comparative Examples 1 to 7 were measured at a current density of 10 mA/cm² and the results were shown in Table 3 below.

**[Table 3]**

| | Driving voltage (V) | EL peak (nm) | Current efficiency (cd/A) |
|---|---|---|---|
| Example 1 | 3.5 | 453 | 8.7 |
| Example 2 | 3.6 | 454 | 8.5 |
| Example 3 | 3.6 | 453 | 8.6 |
| Example 4 | 3.5 | 452 | 8.8 |
| Example 5 | 3.6 | 451 | 8.6 |
| Example 6 | 3.7 | 454 | 8.4 |
| Example 7 | 3.4 | 453 | 8.7 |
| Example 8 | 3.5 | 455 | 8.4 |
| Example 9 | 3.6 | 452 | 8.5 |
| Example 10 | 3.5 | 453 | 8.8 |
| Example 11 | 3.5 | 456 | 8.6 |
| Example 12 | 3.6 | 453 | 8.5 |
| Example 13 | 3.6 | 453 | 8.8 |
| Example 14 | 3.7 | 455 | 8.6 |
| Example 15 | 3.6 | 454 | 8.6 |
| Example 16 | 3.4 | 453 | 8.7 |
| Example 17 | 3.5 | 455 | 8.5 |
| Example 18 | 3.5 | 452 | 8.5 |
| Example 19 | 3.6 | 453 | 8.8 |
| Example 20 | 3.6 | 456 | 8.5 |
| Example 21 | 3.7 | 453 | 8.4 |
| Example 22 | 3.7 | 453 | 8.7 |
| Example 23 | 3.8 | 453 | 8.6 |
| Example 24 | 3.8 | 453 | 8.6 |
| Example 25 | 3.5 | 453 | 8.8 |
| Example 26 | 3.6 | 453 | 8.5 |
| Example 27 | 3.6 | 453 | 8.5 |
| Example 28 | 3.5 | 453 | 8.7 |
| Example 29 | 3.5 | 453 | 8.5 |
| Example 30 | 3.6 | 454 | 8.4 |
| Example 31 | 3.4 | 453 | 8.8 |
| Example 32 | 3.7 | 454 | 8.4 |
| Example 33 | 3.6 | 455 | 8.3 |
| Example 34 | 3.5 | 454 | 8.7 |
| Example 35 | 3.5 | 456 | 8.7 |
| Example 36 | 3.6 | 455 | 8.6 |
| Example 37 | 3.6 | 455 | 8.7 |
| Example 38 | 3.7 | 454 | 8.3 |
| Example 39 | 3.6 | 456 | 8.2 |
| Example 40 | 3.7 | 455 | 8.6 |
| Example 41 | 3.7 | 456 | 8.6 |
| Example 42 | 3.6 | 453 | 8.5 |
| Example 43 | 3.6 | 454 | 8.8 |
| Example 44 | 3.6 | 456 | 8.5 |
| Example 45 | 3.7 | 455 | 8.6 |
| Example 46 | 3.6 | 457 | 8.6 |
| Example 47 | 3.8 | 456 | 8.6 |
| Example 48 | 3.6 | 455 | 8.5 |
| Example 49 | 3.7 | 454 | 8.8 |
| Example 50 | 3.8 | 454 | 8.4 |
| Example 51 | 3.7 | 455 | 8.4 |
| Example 52 | 3.6 | 453 | 8.7 |
| Example 53 | 3.6 | 456 | 8.6 |
| Example 54 | 3.8 | 455 | 8.5 |
| Example 55 | 3.6 | 455 | 8.6 |
| Example 56 | 3.6 | 454 | 8.6 |
| Example 57 | 3.7 | 455 | 8.8 |
| Example 58 | 3.5 | 454 | 8.5 |
| Example 59 | 3.6 | 453 | 8.5 |
| Example 60 | 3.6 | 455 | 8.4 |
| Example 61 | 3.5 | 455 | 8.7 |
| Example 62 | 3.7 | 454 | 8.5 |
| Example 63 | 3.6 | 455 | 8.6 |
| Example 64 | 3.5 | 454 | 8.7 |
| Example 65 | 3.7 | 455 | 8.6 |
| Example 66 | 3.7 | 456 | 8.7 |
| Example 67 | 3.6 | 455 | 8.8 |
| Example 68 | 3.8 | 455 | 8.6 |
| Example 69 | 3.7 | 454 | 8.5 |
| Example 70 | 3.6 | 456 | 8.7 |
| Example 71 | 3.6 | 455 | 8.6 |
| Example 72 | 3.7 | 455 | 8.6 |
| Example 73 | 3.5 | 454 | 8.7 |
| Example 74 | 3.7 | 452 | 8.5 |
| Example 75 | 3.6 | 455 | 8.4 |
| Example 76 | 3.3 | 453 | 8.6 |
| Example 77 | 3.8 | 455 | 8.5 |
| Example 78 | 3.7 | 454 | 8.5 |
| Example 79 | 3.6 | 454 | 8.7 |
| Example 80 | 3.7 | 453 | 8.6 |
| Example 81 | 3.6 | 455 | 8.6 |
| Example 82 | 3.8 | 456 | 8.5 |
| Example 83 | 3.8 | 455 | 8.4 |
| Example 84 | 3.8 | 453 | 8.4 |
| Example 85 | 3.5 | 456 | 8.7 |
| Example 86 | 3.6 | 455 | 8.7 |
| Example 87 | 3.6 | 452 | 8.6 |
| Example 88 | 3.6 | 455 | 8.6 |
| Example 89 | 3.6 | 454 | 8.4 |
| Example 90 | 3.7 | 456 | 8.4 |
| Example 91 | 3.4 | 455 | 8.8 |
| Example 92 | 3.5 | 455 | 8.3 |
| Example 93 | 3.7 | 456 | 8.4 |
| Example 94 | 3.5 | 454 | 8.2 |
| Example 95 | 3.6 | 455 | 8.2 |
| Example 96 | 3.4 | 456 | 8.3 |
| Example 97 | 3.5 | 456 | 8.4 |
| Example 98 | 3.8 | 455 | 8.3 |
| Example 99 | 3.8 | 456 | 8.4 |
| Example 100 | 3.5 | 455 | 8.7 |
| Example 101 | 3.6 | 456 | 8.7 |
| Example 102 | 3.5 | 455 | 8.2 |
| Example 103 | 3.5 | 455 | 8.6 |
| Comparative Example 1 | 4.2 | 455 | 7.7 |
| Comparative Example 2 | 4.4 | 454 | 7.5 |
| Comparative Example 3 | 4.4 | 455 | 7.3 |
| Comparative Example 4 | 4.2 | 455 | 7.4 |
| Comparative Example 5 | 4.3 | 457 | 7.4 |
| Comparative Example 6 | 4.4 | 456 | 7.4 |
| Comparative Example 7 | 4.1 | 456 | 7.6 |

As can be seen from Table 3, the blue organic electroluminescent devices fabricated in Examples 1 to 103 using the compounds according to the present disclosure as the electron transport layer materials exhibited excellent driving voltage, EL peak, and current efficiency, compared to blue organic electroluminescent devices fabricated in Comparative Examples 1 to 7 using the compounds I to O as the electron transport layer materials.

Specifically, the organic light-emitting compounds according to the present disclosure used in the blue organic electroluminescent devices of Examples 1 to 103 have a structure in which an azine moiety with excellent electron transport ability and stability is linked to a cyclohexylfluorene group bonded with a cyano or pyridine group that increases a dipole moment, thereby exhibiting excellent driving voltage and current efficiency, compared to blue organic electroluminescent devices of Comparative Examples 1 and 7 that use, as an electron transport layer material, such as Compound I or Compound O, a compound in which an azine moiety is linked to a cyclohexylfluorene group and that does not contain a cyano group or a pyridine group, a blue organic electroluminescent device of Comparative Example 2 that uses, as an electron transport layer material, a compound, such as Compound J, in which a cyclohexylfluorene group is linked to a cyano group and that does not contain an azine moiety, blue organic electroluminescent devices of Comparative Examples 3 and 4 that use, as an electron transport layer material, a compound, such as Compound K or L, in which a cyclohexylfluorene group is linked to a cyano group and that contains an azine moiety, wherein the cyclohexylfluorene group does not bind to the azine moiety in the ortho position, and blue organic electroluminescent devices of Comparative Examples 5 and 6 that use, as an electron transport layer material, a compound, such as Compound M or N, in which a cyclohexylfluorene group is linked to a cyano group or a pyridine group and that contains an azine moiety, wherein the cyclohexylfluorene group is directly linked to the azine moiety without a linker.

### [Examples and Comparative Examples] - 2

### [Examples 104 to 108 and Comparative Example 8]: Fabrication of blue organic electroluminescent devices

The compounds synthesized in Synthesis Examples were purified by sublimation to high purity using a commonly known method and then blue organic electroluminescent devices were fabricated in accordance with the following process.

First, a glass substrate coated to a thickness of 1,200 Å with indium tin oxide (ITO) was subjected to ultrasonic cleaning with distilled water. After cleaning with distilled water, the glass substrate was subjected to ultrasonic cleaning with a solvent such as isopropyl alcohol, acetone, or methanol, followed by drying and cleaning with UV using a UV OZONE cleaner (Power sonic 405, Hwashin Tech) for 5 minutes to manufacture a substrate with an ITO transparent electrode. The manufactured substrate was transferred to a vacuum evaporator.

A hole injection layer, a hole transport layer, a hole transport auxiliary layer, a light-emitting layer, an electron transport auxiliary layer, an electron transport layer, an electron injection layer, and a cathode were laminated in that order on the ITO transparent electrode (anode) of the substrate prepared as above, to fabricate an organic electroluminescent device. Specifically, the hole injection layer was formed by co-depositing Compound A and Compound B at a weight ratio of 98:2 to a thickness of 100 Å on the anode, the hole transport layer was formed by depositing Compound A on the hole injection layer to a thickness of 1,400 Å, the hole transport auxiliary layer was formed by depositing Compound C to a thickness of 50 Å on the hole transport layer, the light-emitting layer was formed by co-depositing Compound D and Compound E at a weight ratio of 98:2 to a thickness of 200 Å on the hole transport auxiliary layer, the electron transport auxiliary layer was formed by depositing material for electron transport auxiliary layer to a thickness of 50 Å on the light-emitting layer, the electron transport layer was formed by co-depositing Compound G and Compound H at a weight ratio of 1:1 to a thickness of 300 Å on the electron transport auxiliary layer, the electron injection layer was formed by depositing LiF to a thickness of 10 Å on the electron transport layer, and the cathode was formed by depositing Al to a thickness of 1,000 Å on the electron injection layer. The structures of Compounds A to H are shown in Table 1 below and the material for the electron transport auxiliary layer is shown in Table 4 below.

**[Table 4]**

| | Material for electron transport auxiliary layer | |
|---|---|---|
| Example 104 | Compound 518 | |
| Example 105 | Compound 635 | |
| Example 106 | Compound 646 | |
| Example 107 | Compound 714 | |
| Example 108 | Compound 761 | |
| Comparative Example 8 | Compound F | 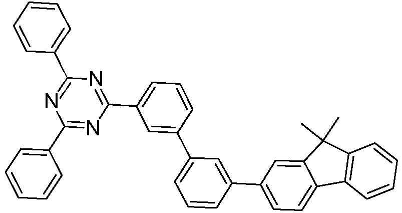 |

### [Experimental Example 2]: Evaluation of performance of blue organic electroluminescent devices of Examples 104 to 108 and Comparative Example 8

The driving voltage, EL peak, and current efficiency of the organic electroluminescent devices fabricated in Examples 104 to 108 and Comparative Example 8 were measured at a current density of 10 mA/cm² and the results are shown in Table 5 below.

**[Table 5]**

| | Driving voltage (V) | EL peak (nm) | Current efficiency (cd/A) |
|---|---|---|---|
| Example 104 | 4.0 | 453 | 7.5 |
| Example 105 | 3.9 | 455 | 7.7 |
| Example 106 | 3.8 | 456 | 7.8 |
| Example 107 | 4.1 | 453 | 7.6 |
| Example 108 | 4.0 | 454 | 7.7 |
| Comparative Example 8 | 4.5 | 456 | 7.0 |

As can be seen from Table 5, the blue organic electroluminescent devices fabricated in Examples 104 to 108 using the compounds according to the present disclosure as the electron transport auxiliary layer materials exhibited excellent driving voltage, EL peak, and current efficiency, compared to the blue organic electroluminescent device fabricated in Comparative Example 8 using the compound F as the electron transport layer material.

## Claims

1. An organic light-emitting compound represented by the following Formula 1: 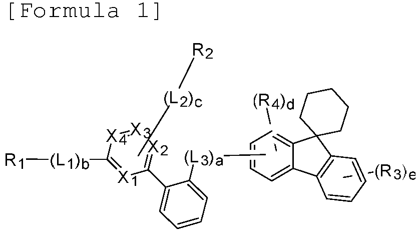 wherein
one of X₁ to X₄ is C-(L₂)_{c}-R₂, the others are each independently N or CR, and at least two of X₁ to X₄ are N, wherein R is hydrogen, an alkyl group containing 1 to 40 carbon atoms, an aryl group containing 6 to 60 carbon atoms, or a heteroaryl group containing 2 to 60 carbon atoms, each of which is unsubstituted or substituted,
R₁ and R₂ are each independently hydrogen, an alkenyl group containing 2 to 40 carbon atoms, an alkynyl group containing 2 to 40 carbon atoms, a cycloalkyl group containing 3 to 40 carbon atoms, a heterocycloalkyl group containing 1 to 40 carbon atoms, an alkyl group containing 1 to 40 carbon atoms, an aryl group containing 6 to 60 carbon atoms, a heteroaryl group containing 2 to 60 carbon atoms, an alkylsilyl group containing 1 to 40 carbon atoms, an arylsilyl group containing 6 to 60 carbon atoms, an alkyl boron group containing 1 to 40 carbon atoms, an aryl boron group containing 6 to 60 carbon atoms, an arylphosphine group containing 6 to 60 carbon atoms, an alkylphosphine oxide group containing 1 to 40 carbon atoms, an arylphosphine oxide group containing 6 to 60 carbon atoms, an alkylamine group containing 1 to 40 carbon atoms, or an arylamine group containing 6 to 60 carbon atoms, each of which is unsubstituted or substituted,
L₁ to L₃ are each independently a single bond, an arylene group containing 6 to 60 carbon atoms, or a heteroarylene group containing 2 to 60 carbon atoms, each of which is unsubstituted or substituted,
R₃ and R₄ are each independently an aryl group containing 6 to 60 carbon atoms substituted with a cyano group, an aryl group containing 6 to 60 carbon atoms substituted with a nitrogen-containing heteroaryl group containing 2 to 60 carbon atoms, a nitrogen-containing heteroaryl group containing 2 to 60 carbon atoms, deuterium or a cyano group, each of which is unsubstituted or substituted,
a to c are each independently an integer from 0 to 5, and
d is an integer of 0 to 3, and e is an integer of 0 to 4, with the proviso of d+e ≥ 1.

2. The organic light-emitting compound according to claim 1, wherein
one of X₁ to X₄ is C- (L₂)_{c}-R₂, the others are each independently N or CR, and at least two of X₁ to X₄ are N, wherein R is hydrogen, an alkyl group containing 1 to 20 carbon atoms, an aryl group containing 6 to 30 carbon atoms, or a heteroaryl group containing 2 to 30 carbon atoms,
R₁ and R₂ are each independently hydrogen, an alkenyl group containing 2 to 40 carbon atoms, an alkynyl group containing 2 to 20 carbon atoms, a cycloalkyl group containing 3 to 20 carbon atoms, a heterocycloalkyl group containing 1 to 20 carbon atoms, an alkyl group containing 1 to 20 carbon atoms, an aryl group containing 6 to 30 carbon atoms, a heteroaryl group containing 2 to 30 carbon atoms, an alkylsilyl group containing 1 to 20 carbon atoms, an arylsilyl group containing 6 to 30 carbon atoms, an alkyl boron group containing 1 to 20 carbon atoms, an aryl boron group containing 6 to 30 carbon atoms, an arylphosphine group containing 6 to 30 carbon atoms, an arylphosphine oxide group containing 1 to 20 carbon atoms, an arylphosphine oxide group containing 6 to 30 carbon atoms, an alkylamine group containing 1 to 20 carbon atoms, or an arylamine group containing 6 to 30 carbon atoms, each of which is unsubstituted or substituted with deuterium, an alkenyl group containing 2 to 20 carbon atoms, an alkynyl group containing 2 to 20 carbon atoms, a cycloalkyl group containing 3 to 20 carbon atoms, a heterocycloalkyl group containing 1 to 20 carbon atoms, an alkyl group containing 1 to 20 carbon atoms, an aryl group containing 6 to 30 carbon atoms, a heteroaryl group containing 2 to 30 carbon atoms, an alkylsilyl group containing 1 to 20 carbon atoms, an arylsilyl group containing 6 to 30 carbon atoms, an alkyl boron group having 1 to 20 carbon atoms, an aryl boron group having 6 to 30 carbon atoms, an arylphosphine group having 6 to 30 carbon atoms, an alkylphosphine oxide group having 1 to 20 carbon atoms, an arylphosphine oxide group having 6 to 30 carbon atoms, an alkylamine group having 1 to 20 carbon atoms, or an arylamine group having 6 to 30 carbon atoms,
L₁ to L₃ are each independently a single bond, an arylene group containing 6 to 30 carbon atoms, or a heteroarylene group containing 2 to 30 carbon atoms,
R₃ and R₄ are each independently an aryl group containing 6 to 30 carbon atoms substituted with a cyano group, an aryl group containing 6 to 30 carbon atoms substituted with a nitrogen-containing heteroaryl group containing 2 to 30 carbon atoms, a nitrogen-containing heteroaryl group containing 2 to 30 carbon atoms, deuterium or a cyano group,
a to c are each independently an integer from 0 to 2, and
d is an integer of 0 to 2 and e is an integer of 0 to 2, with the proviso of d+e ≥ 1.

3. The organic light-emitting compound according to claim 1, wherein
one of X₁ to X₄ is C- (L₂)_{c}-R₂, the others are each independently N or CH, and at least two of X₁ to X₄ are N,
R₁ and R₂ are each independently hydrogen, an aryl group containing 6 to 30 carbon atoms, or a heteroaryl group containing 2 to 30 carbon atoms, each of which is unsubstituted or substituted with a cycloalkyl group containing 3 to 20 carbon atoms, an alkyl group containing 1 to 20 carbon atoms, an aryl group containing 6 to 30 carbon atoms, a heteroaryl group containing 2 to 30 carbon atoms, an alkylsilyl group containing 1 to 20 carbon atoms, an arylsilyl group containing 6 to 30 carbon atoms, an alkylphosphine oxide group containing 1 to 20 carbon atoms, or an arylphosphine oxide group containing 6 to 30 carbon atoms,
L₁ to L₃ are each independently a single bond, an arylene group containing 6 to 30 carbon atoms, or a heteroarylene group containing 2 to 30 carbon atoms,
R₃ and R₄ are each independently an aryl group containing 6 to 30 carbon atoms substituted with a cyano group, a nitrogen-containing heteroaryl group containing 2 to 30 carbon atoms, or a cyano group,
a to c are each independently an integer from 0 to 2, and
d is an integer of 0 to 2 and e is an integer of 0 to 2, with the proviso of d+e ≥ 1.

4. The organic light-emitting compound according to claim 1, wherein Formula 1 is represented by any one selected from Formulas 2 to 9 below: 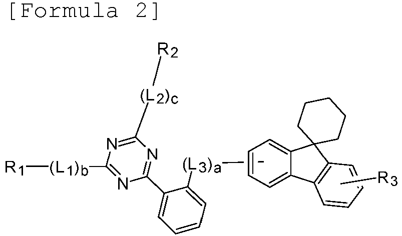 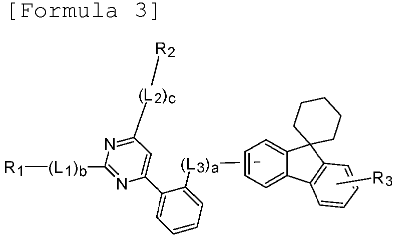 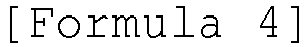 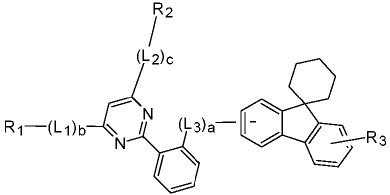 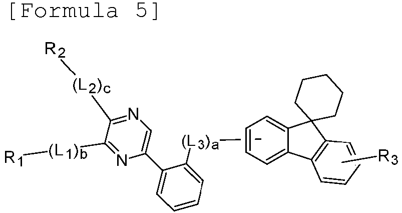 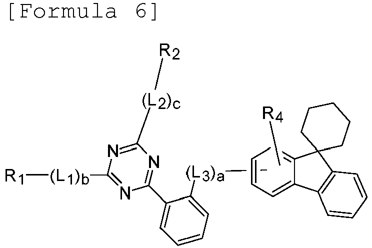 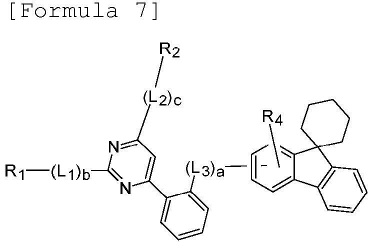 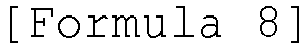 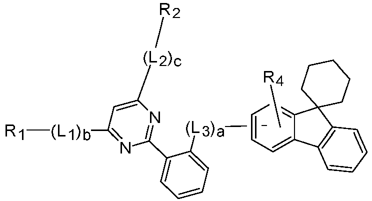 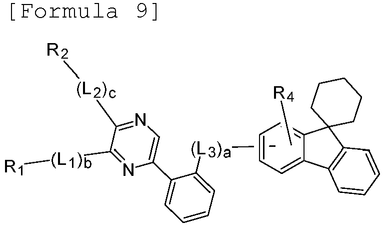 wherein in each of formulas 2 to 9,
R₁ and R₂ are each independently hydrogen, an aryl group containing 6 to 30 carbon atoms, or a heteroaryl group containing 2 to 30 carbon atoms, each of which is unsubstituted or substituted with a cycloalkyl group containing 3 to 20 carbon atoms, an alkyl group containing 1 to 20 carbon atoms, an aryl group containing 6 to 30 carbon atoms, a heteroaryl group containing 2 to 30 carbon atoms, an alkylsilyl group containing 1 to 20 carbon atoms, an arylsilyl group containing 6 to 30 carbon atoms, an alkylphosphine oxide group containing 1 to 20 carbon atoms, or an arylphosphine oxide group containing 6 to 30 carbon atoms,
L₁ to L₃ are each independently a single bond, an arylene group containing 6 to 30 carbon atoms, or a heteroarylene group containing 2 to 30 carbon atoms,
R₃ and R₄ are each independently an aryl group containing 6 to 30 carbon atoms substituted with a cyano group, a nitrogen-containing heteroaryl group containing 2 to 30 carbon atoms, or a cyano group, and
a to c are each independently an integer from 0 to 2.

5. The organic light-emitting compound according to claim 4, wherein Formula 1 is represented by any one selected from Formulas 2 to 7 below. 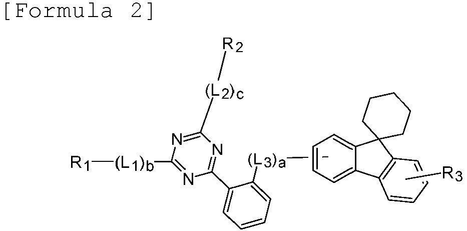 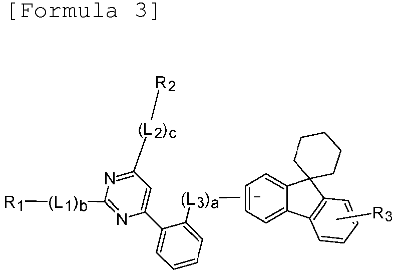 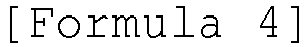 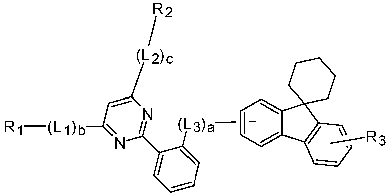 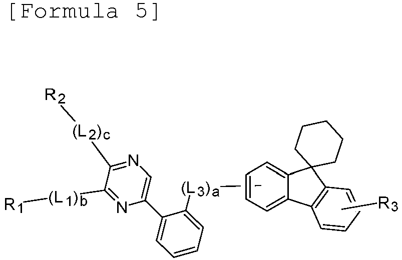 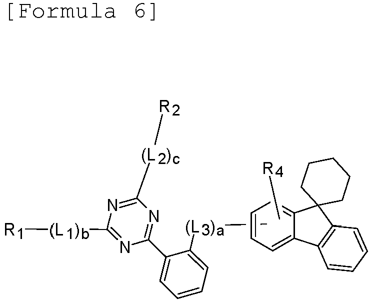 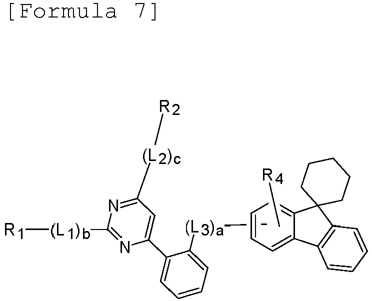 wherein in each of formulas 2 to 7,
R₁ and R₂ are each independently hydrogen, a phenyl group, a biphenyl group, a naphthyl group, a terphenyl group, a fluorenyl group, a pyridyl group, a carbazolyl group, a dibenzothiophenyl group, or a dibenzofuranyl group, each of which is unsubstituted or substituted with a methyl group, a phenyl group, a cyclohexyl group, an adamantyl group, a pyridyl group, a carbazolyl group, a dibenzothiophenyl group, a dibenzofuranyl group, a trimethylsilyl group, a triphenylsilyl group, a dimethylphosphine oxide group or a diphenylphosphine oxide group,
L₁ to L₃ are each independently a single bond, a phenylene group, a biphenylene group, a naphthylene group, a terphenylene group, a dibenzofuranylene group, or a dibenzothiophenylene group,
R₃ and R₄ are each independently a phenyl group substituted with a cyano group, a pyridyl group, or a cyano group, and
a to c are each independently be an integer of 0 to 2.

6. The organic light-emitting compound according to claim 1, wherein the organic light-emitting compound represented by Formula 1 is any one selected from the following Compounds 001 to 774.

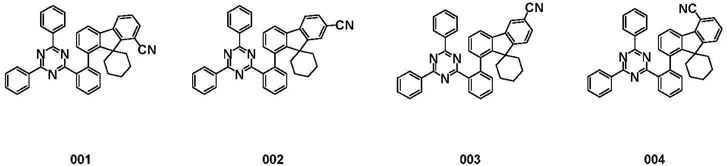

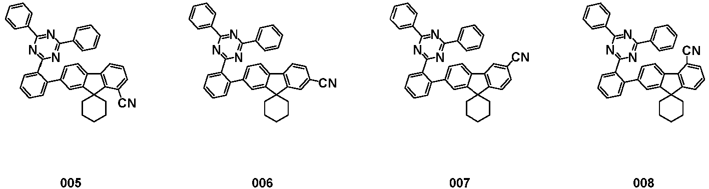

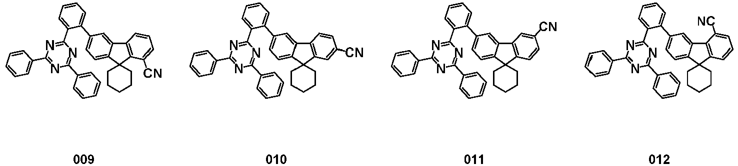

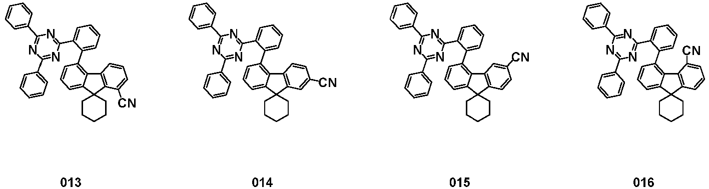

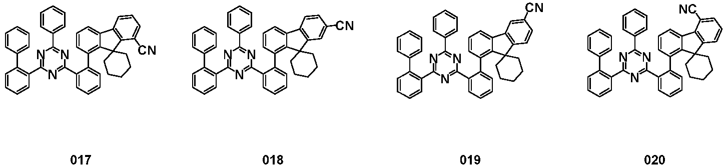

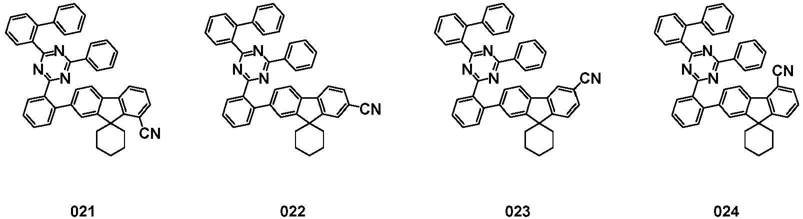

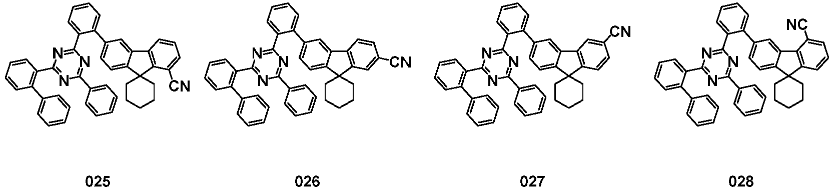

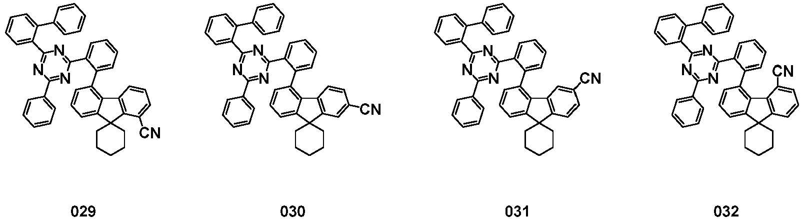

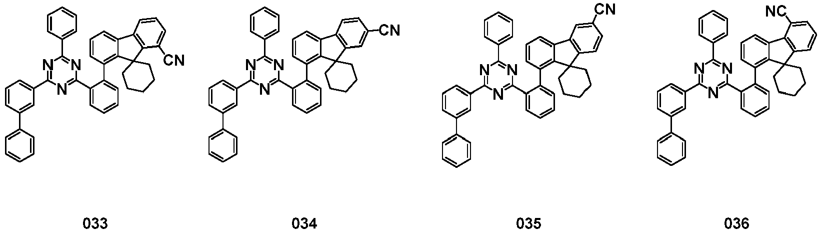

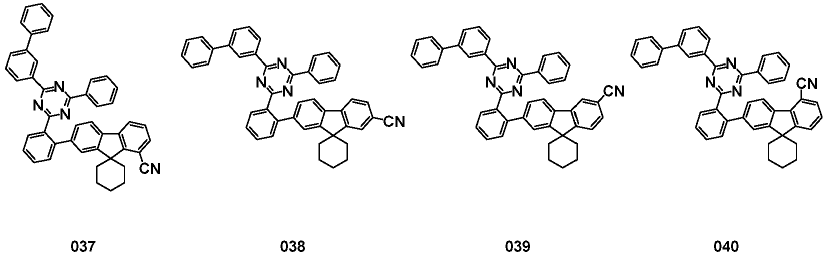

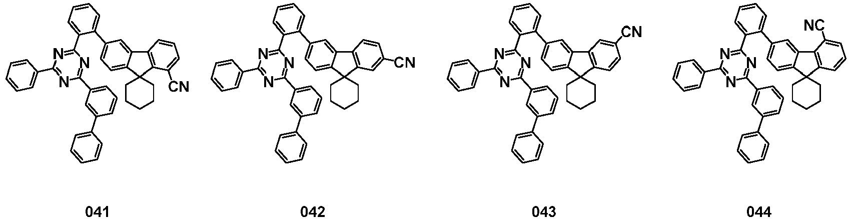

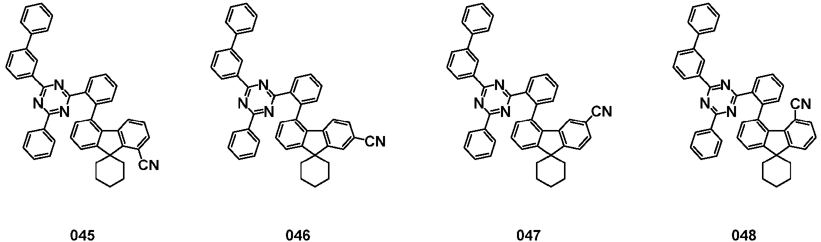

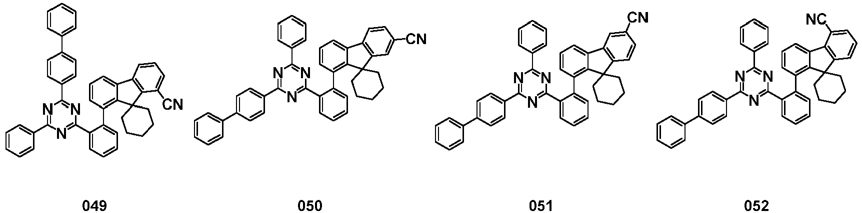

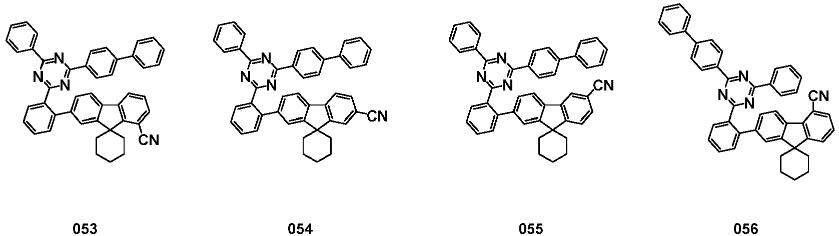

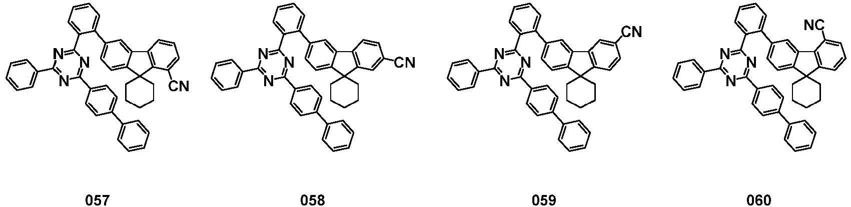

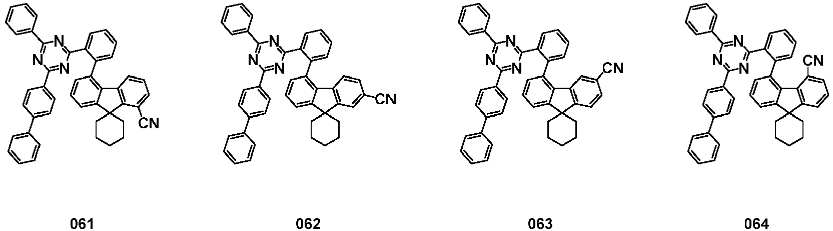

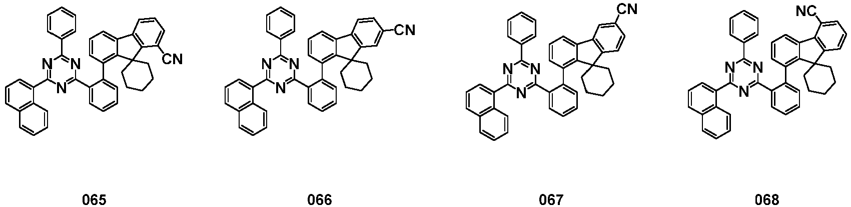

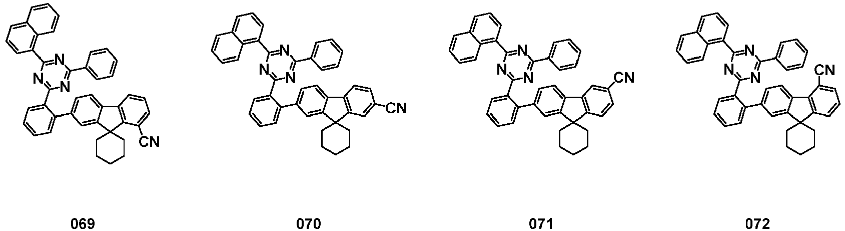

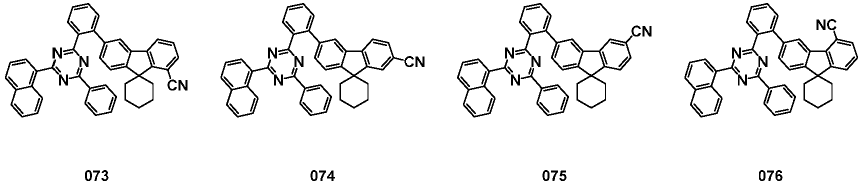

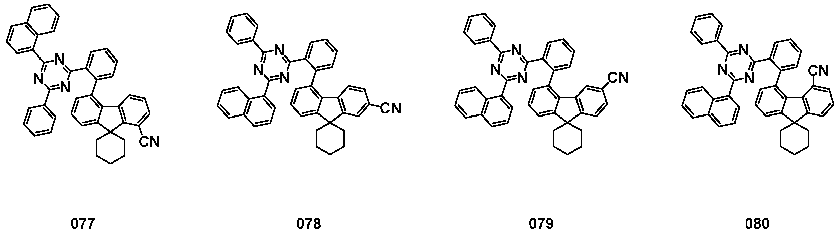

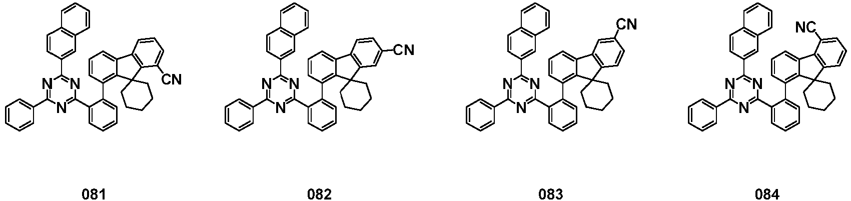

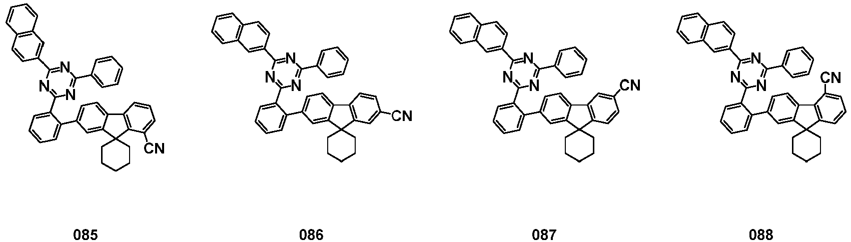

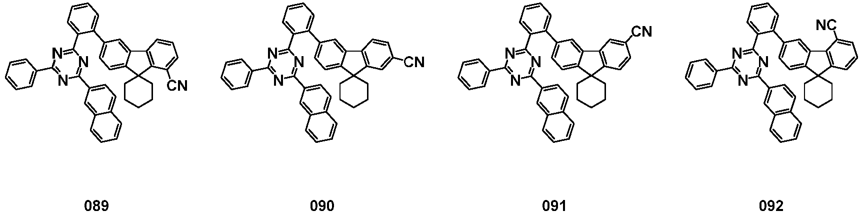

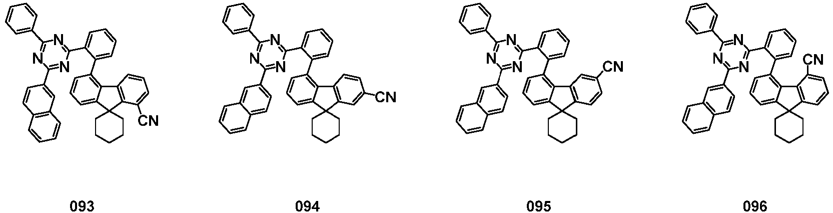

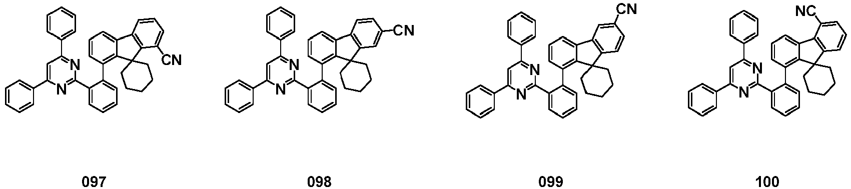

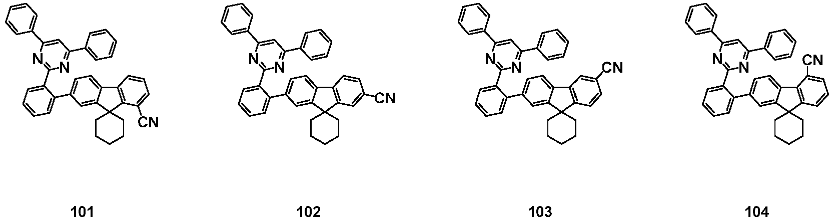

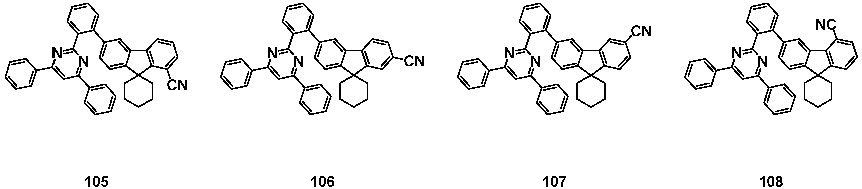

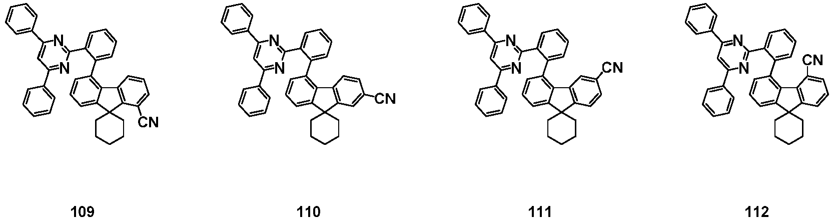

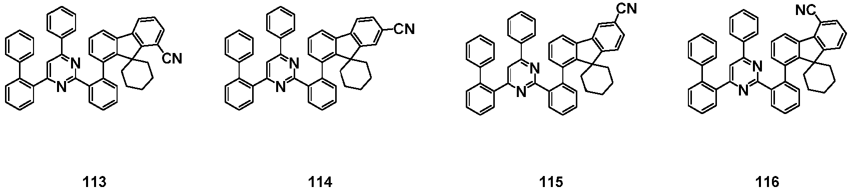

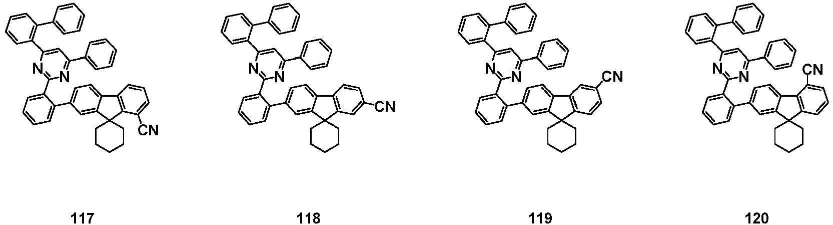

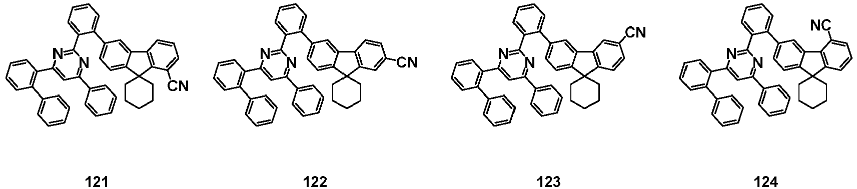

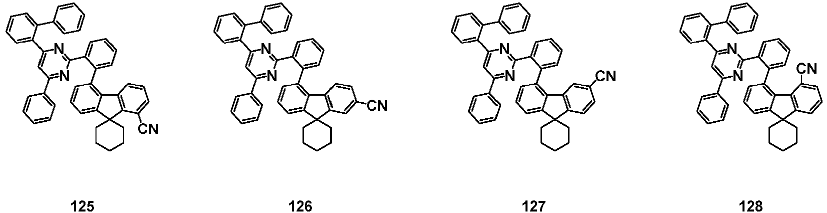

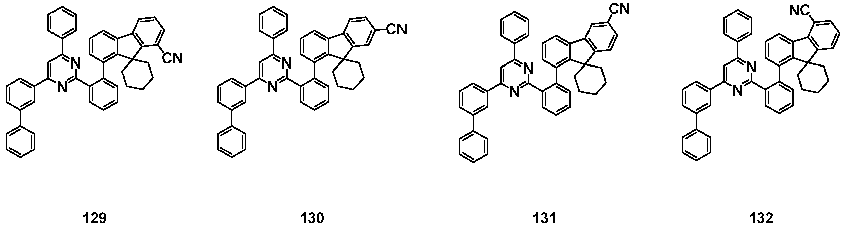

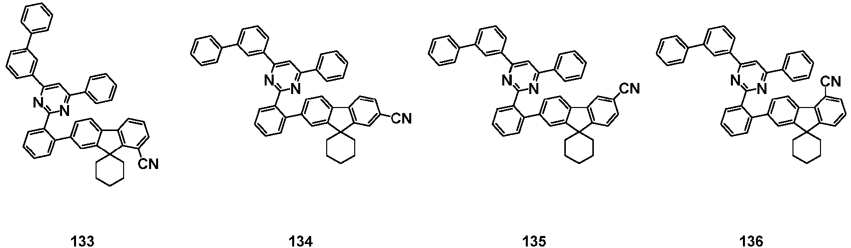

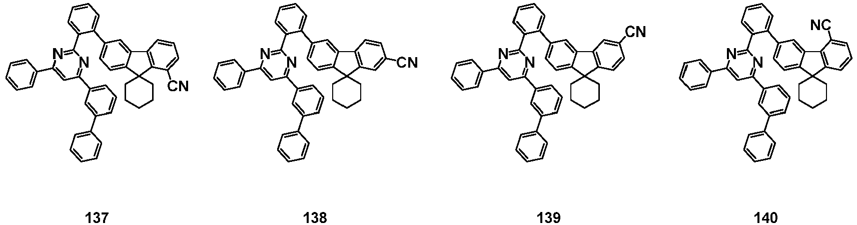

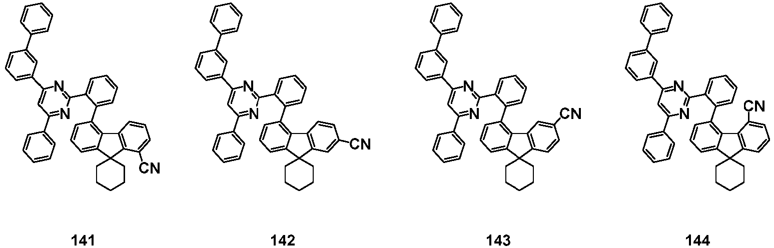

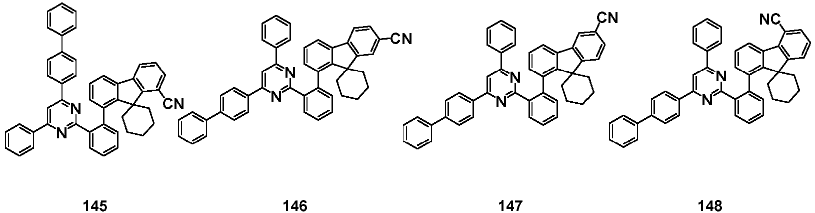

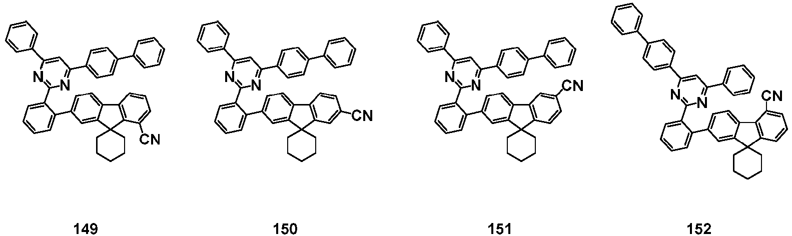

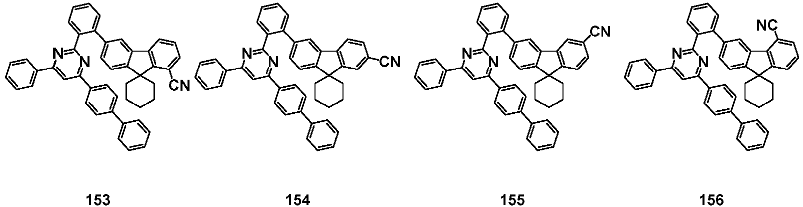

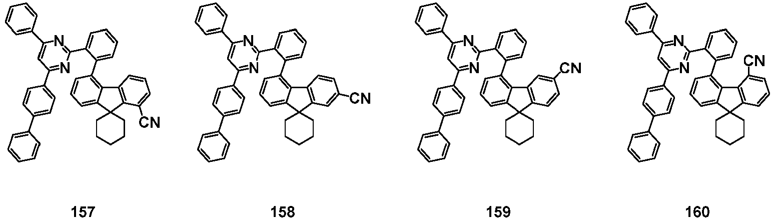

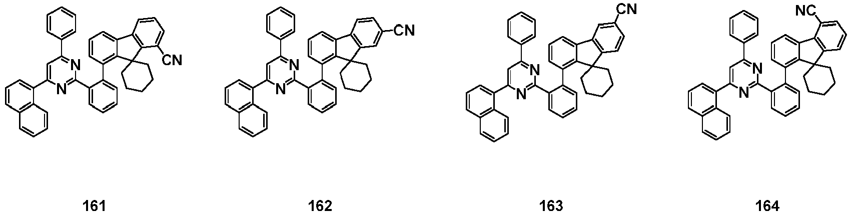

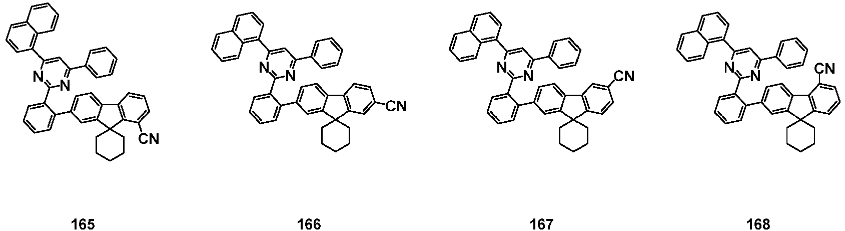

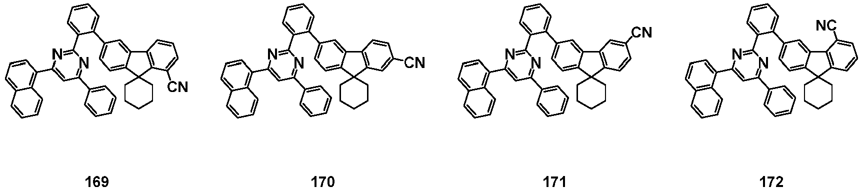

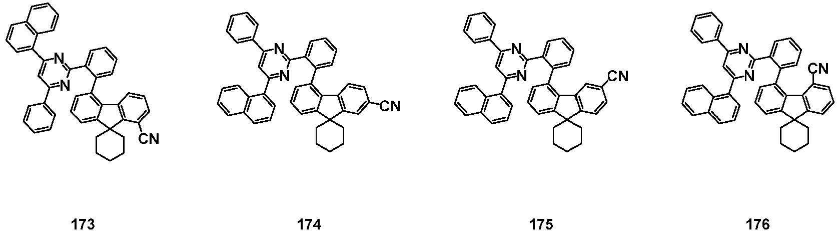

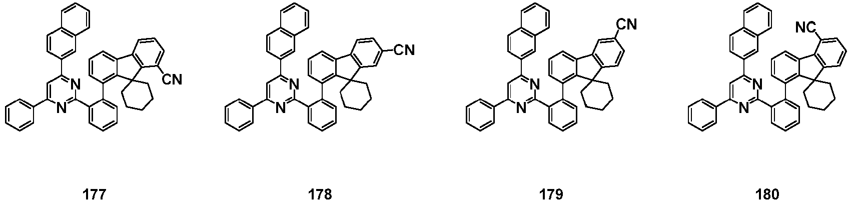

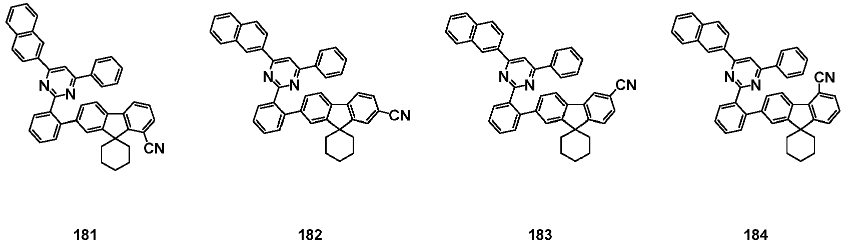

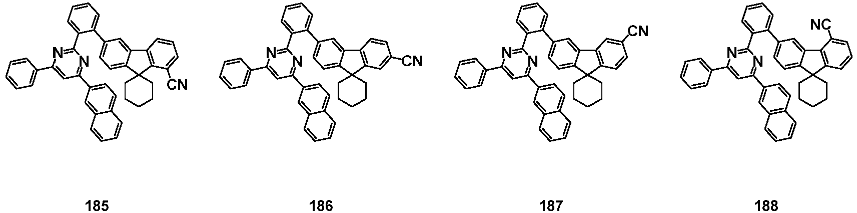

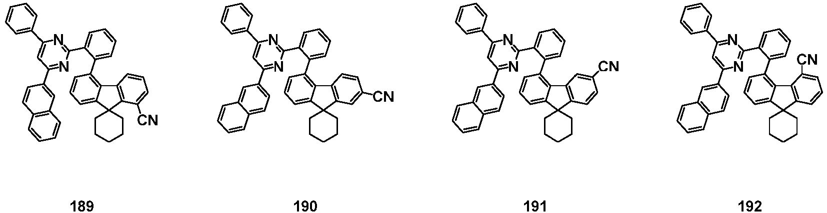

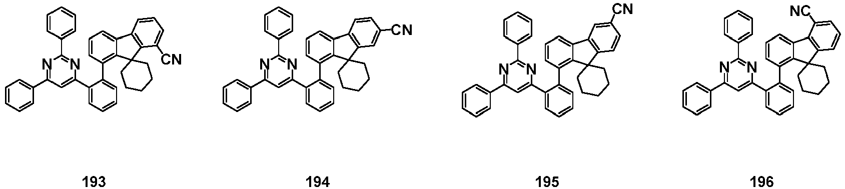

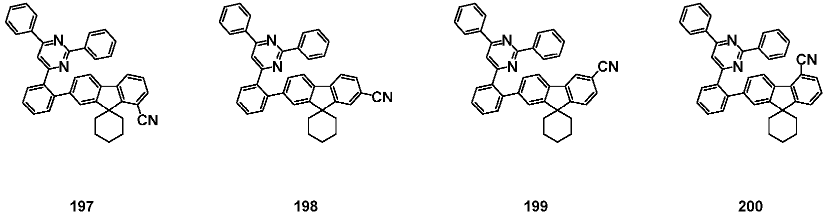

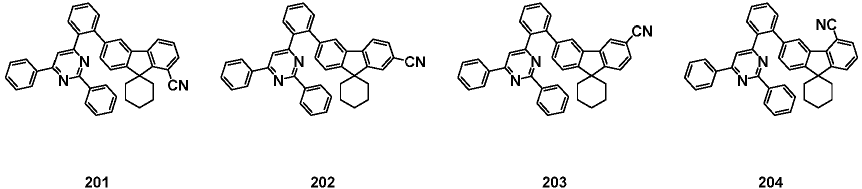

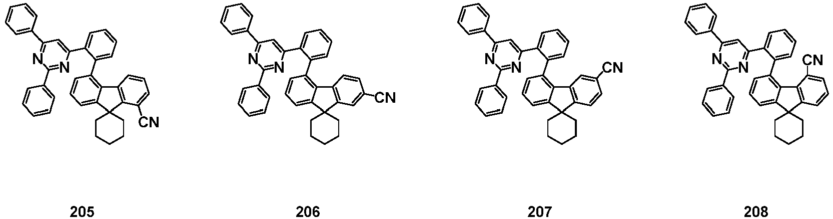

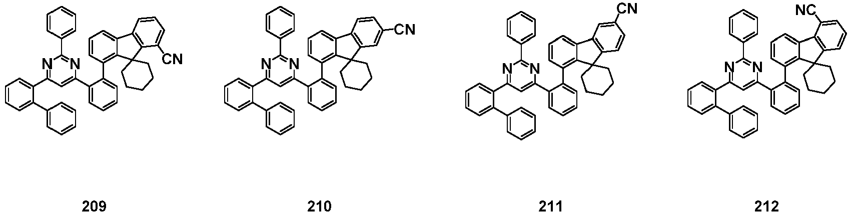

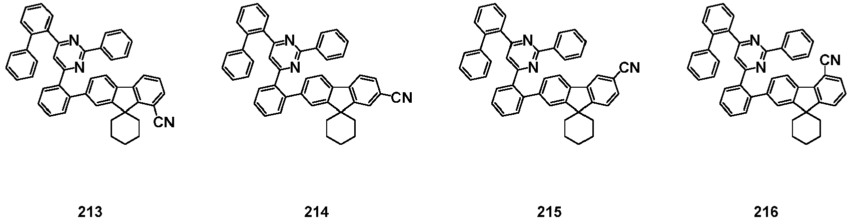

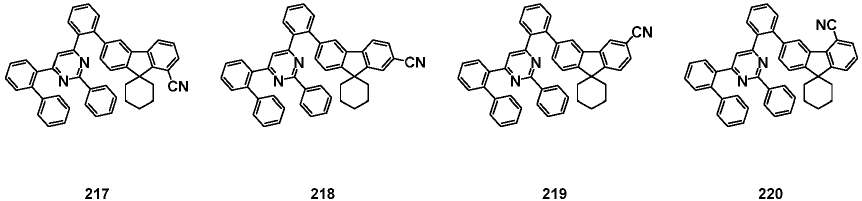

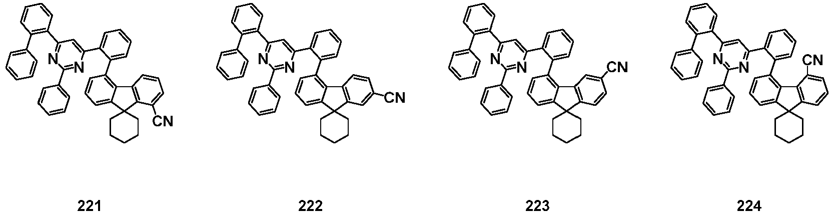

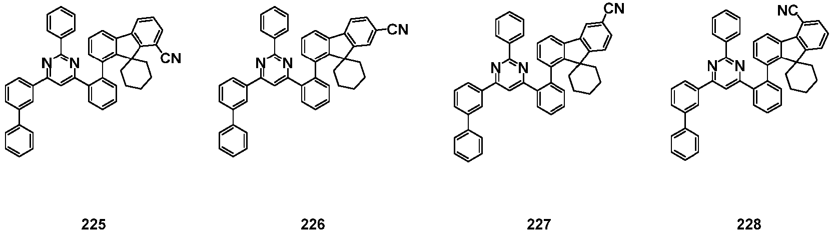

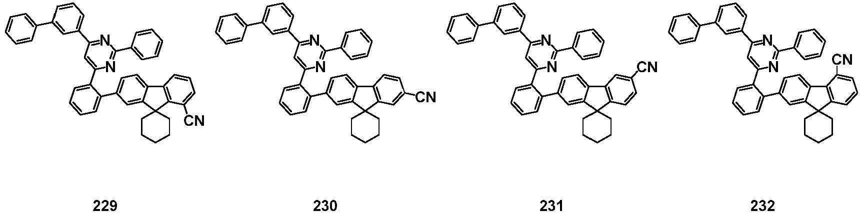

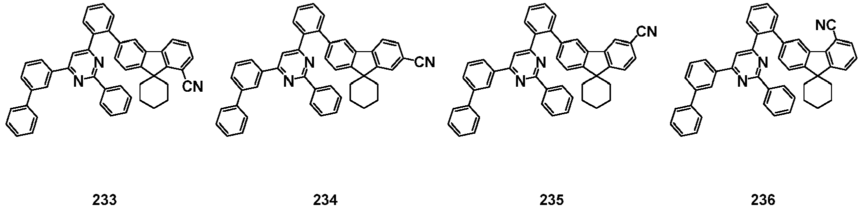

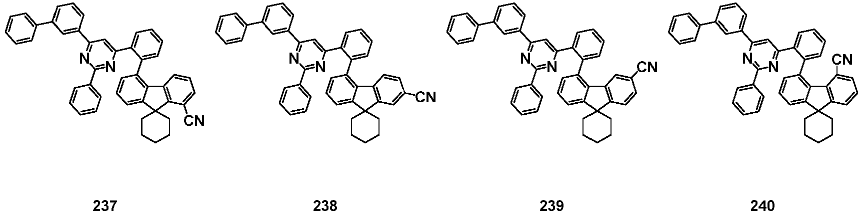

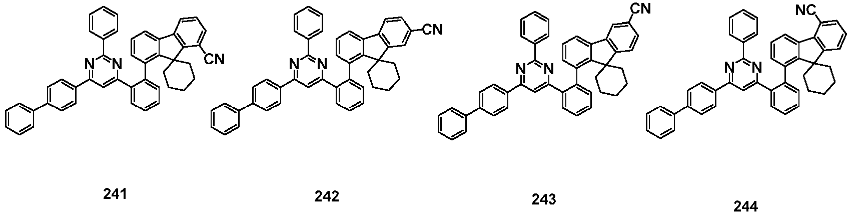

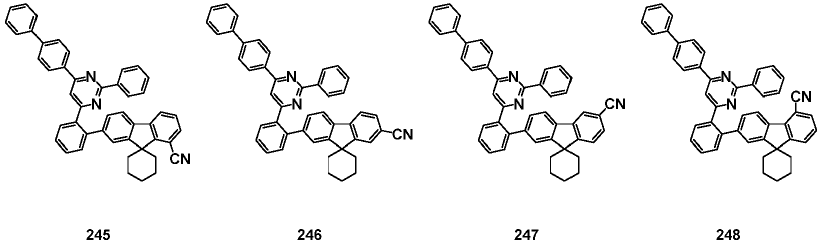

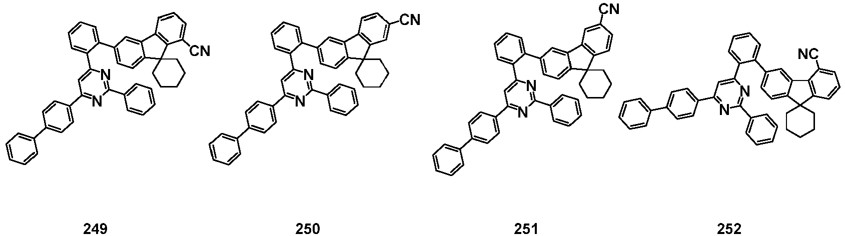

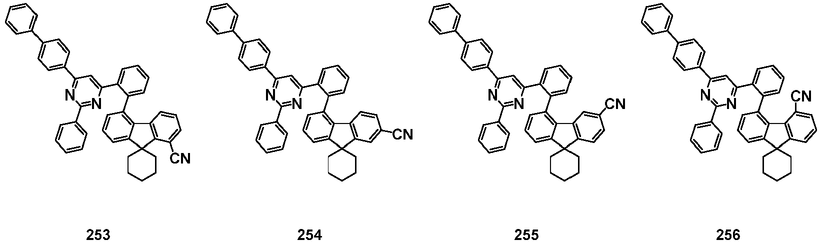

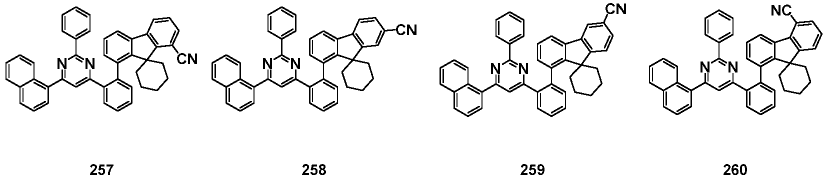

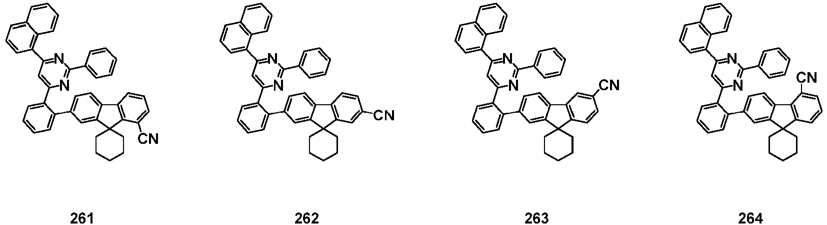

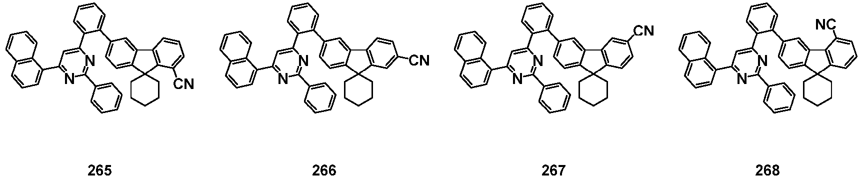

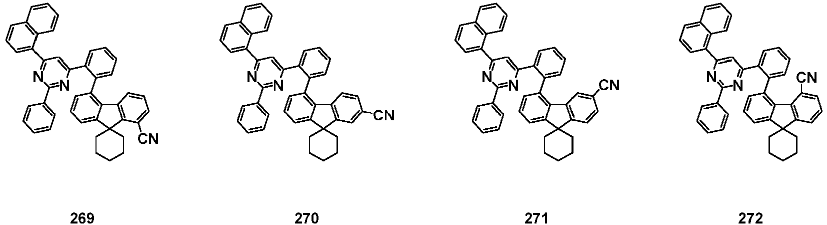

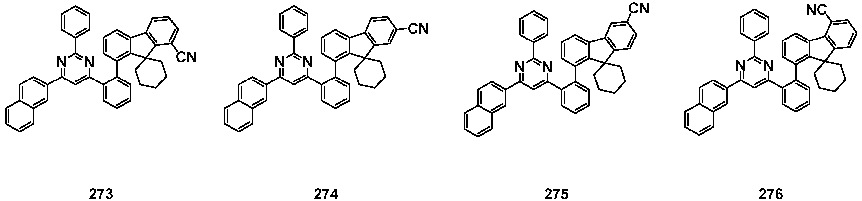

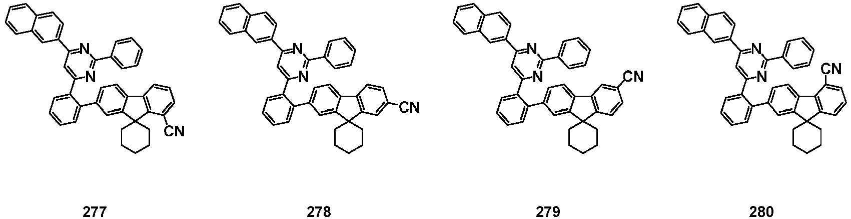

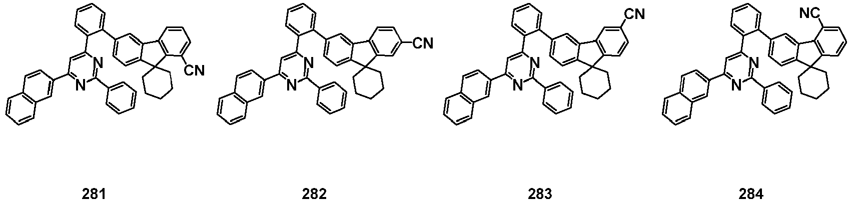

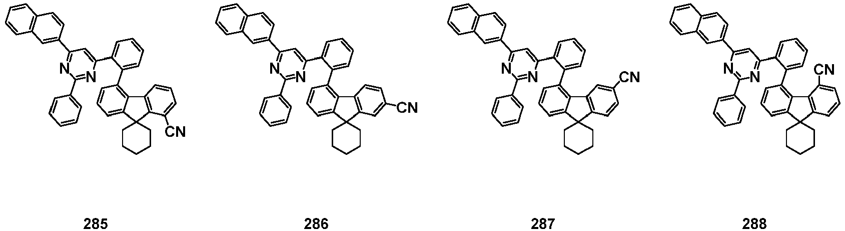

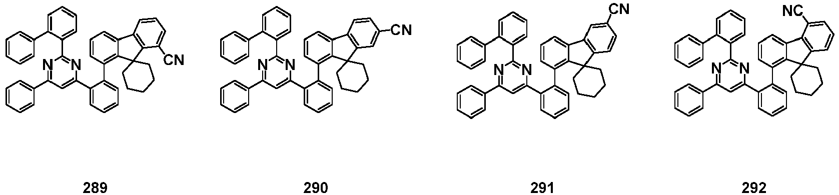

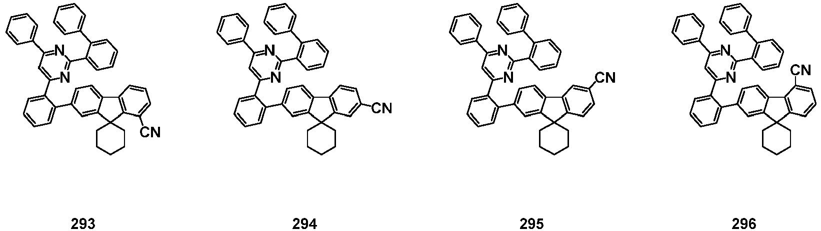

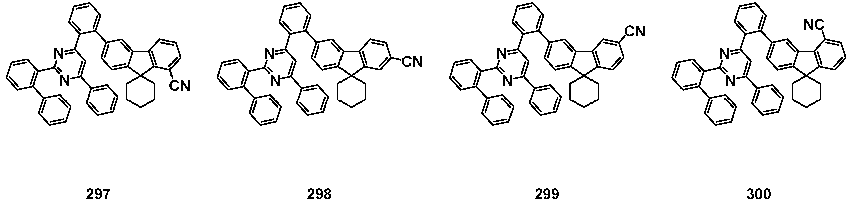

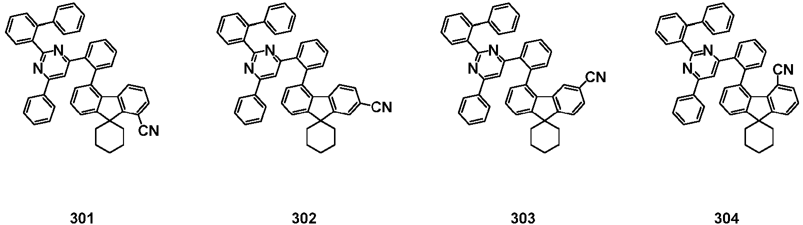

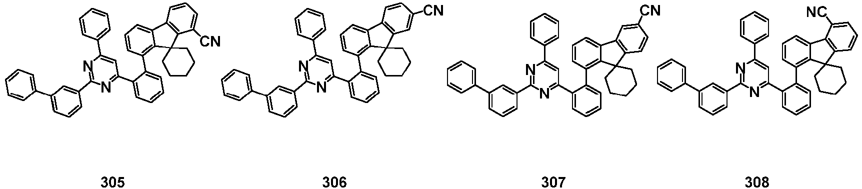

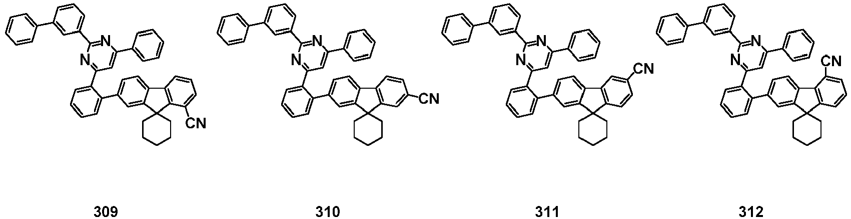

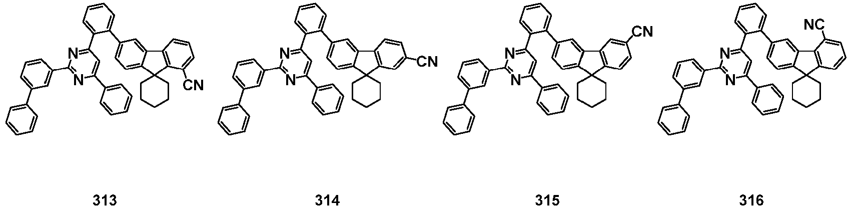

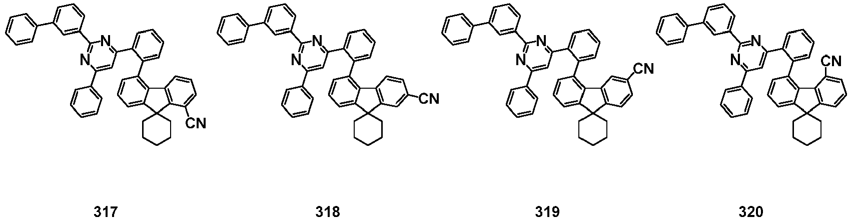

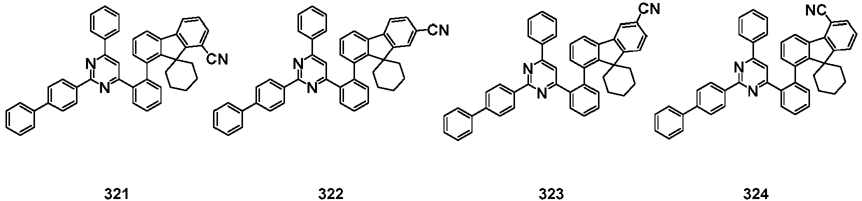

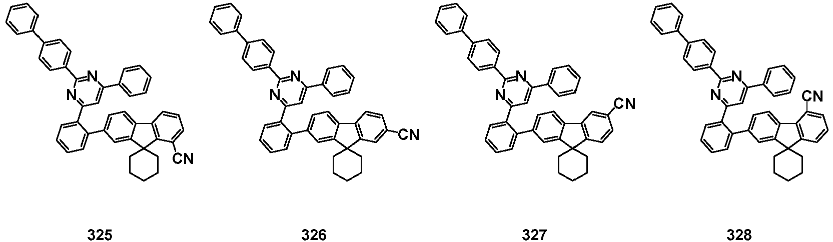

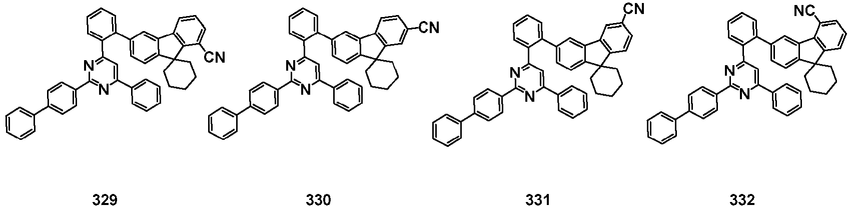

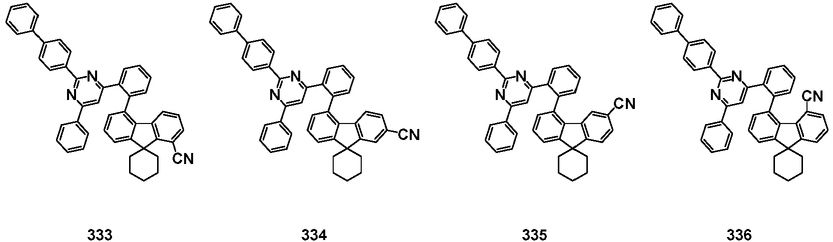

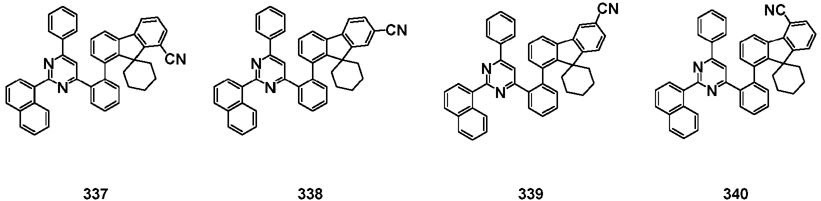

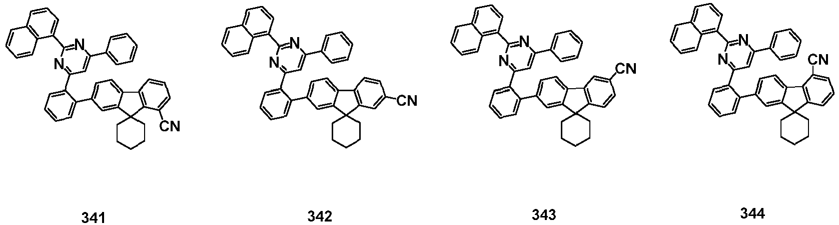

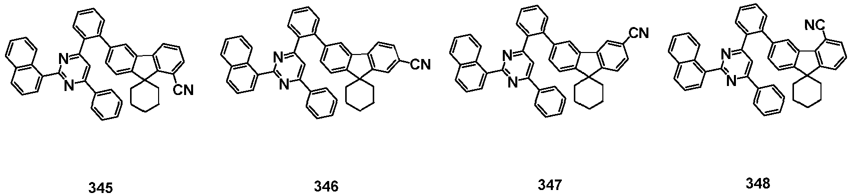

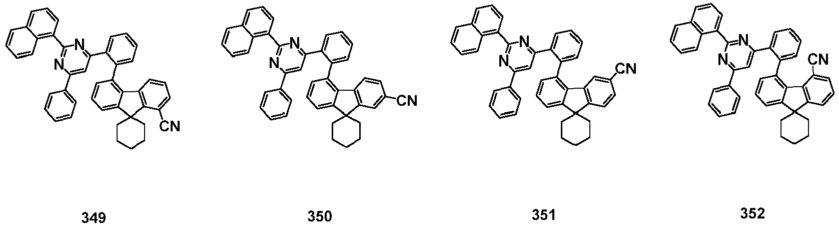

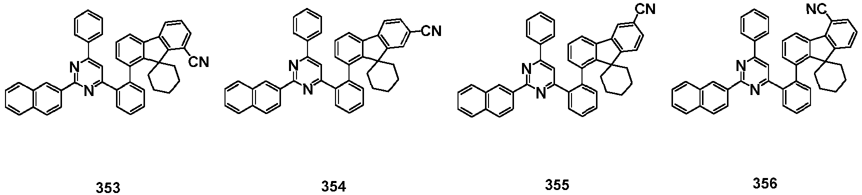

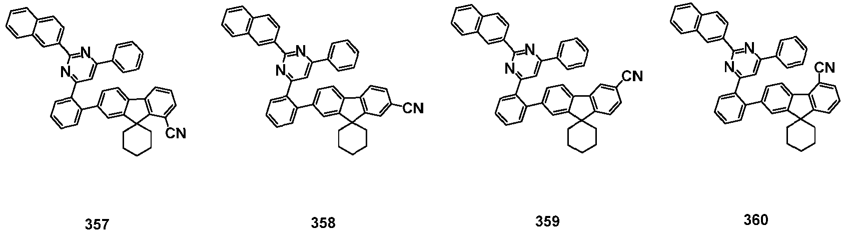

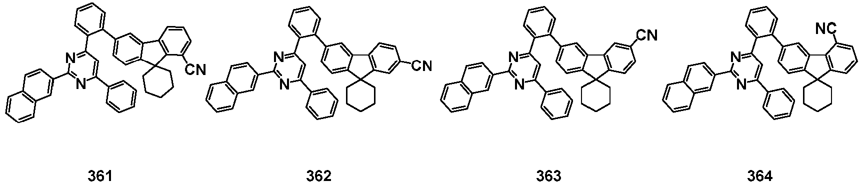

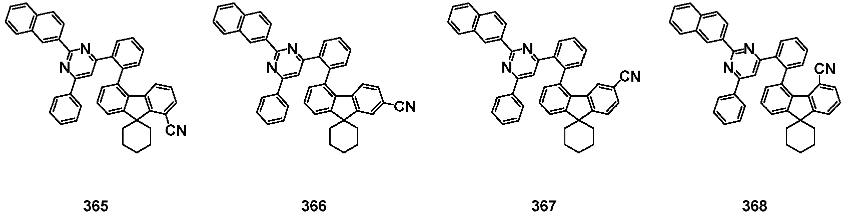

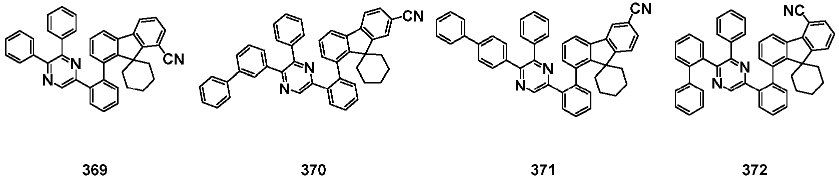

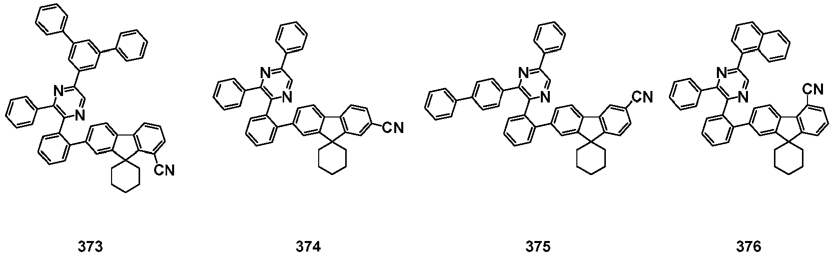

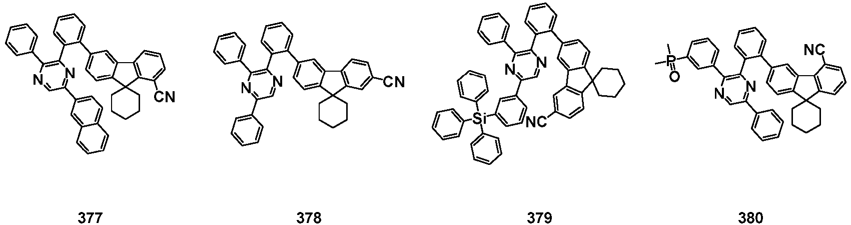

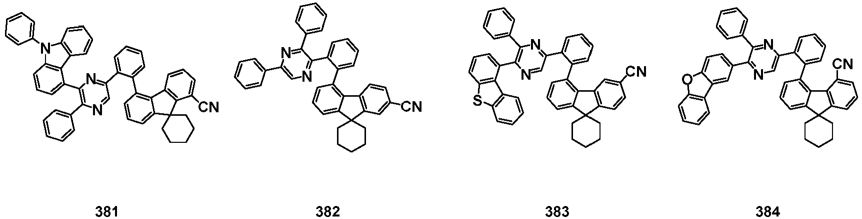

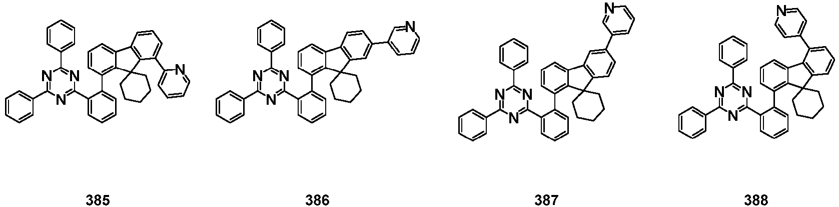

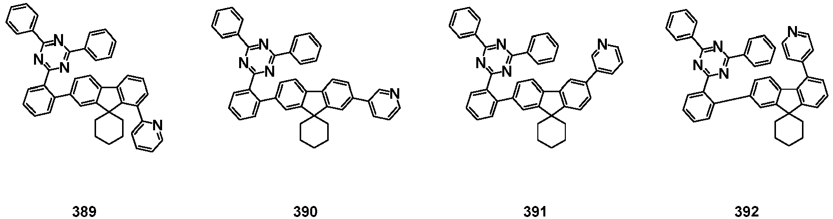

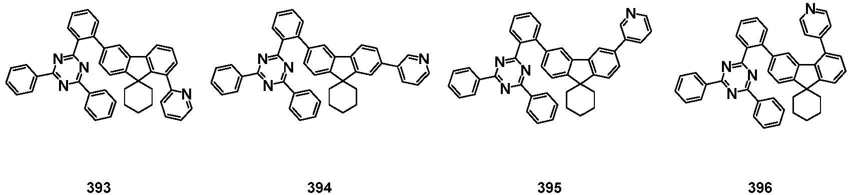

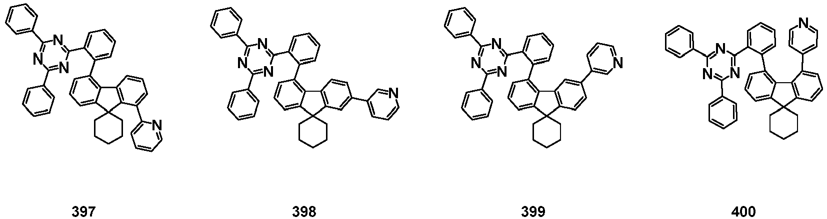

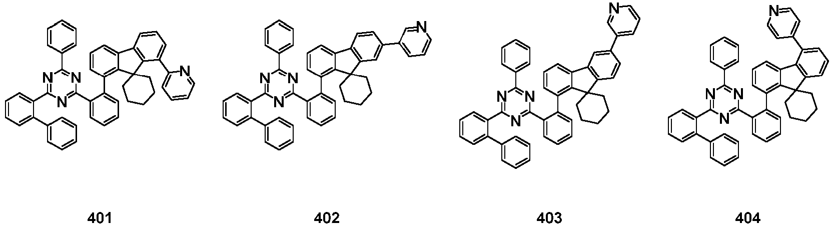

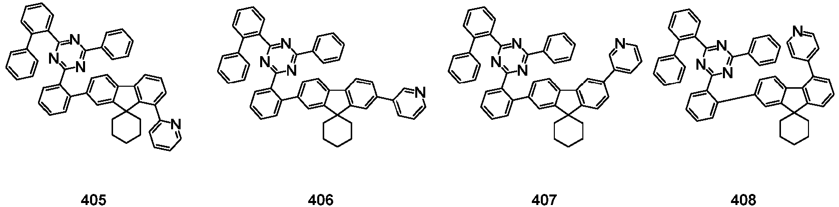

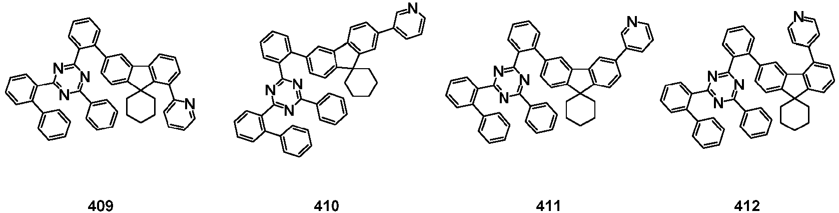

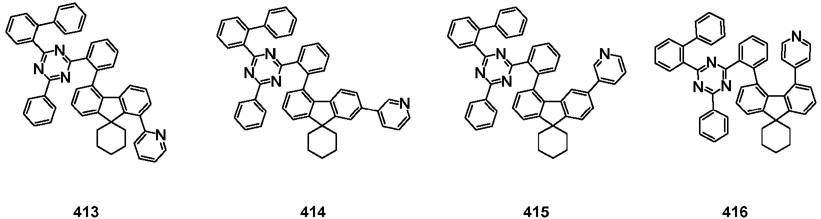

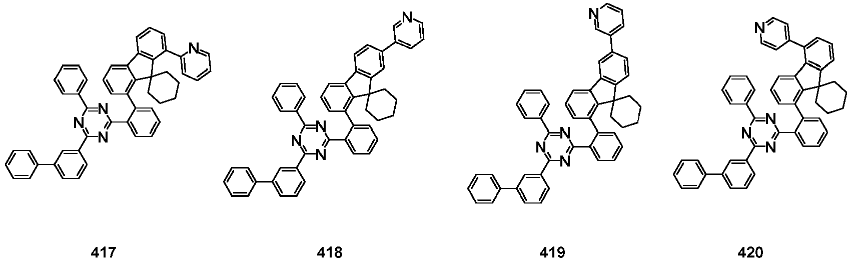

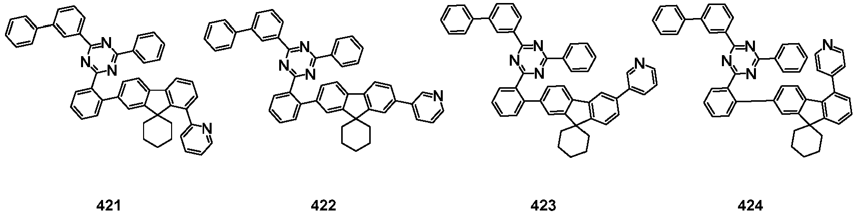

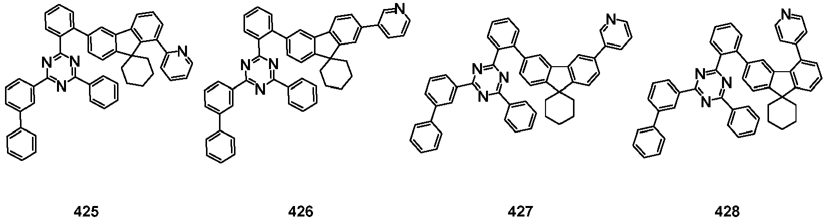

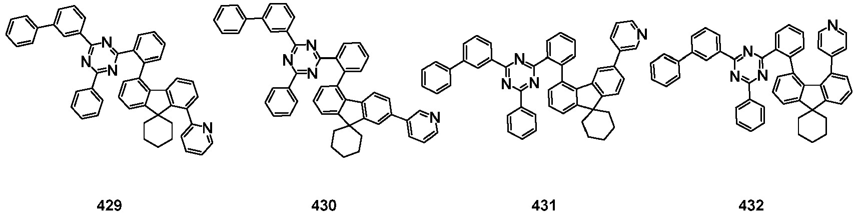

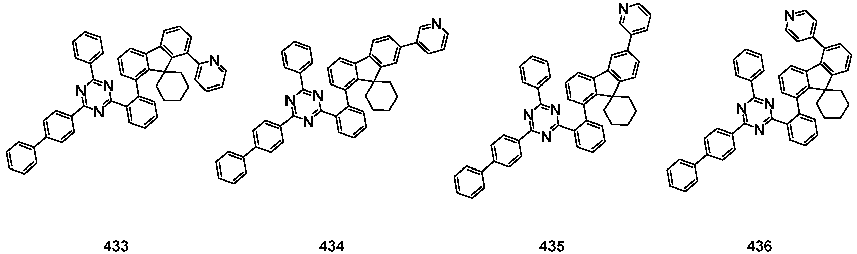

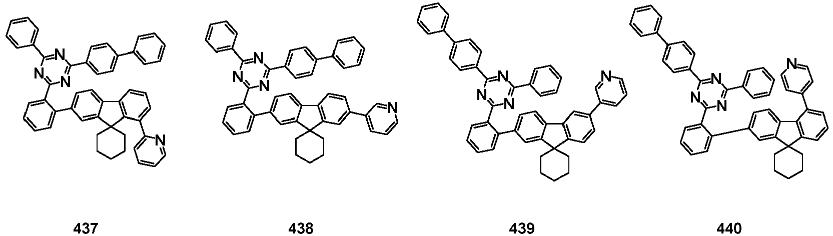

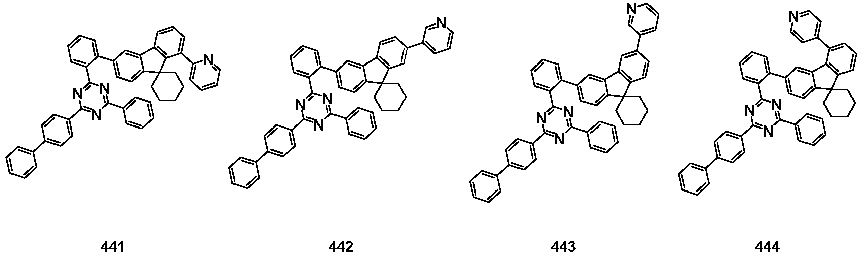

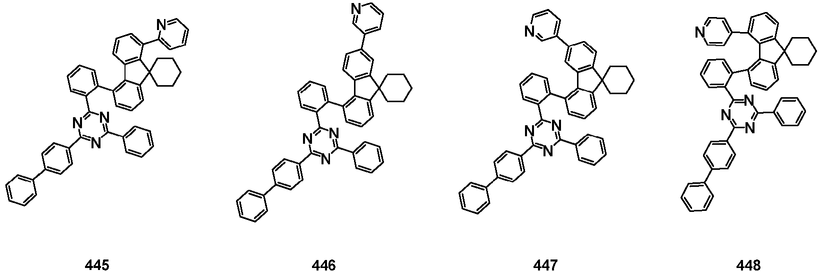

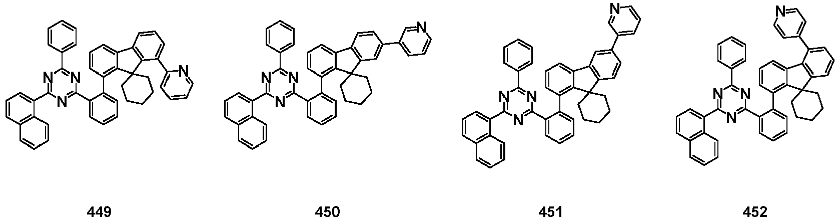

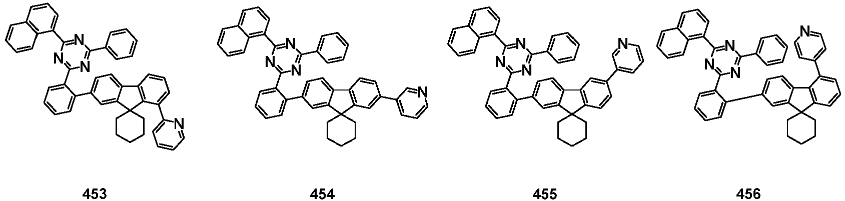

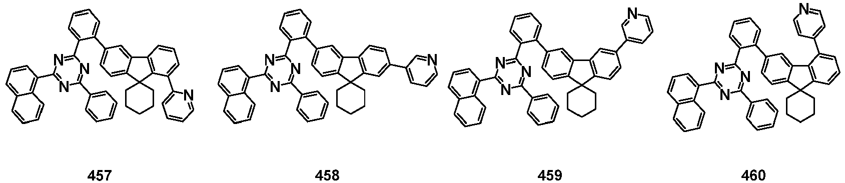

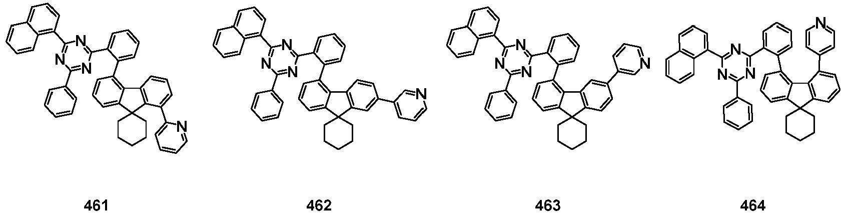

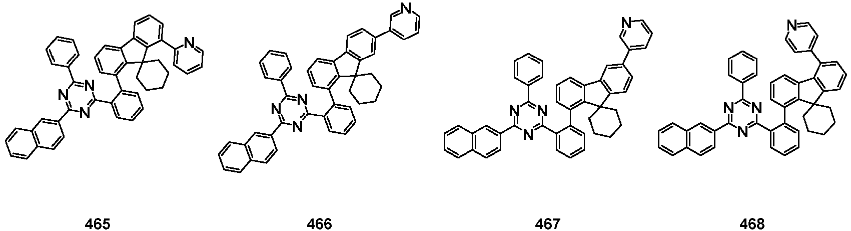

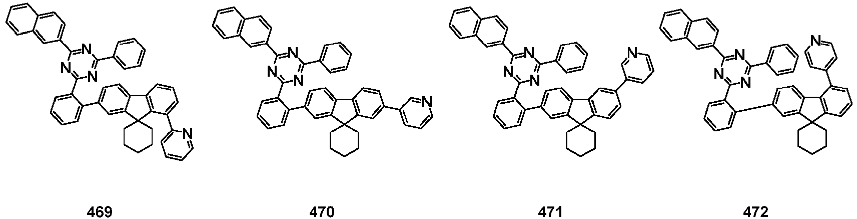

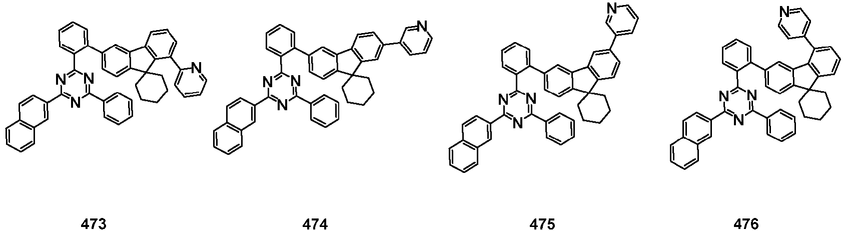

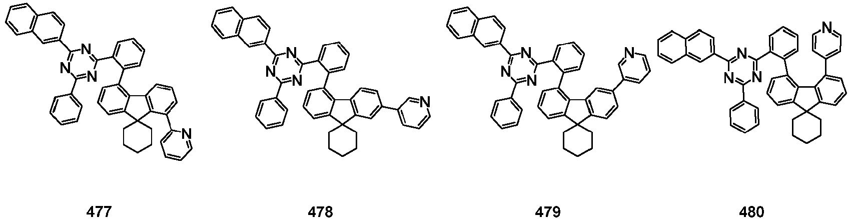

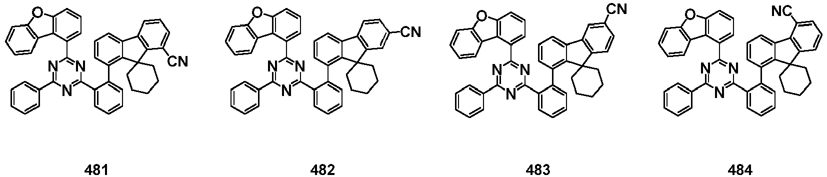

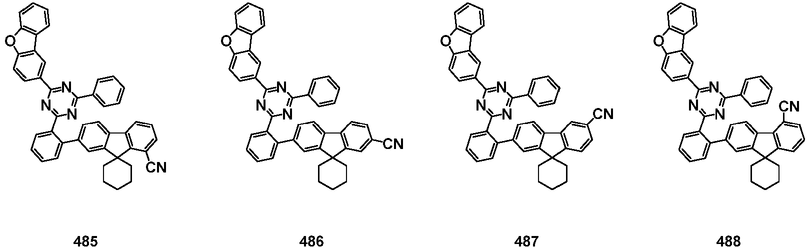

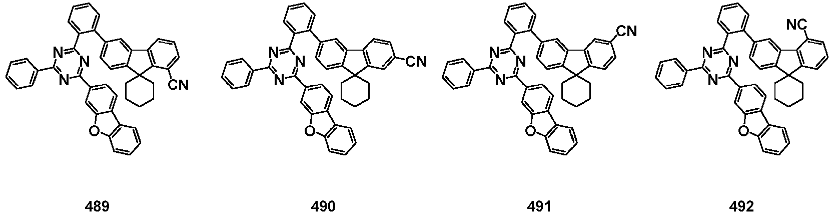

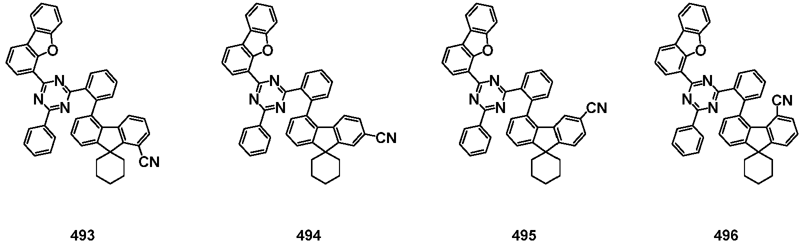

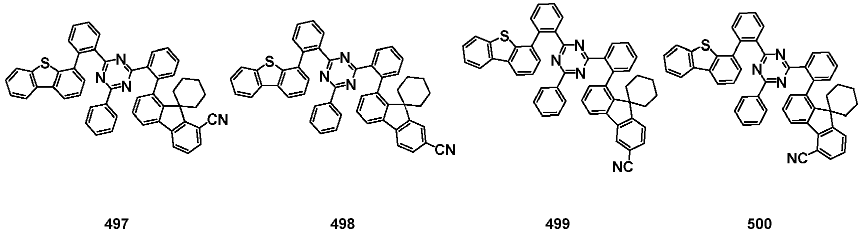

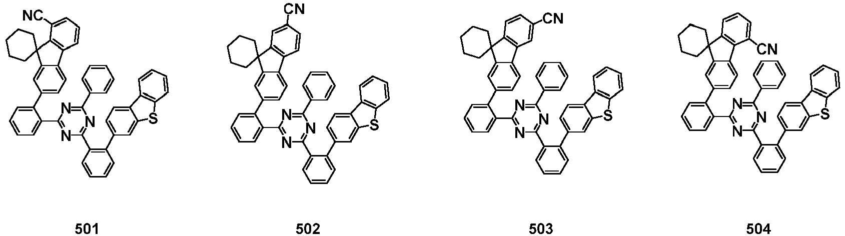

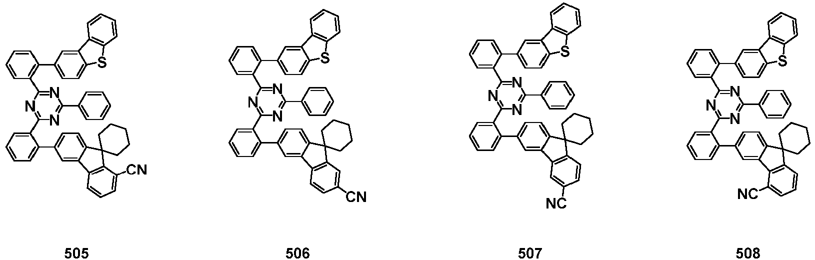

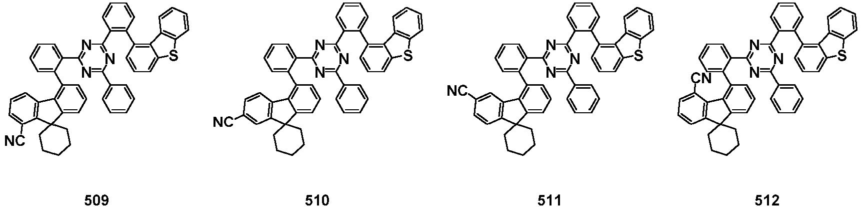

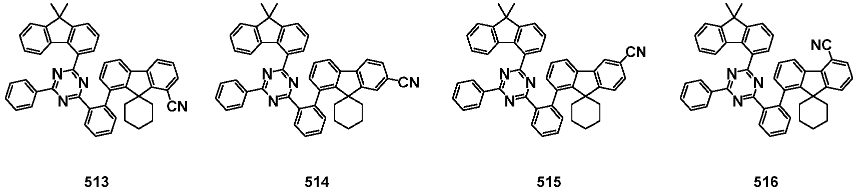

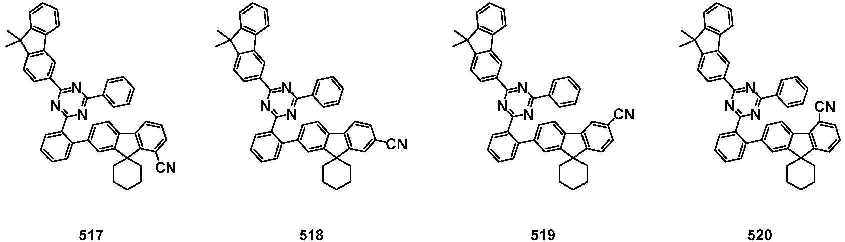

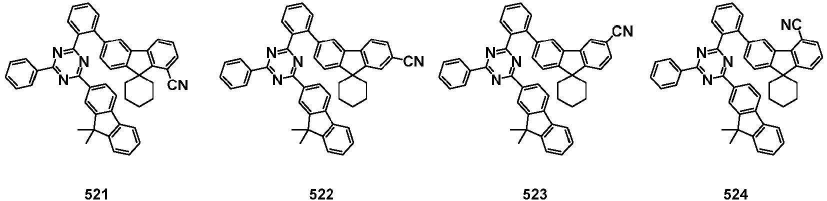

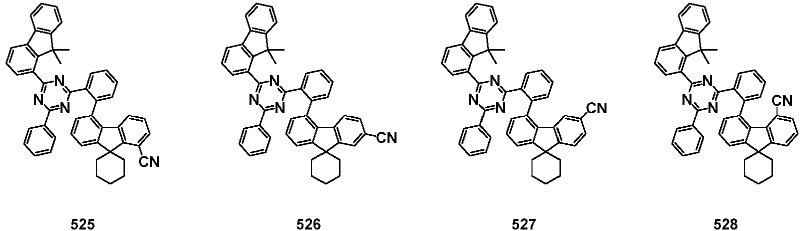

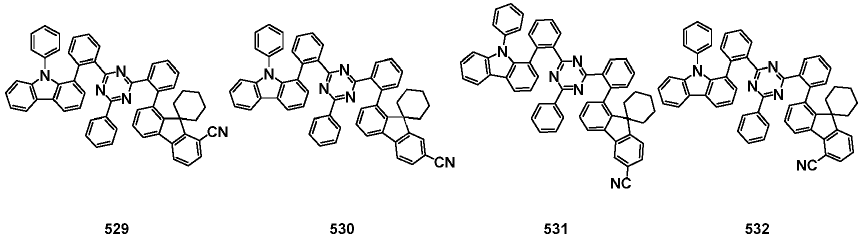

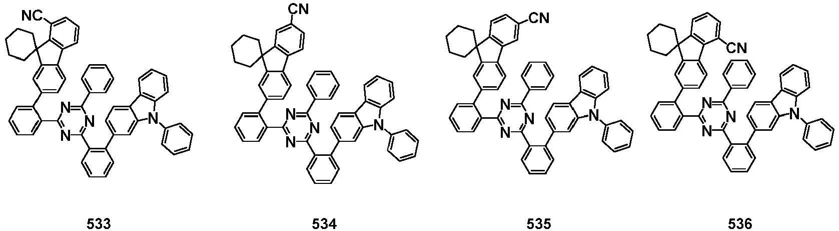

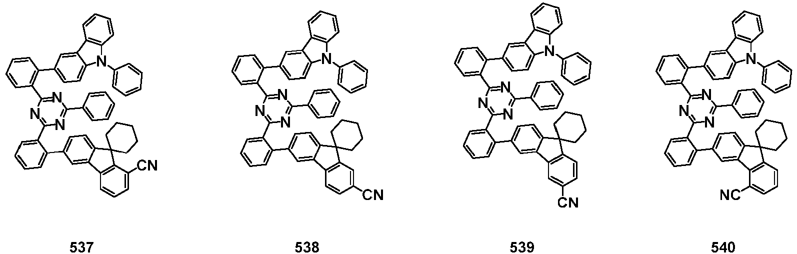

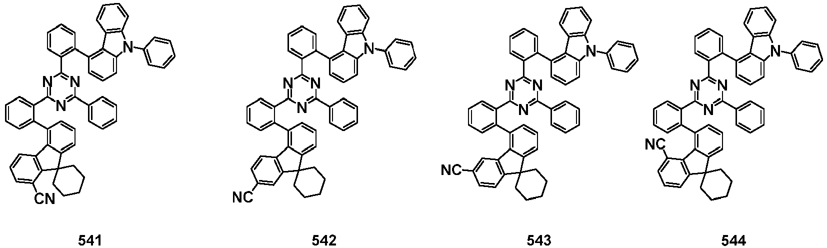

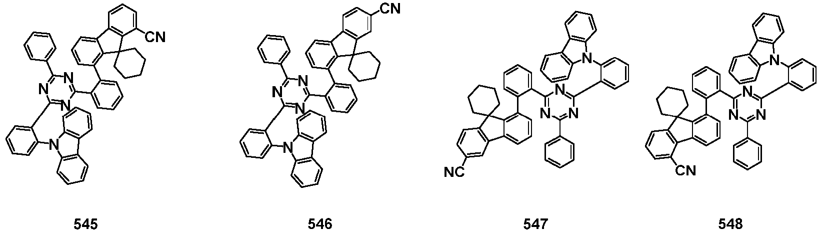

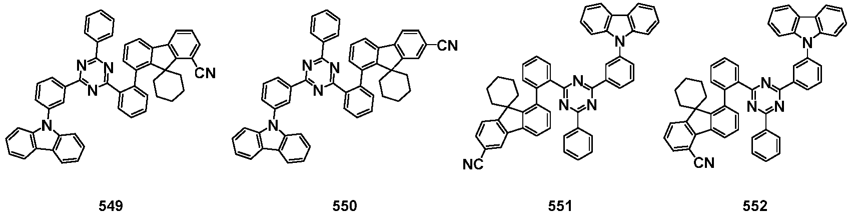

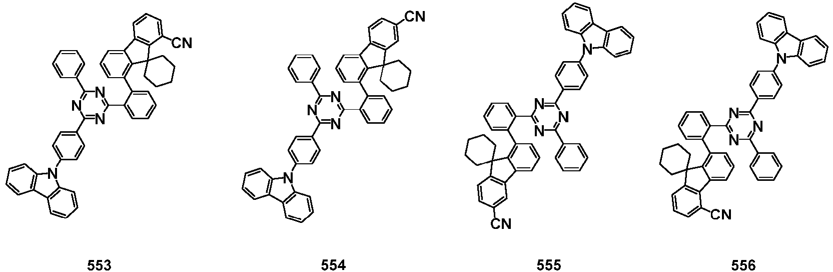

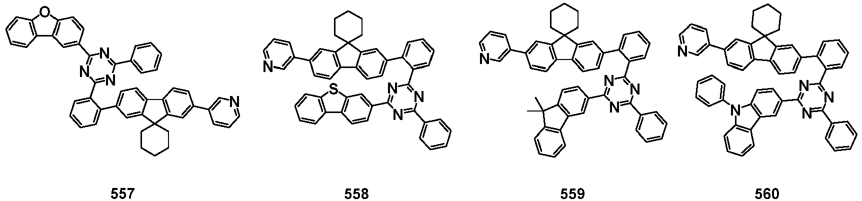

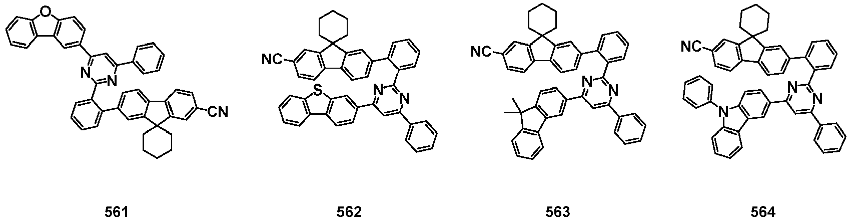

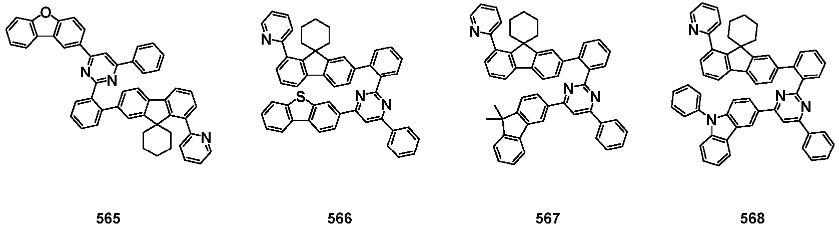

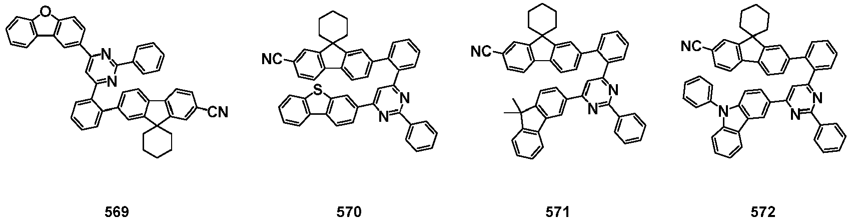

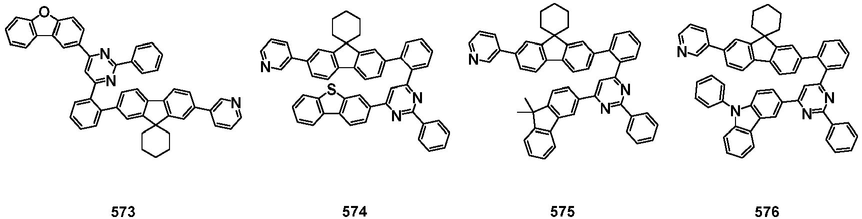

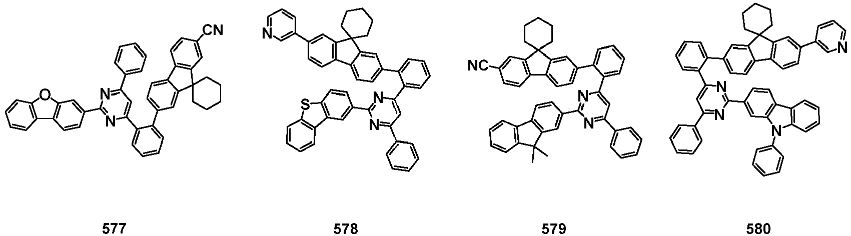

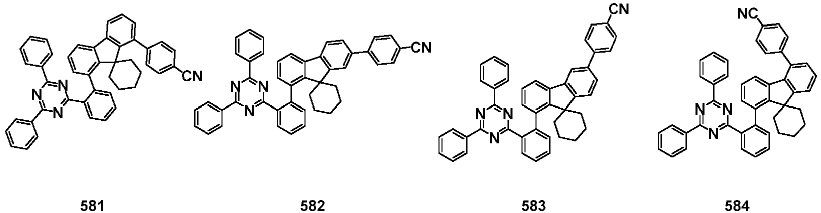

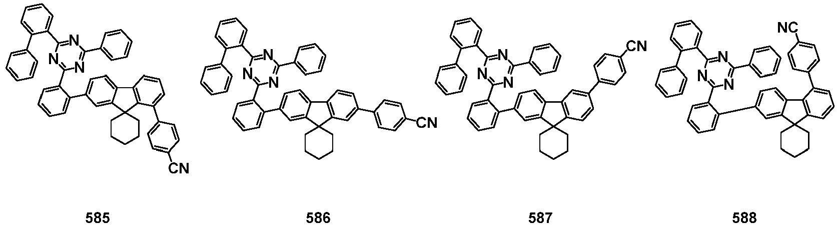

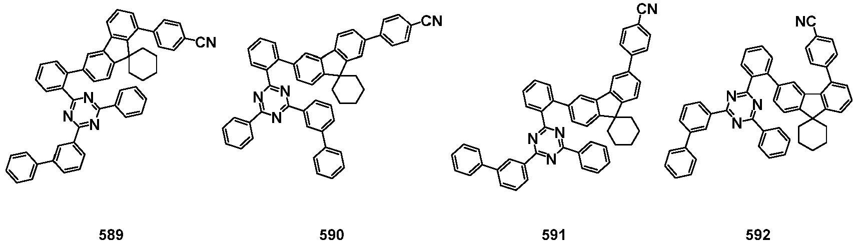

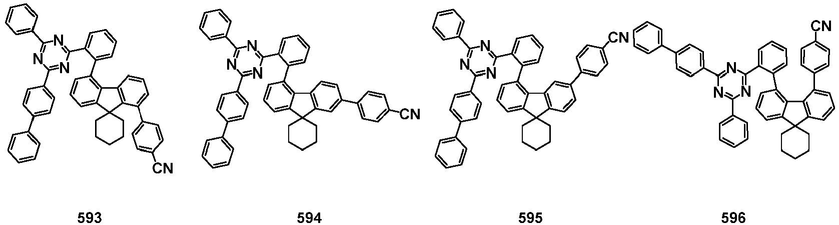

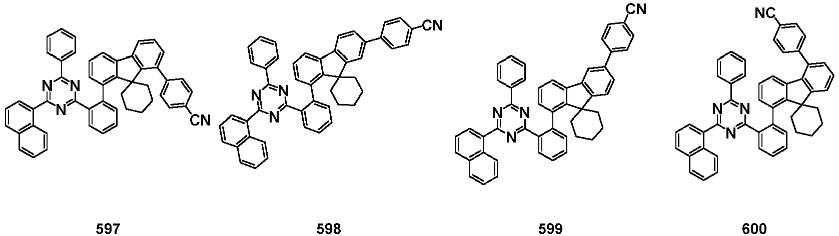

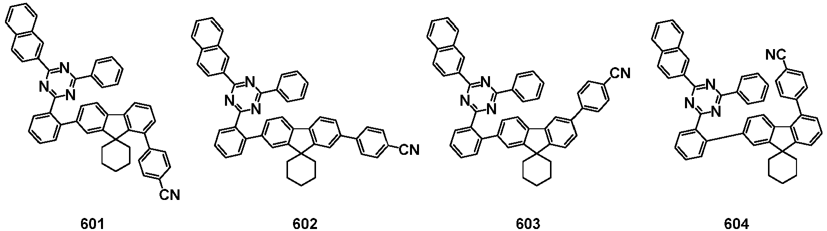

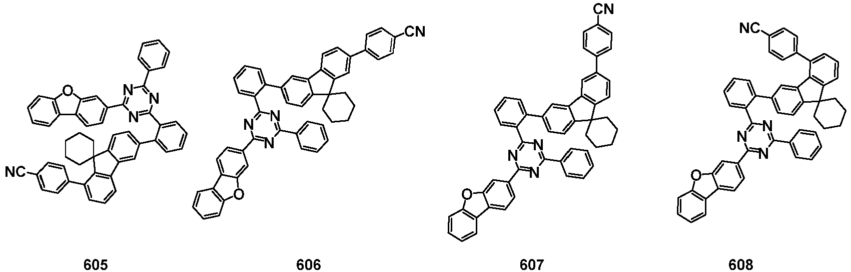

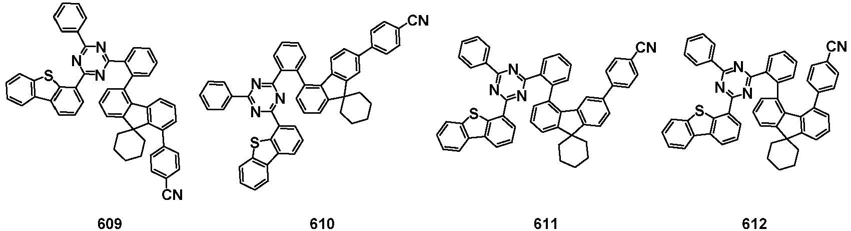

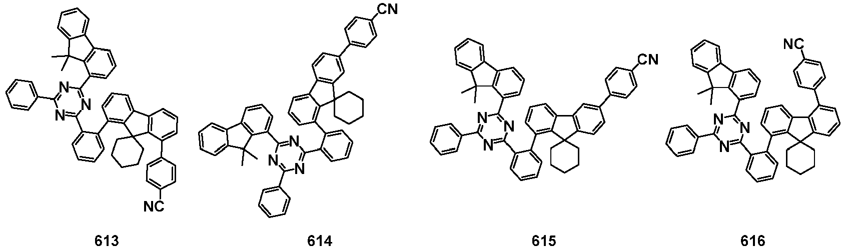

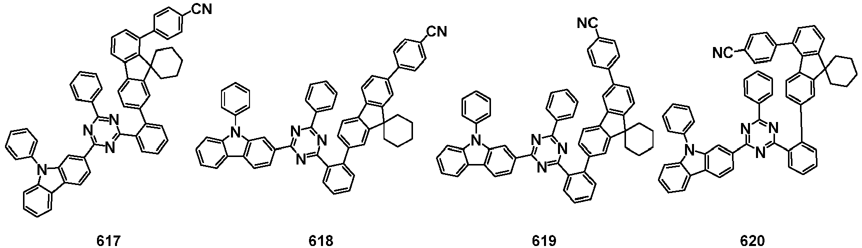

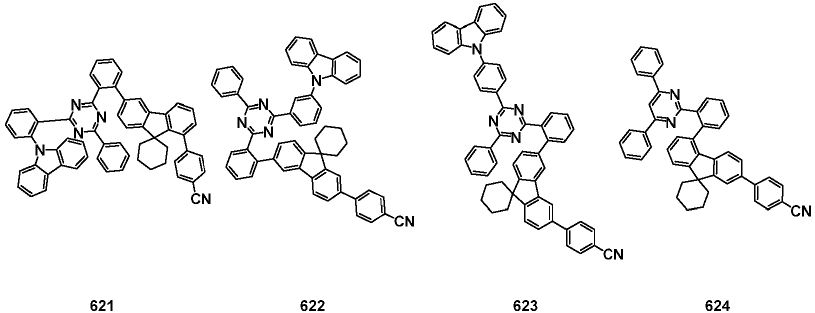

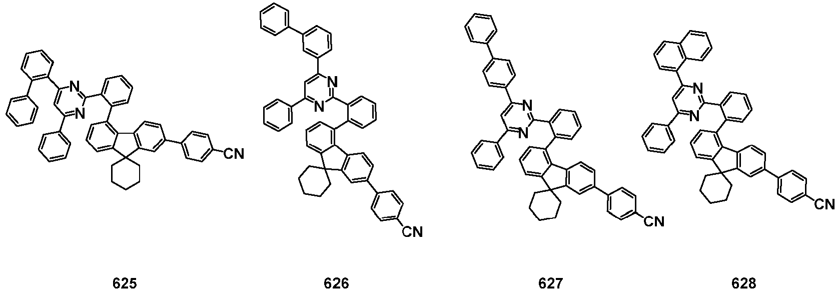

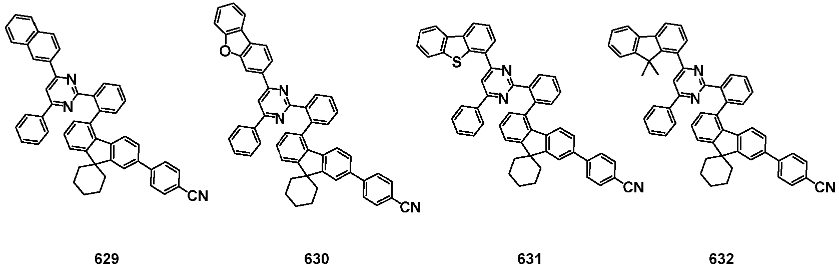

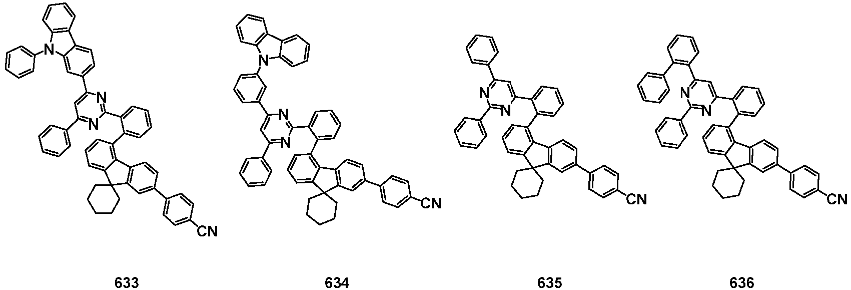

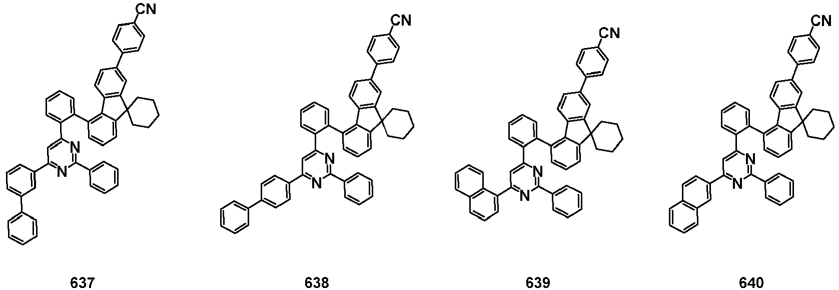

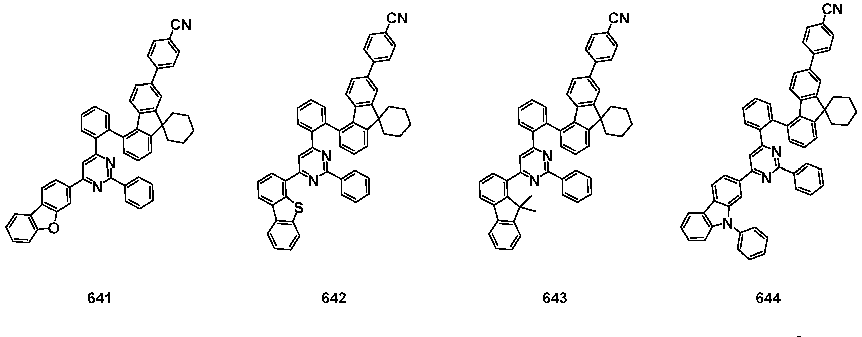

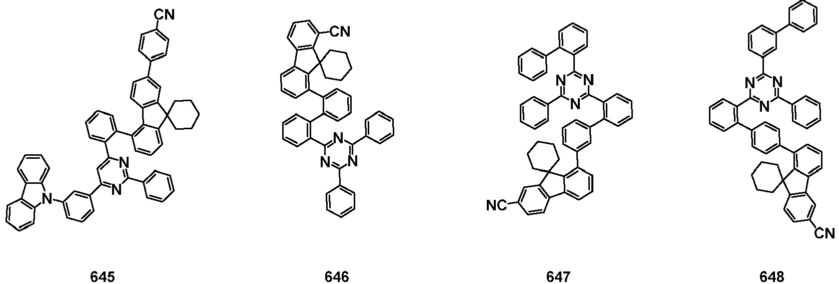

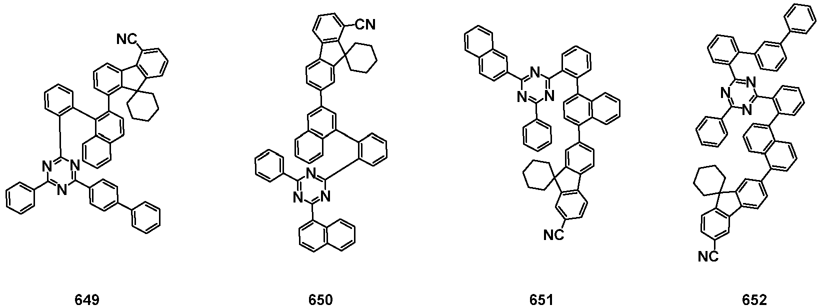

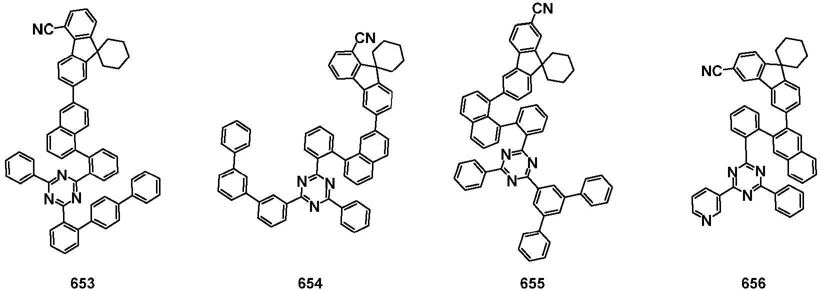

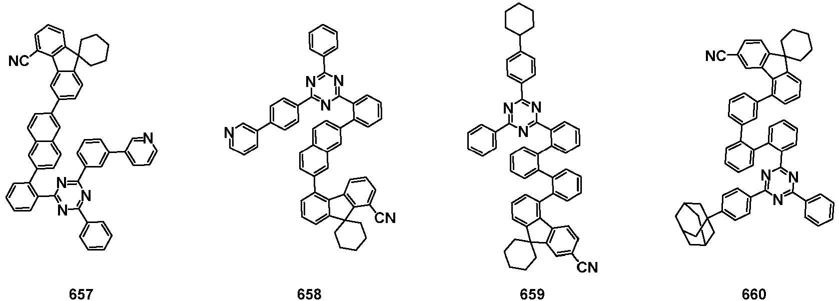

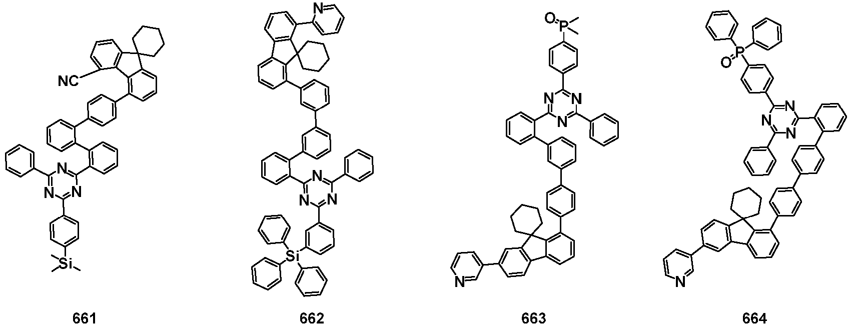

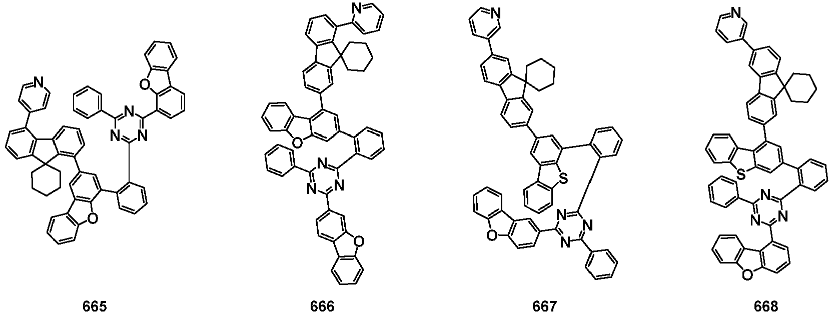

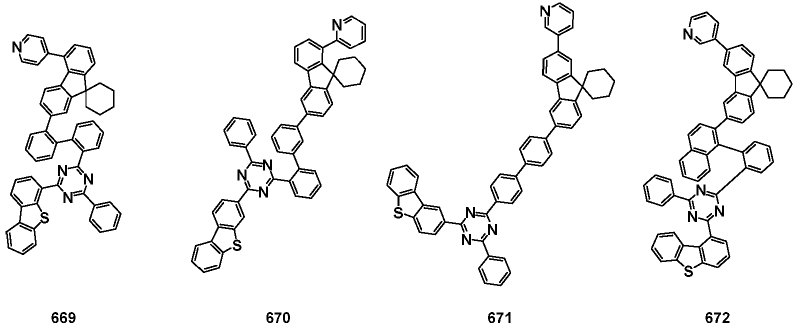

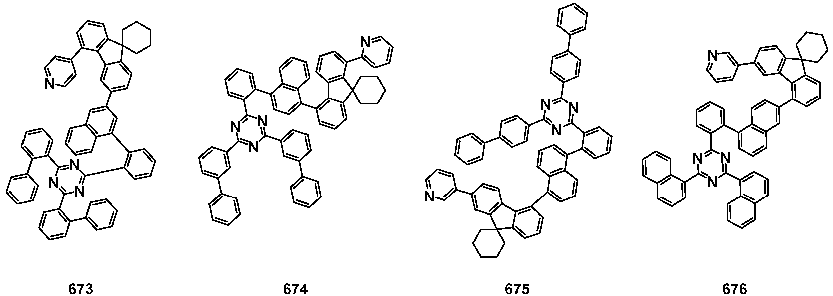

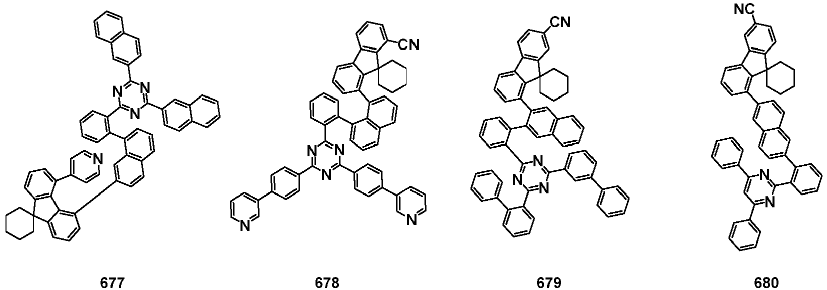

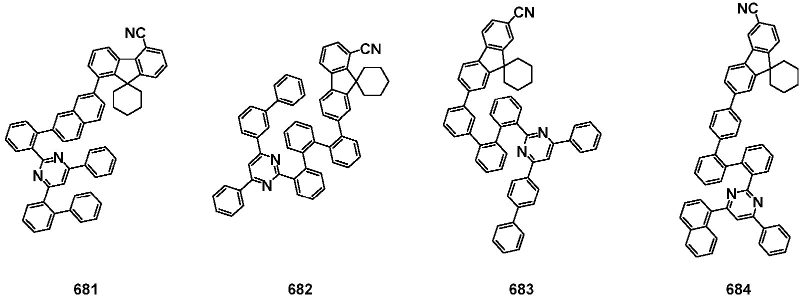

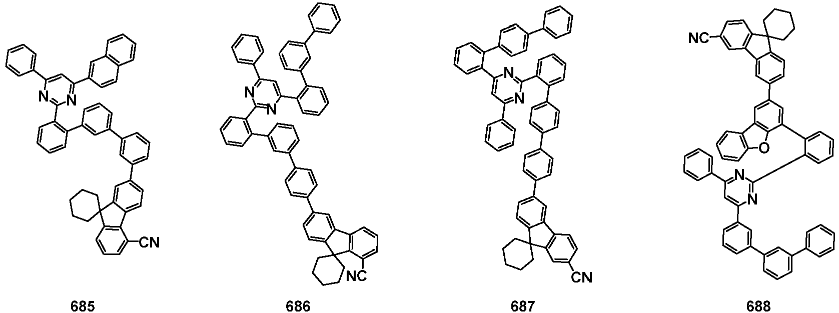

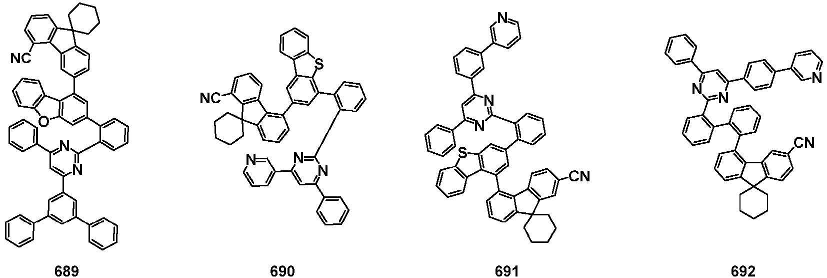

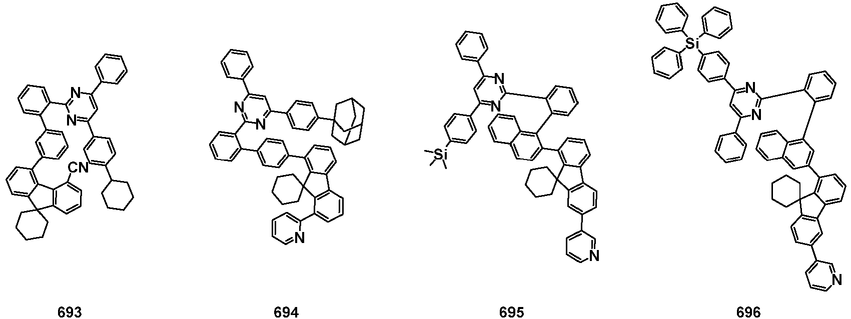

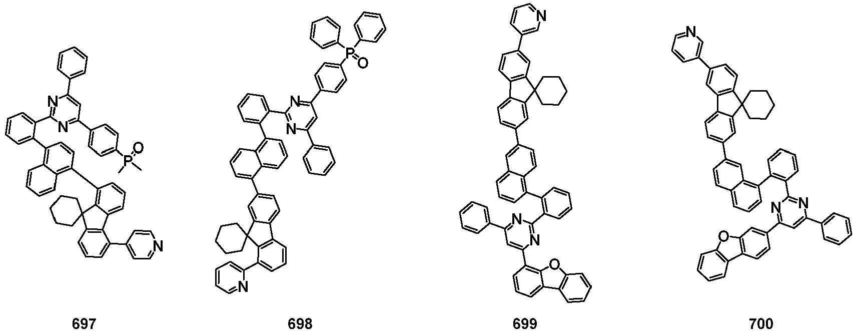

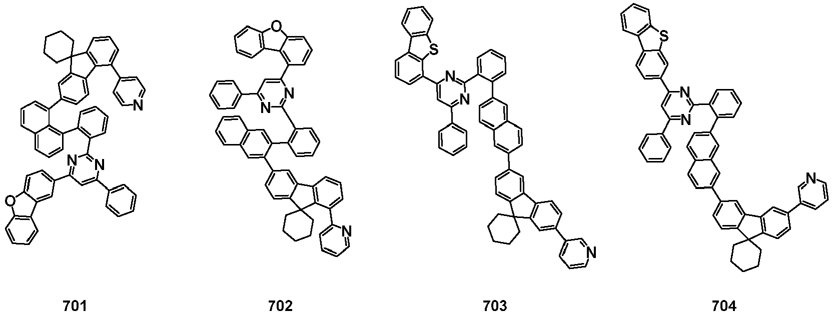

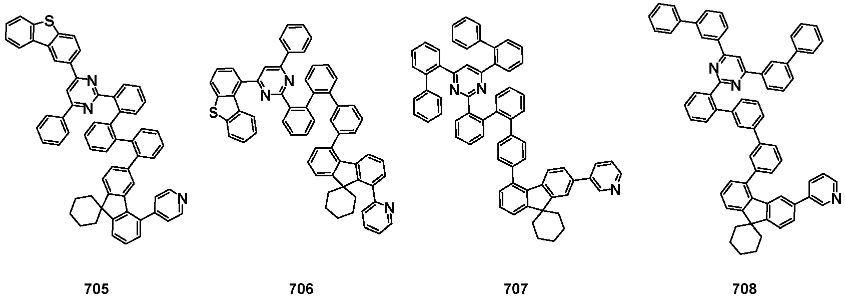

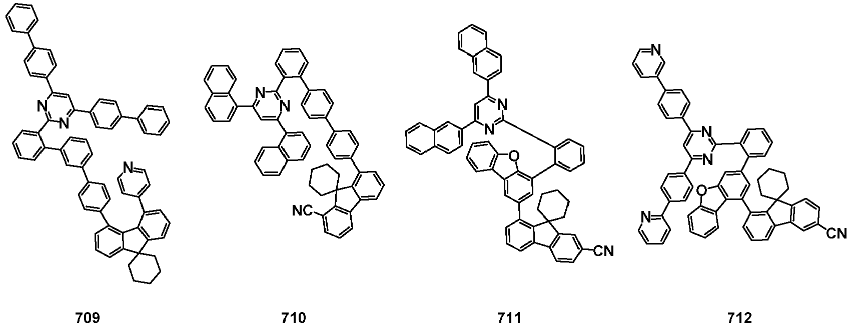

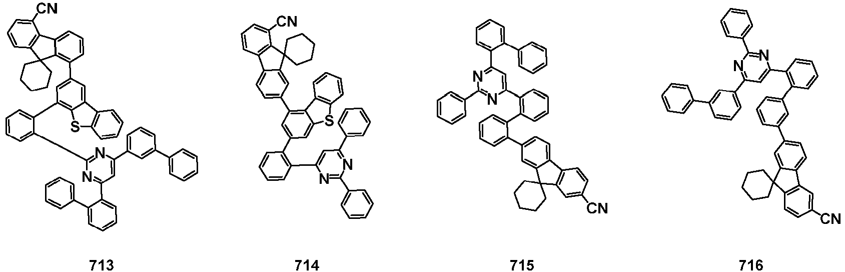

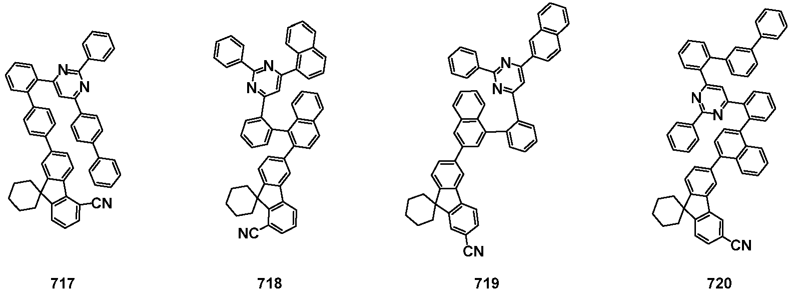

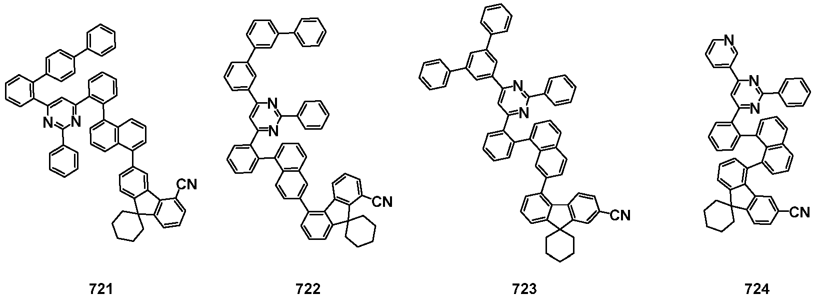

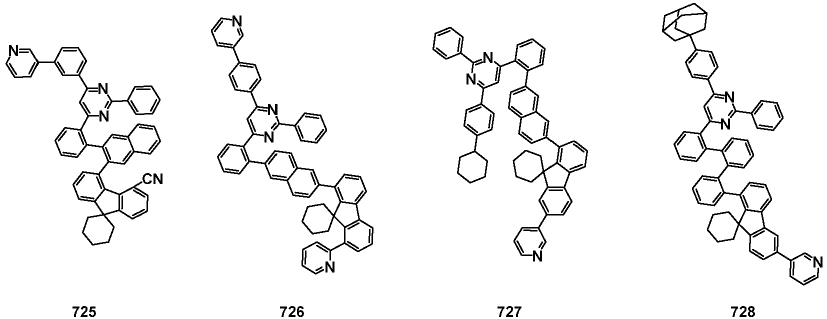

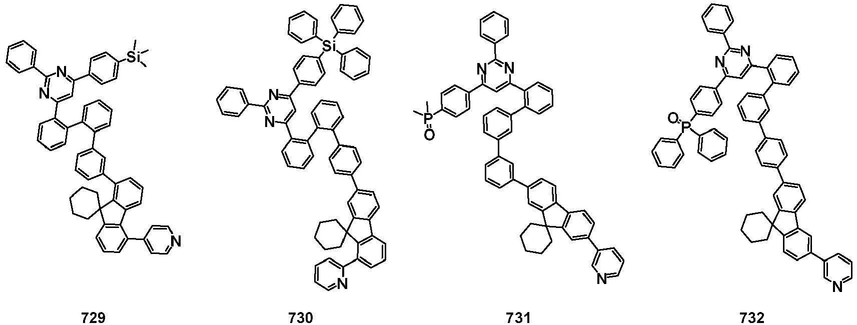

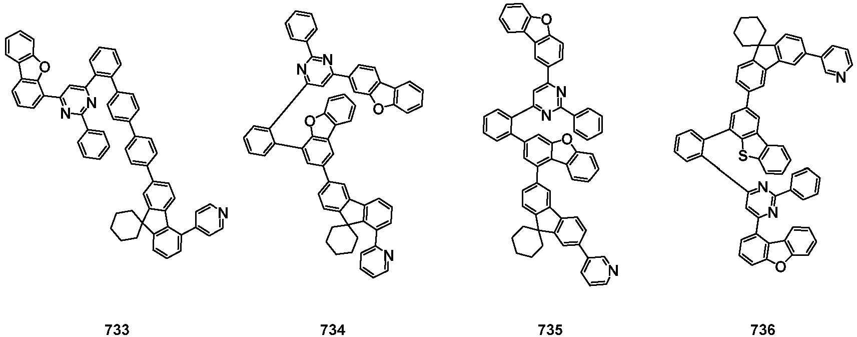

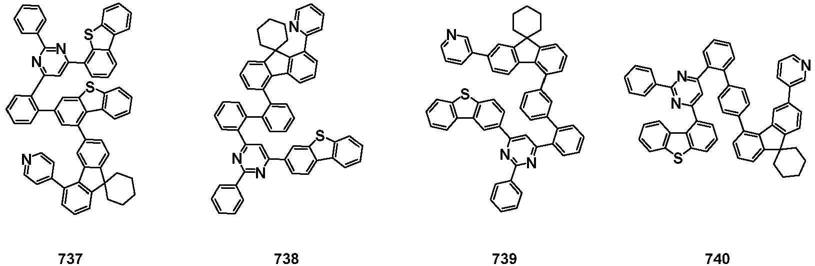

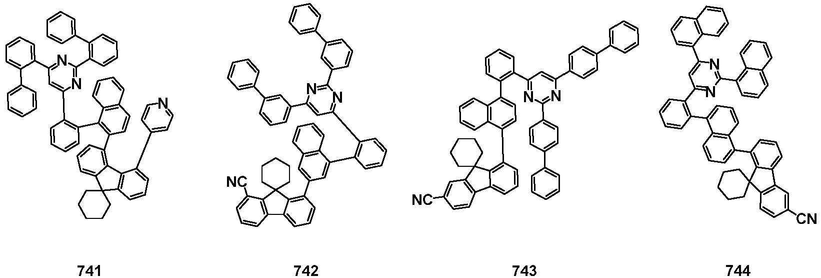

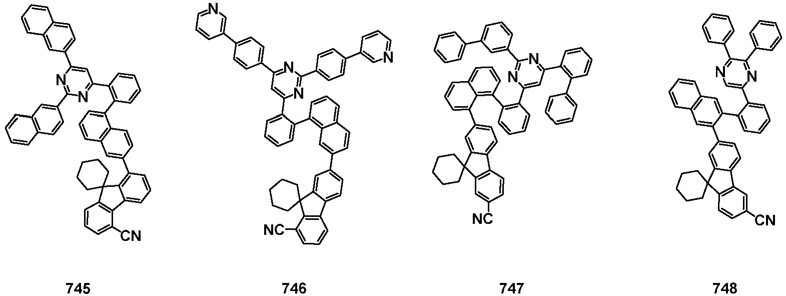

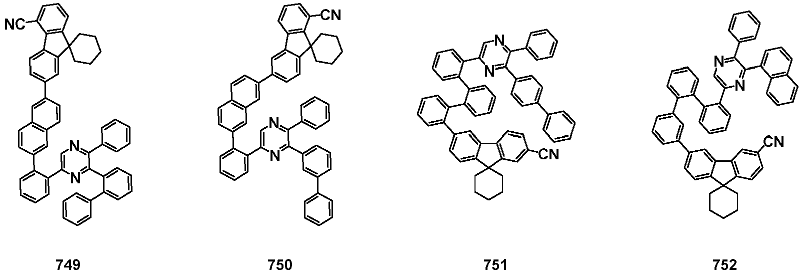

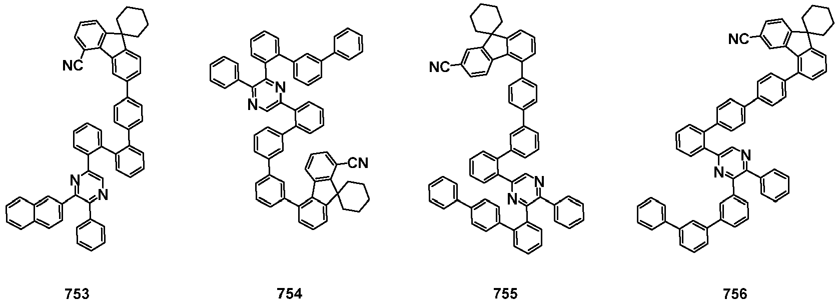

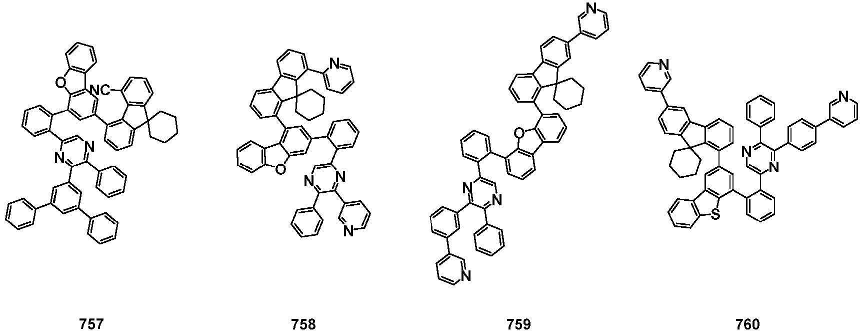

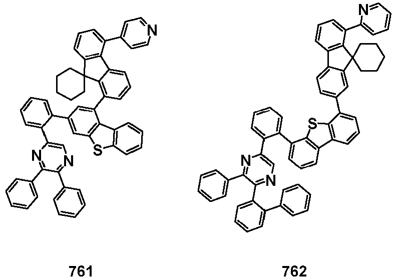

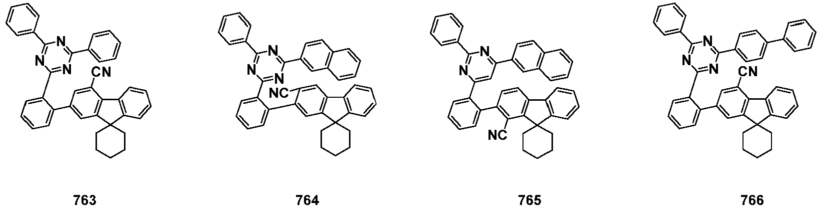

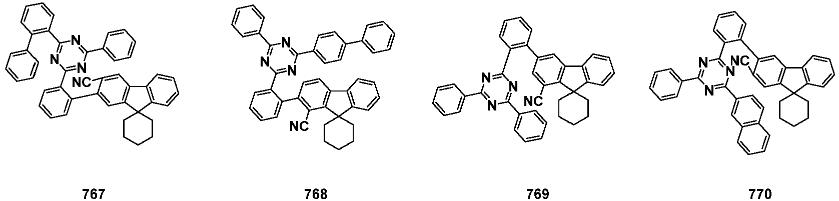

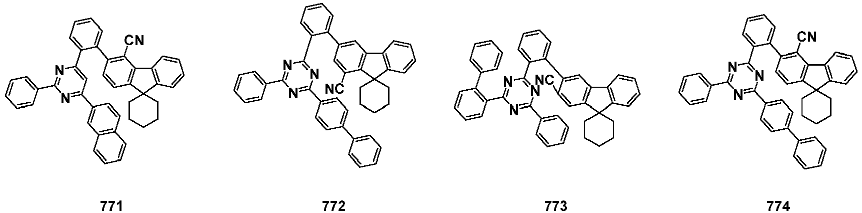

7. An organic electroluminescent device comprising the organic light-emitting compound according to any one of claims 1 to 6.

8. The organic electroluminescent device according to claim 7, comprising:
an anode;
a cathode;
a light-emitting layer disposed between the cathode and the anode; and
an electron transport region disposed between the cathode and the light-emitting layer,
wherein the electron transport region comprises the organic light-emitting compound.

9. The organic electroluminescent device according to claim 8, wherein the electron transport region comprises at least one of an electron transport layer or an electron transport auxiliary layer, and
the organic electroluminescent compound is contained in at least one layer of the electron transport layer or the electron transport auxiliary layer.

10. Use of the organic light-emitting compound according to any one of claims 1 to 6 for an organic electroluminescent device.

11. The use according to claim 10, wherein the organic light-emitting compound is used as an electron transport material for the organic electroluminescent device.

## Patentansprüche

1. Eine organische lichtemittierende Verbindung, dargestellt durch die folgende Formel 1: 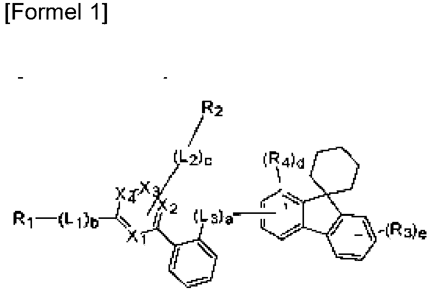 wobei
eines von X₁ bis X₄ C-(L₂)_{c}-R₂ ist, die anderen jeweils unabhängig voneinander N oder CR sind und mindestens zwei von X₁ bis X₄ N sind, wobei R Wasserstoff ist, eine Alkylgruppe, die 1 bis 40 Kohlenstoffatome enthält, eine Arylgruppe, die 6 bis 60 Kohlenstoffatome enthält, oder eine Heteroarylgruppe, die 2 bis 60 Kohlenstoffatome enthält, ist, die jeweils unsubstituiert oder substituiert sind,
R₁ und R₂ jeweils unabhängig voneinander Wasserstoff, eine Alkenylgruppe, die 2 bis 40 Kohlenstoffatome enthält, eine Alkinylgruppe, die 2 bis 40 Kohlenstoffatome enthält, eine Cycloalkylgruppe, die 3 bis 40 Kohlenstoffatome enthält, eine Heterocycloalkylgruppe, die 1 bis 40 Kohlenstoffatome enthält, eine Alkylgruppe, die 1 bis 40 Kohlenstoffatome enthält, eine Arylgruppe, die 6 bis 60 Kohlenstoffatome enthält, eine Heteroarylgruppe, die 2 bis 60 Kohlenstoffatome enthält, eine Alkylsilylgruppe, die 1 bis 40 Kohlenstoffatome enthält, eine Arylsilylgruppe, die 6 bis 60 Kohlenstoffatome enthält, eine Alkylborongruppe, die 1 bis 40 Kohlenstoffatome enthält, eine Arylborongruppe, die 6 bis 60 Kohlenstoffatome enthält, eine Arylphosphingruppe, die 6 bis 60 Kohlenstoffatome enthält, eine Alkylphosphinoxidgruppe, die 1 bis 40 Kohlenstoffatome enthält, eine Arylphosphinoxidgruppe, die 6 bis 60 Kohlenstoffatome enthält, eine Alkylaminogruppe, die 1 bis 40 Kohlenstoffatome enthält oder eine Arylaminogruppe, die 6 bis 60 Kohlenstoffatome enthält, die jeweils unsubstituiert oder substituiert sind, sind,
L₁ bis L₃ jeweils unabhängig voneinander eine Einfachbindung, eine Arylengruppe, die 6 bis 60 Kohlenstoffatome enthält, oder eine Heteroarylengruppe, die 2 bis 60 Kohlenstoffatome enthält, die jeweils unsubstituiert oder substituiert sind, sind,
R₃ und R₄ jeweils unabhängig voneinander eine Arylgruppe, die 6 bis 60 Kohlenstoffatome enthält, die mit einer Cyanogruppe substituiert ist, eine Arylgruppe, die 6 bis 60 Kohlenstoffatome enthält, die mit einer stickstoffhaltigen Heteroarylgruppe, die 2 bis 60 Kohlenstoffatome enthält,
substituiert ist, eine stickstoffhaltige Heteroarylgruppe, die 2 bis 60 Kohlenstoffatome enthält, Deuterium oder eine Cyanogruppe sind, die jeweils unsubstituiert oder substituiert sind,
a bis c jeweils unabhängig voneinander eine ganze Zahl von 0 bis 5 sind,
und
d eine ganze Zahl von 0 bis 3 ist, und e eine ganze Zahl von 0 bis 4 ist, mit der Maßgabe, dass d+e ≥ 1 ist.

2. Organische lichtemittierende Verbindung nach Anspruch 1, wobei eines von X₁ bis X₄ C-(L₂)_{c}-R₂ ist, die anderen jeweils unabhängig voneinander N oder CR sind und mindestens zwei von X₁ bis X₄ N sind, wobei R Wasserstoff, eine Alkylgruppe, die 1 bis 20 Kohlenstoffatome enthält, eine Arylgruppe, die 6 bis 30 Kohlenstoffatome enthält, oder eine Heteroarylgruppe, die 2 bis 30 Kohlenstoffatome enthält, ist,
R₁ und R₂ jeweils unabhängig voneinander Wasserstoff, eine Alkenylgruppe, die 2 bis 40 Kohlenstoffatome enthält, eine Alkinylgruppe, die 2 bis 20 Kohlenstoffatome enthält, eine Cycloalkylgruppe, die 3 bis 20 Kohlenstoffatome enthält, eine Heterocycloalkylgruppe, die 1 bis 20 Kohlenstoffatome enthält, eine Alkylgruppe, die 1 bis 20 Kohlenstoffatome enthält, eine Arylgruppe, die 6 bis 30 Kohlenstoffatome enthält, eine Heteroarylgruppe, die 2 bis 30 Kohlenstoffatome enthält, eine Alkylsilylgruppe, die 1 bis 20 Kohlenstoffatome enthält, eine Arylsilylgruppe, die 6 bis 30 Kohlenstoffatome enthält, eine Alkylborongruppe, die 1 bis 20 Kohlenstoffatome enthält, eine Arylborongruppe, die 6 bis 30 Kohlenstoffatome enthält, eine Arylphosphingruppe, die 6 bis 30 Kohlenstoffatome enthält, eine Arylphosphinoxidgruppe, die 1 bis 20 Kohlenstoffatome enthält, eine Arylphosphinoxidgruppe, die 6 bis 30 Kohlenstoffatome enthält, eine Alkylamingruppe, die 1 bis 20 Kohlenstoffatome enthält, oder eine Arylamingruppe, die 6 bis 30 Kohlenstoffatome enthält, von denen jede unsubstituiert oder mit Deuterium substituiert ist, eine Alkenylgruppe, die 2 bis 20 Kohlenstoffatome enthält, eine Alkinylgruppe, die 2 bis 20 Kohlenstoffatome enthält, eine Cycloalkylgruppe, die 3 bis 20 Kohlenstoffatome enthält, eine Heterocycloalkylgruppe, die 1 bis 20 Kohlenstoffatome enthält, eine Alkylgruppe, die 1 bis 20 Kohlenstoffatome enthält, eine Arylgruppe, die 6 bis 30 Kohlenstoffatome enthält, eine Heteroarylgruppe, die 2 bis 30 Kohlenstoffatome enthält, eine Alkylsilylgruppe, die 1 bis 20 Kohlenstoffatome enthält, eine Arylsilylgruppe, die 6 bis 30 Kohlenstoffatome enthält, eine Alkylborongruppe, die 1 bis 20 Kohlenstoffatome enthält, eine Arylborongruppe, die 6 bis 30 Kohlenstoffatome enthält, eine Arylphosphingruppe, die 6 bis 30 Kohlenstoffatome aufweist, eine Alkylphosphinoxidgruppe, die 1 bis 20 Kohlenstoffatome aufweist, eine Arylphosphinoxidgruppe, die 6 bis 30 Kohlenstoffatome aufweist, eine Alkylaminogruppe, die 1 bis 20 Kohlenstoffatome aufweist, oder eine Arylaminogruppe, die 6 bis 30 Kohlenstoffatome aufweist, sind,
L₁ bis L₃ jeweils unabhängig voneinander eine Einfachbindung, eine Arylengruppe, die 6 bis 30 Kohlenstoffatome enthält, oder eine Heteroarylengruppe, die 2 bis 30 Kohlenstoffatome enthält, sind,
R₃ und R₄ jeweils unabhängig voneinander eine Arylgruppe, die 6 bis 30 Kohlenstoffatome enthält, die mit einer Cyanogruppe substituiert ist, eine Arylgruppe, die 6 bis 30 Kohlenstoffatome enthält, die mit einer stickstoffhaltigen Heteroarylgruppe, die 2 bis 30 Kohlenstoffatome enthält, substituiert ist, eine stickstoffhaltige Heteroarylgruppe, die 2 bis 30 Kohlenstoffatome enthält, Deuterium oder eine Cyanogruppe sind, die jeweils unsubstituiert oder substituiert sind,
a bis c jeweils unabhängig voneinander eine ganze Zahl von 0 bis 2 sind,
und
d eine ganze Zahl von 0 bis 2 ist, und e eine ganze Zahl von 0 bis 2 ist, mit der Maßgabe, dass d+e ≥ 1 ist.

3. Organische lichtemittierende Verbindung nach Anspruch 1, wobei eines von X₁ bis X₄ C-(L₂)_{c}-R₂ ist, die anderen jeweils unabhängig voneinander N oder CH sind und mindestens zwei von X₁ bis X₄ N sind,
R₁ und R₂ jeweils unabhängig voneinander Wasserstoff, eine Arylgruppe, die 6 bis 30 Kohlenstoffatome enthält, oder eine Heteroarylgruppe, die 2 bis 30 Kohlenstoffatome enthält, die jeweils unsubstituiert oder mit einer Cycloalkylgruppe, die 3 bis 20 Kohlenstoffatome enthält, einer Alkylgruppe, die 1 bis 20 Kohlenstoffatome enthält, einer Arylgruppe, die 6 bis 30 Kohlenstoffatome enthält, einer Heteroarylgruppe, die 2 bis 30 Kohlenstoffatome enthält, einer Alkylsilylgruppe, die 1 bis 20 Kohlenstoffatome enthält, einer Arylsilylgruppe, die 6 bis 30 Kohlenstoffatome enthält, einer Alkylphosphinoxidgruppe, die 1 bis 20 Kohlenstoffatome enthält, oder einer Arylphosphinoxidgruppe, die 6 bis 30 Kohlenstoffatome enthält, substituiert sind,
L₁ bis L₃ jeweils unabhängig voneinander eine Einfachbindung, eine Arylengruppe, die 6 bis 30 Kohlenstoffatome enthält, oder eine Heteroarylengruppe, die 2 bis 30 Kohlenstoffatome enthält, sind,
R₃ und R₄ jeweils unabhängig voneinander eine Arylgruppe, die 6 bis 30 Kohlenstoffatome enthält, die mit einer Cyanogruppe substituiert ist, eine stickstoffhaltige Heteroarylgruppe, die 2 bis 30 Kohlenstoffatome enthält, oder eine Cyanogruppe sind,
a bis c jeweils unabhängig voneinander eine ganze Zahl von 0 bis 2 sind,
und
d eine ganze Zahl von 0 bis 2 ist, und e eine ganze Zahl von 0 bis 2 ist, mit der Maßgabe, dass d+e ≥ 1 ist.

4. Organische lichtemittierende Verbindung nach Anspruch 1, wobei die Formel 1 durch eine der folgenden Formeln 2 bis 9 dargestellt wird: 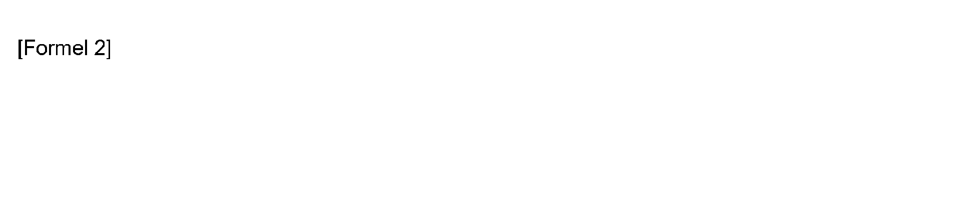 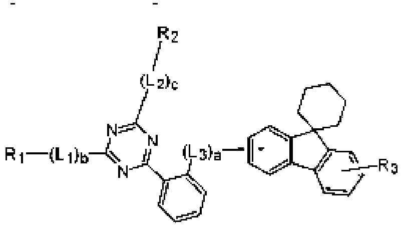 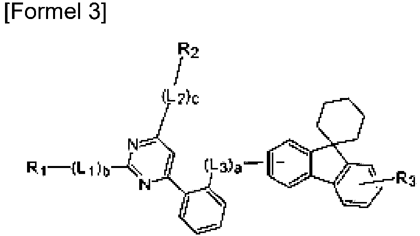 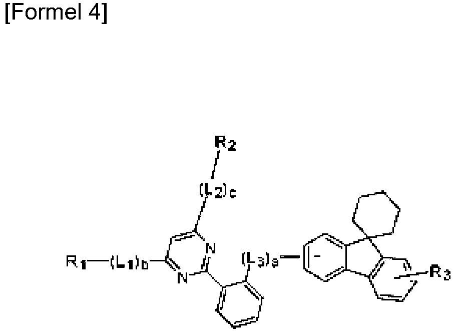 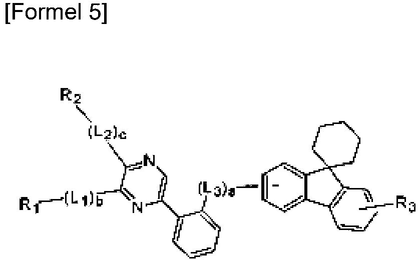 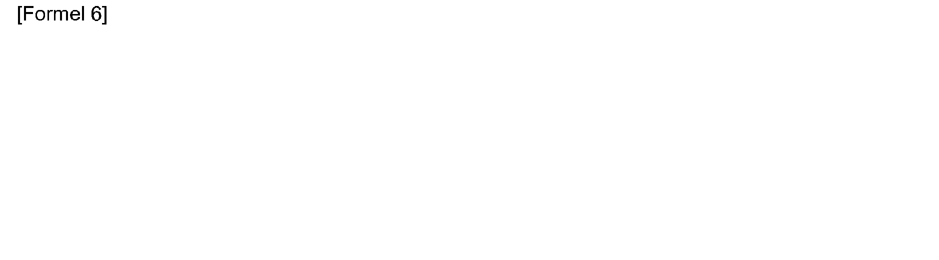 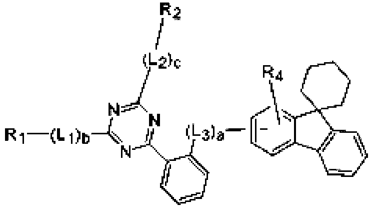 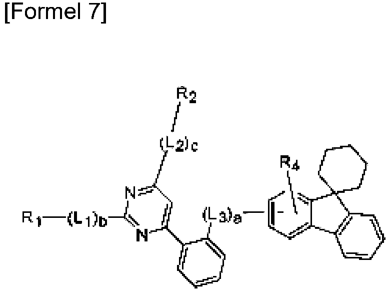 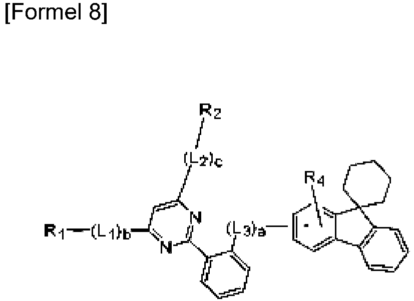 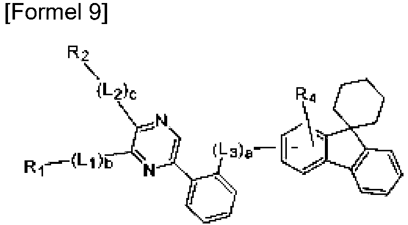 wobei in jeder der Formeln 2 bis 9
R₁ und R₂ jeweils unabhängig voneinander Wasserstoff, eine Arylgruppe, die 6 bis 30 Kohlenstoffatome enthält, oder eine Heteroarylgruppe, die 2 bis 30 Kohlenstoffatome enthält, die jeweils unsubstituiert oder mit einer Cycloalkylgruppe, die 3 bis 20 Kohlenstoffatome enthält, einer Alkylgruppe, die 1 bis 20 Kohlenstoffatome enthält, einer Arylgruppe, die 6 bis 30 Kohlenstoffatome enthält, einer Heteroarylgruppe, die 2 bis 30 Kohlenstoffatome enthält, einer Alkylsilylgruppe, die 1 bis 20 Kohlenstoffatome enthält, einer Arylsilylgruppe, die 6 bis 30 Kohlenstoffatome enthält, einer Alkylphosphinoxidgruppe, die 1 bis 20 Kohlenstoffatome enthält, oder einer Arylphosphinoxidgruppe, die 6 bis 30 Kohlenstoffatome enthält, substituiert sind,
L₁ bis L₃ jeweils unabhängig voneinander eine Einfachbindung, eine Arylengruppe, die 6 bis 30 Kohlenstoffatome enthält, oder eine Heteroarylengruppe, die 2 bis 30 Kohlenstoffatome enthält, sind,
R₃ und R₄ jeweils unabhängig voneinander eine Arylgruppe, die 6 bis 30 Kohlenstoffatome enthält, die mit einer Cyanogruppe substituiert ist, eine stickstoffhaltige Heteroarylgruppe, die 2 bis 30 Kohlenstoffatome enthält, oder eine Cyanogruppe sind, und
a bis c jeweils unabhängig voneinander eine ganze Zahl von 0 bis 2 sind.

5. Organische lichtemittierende Verbindung nach Anspruch 4, wobei die Formel 1 durch eine der folgenden Formeln 2 bis 7 dargestellt wird. 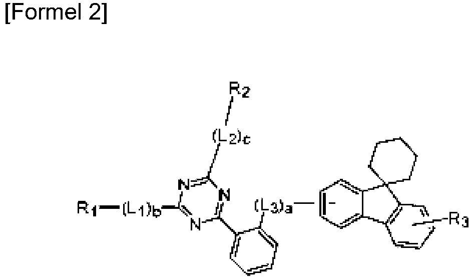 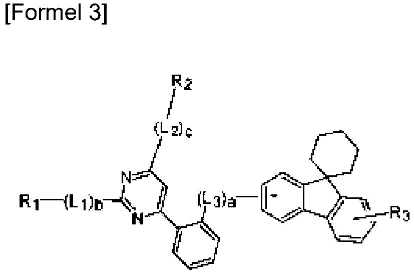 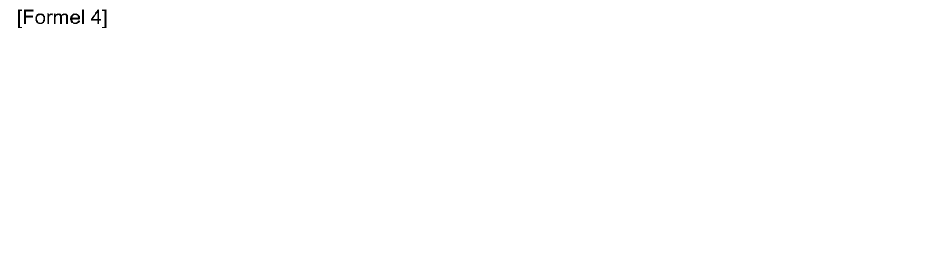 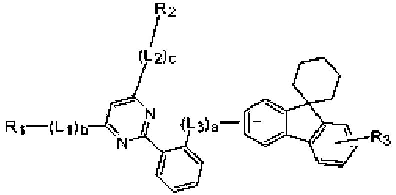 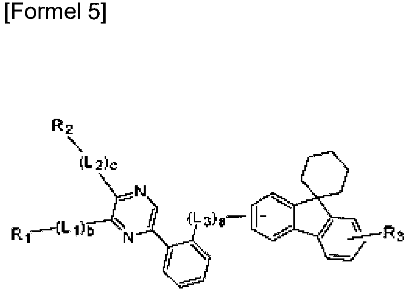 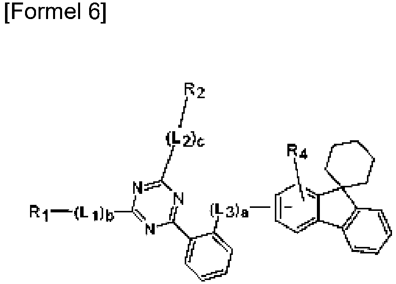 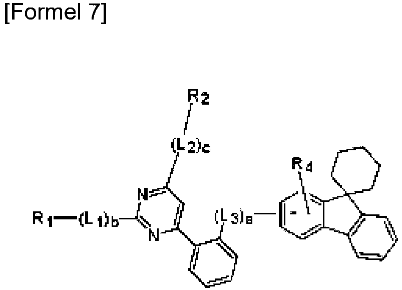 wobei in jeder der Formeln 2 bis 7
R₁ und R₂ jeweils unabhängig voneinander Wasserstoff, eine Phenylgruppe, eine Biphenylgruppe, eine Naphthylgruppe, eine Terphenylgruppe, eine Fluorenylgruppe, eine Pyridylgruppe, eine Carbazolylgruppe, eine Dibenzothiophenylgruppe oder eine Dibenzofuranylgruppe sind, die jeweils unsubstituiert oder mit einer Methylgruppe, einer Phenylgruppe, einer Cyclohexylgruppe, einer Adamantylgruppe, einer Pyridylgruppe, einer Carbazolylgruppe, einer Dibenzothiophenylgruppe, einer Dibenzofuranylgruppe, einer Trimethylsilylgruppe, einer Triphenylsilylgruppe, einer Dimethylphosphinoxidgruppe oder einer Diphenylphosphinoxidgruppe substituiert ist,
L₁ bis L₃ jeweils unabhängig voneinander eine Einfachbindung, eine Phenylengruppe, eine Biphenylengruppe, eine Naphthylengruppe, eine Terphenylengruppe, eine Dibenzofuranylengruppe oder eine Dibenzothiophenylengruppe sind,
R₃ und R₄ jeweils unabhängig voneinander eine Phenylgruppe sind, die mit einer Cyanogruppe, einer Pyridylgruppe oder einer Cyanogruppe substituiert ist, und
a bis c jeweils unabhängig voneinander eine ganze Zahl von 0 bis 2 sind.

6. Organische lichtemittierende Verbindung nach Anspruch 1, wobei die durch Formel 1 dargestellte organische lichtemittierende Verbindung eine beliebige der folgenden Verbindungen 001 bis 774 ist.

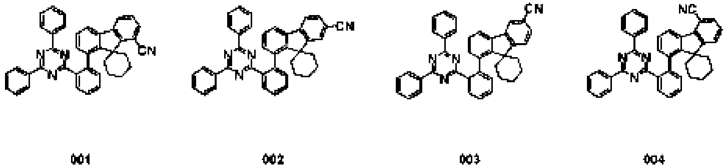

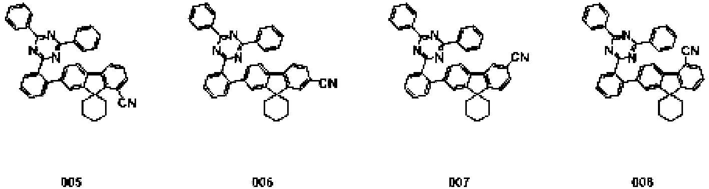

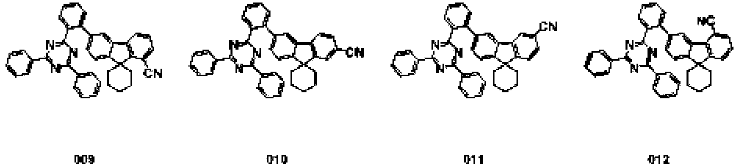

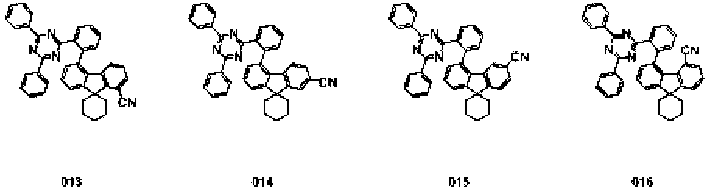

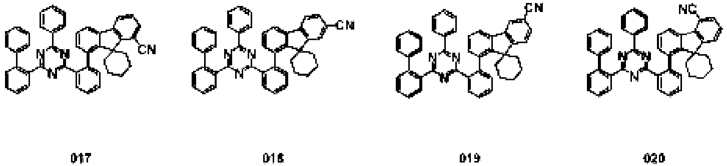

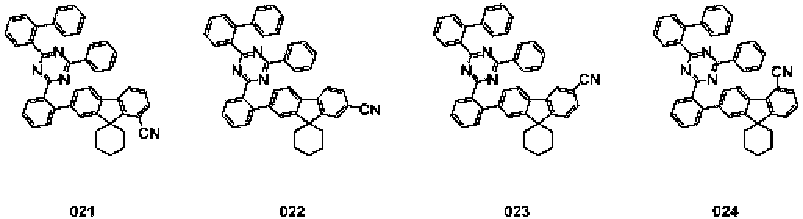

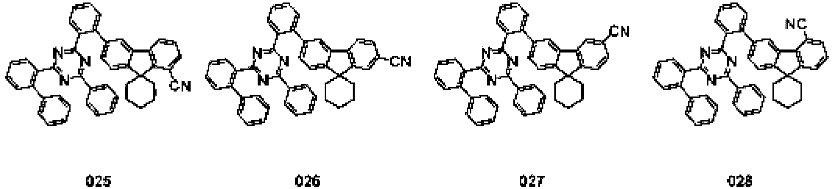

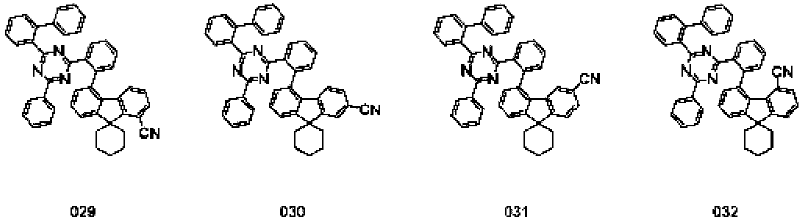

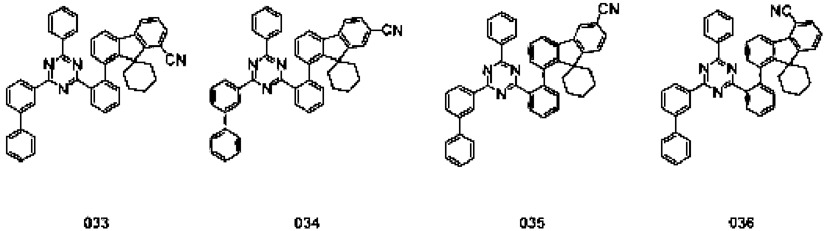

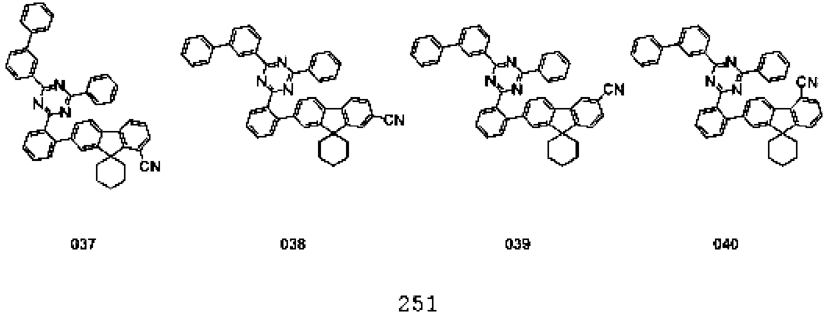

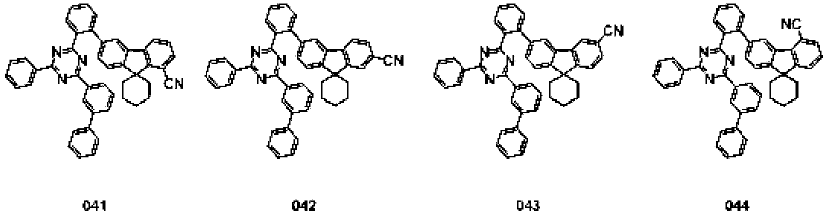

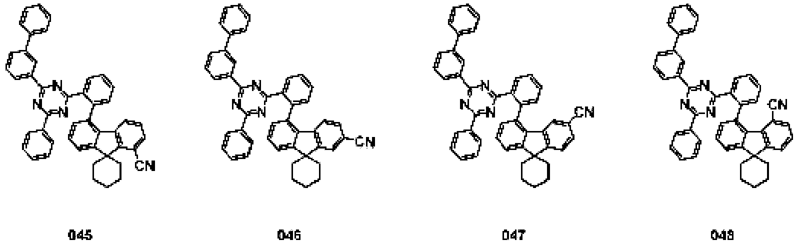

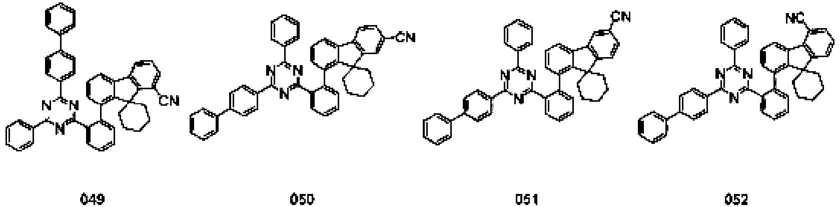

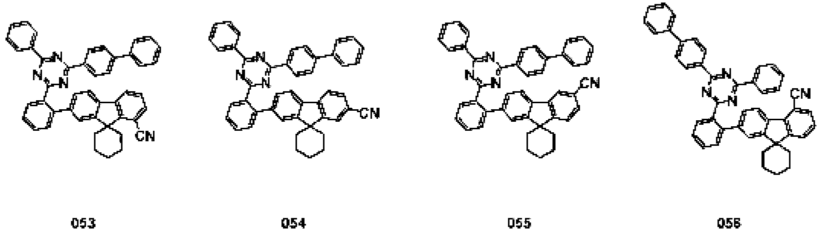

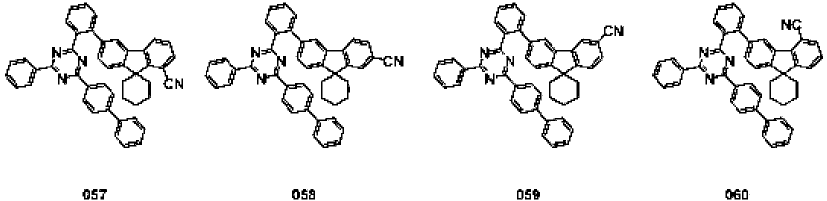

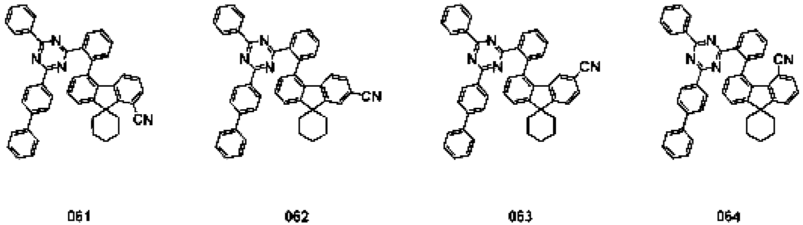

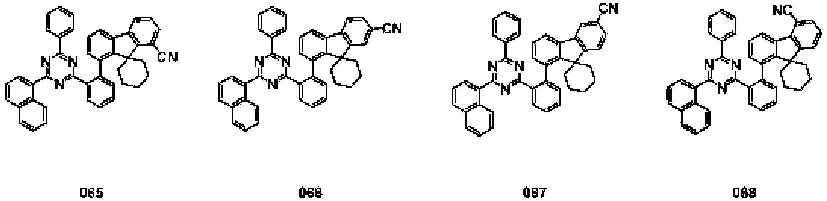

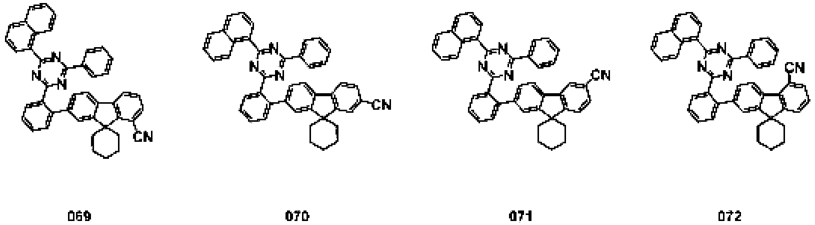

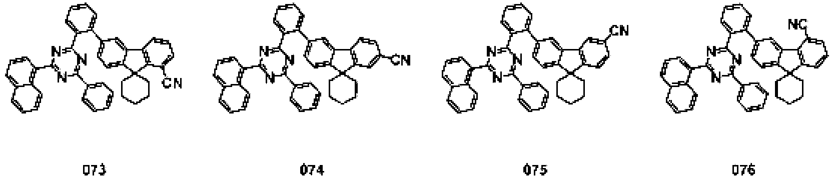

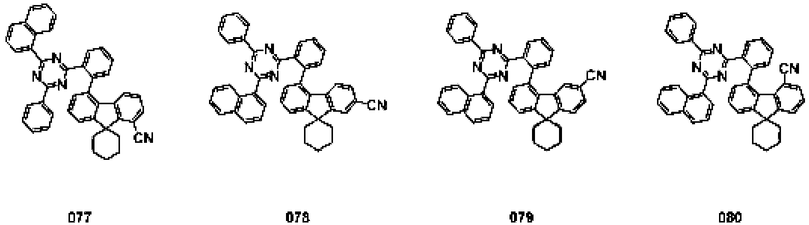

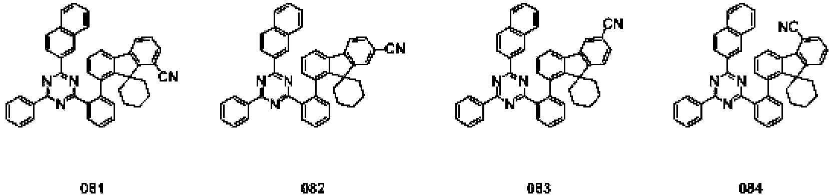

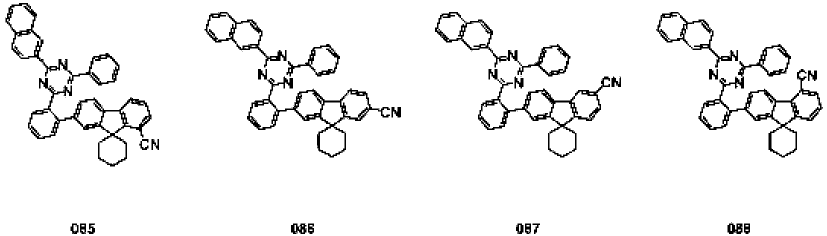

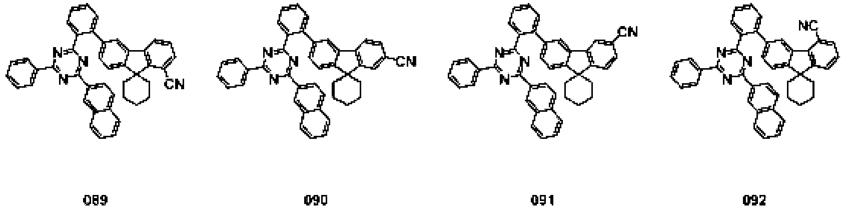

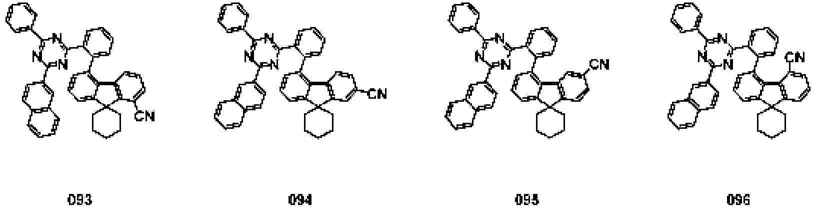

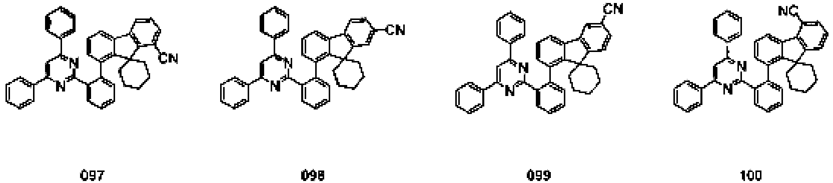

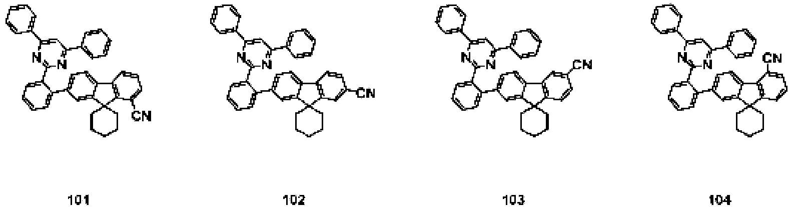

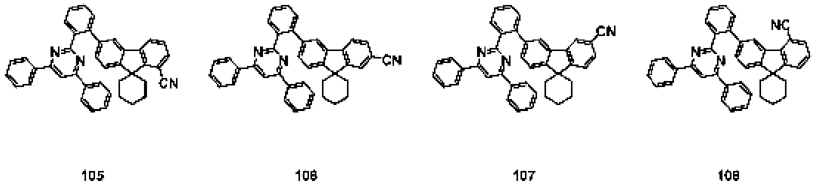

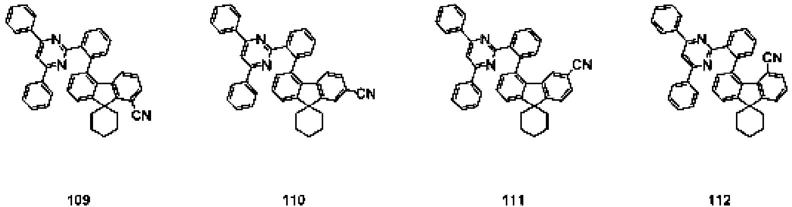

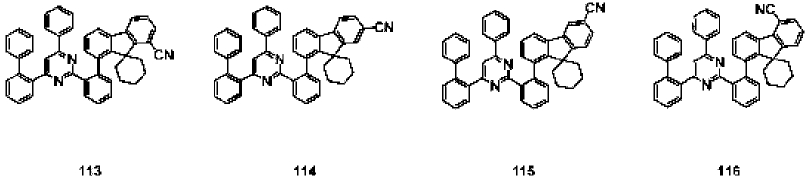

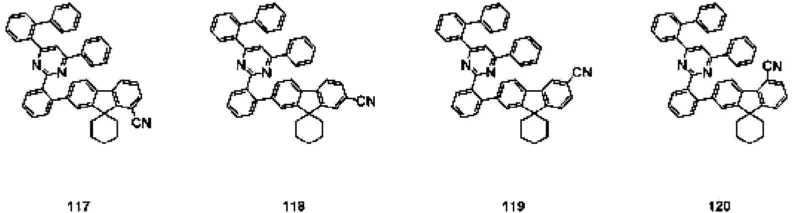

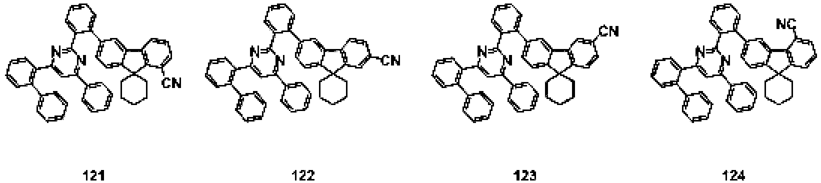

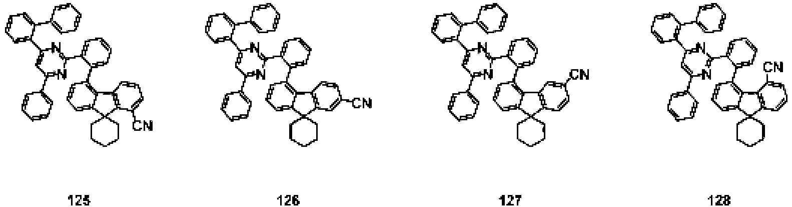

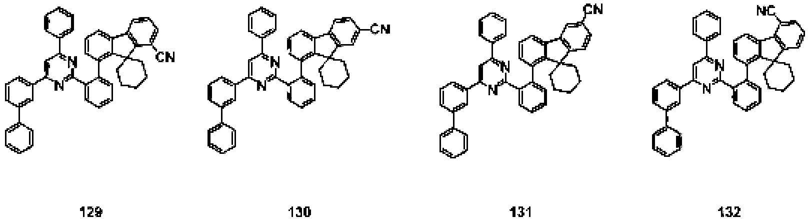

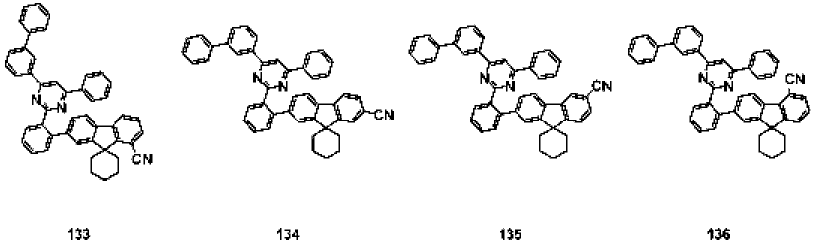

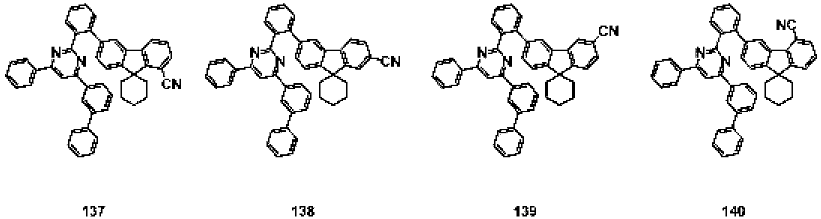

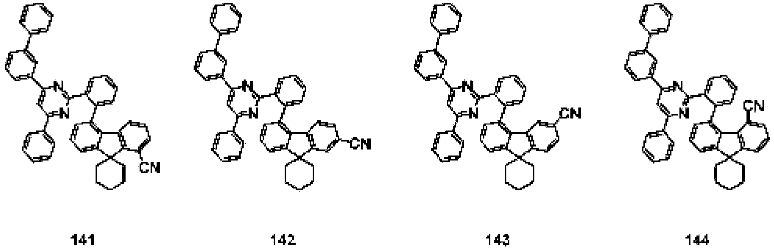

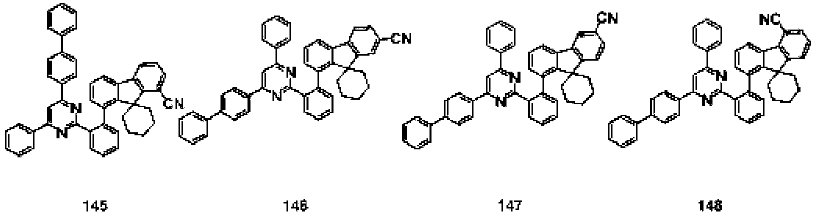

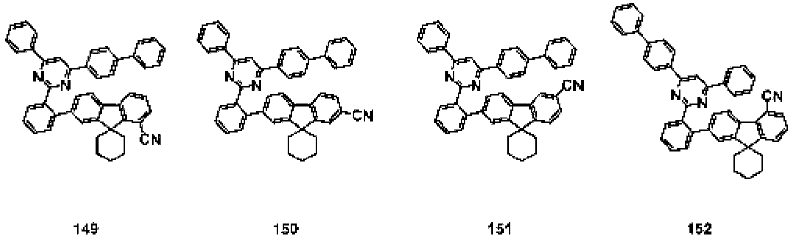

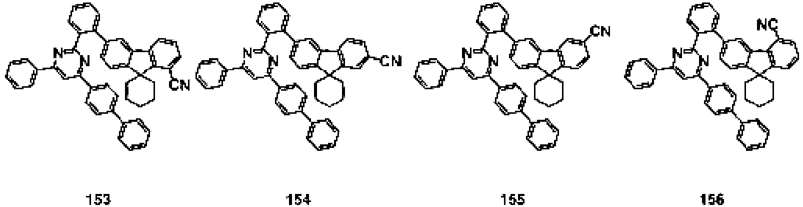

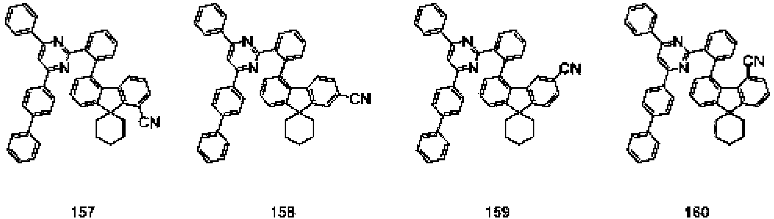

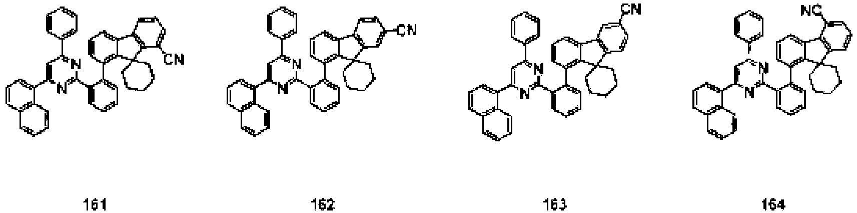

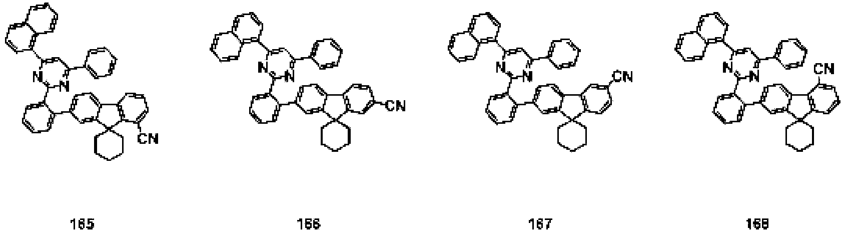

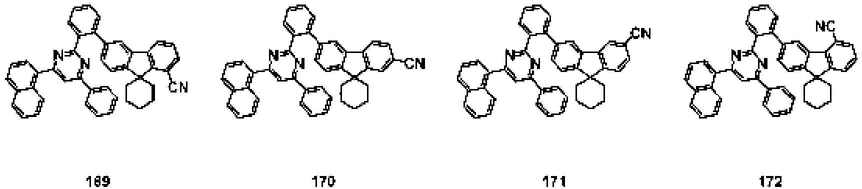

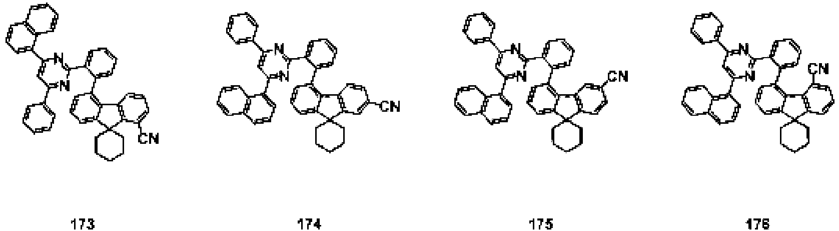

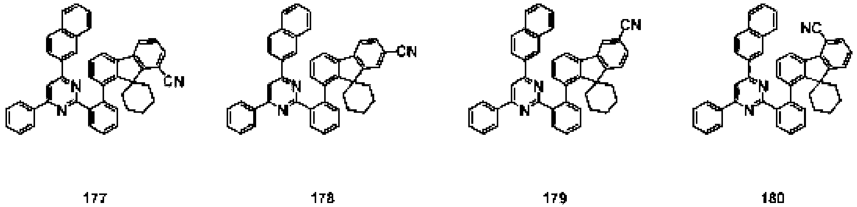

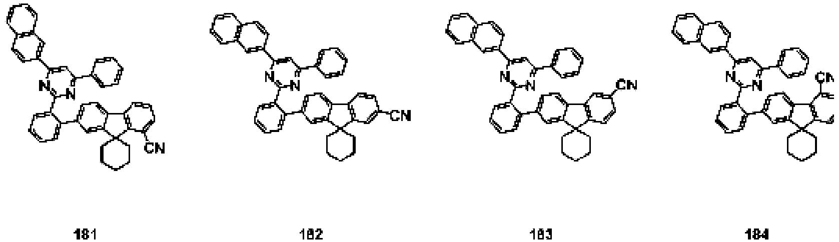

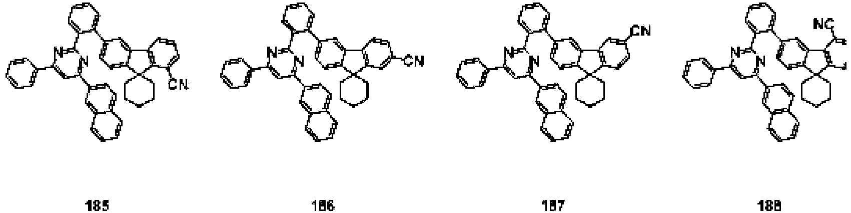

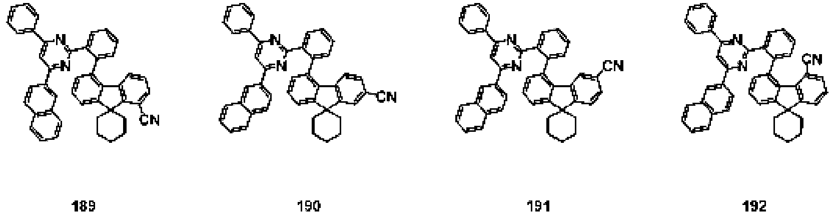

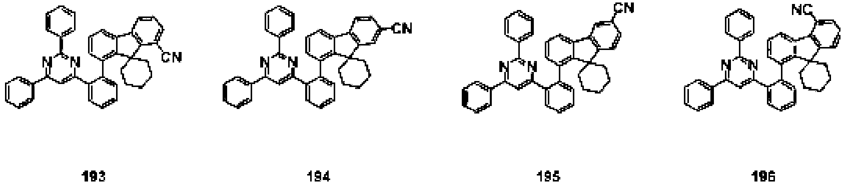

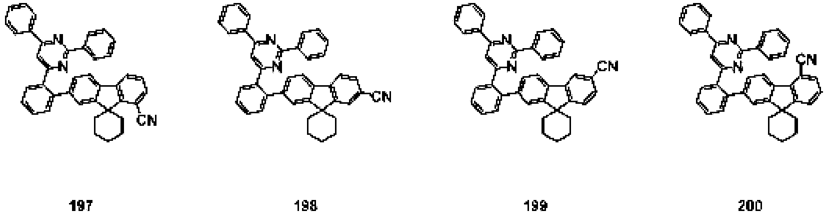

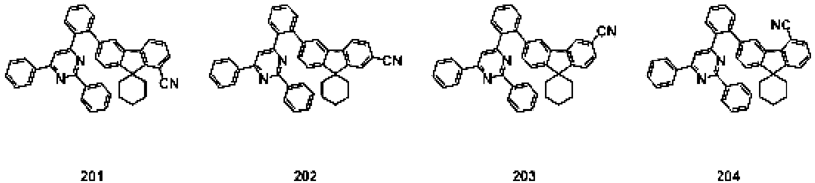

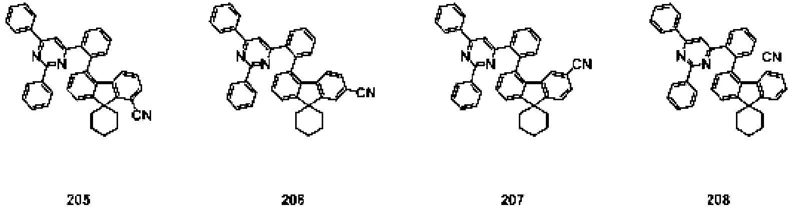

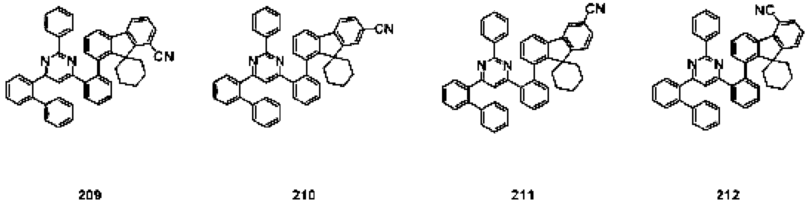

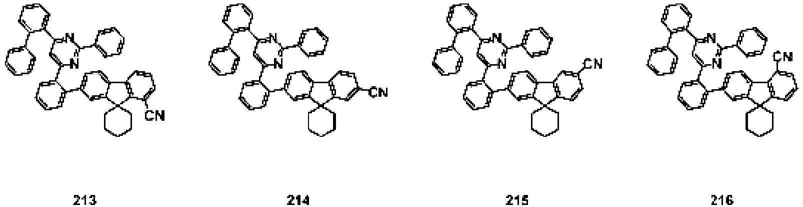

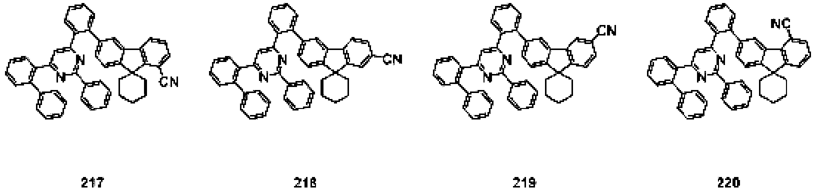

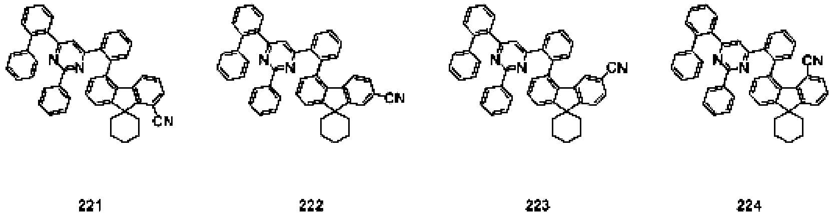

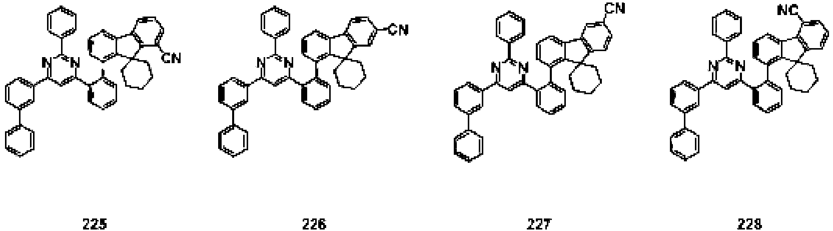

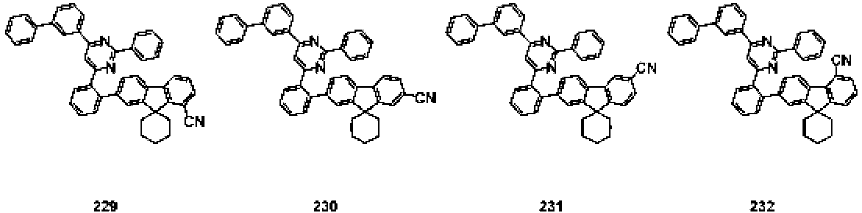

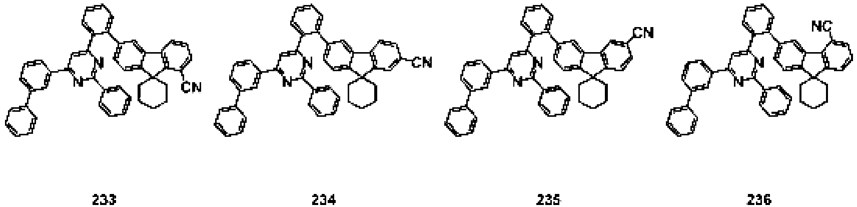

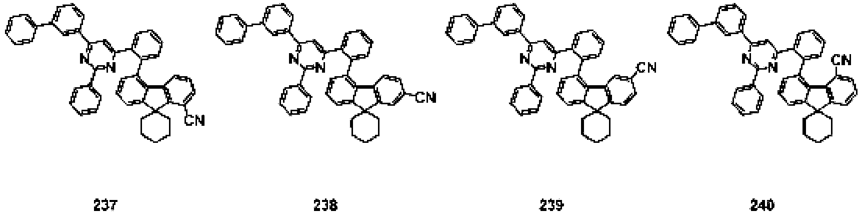

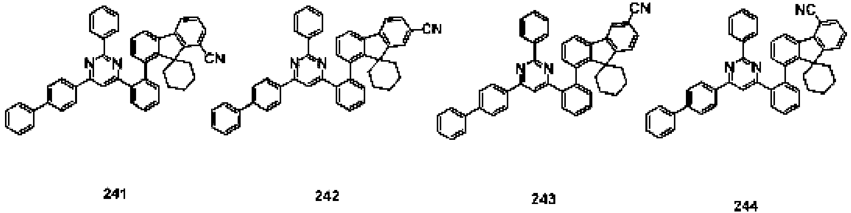

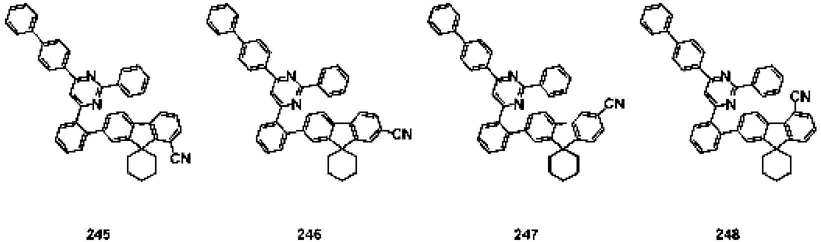

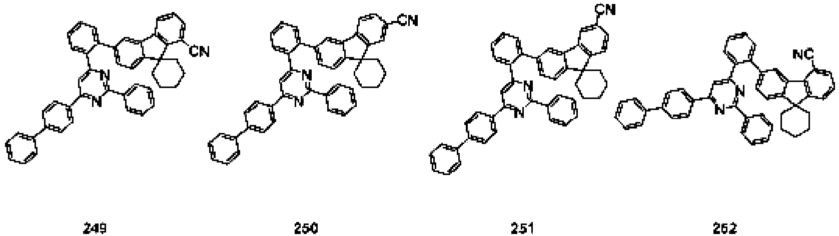

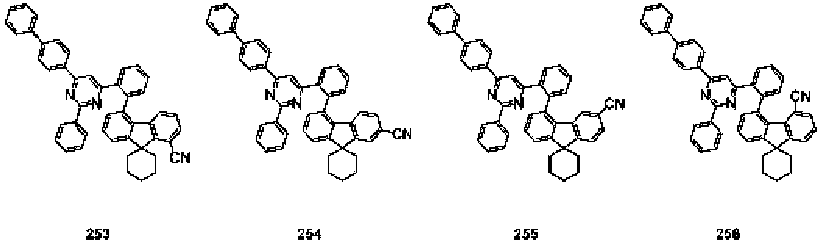

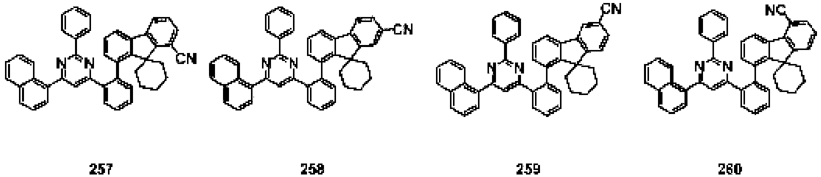

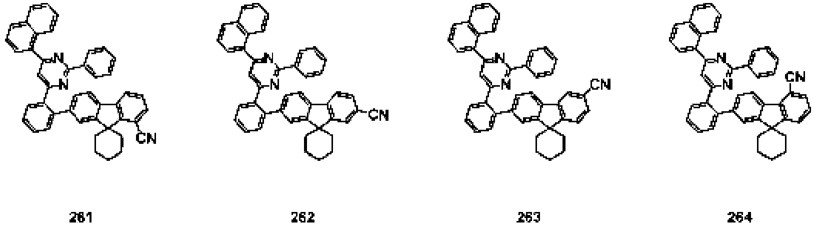

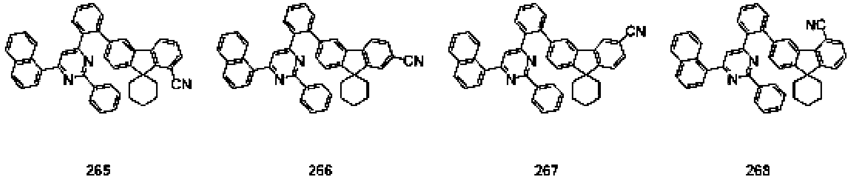

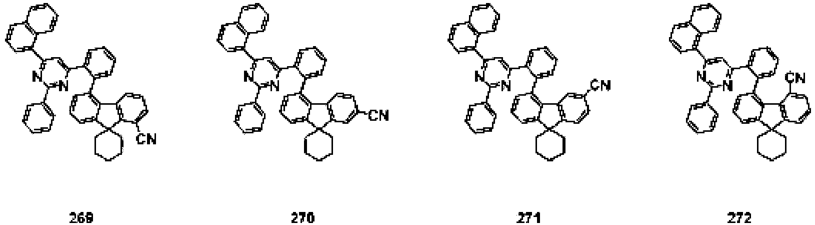

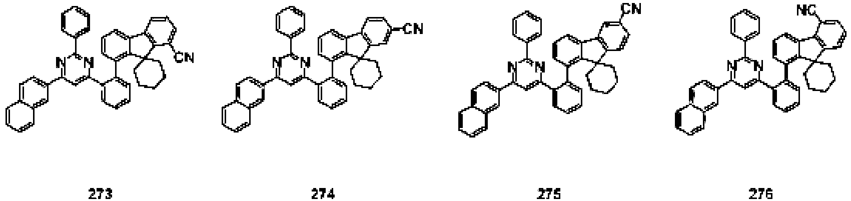

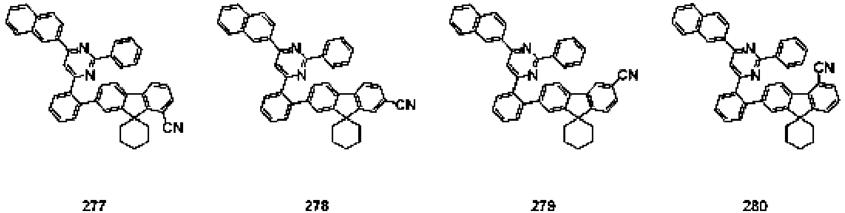

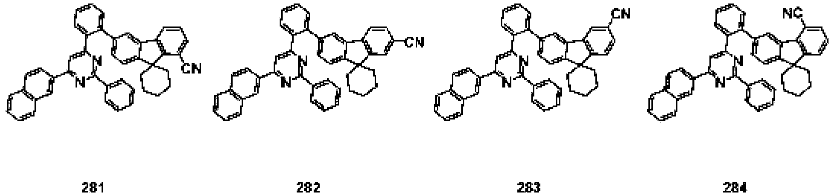

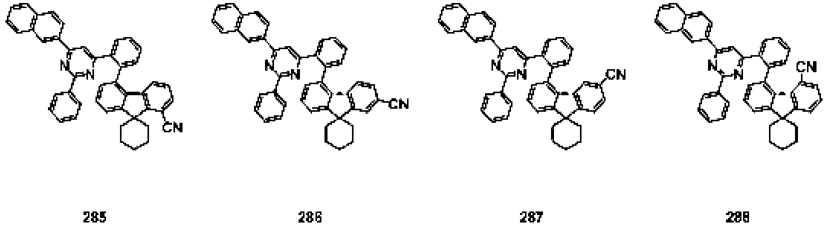

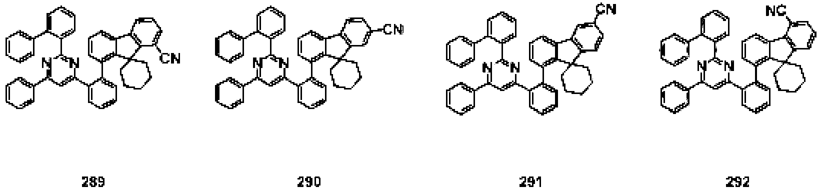

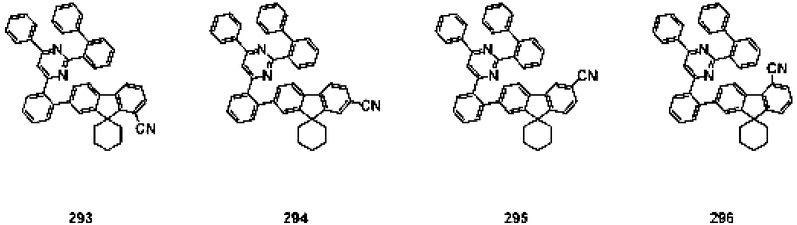

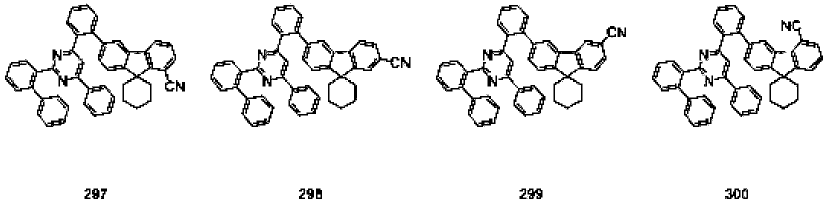

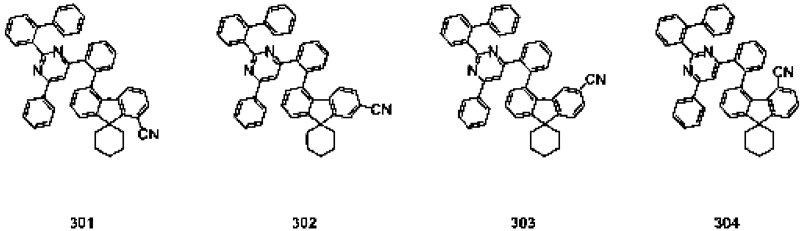

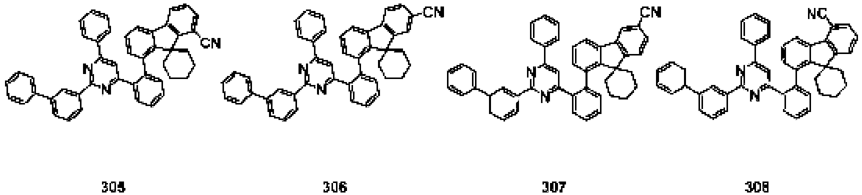

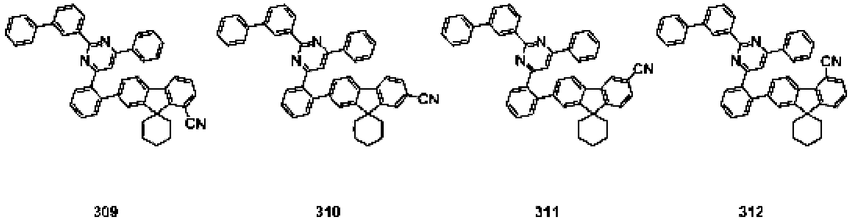

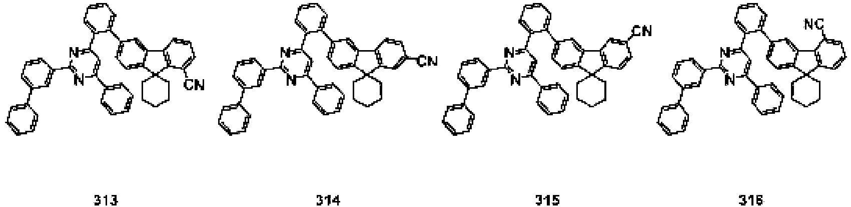

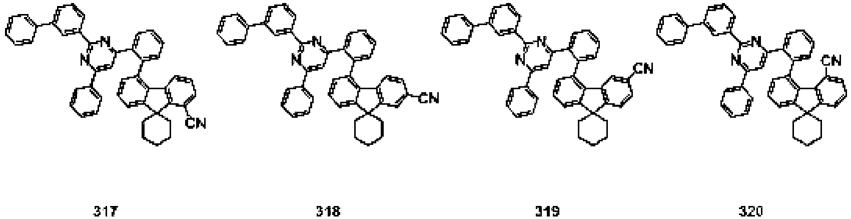

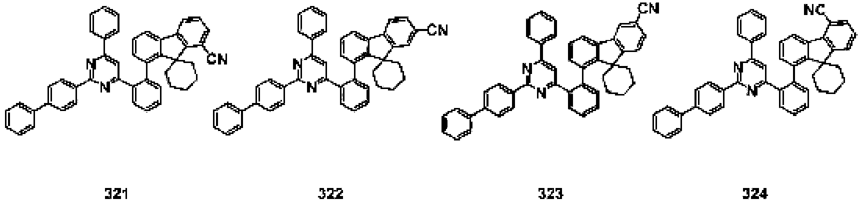

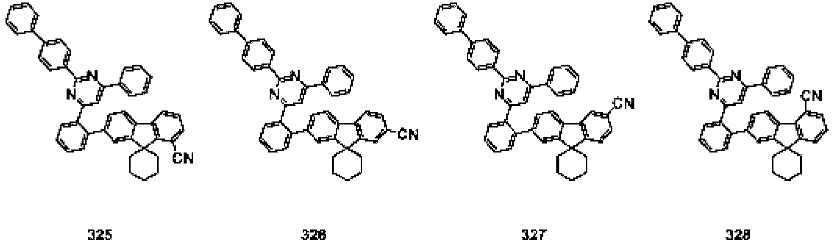

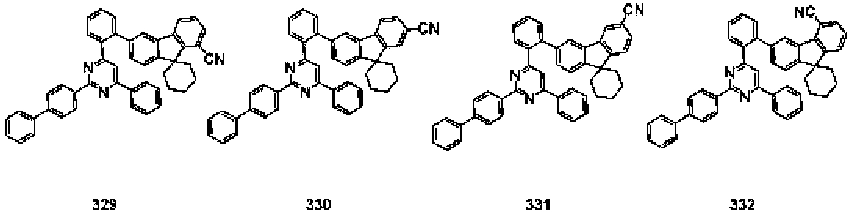

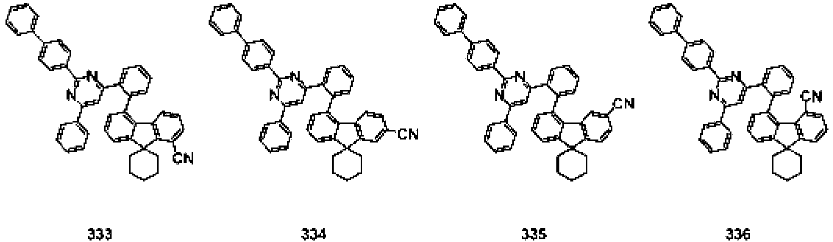

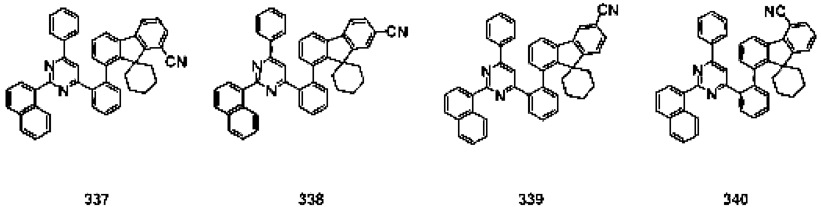

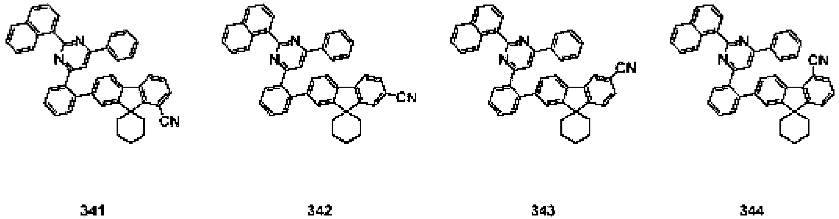

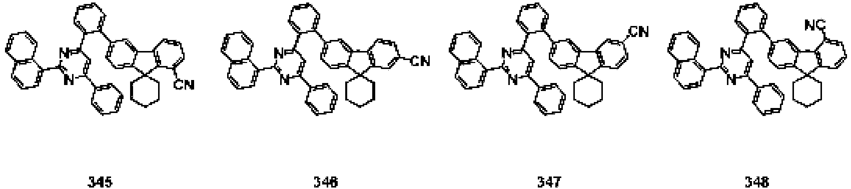

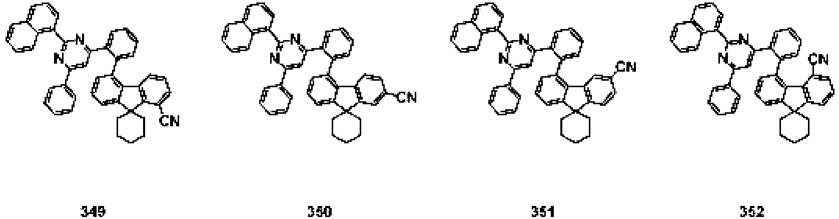

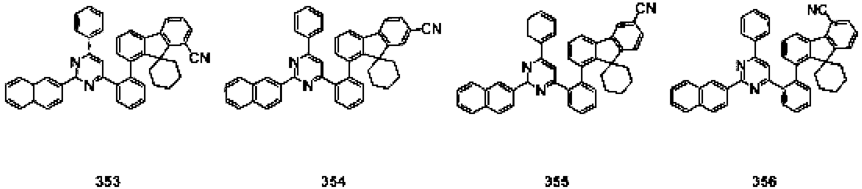

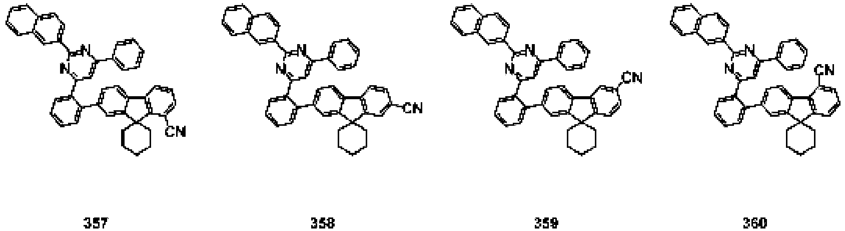

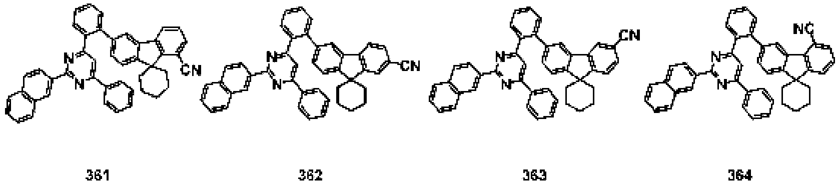

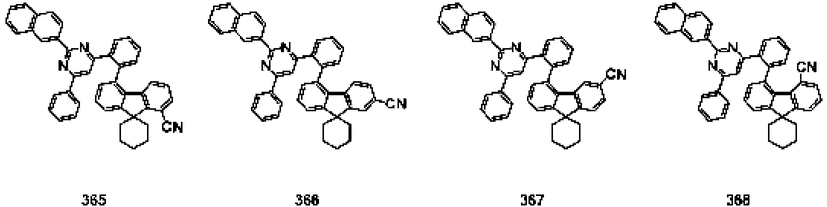

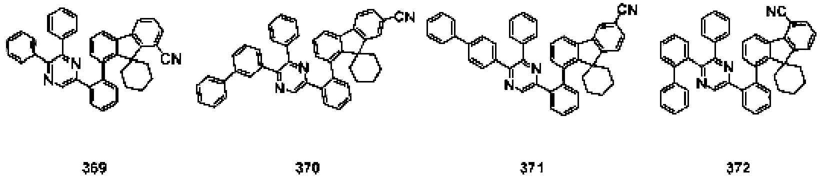

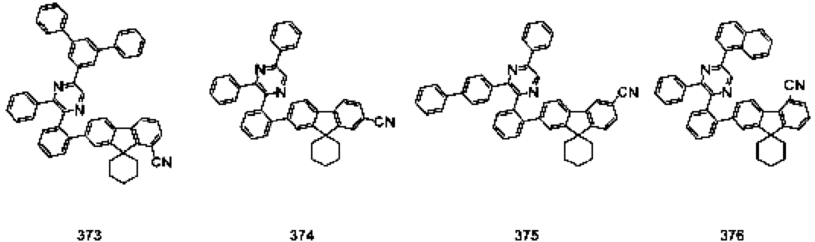

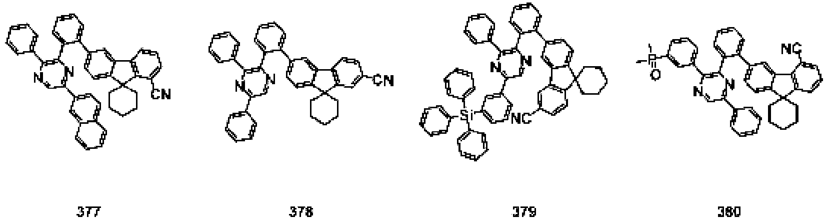

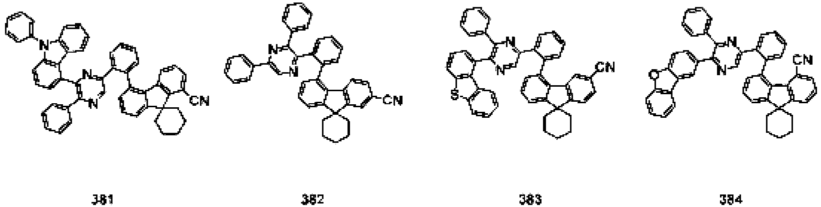

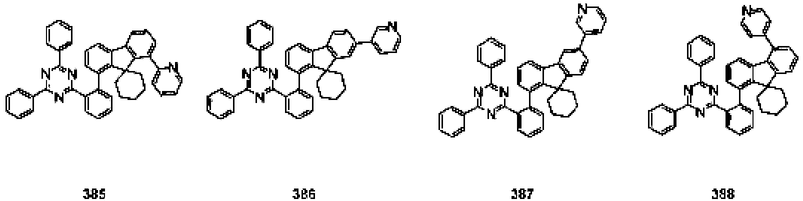

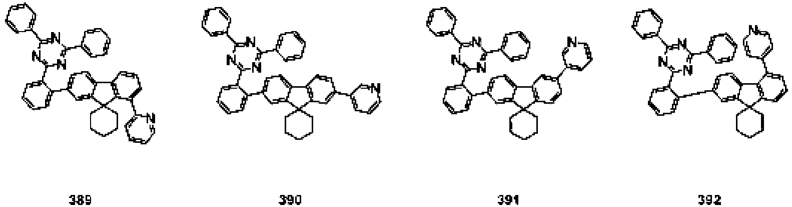

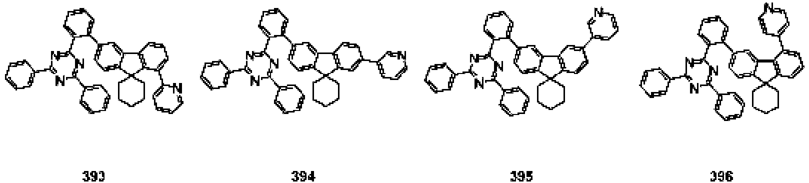

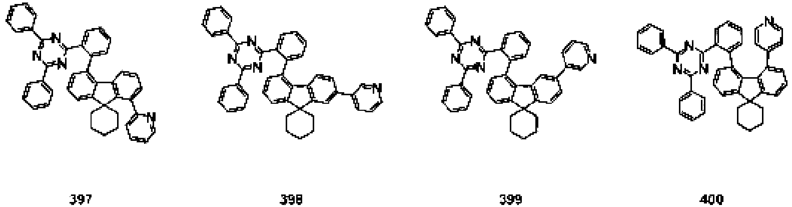

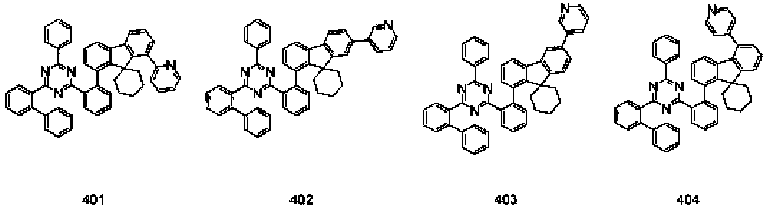

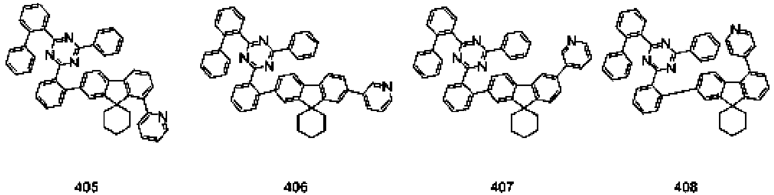

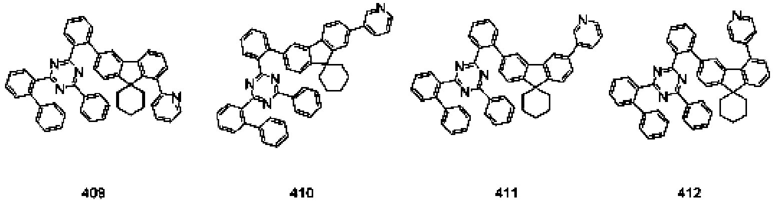

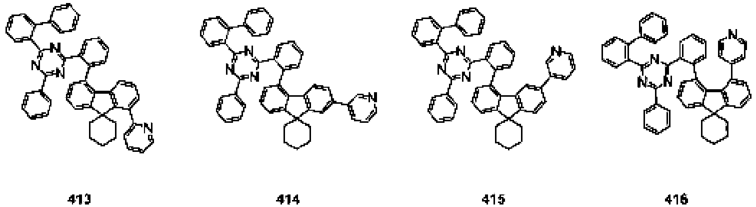

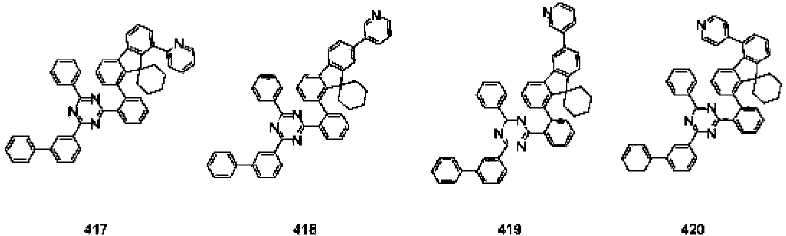

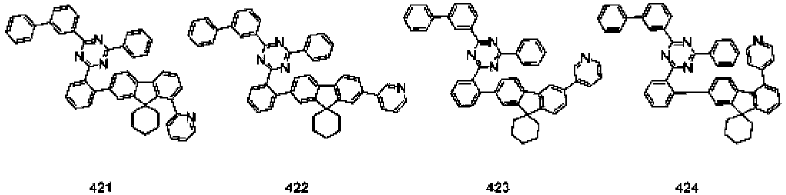

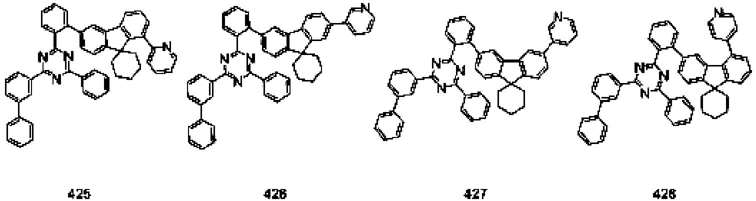

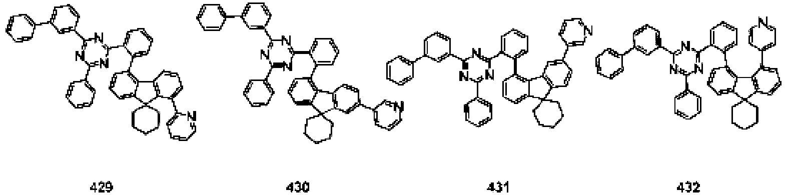

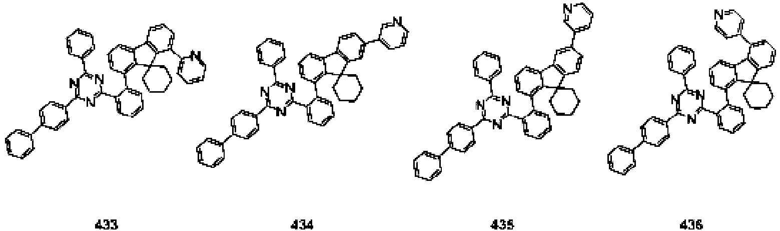

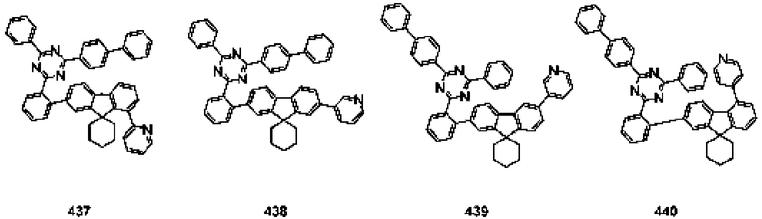

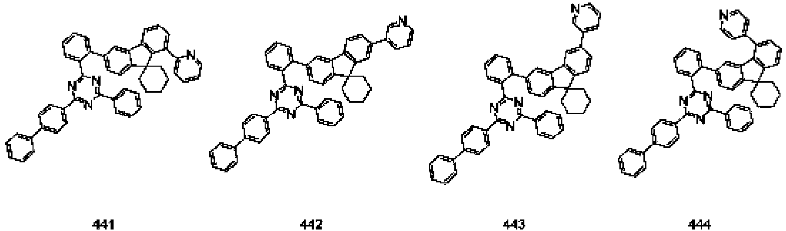

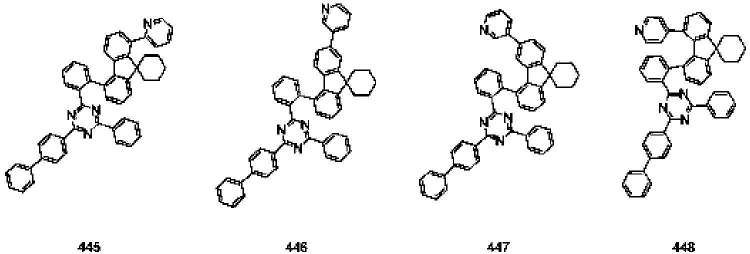

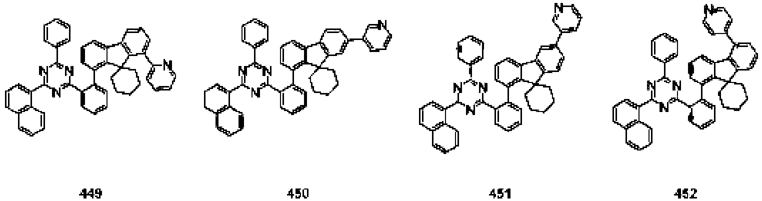

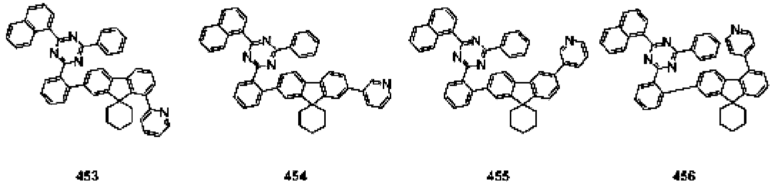

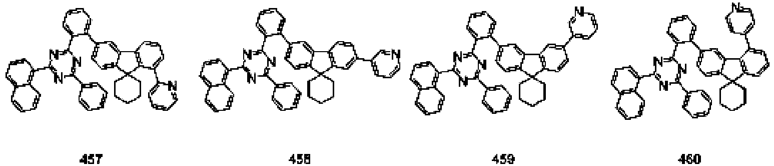

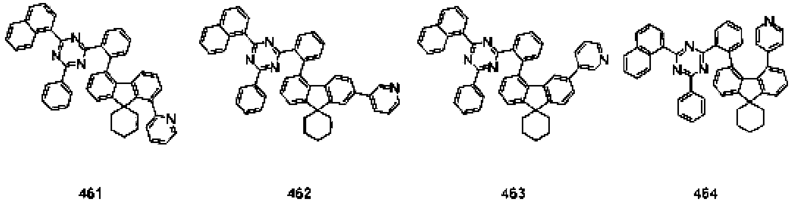

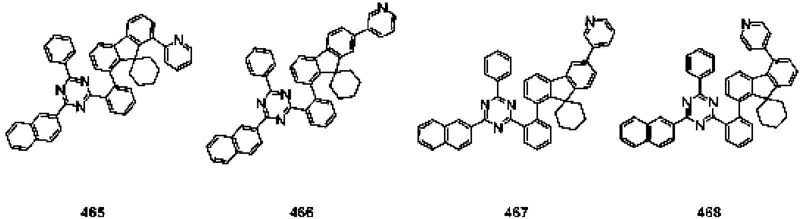

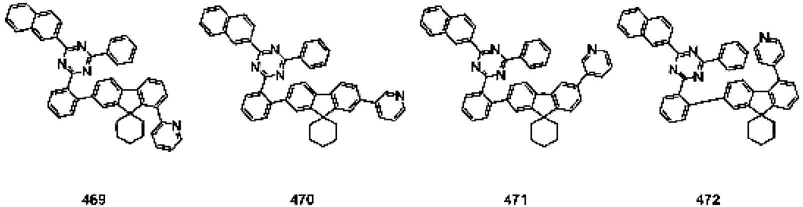

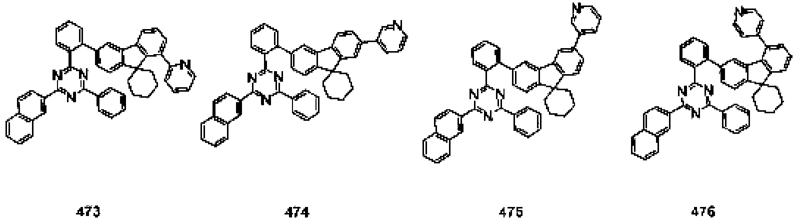

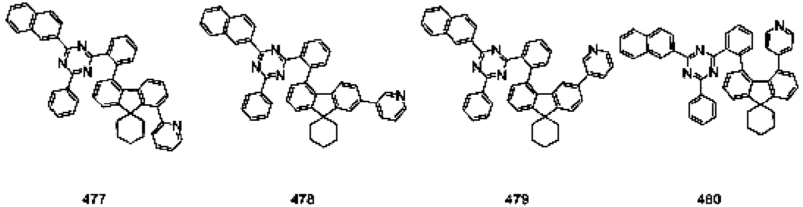

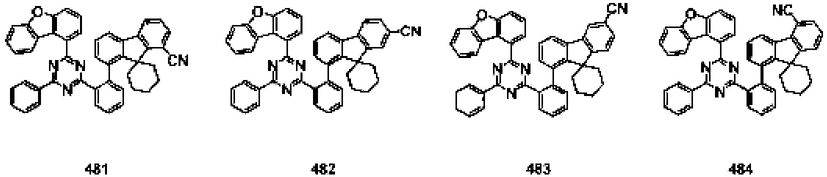

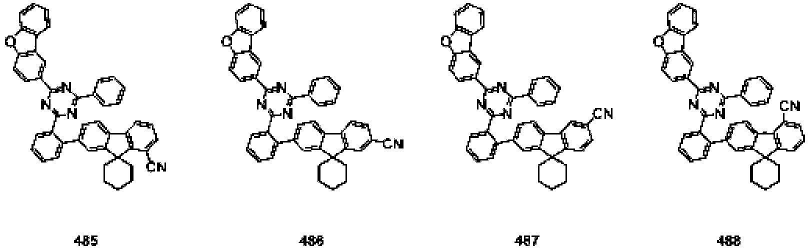

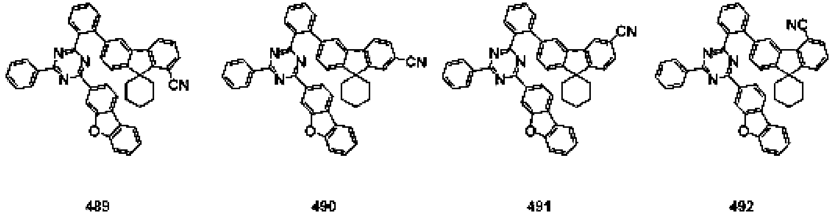

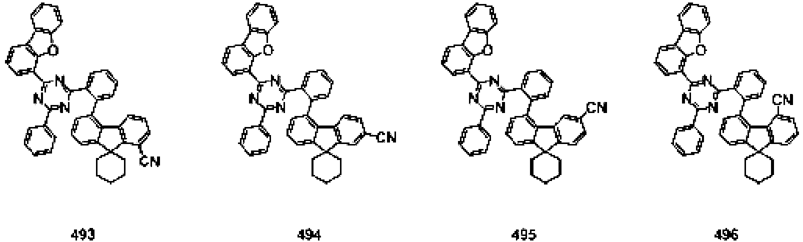

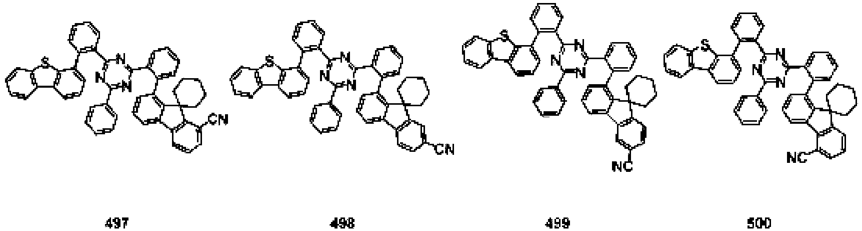

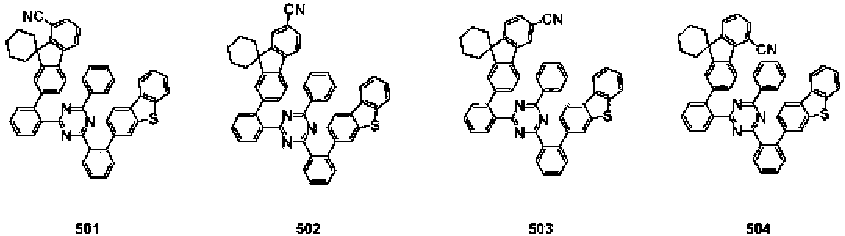

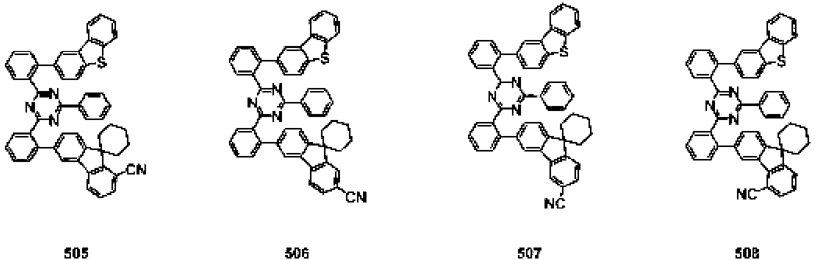

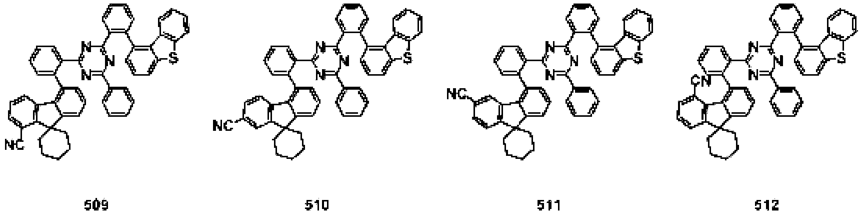

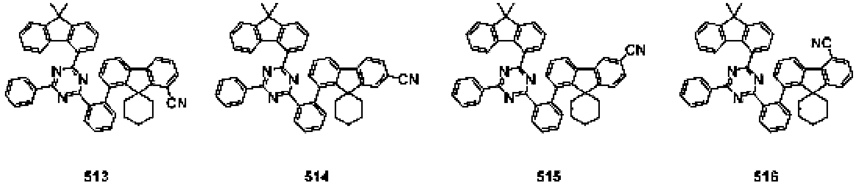

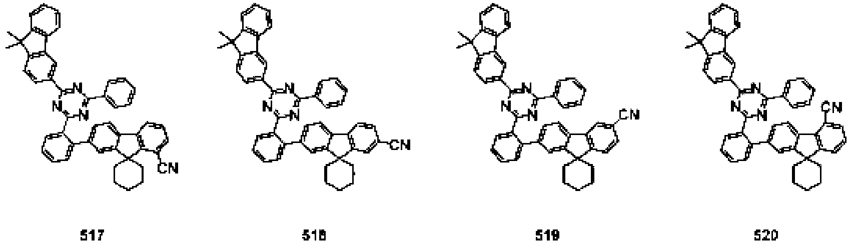

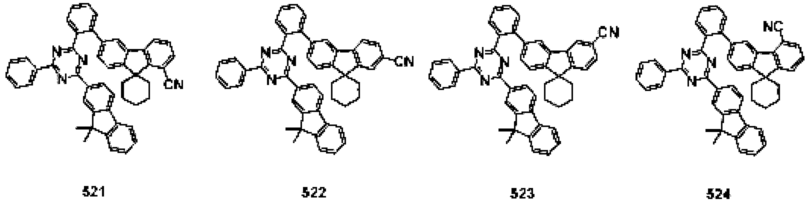

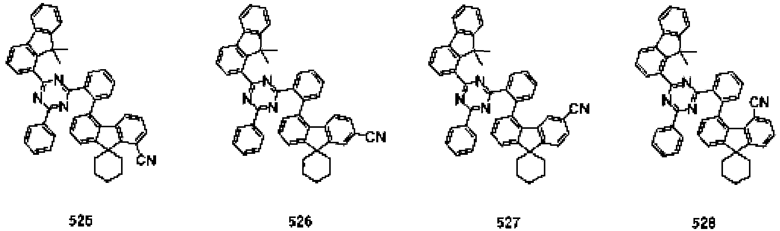

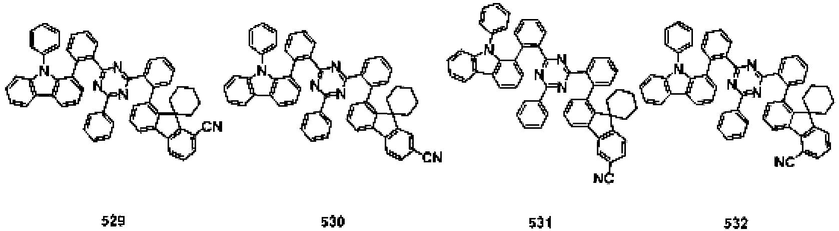

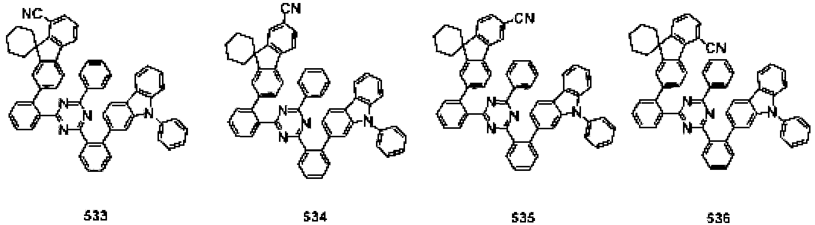

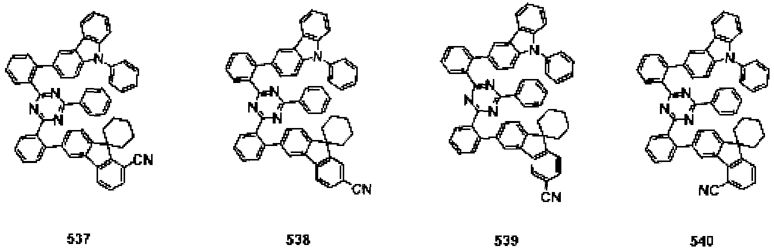

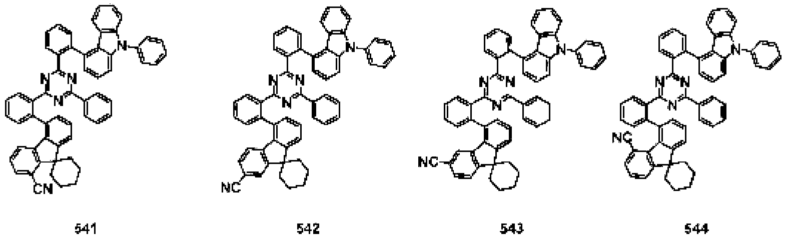

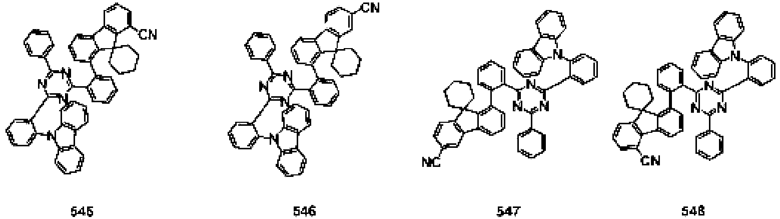

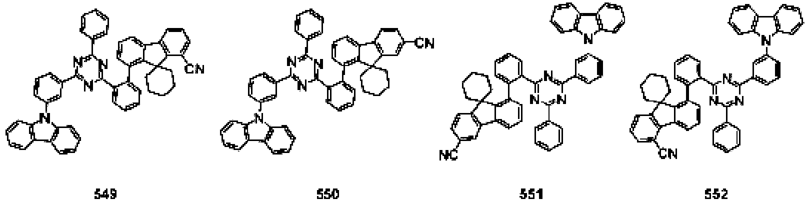

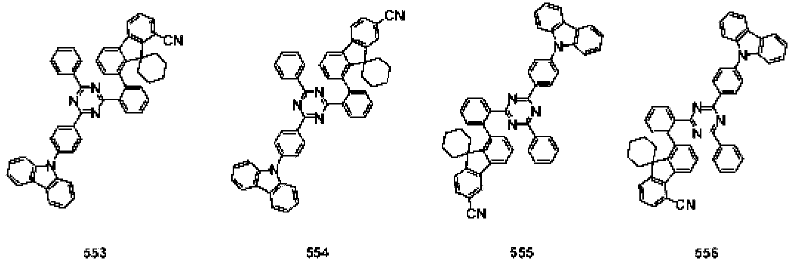

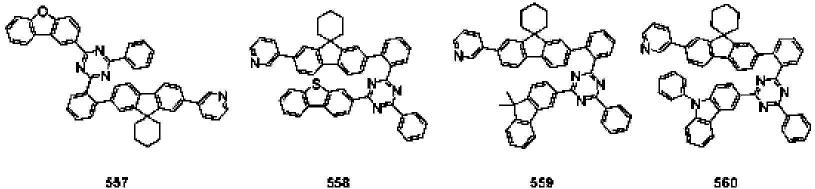

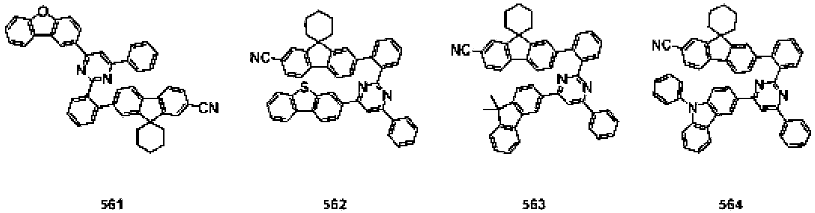

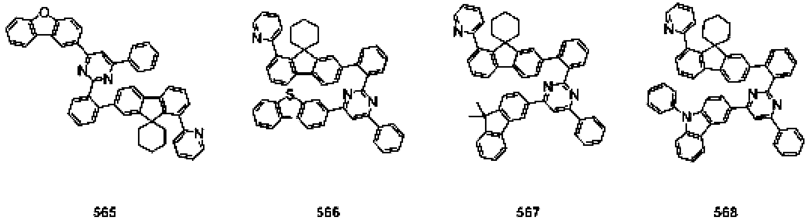

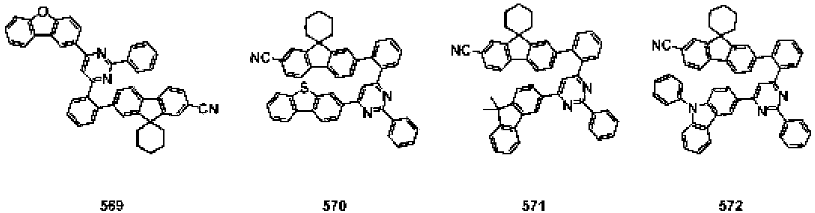

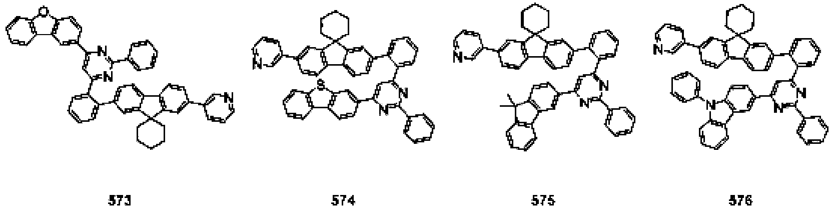

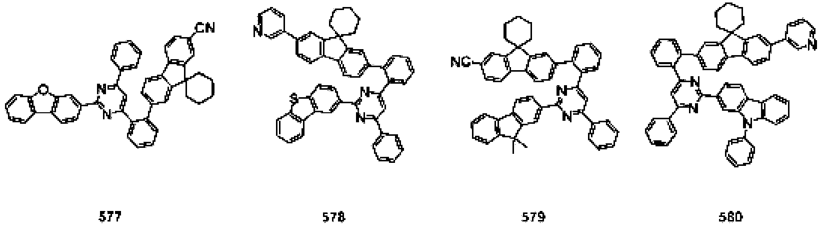

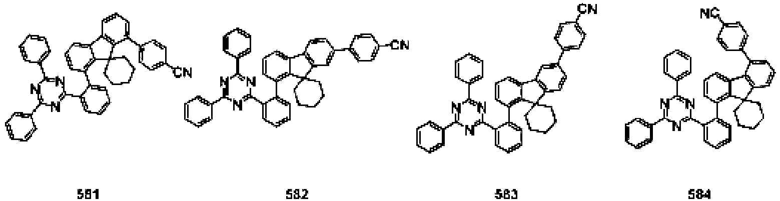

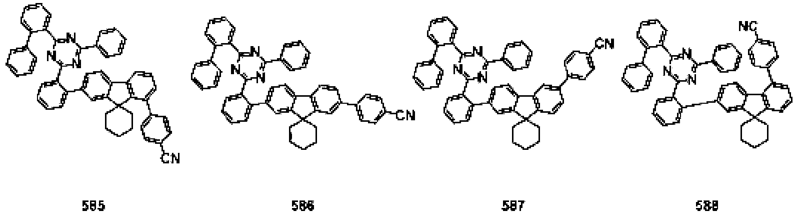

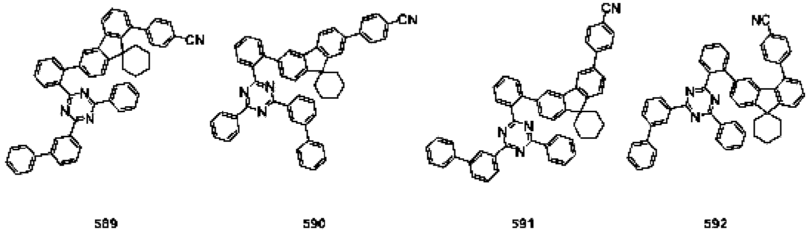

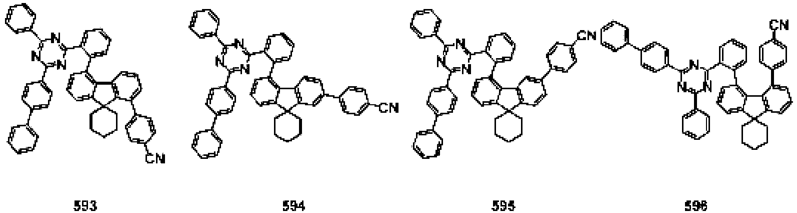

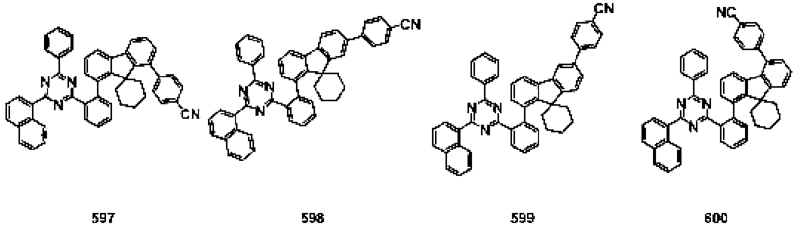

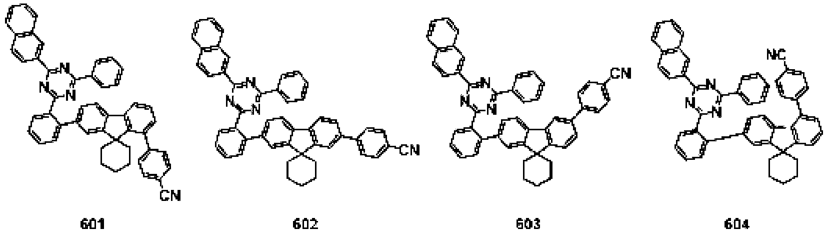

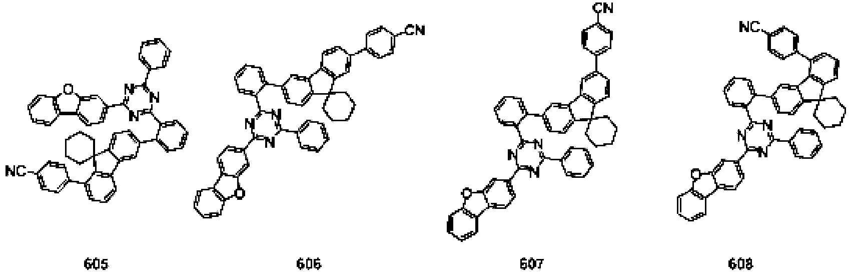

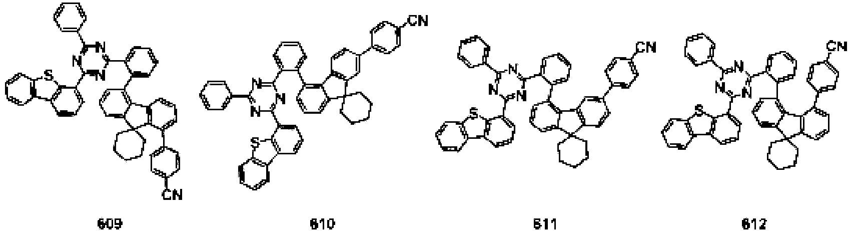

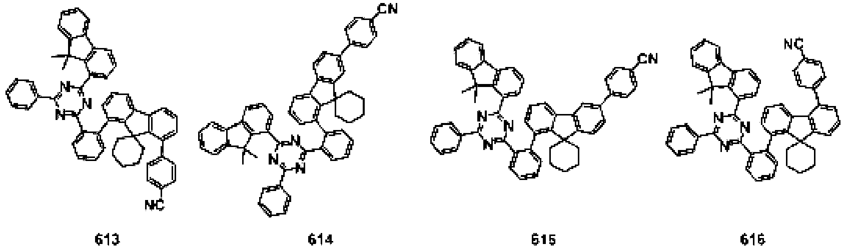

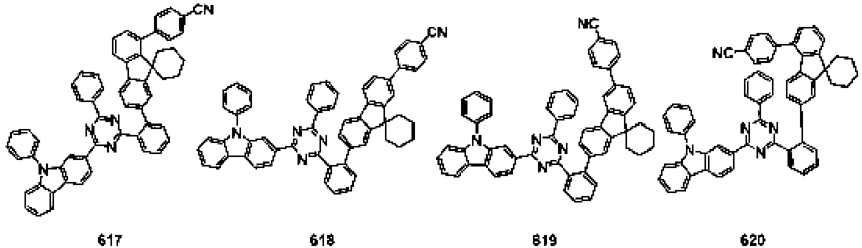

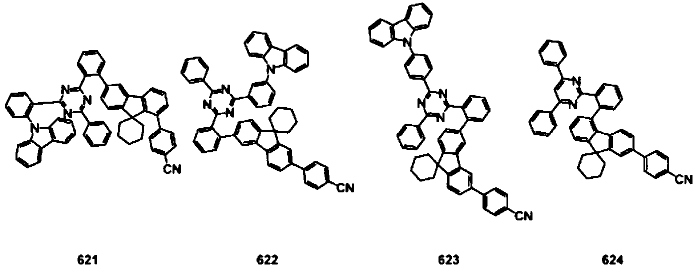

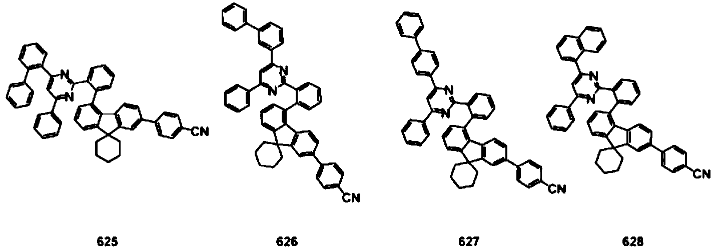

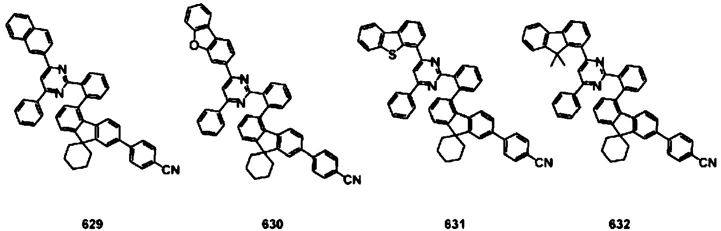

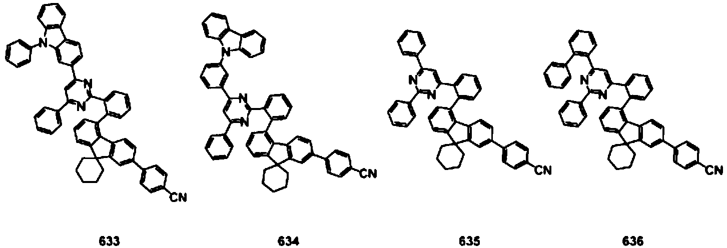

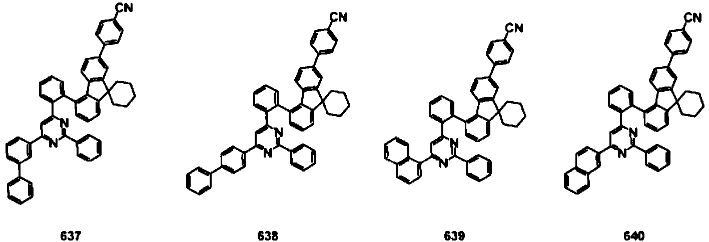

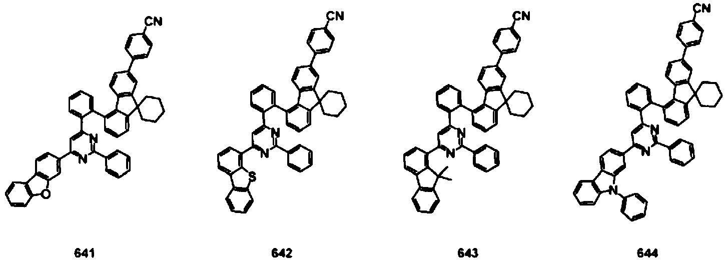

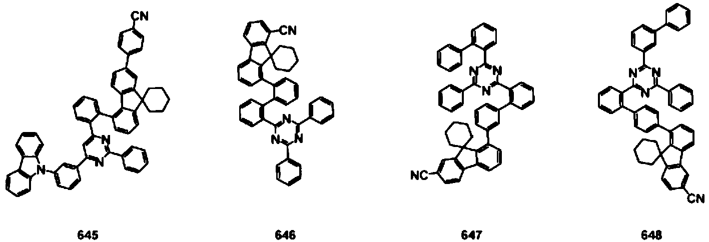

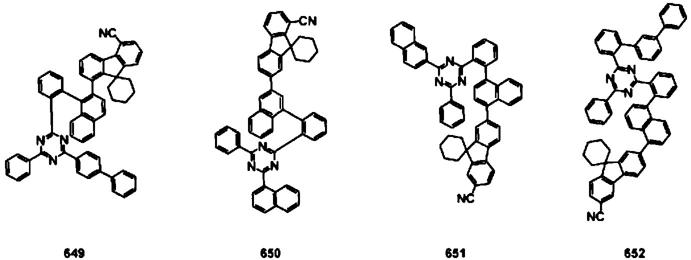

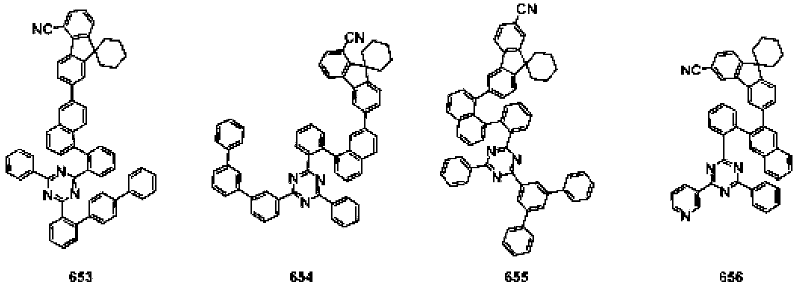

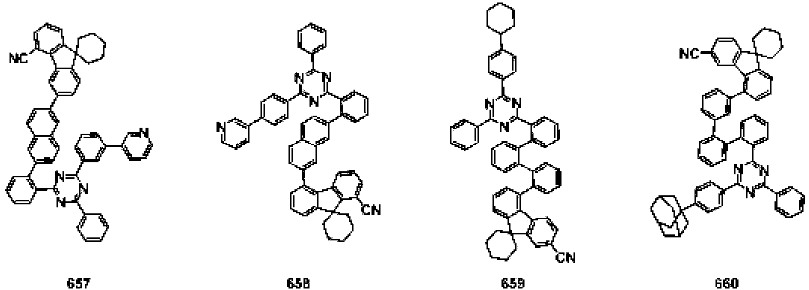

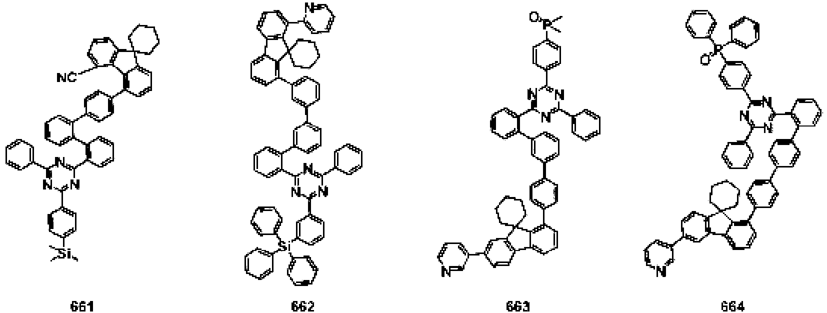

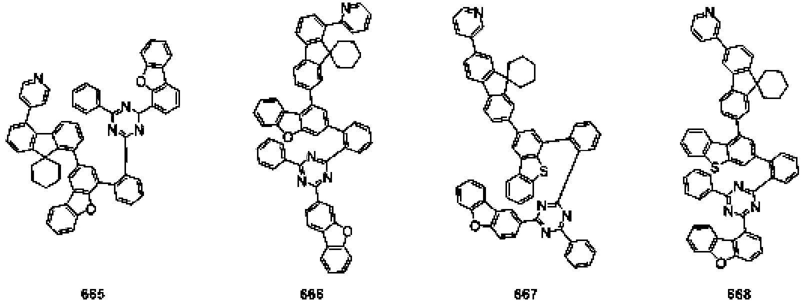

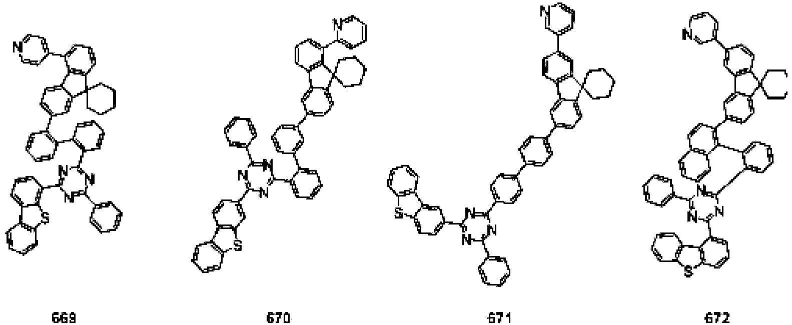

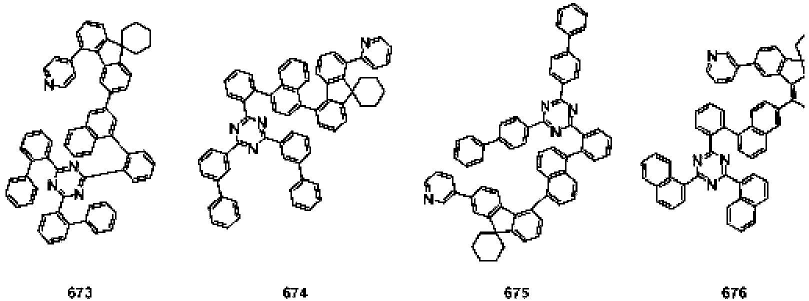

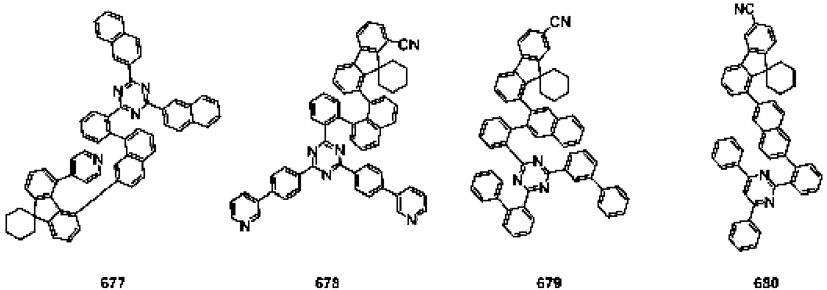

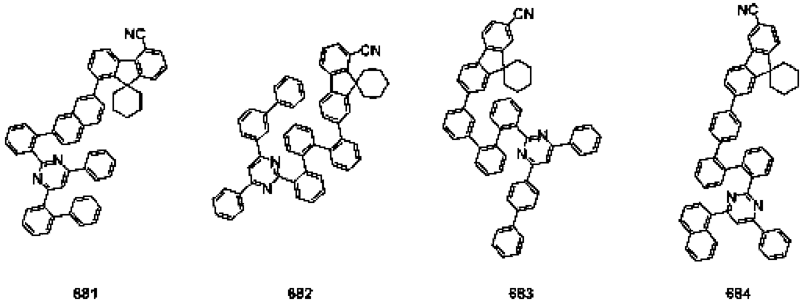

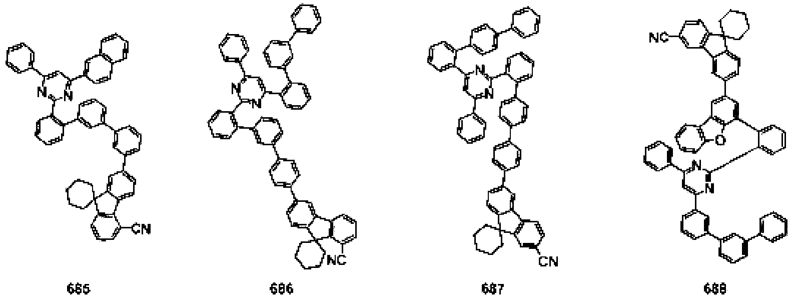

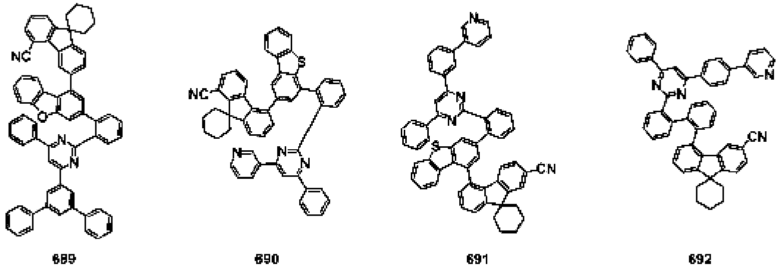

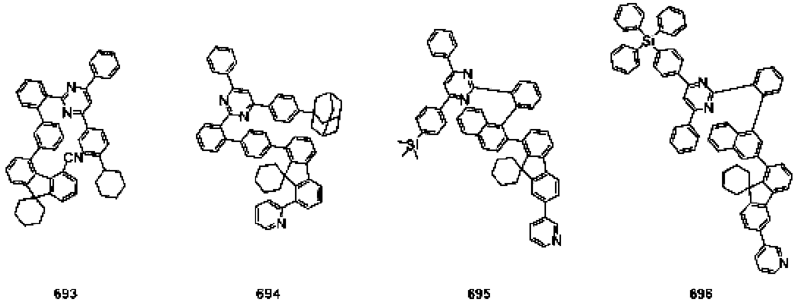

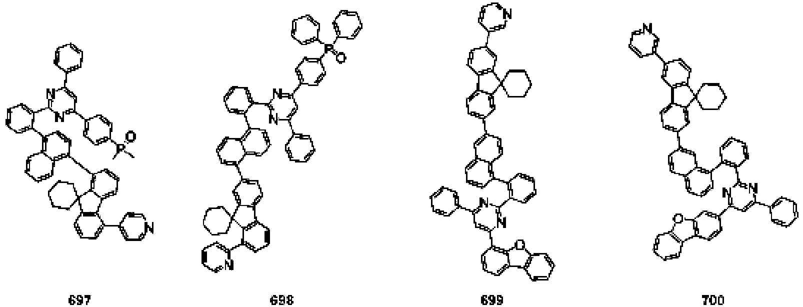

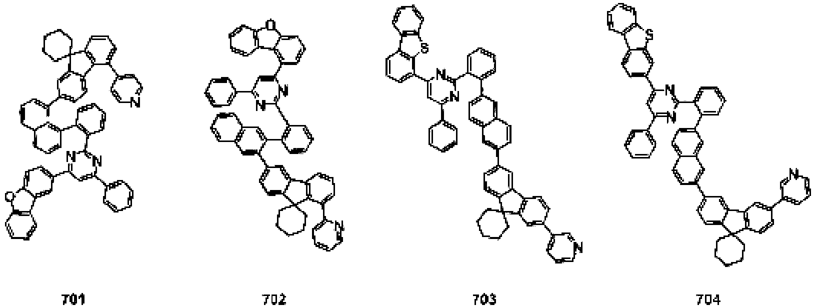

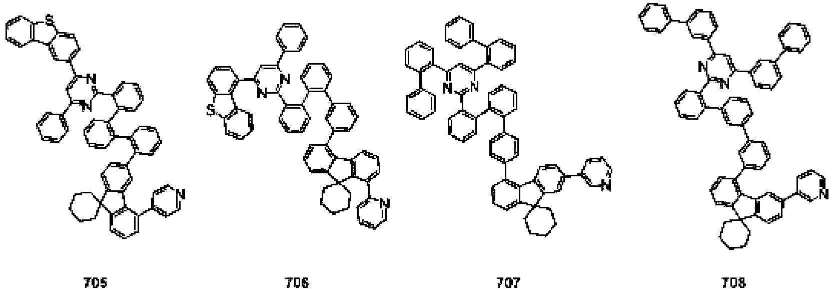

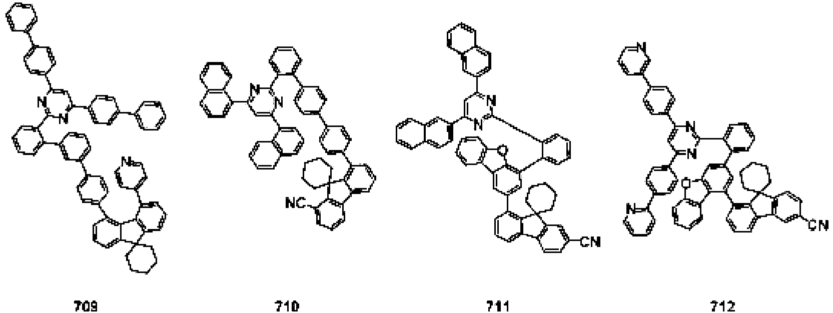

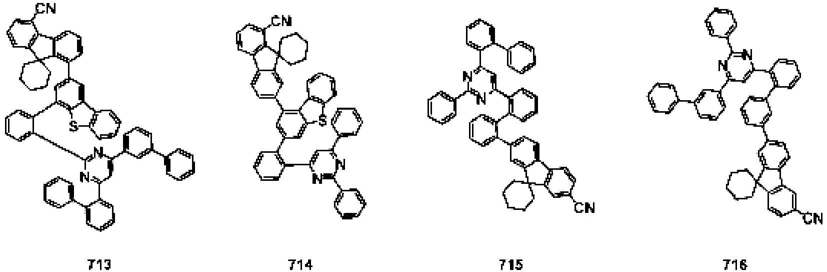

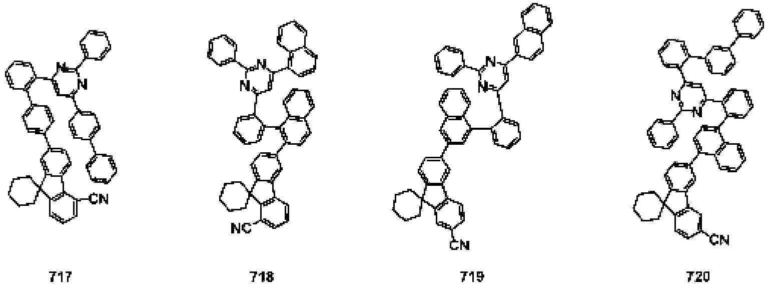

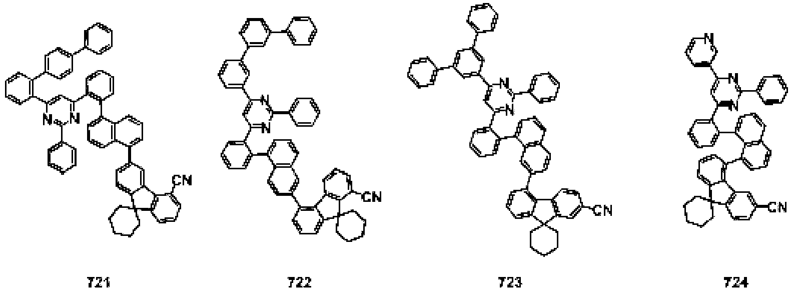

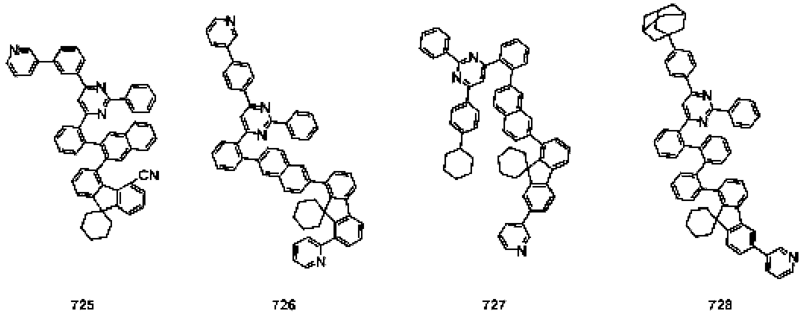

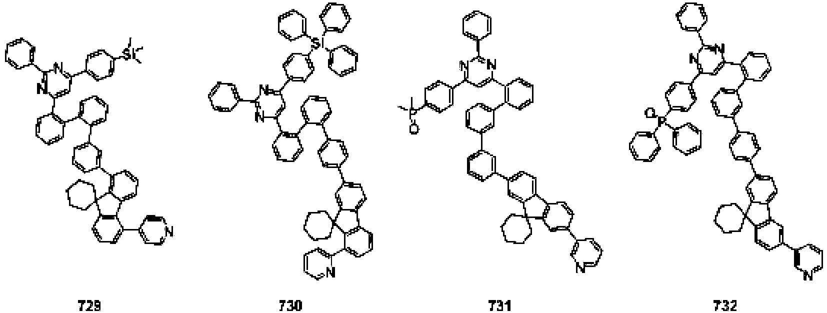

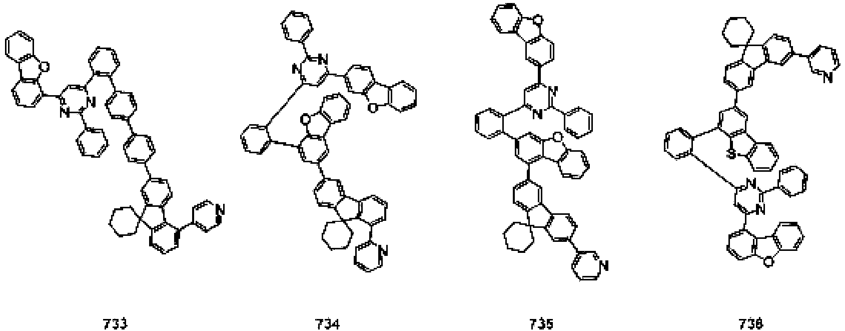

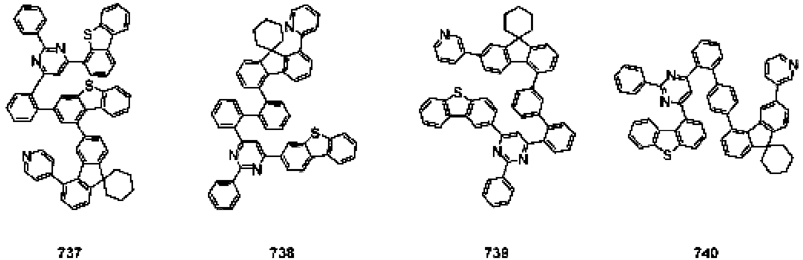

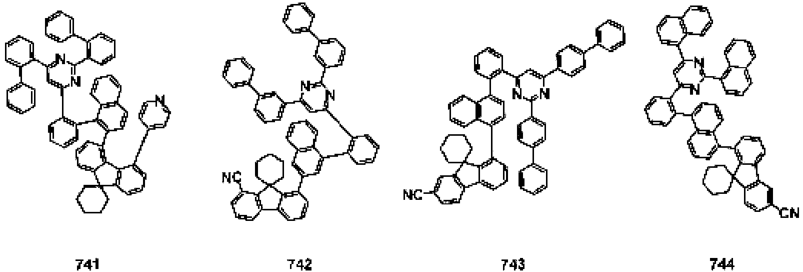

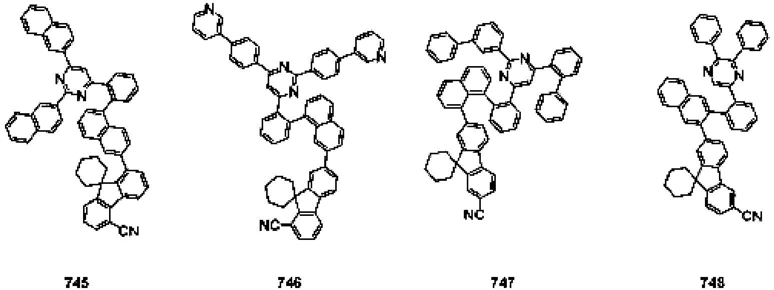

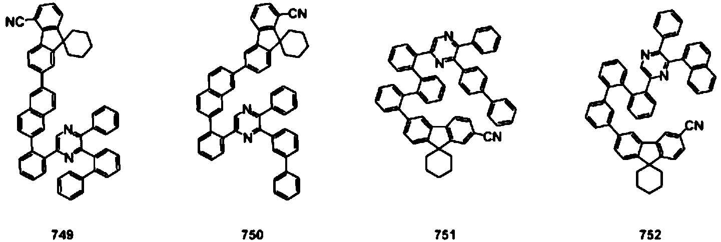

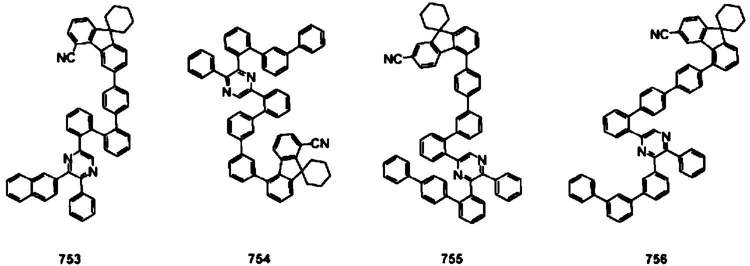

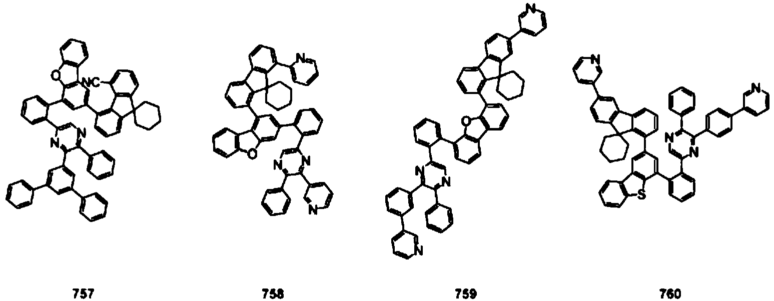

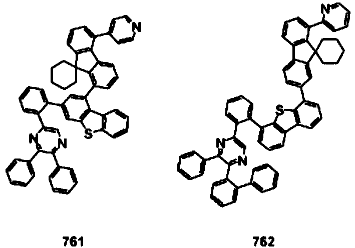

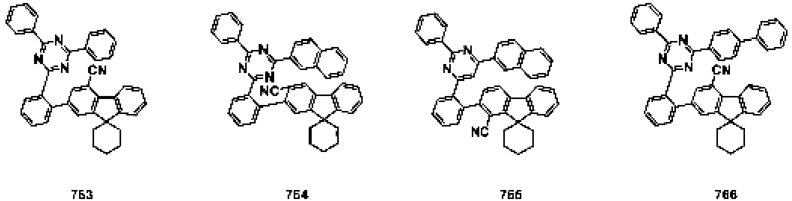

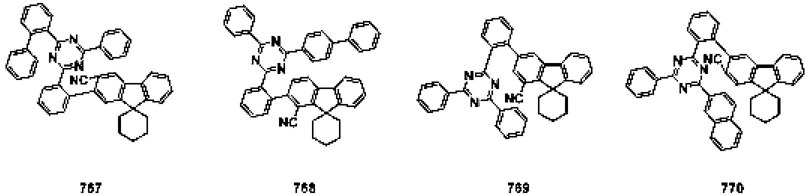

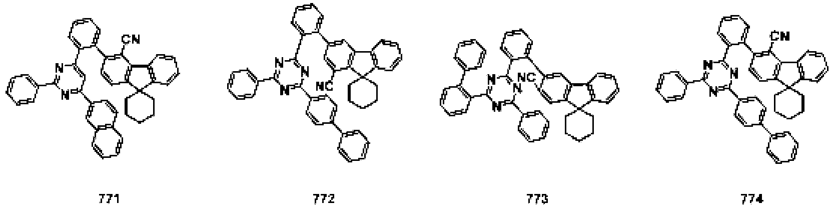

7. Organische Elektrolumineszenzvorrichtung, die die organische lichtemittierende Verbindung nach einem der Ansprüche 1 bis 6 umfasst.

8. Organische Elektrolumineszenzvorrichtung nach Anspruch 7, umfassend:
eine Anode;
eine Kathode;
eine zwischen der Kathode und der Anode angeordnete lichtemittierende Schicht; und
einen zwischen der Kathode und der lichtemittierenden Schicht angeordneten Elektronentransportbereich,
wobei der Elektronentransportbereich die organische lichtemittierende Verbindung umfasst.

9. Organische Elektrolumineszenzvorrichtung nach Anspruch 8, wobei der Elektronentransportbereich mindestens eine Elektronentransportschicht oder eine Elektronentransport-Hilfsschicht umfasst und
die organische Elektrolumineszenzverbindung in mindestens einer Schicht der Elektronentransportschicht oder der Elektronentransport-Hilfsschicht enthalten ist.

10. Verwendung der organischen lichtemittierenden Verbindung nach einem der Ansprüche 1 bis 6 für eine organische Elektrolumineszenzvorrichtung.

11. Verwendung nach Anspruch 10, wobei die organische lichtemittierende Verbindung als Elektronentransportmaterial für die organische Elektrolumineszenzvorrichtung verwendet wird.

## Revendications

1. Composé électroluminescent organique représenté par la formule 1 suivante : 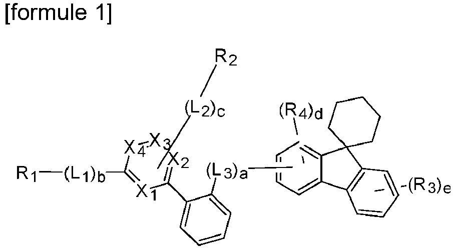 dans laquelle
l'un parmi X₁ à X₄ représente C-(L₂)_{c}R₂, les autres représentent chacun indépendamment N ou CR, et au moins deux parmi X₁ à X₄ représentent N, et dans laquelle R représente l'hydrogène, un groupe alkyle contenant 1 à 40 atomes de carbone, un groupe aryle contenant 6 à 60 atomes de carbone ou un groupe hétéroaryle contenant 2 à 60 atomes de carbone, chacun d'entre eux étant non substitué ou substitué,
R₁ et R₂ représentent chacun indépendamment l'hydrogène, un groupe alcényle contenant 2 à 40 atomes de carbone, un groupe alcynyle contenant 2 à 40 atomes de carbone, un groupe cycloalkyle contenant 3 à 40 atomes de carbone, un groupe hétérocycloalkyle contenant 1 à 40 atomes de carbone, un groupe alkyle contenant 1 à 40 atomes de carbone, un groupe aryle contenant 6 à 60 atomes de carbone, un groupe hétéroaryle contenant 2 à 60 atomes de carbone, un groupe alkylsilyle contenant 1 à 40 atomes de carbone, un groupe arylsilyle contenant 6 à 60 atomes de carbone, un groupe alkylbore contenant 1 à 40 atomes de carbone, un groupe arylbore contenant 6 à 60 atomes de carbone, un groupe arylphosphine contenant 6 à 60 atomes de carbone, un groupe oxyde d'alkylphosphine contenant 1 à 40 atomes de carbone, un groupe oxyde d'arylphosphine contenant 6 à 60 atomes de carbone, un groupe alkylamine contenant 1 à 40 atomes de carbone ou un groupe arylamine contenant 6 à 60 atomes de carbone, chacun d'entre eux étant non substitué ou substitué,
L₁ à L₃ représentent chacun indépendamment une liaison simple, un groupe arylène contenant 6 à 60 atomes de carbone ou un groupe hétéroarylène contenant 2 à 60 atomes de carbone, chacun d'entre eux étant non substitué ou substitué,
R₃ et R₄ représentent chacun indépendamment un groupe aryle contenant 6 à 60 atomes de carbone substitué par un groupe cyano, un groupe aryle contenant 6 à 60 atomes de carbone substitué par un groupe hétéroaryle contenant de l'azote contenant 2 à 60 atomes de carbone, un groupe hétéroaryle contenant de l'azote contenant 2 à 60 atomes de carbone, le deutérium ou un groupe cyano, chacun d'entre eux étant non substitué ou substitué,
a à c représentent chacun indépendamment un nombre entier compris entre 0 et 5, et
d représente un nombre entier compris entre 0 et 3, et e représente un nombre entier compris entre 0 et 4, à condition que d+e ≥ 1.

2. Composé électroluminescent organique selon la revendication 1, dans lequel
l'un parmi X₁ à X₄ représente C-(L₂)_{c}R₂, les autres représentent chacun indépendamment N ou CR, et au moins deux parmi X₁ à X₄ représentent N, et dans laquelle R représente l'hydrogène, un groupe alkyle contenant 1 à 20 atomes de carbone, un groupe aryle contenant 6 à 30 atomes de carbone ou un groupe hétéroaryle contenant 2 à 30 atomes de carbone,
R₁ et R₂ représentent chacun indépendamment l'hydrogène, un groupe alcényle contenant 2 à 40 atomes de carbone, un groupe alcynyle contenant 2 à 20 atomes de carbone, un groupe cycloalkyle contenant 3 à 20 atomes de carbone, un groupe hétérocycloalkyle contenant 1 à 20 atomes de carbone, un groupe alkyle contenant 1 à 20 atomes de carbone, un groupe aryle contenant 6 à 30 atomes de carbone, un groupe hétéroaryle contenant 2 à 30 atomes de carbone, un groupe alkylsilyle contenant 1 à 20 atomes de carbone, un groupe arylsilyle contenant 6 à 30 atomes de carbone, un groupe alkylbore contenant 1 à 20 atomes de carbone, un groupe arylbore contenant 6 à 30 atomes de carbone, un groupe arylphosphine contenant 6 à 30 atomes de carbone, un groupe oxyde d'arylphosphine contenant 1 à 20 atomes de carbone, un groupe oxyde d'arylphosphine contenant 6 à 30 atomes de carbone, un groupe alkylamine contenant 1 à 20 atomes de carbone ou un groupe arylamine contenant 6 à 30 atomes de carbone, chacun d'entre eux étant non substitué ou substitué par du deutérium, un groupe alcényle contenant 2 à 20 atomes de carbone, un groupe alcynyle contenant 2 à 20 atomes de carbone, un groupe cycloalkyle contenant 3 à 20 atomes de carbone, un groupe hétérocycloalkyle contenant 1 à 20 atomes de carbone, un groupe alkyle contenant 1 à 20 atomes de carbone, un groupe aryle contenant 6 à 30 atomes de carbone, un groupe hétéroaryle contenant 2 à 30 atomes de carbone, un groupe alkylsilyle présentant 1 à 20 atomes de carbone, un groupe arylsilyle présentant 6 à 30 atomes de carbone, un groupe alkylbore présentant 1 à 20 atomes de carbone, un groupe arylbore présentant 6 à 30 atomes de carbone, un groupe arylphosphine présentant 6 à 30 atomes de carbone, un groupe oxyde d'alkylphosphine présentant 1 à 20 atomes de carbone, un groupe oxyde d'arylphosphine présentant 6 à 30 atomes de carbone, un groupe alkylamine ayant 1 à 20 atomes de carbone ou un groupe arylamine ayant 6 à 30 atomes de carbone,
L₁ à L₃ représentent chacun indépendamment une liaison simple, un groupe arylène contenant 6 à 30 atomes de carbone ou un groupe hétéroarylène contenant 2 à 30 atomes de carbone,
R₃ et R₄ représentent chacun indépendamment un groupe aryle contenant 6 à 30 atomes de carbone substitué par un groupe cyano, un groupe aryle contenant 6 à 30 atomes de carbone substitué par un groupe hétéroaryle contenant de l'azote contenant 2 à 30 atomes de carbone, un groupe hétéroaryle contenant de l'azote contenant 2 à 30 atomes de carbone, le deutérium ou un groupe cyano,
a à c représentent chacun indépendamment un nombre entier compris entre 0 et 2, et
d représente un nombre entier compris entre 0 et 2 et e représente un nombre entier compris entre 0 et 2, à condition que d+e ≥ 1.

3. Composé électroluminescent organique selon la revendication 1, dans lequel
l'un parmi X₁ à X₄ représente C-(L₂)_{c}R₂, les autres représentent chacun indépendamment N ou CR, et au moins deux parmi X₁ à X₄ représentent N,
R₁ et R₂ représentent chacun indépendamment l'hydrogène, un groupe aryle contenant 6 à 30 atomes de carbone ou un groupe hétéroaryle contenant 2 à 30 atomes de carbone, chacun d'entre eux étant non substitué ou substitué par un groupe cycloalkyle contenant 3 à 20 atomes de carbone, un groupe alkyle contenant 1 à 20 atomes de carbone, un groupe aryle contenant 6 à 30 atomes de carbone, un groupe hétéroaryle contenant 2 à 30 atomes de carbone, un groupe alkylsilyle contenant 1 à 20 atomes de carbone, un groupe arylsilyle contenant 6 à 30 atomes de carbone, un groupe oxyde d'alkylphosphine contenant 1 à 20 atomes de carbone ou un groupe oxyde d'arylphosphine contenant 6 à 30 atomes de carbone,
L₁ à L₃ représentent chacun indépendamment une liaison simple, un groupe arylène contenant 6 à 30 atomes de carbone ou un groupe hétéroarylène contenant 2 à 30 atomes de carbone,
R₃ et R₄ représentent chacun indépendamment un groupe aryle contenant 6 à 30 atomes de carbone substitué par un groupe cyano, un groupe hétéroaryle contenant de l'azote contenant 2 à 30 atomes de carbone ou un groupe cyano,
a à c représentent chacun indépendamment un nombre entier compris entre 0 et 2, et
d représente un nombre entier compris entre 0 et 2 et e représente un nombre entier compris entre 0 et 2, à condition que d+e ≥ 1.

4. Composé électroluminescent organique selon la revendication 1, dans lequel la formule 1 est représentée par l'une quelconque des formules 2 à 9 ci-dessous : 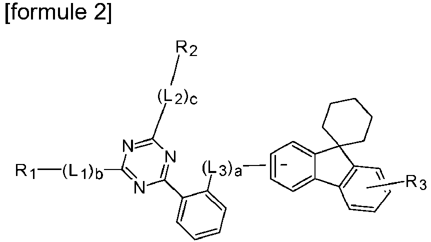 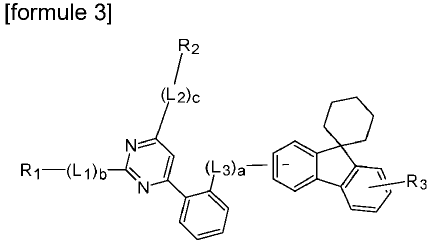 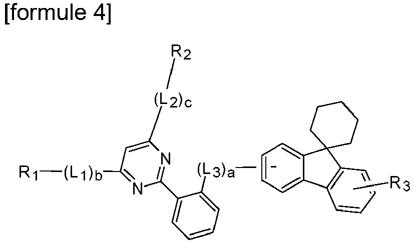 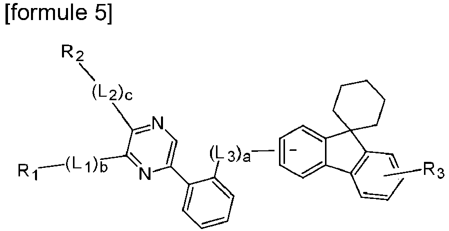 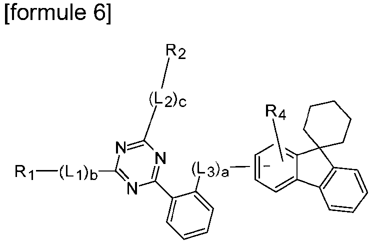 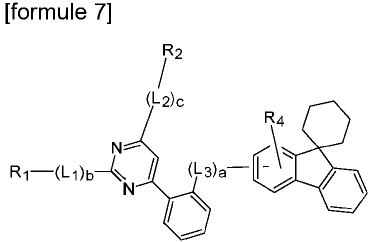 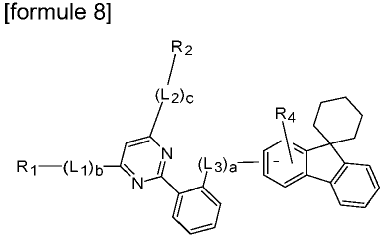 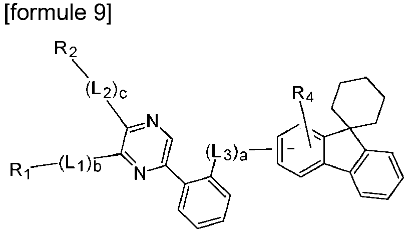 dans lequel, dans chacune des formules 2 à 9,
R₁ et R₂ représentent chacun indépendamment l'hydrogène, un groupe aryle contenant 6 à 30 atomes de carbone ou un groupe hétéroaryle contenant 2 à 30 atomes de carbone, chacun d'entre eux étant non substitué ou substitué par un groupe cycloalkyle contenant 3 à 20 atomes de carbone, un groupe alkyle contenant 1 à 20 atomes de carbone, un groupe aryle contenant 6 à 30 atomes de carbone, un groupe hétéroaryle contenant 2 à 30 atomes de carbone, un groupe alkylsilyle contenant 1 à 20 atomes de carbone, un groupe arylsilyle contenant 6 à 30 atomes de carbone, un groupe oxyde d'alkylphosphine contenant 1 à 20 atomes de carbone ou un groupe oxyde d'arylphosphine contenant 6 à 30 atomes de carbone,
L₁ à L₃ représentent chacun indépendamment une liaison simple, un groupe arylène contenant 6 à 30 atomes de carbone ou un groupe hétéroarylène contenant 2 à 30 atomes de carbone,
R₃ et R₄ représentent chacun indépendamment un groupe aryle contenant 6 à 30 atomes de carbone substitué par un groupe cyano, un groupe hétéroaryle contenant de l'azote contenant 2 à 30 atomes de carbone ou un groupe cyano, et
a à c représentent chacun indépendamment un nombre entier compris entre 0 et 2.

5. Composé électroluminescent organique selon la revendication 4, dans lequel la formule 1 est représentée par l'une quelconque des formules 2 à 7 ci-dessous : 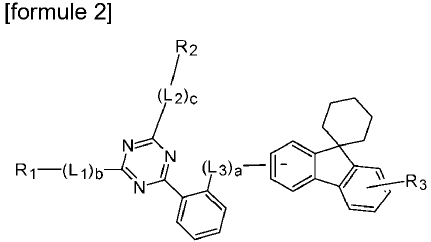 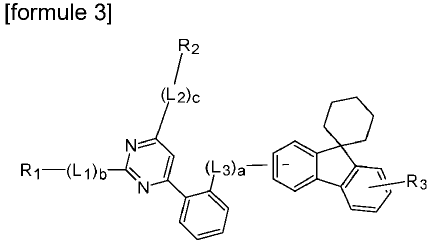 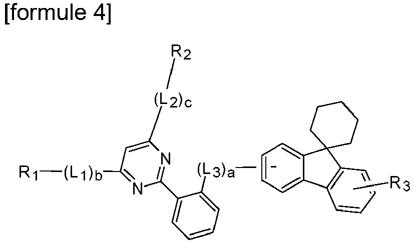 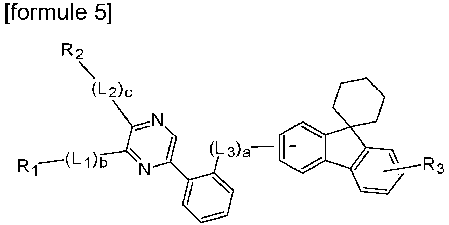 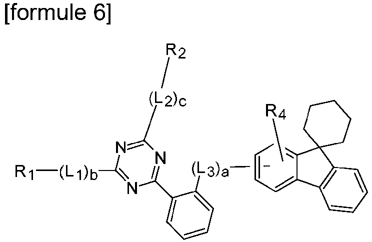 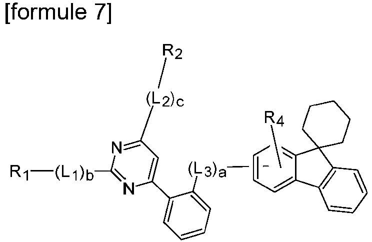 où, dans chacune des formules 2 à 7,
R₁ et R₂ représentent chacun indépendamment l'hydrogène, un groupe phényle, un groupe biphényle, un groupe naphtyle, un groupe terphényle, un groupe fluorényle, un groupe pyridyle, un groupe carbazolyle, un groupe dibenzothiophényle ou un groupe dibenzofuranyle, chacun d'entre eux étant non substitué ou substitué par un groupe méthyle, un groupe phényle, un groupe cyclohexyle, un groupe adamantyle, un groupe pyridyle, un groupe carbazolyle, un groupe dibenzothiophényle, un groupe dibenzofuranyle, un groupe triméthylsilyle, un groupe triphénylsilyle, un groupe oxyde de diméthylphosphine ou un groupe oxyde de diphénylphosphine,
L₁ à L₃ représentent chacun indépendamment une liaison simple, un groupe phénylène, un groupe biphénylène, un groupe naphtylène, un groupe terphénylène, un groupe dibenzofuranylène ou un groupe dibenzothiophénylène,
R₃ et R₄ représentent chacun indépendamment un groupe phényle substitué par un groupe cyano, un groupe pyridyle ou un groupe cyano, et
a à c représentent chacun indépendamment un nombre entier compris entre 0 et 2.

6. Composé électroluminescent organique selon la revendication 1, le composé électroluminescent organique représenté par la formule 1 étant l'un quelconque choisi parmi les composés 001 à 774 suivants.

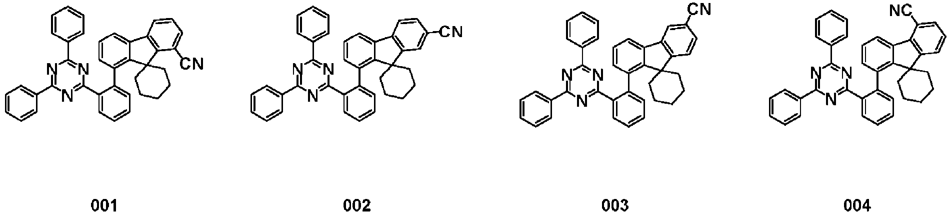

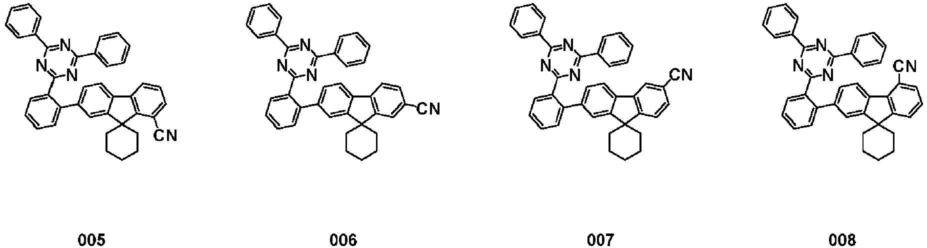

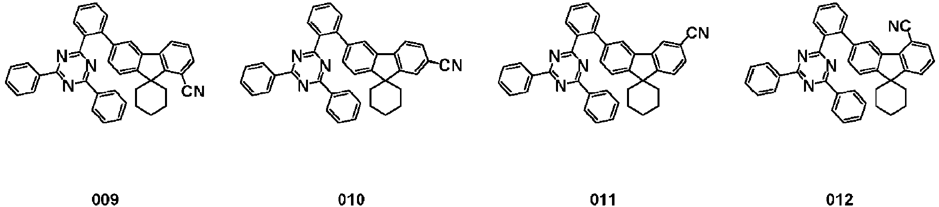

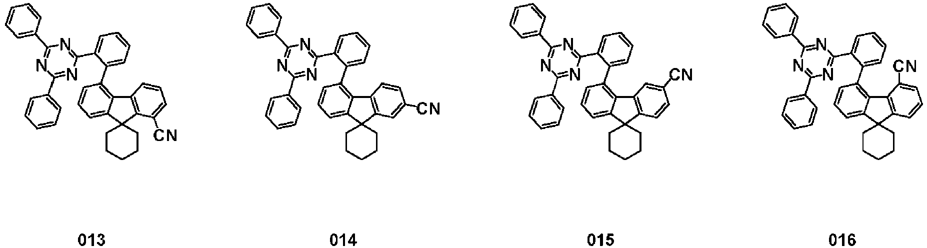

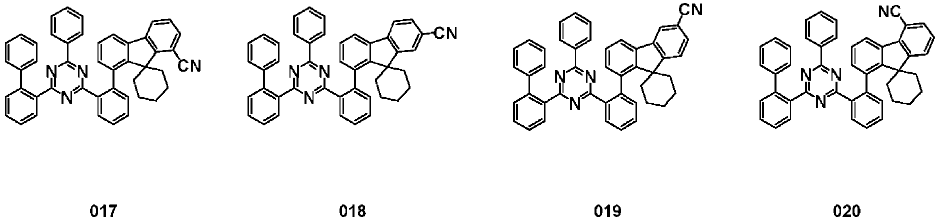

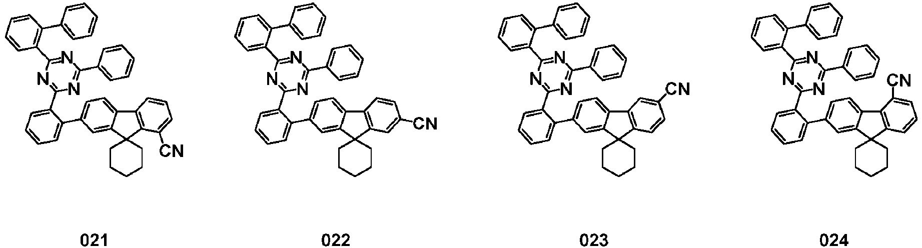

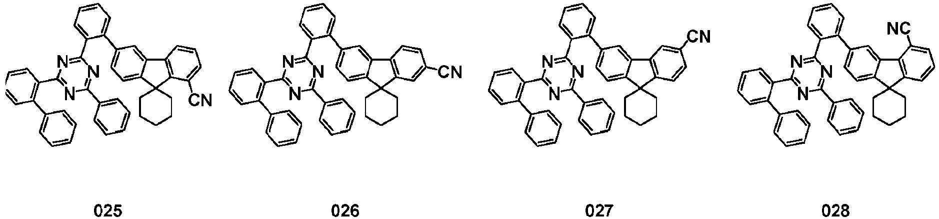

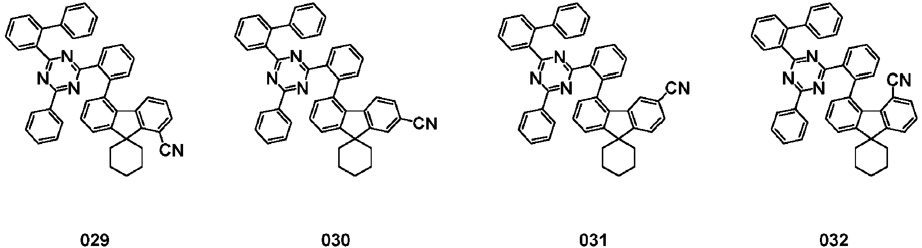

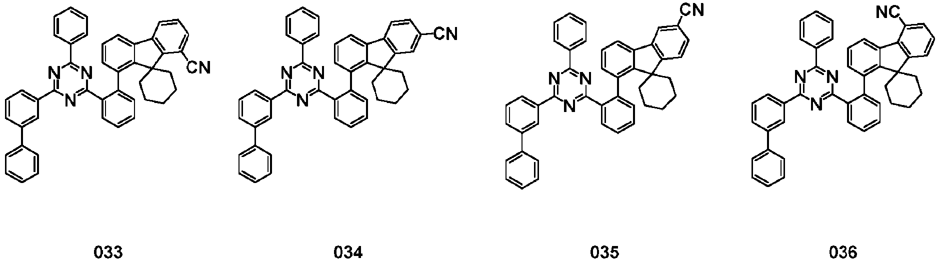

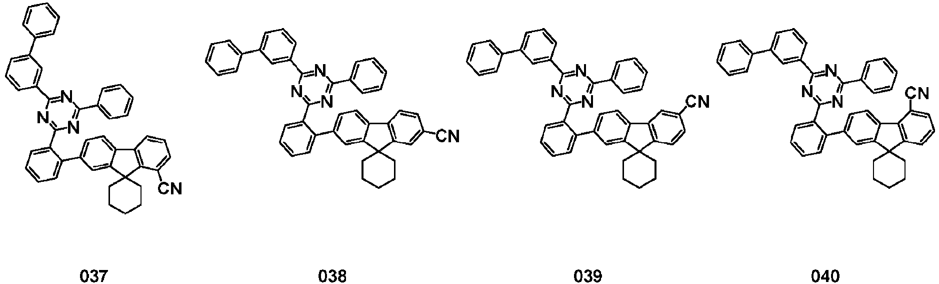

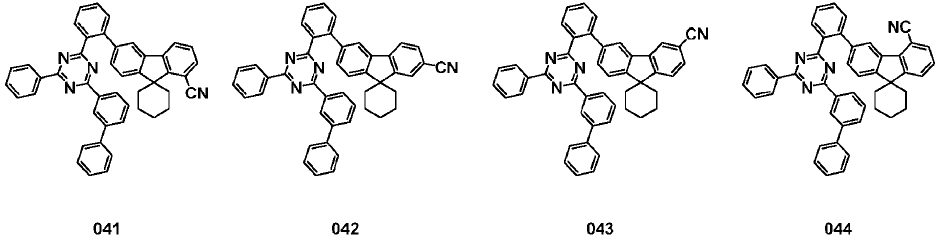

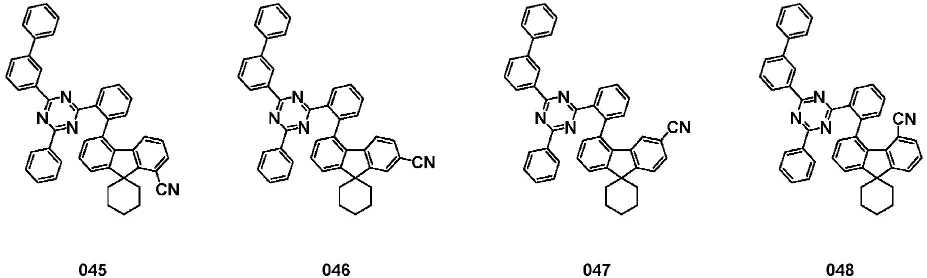

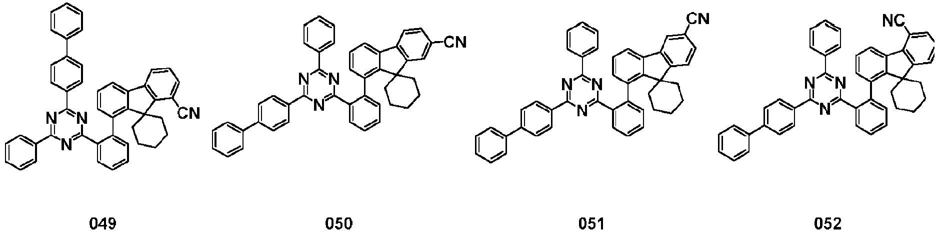

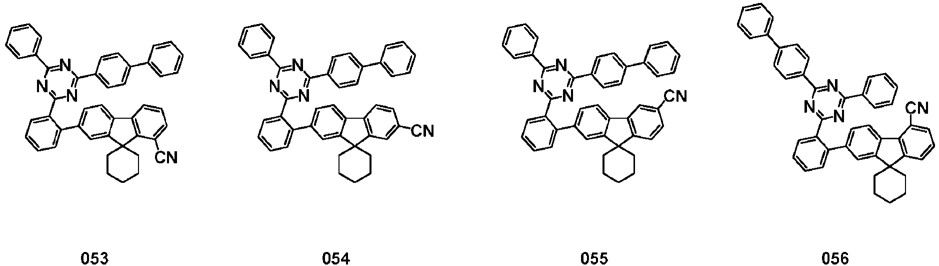

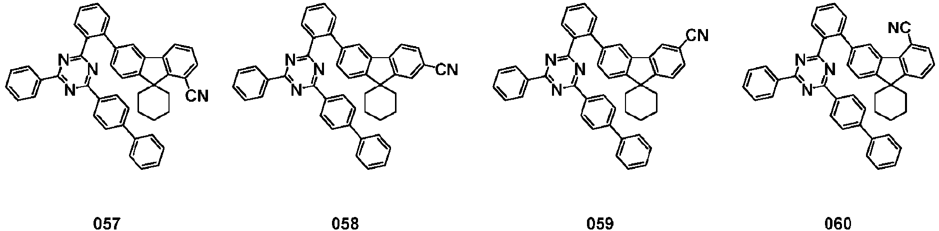

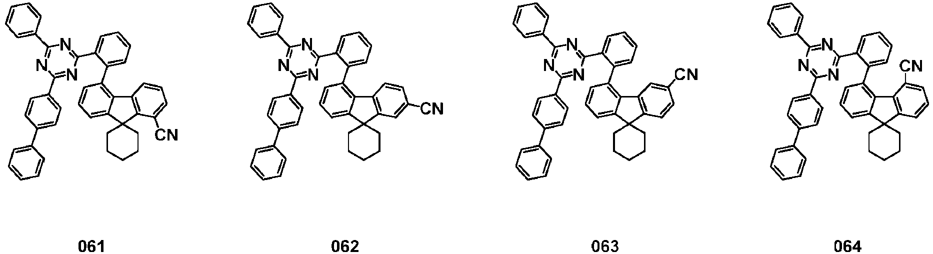

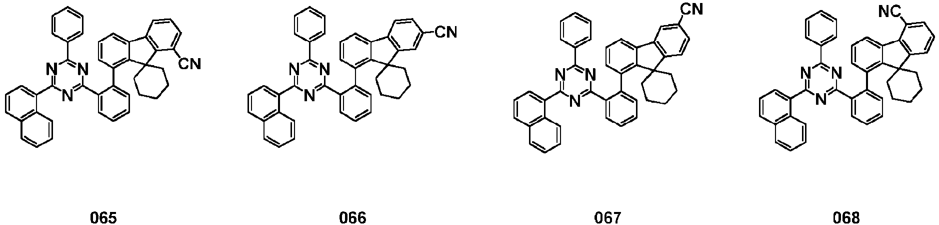

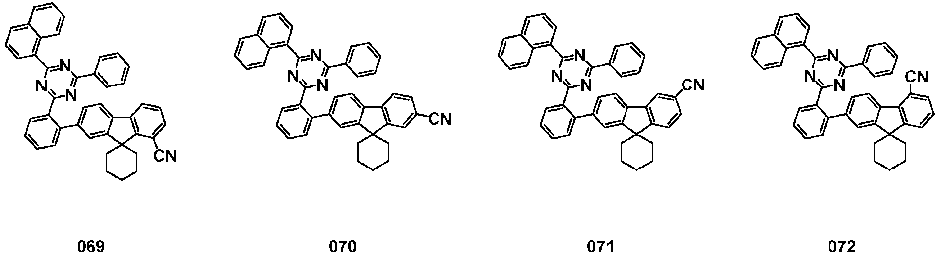

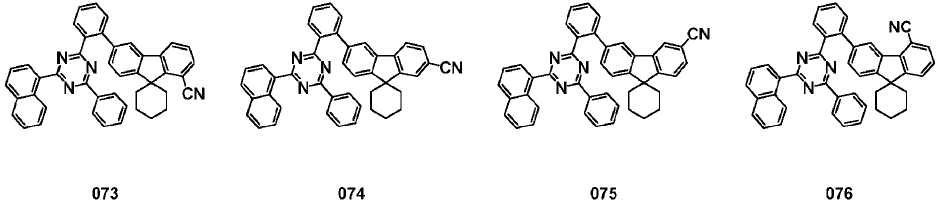

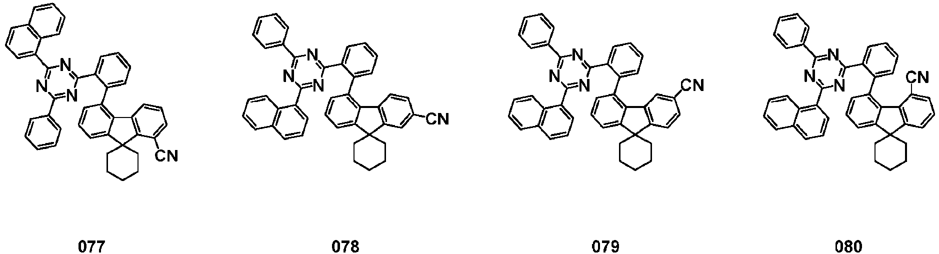

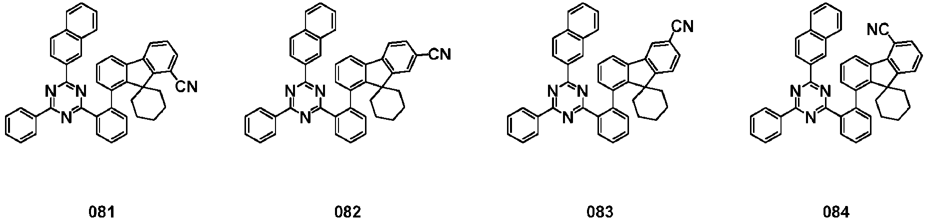

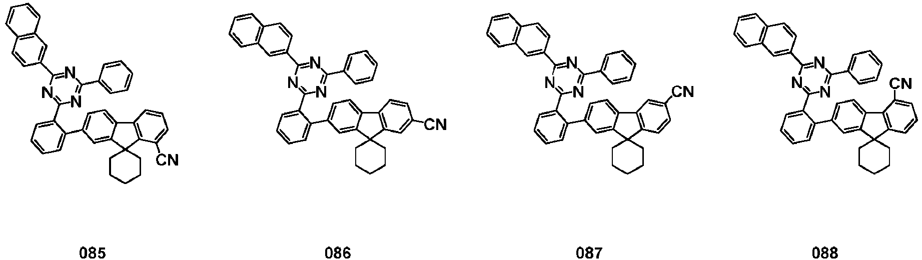

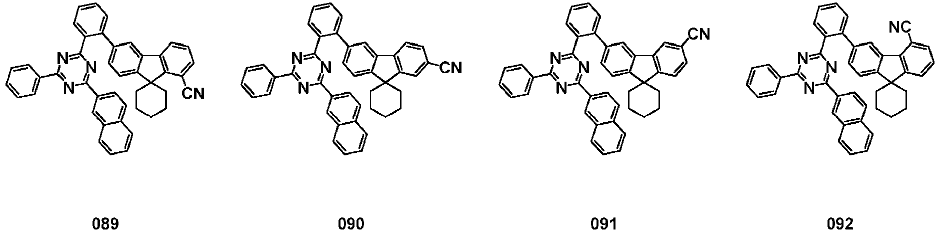

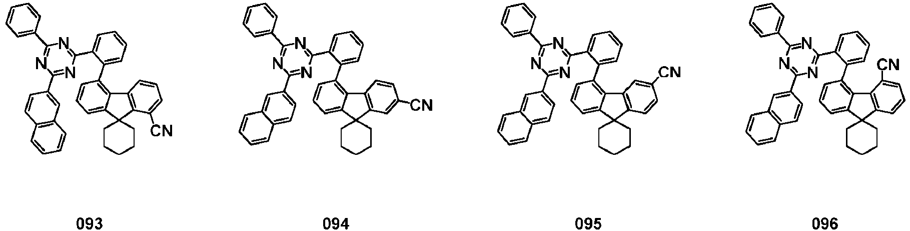

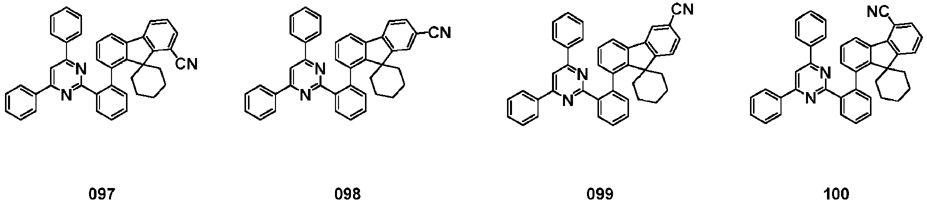

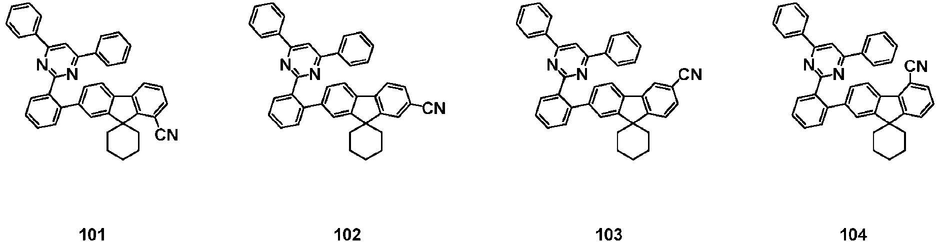

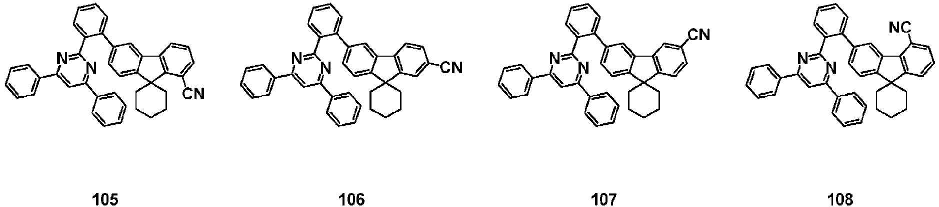

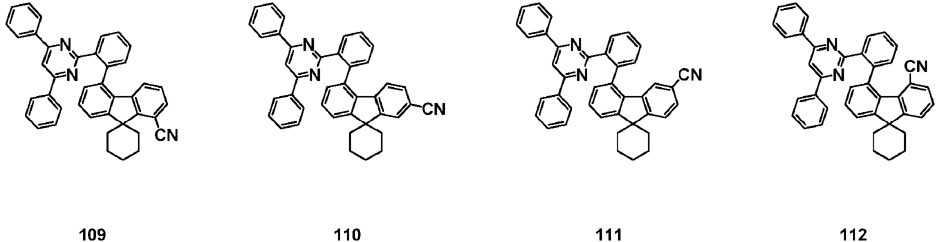

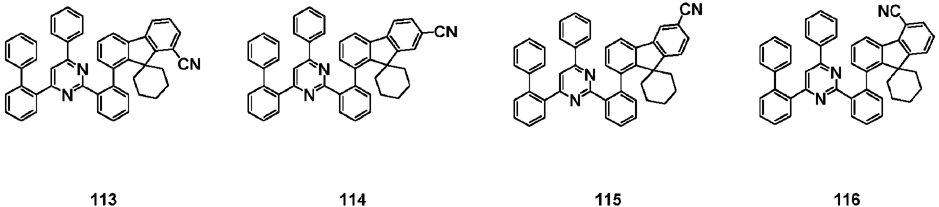

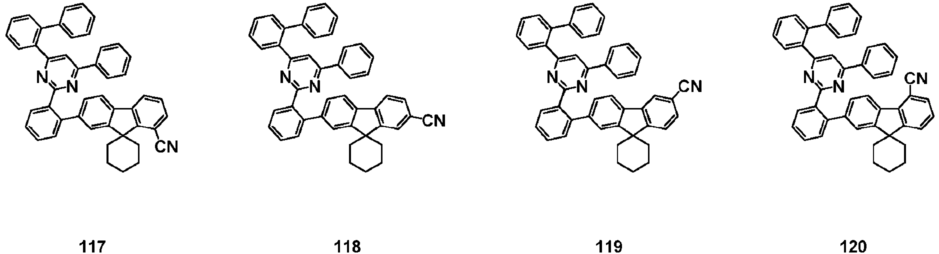

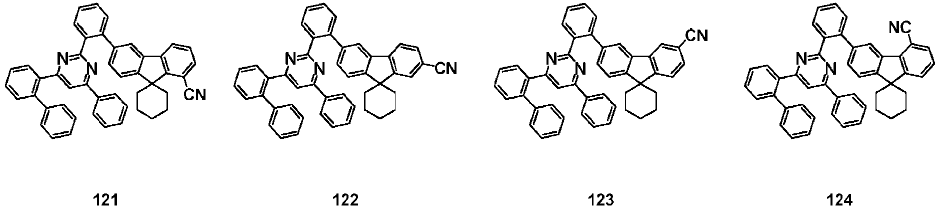

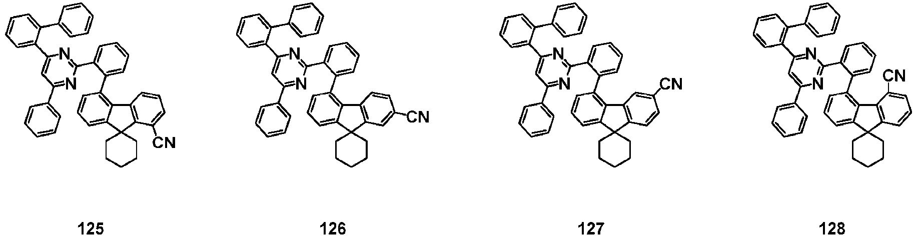

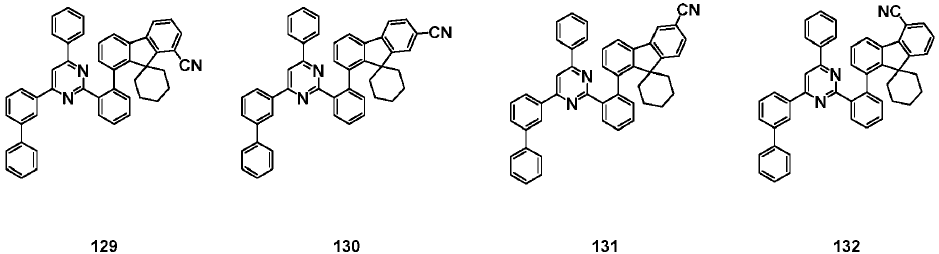

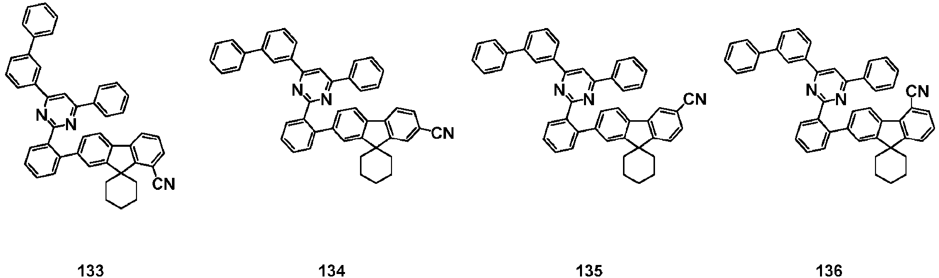

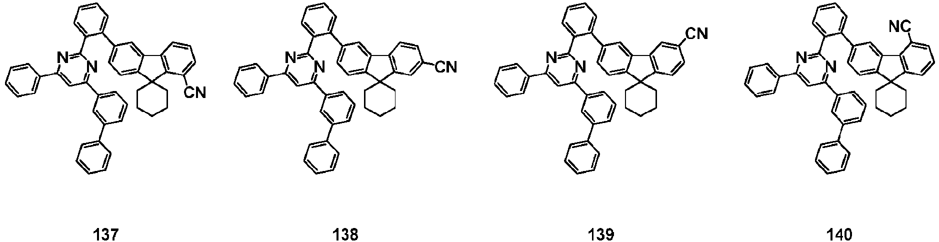

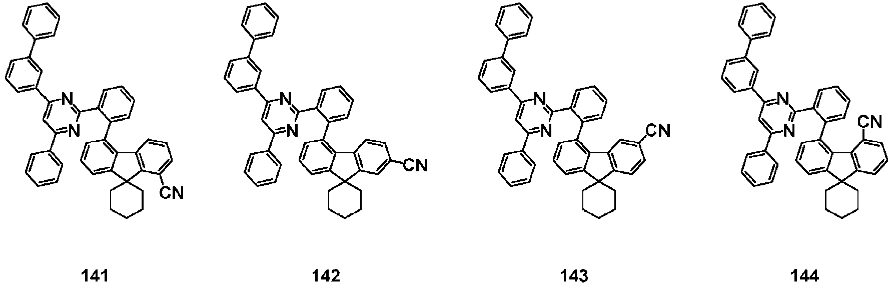

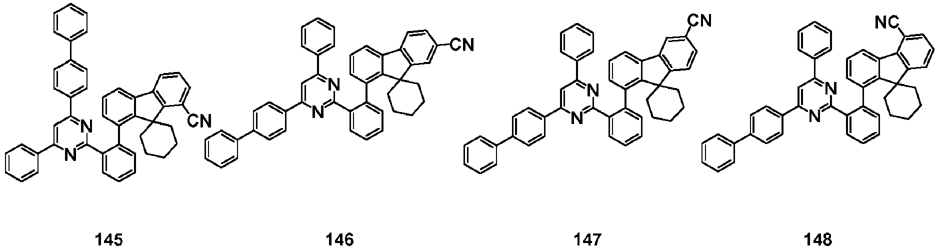

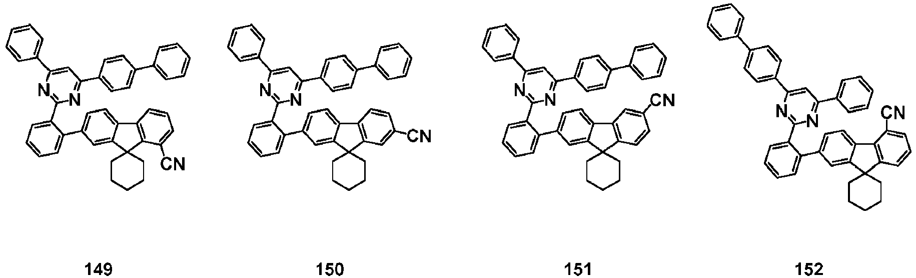

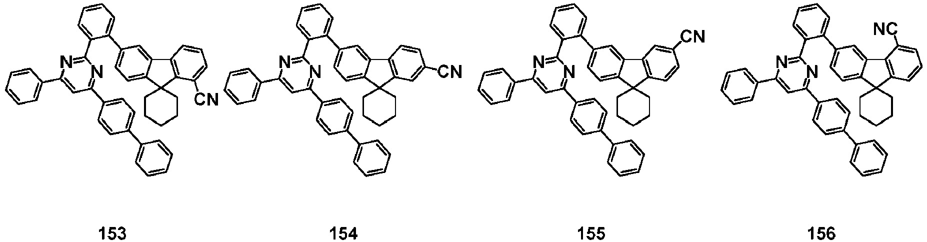

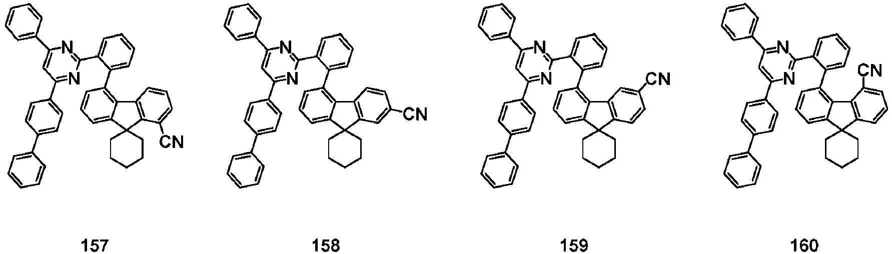

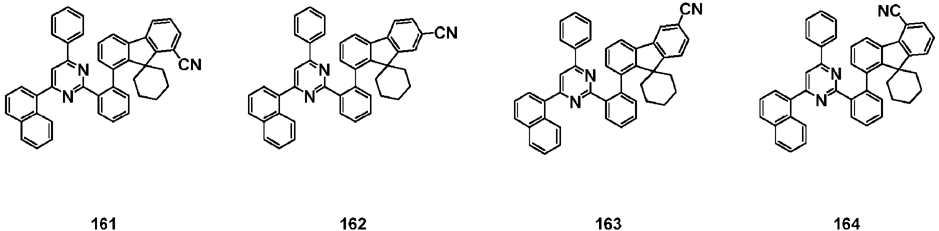

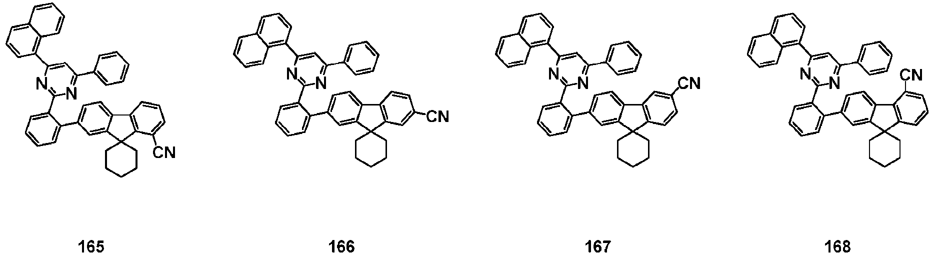

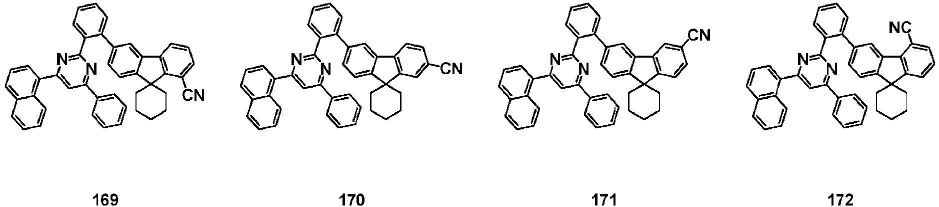

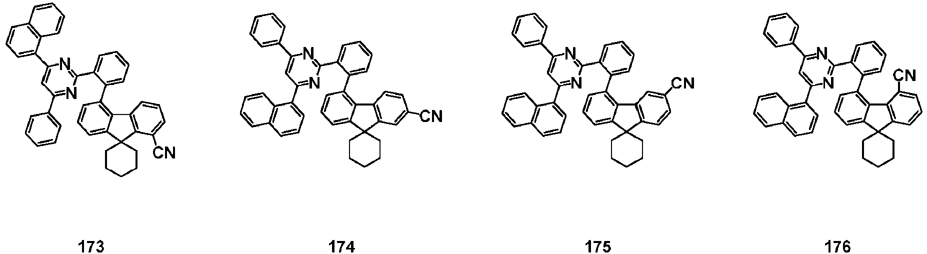

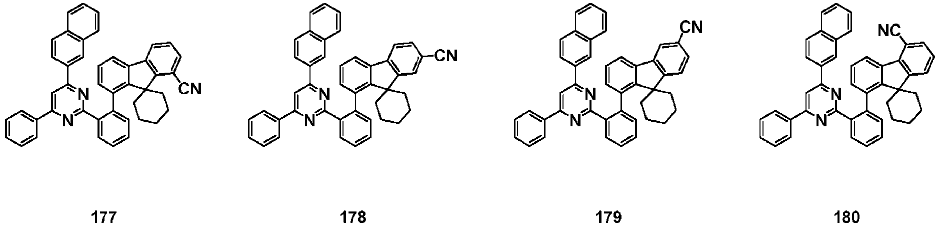

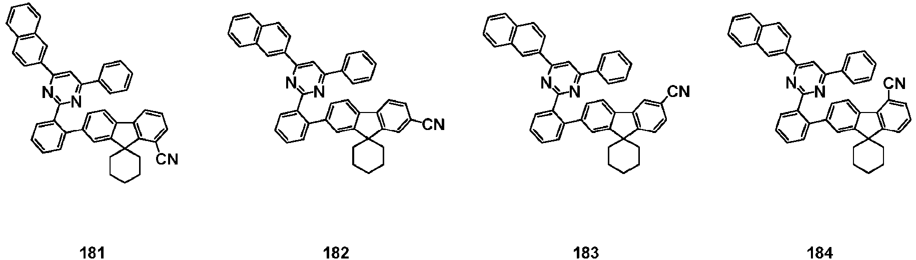

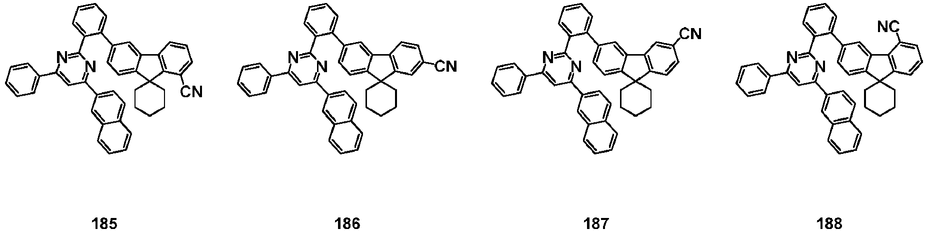

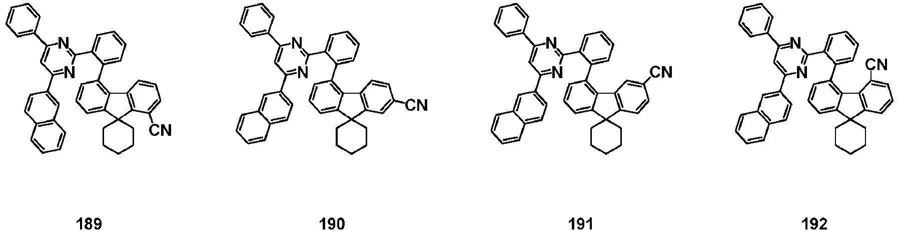

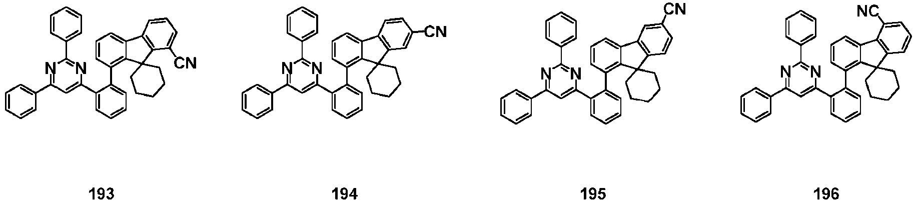

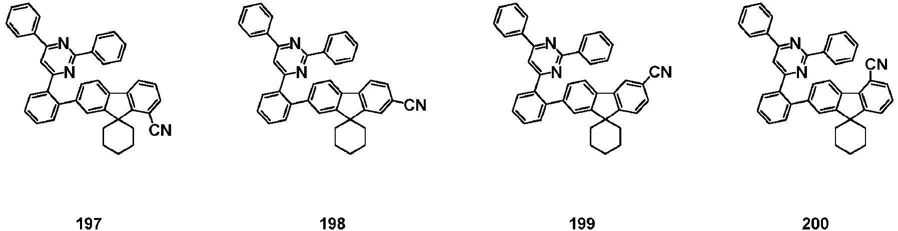

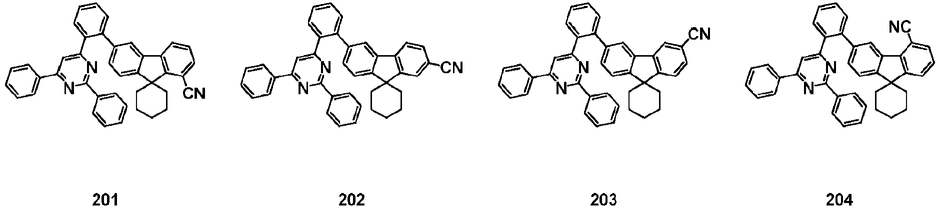

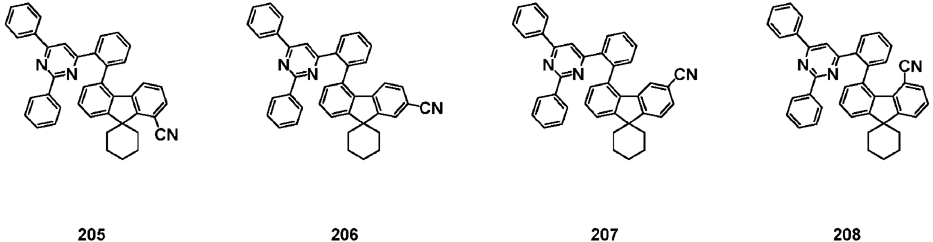

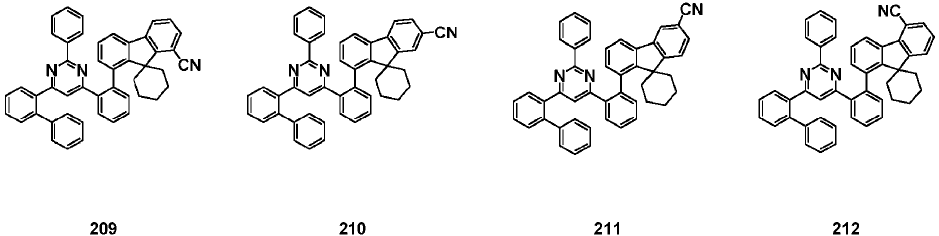

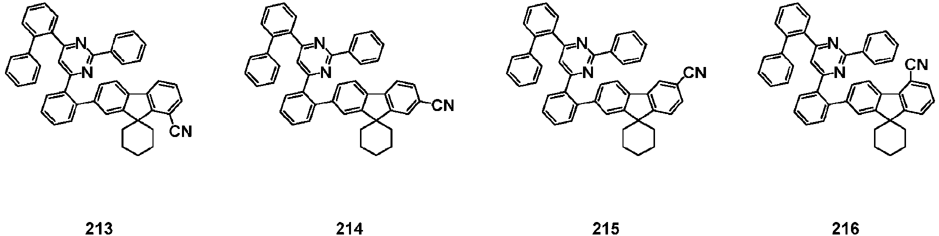

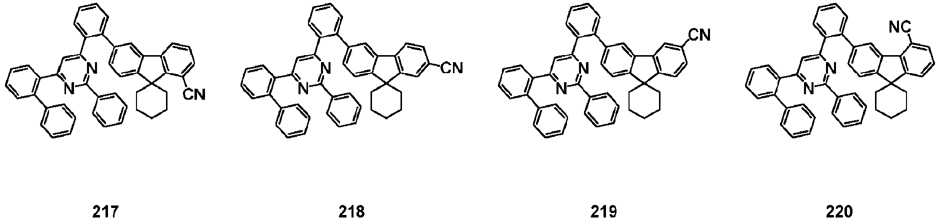

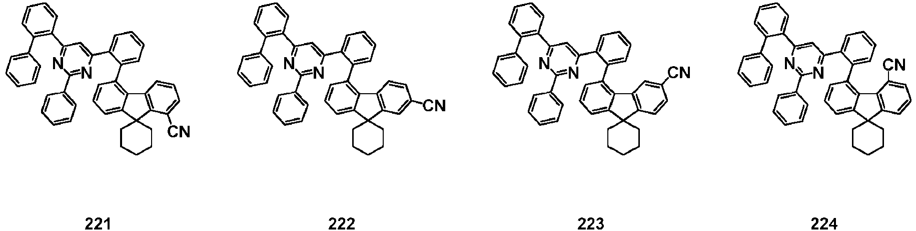

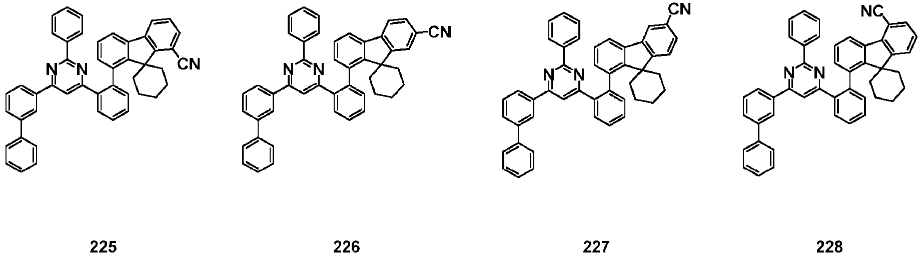

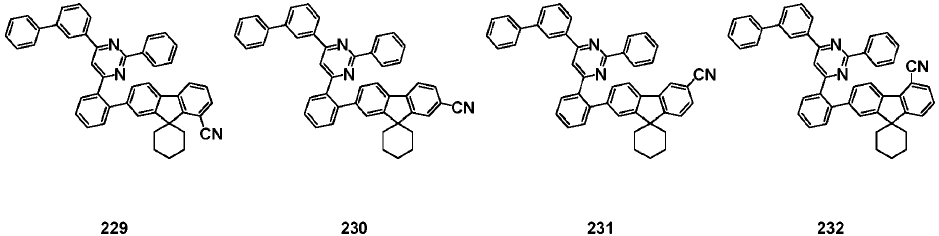

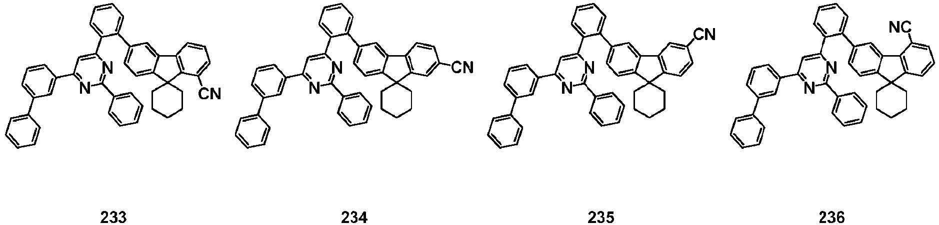

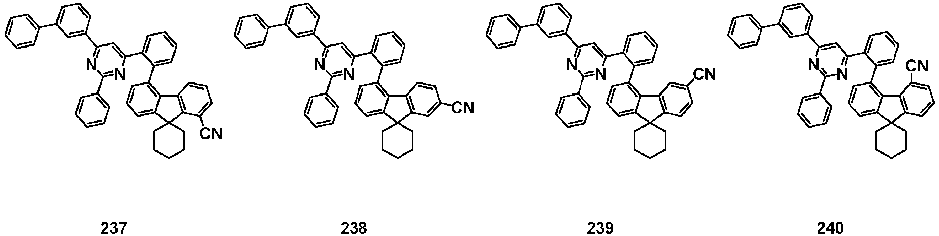

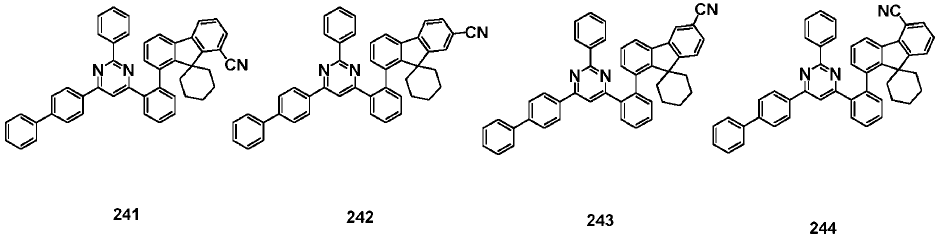

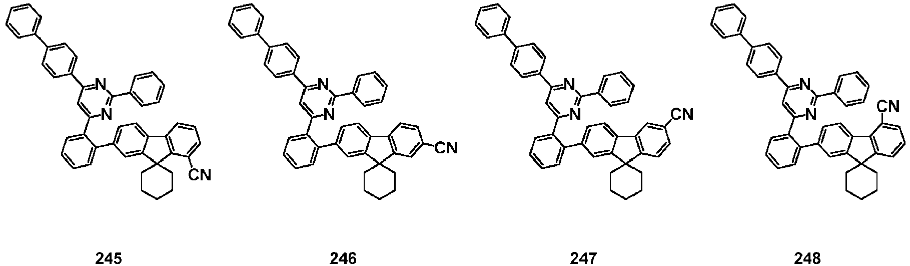

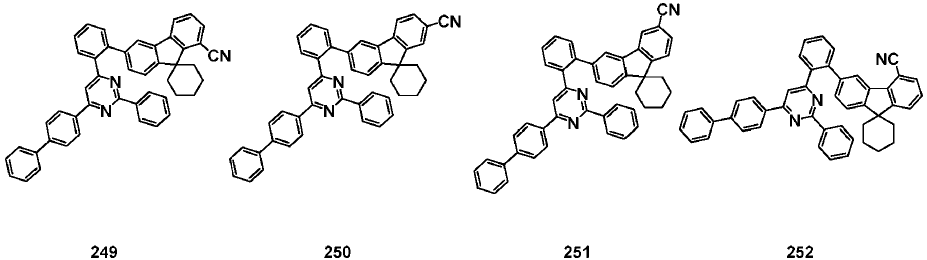

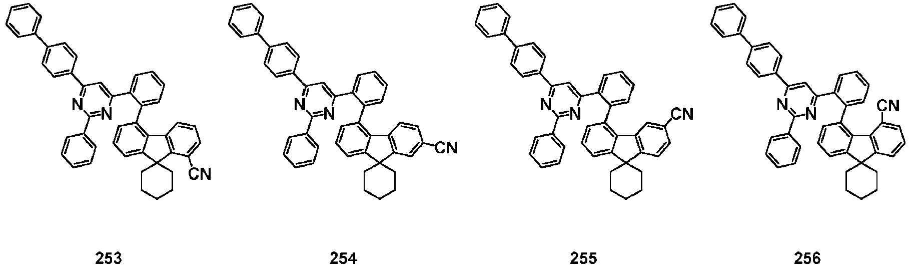

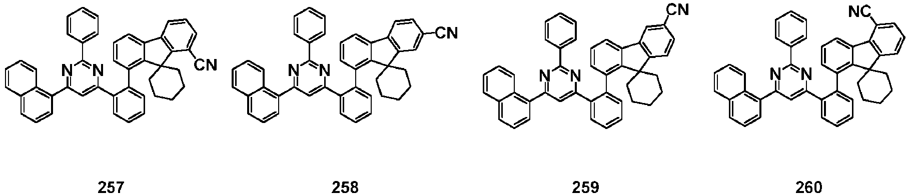

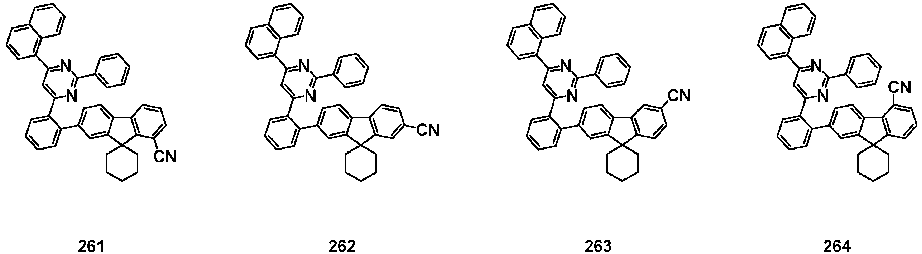

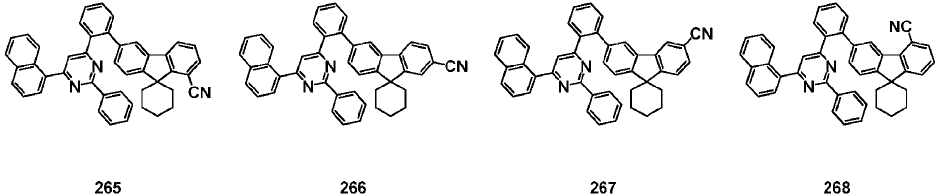

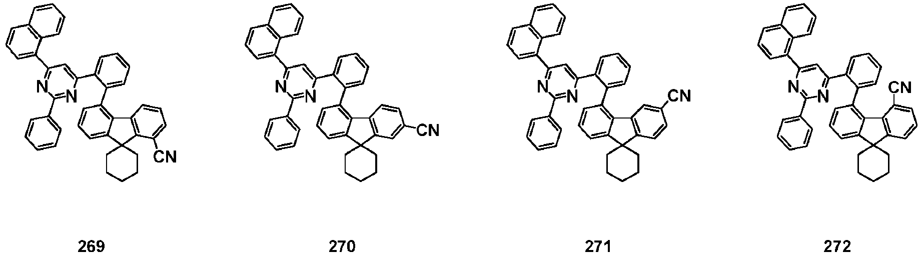

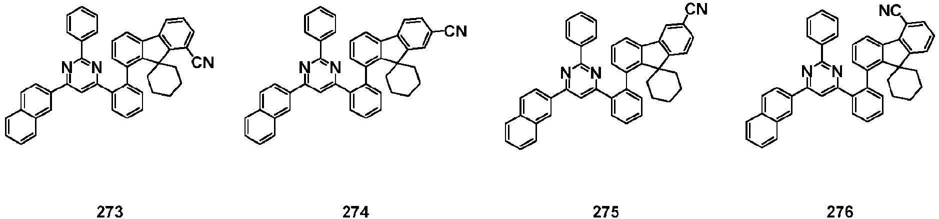

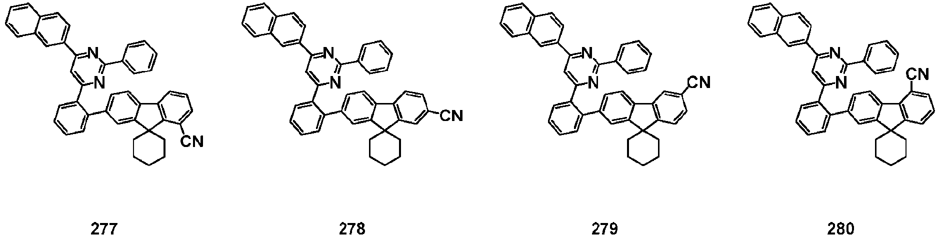

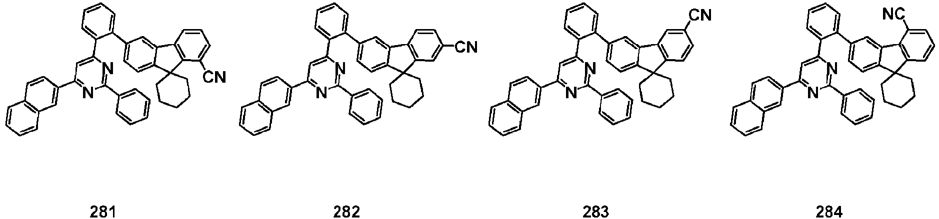

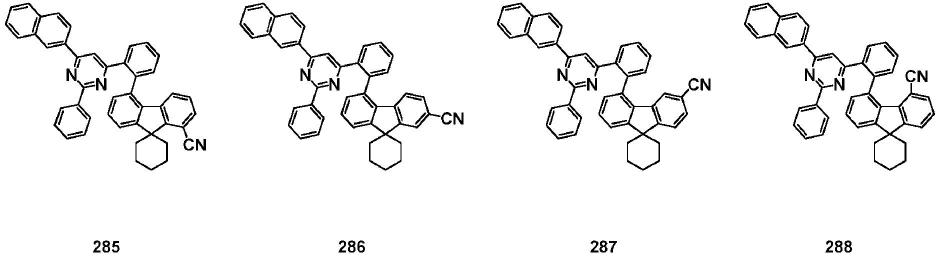

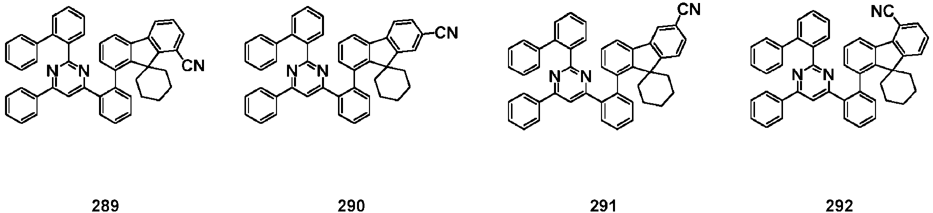

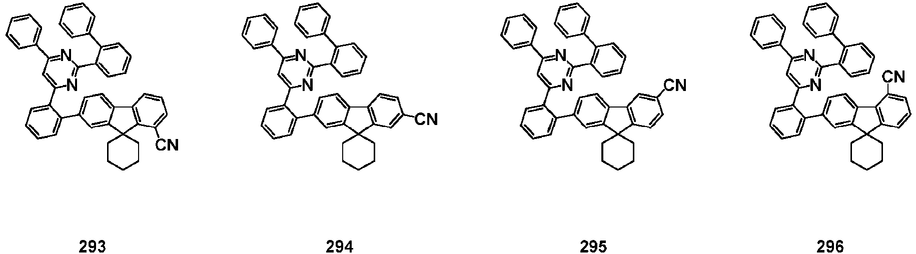

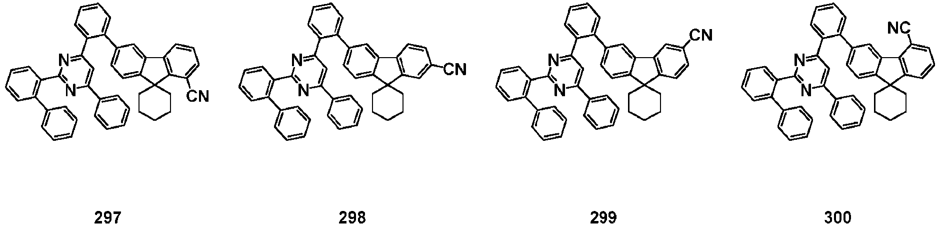

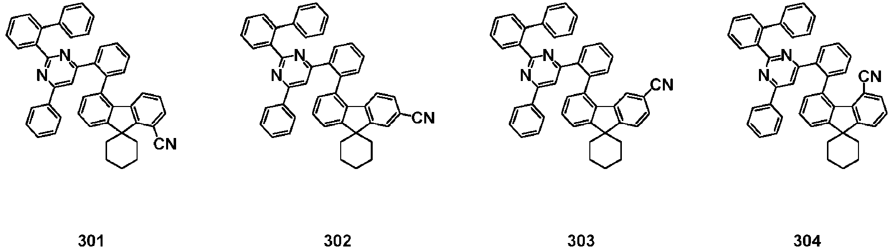

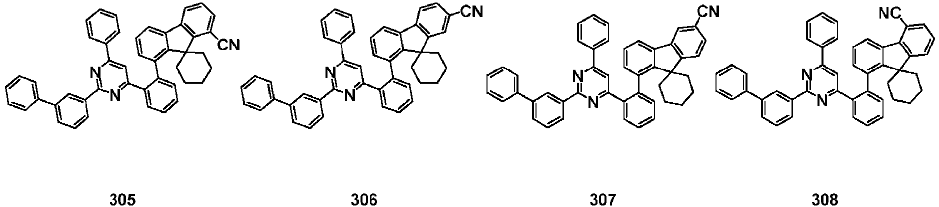

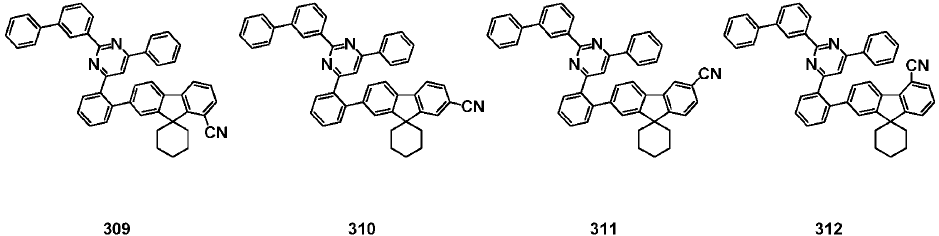

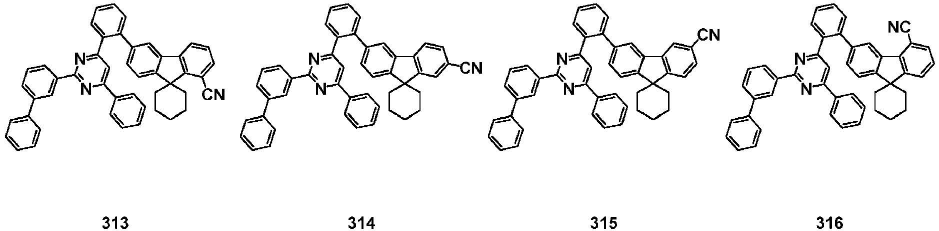

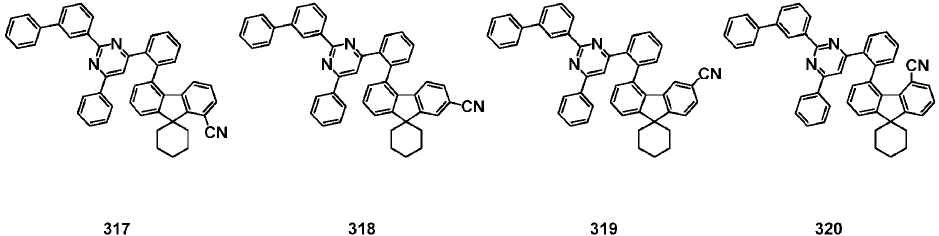

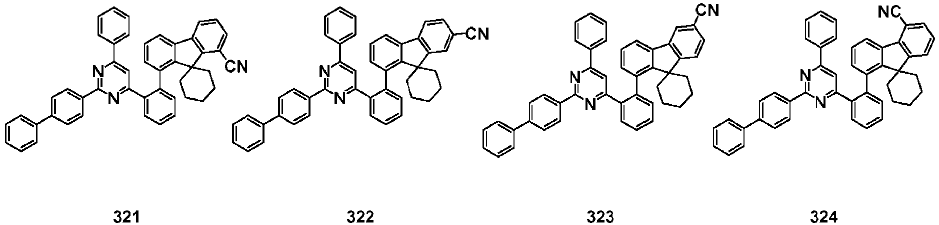

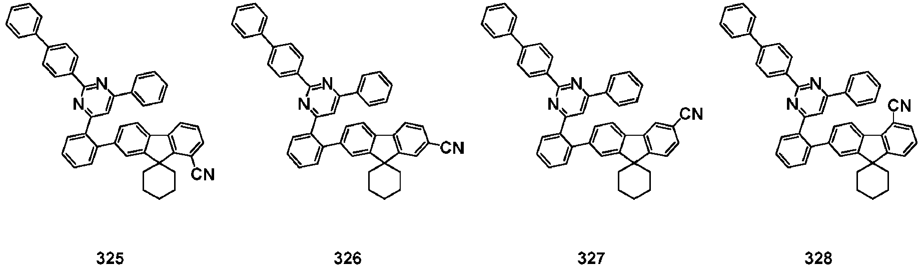

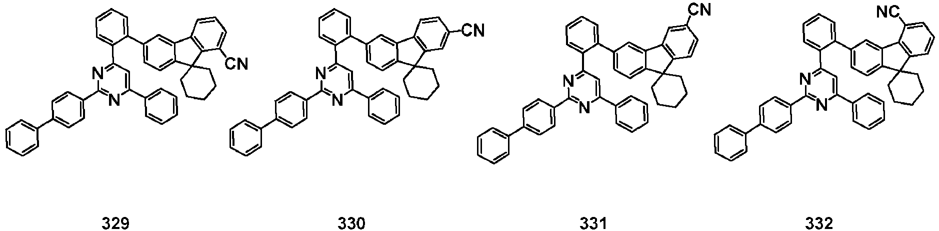

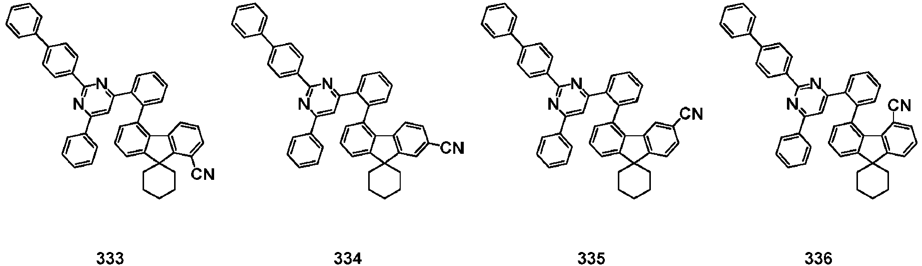

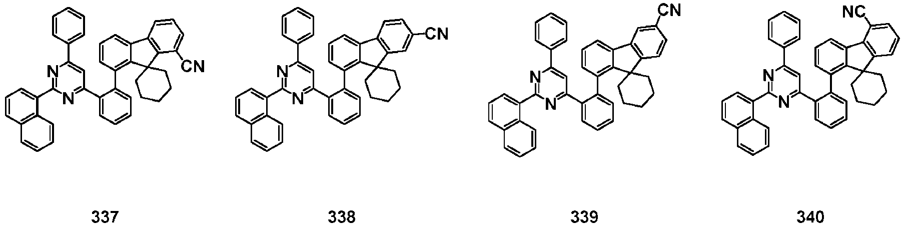

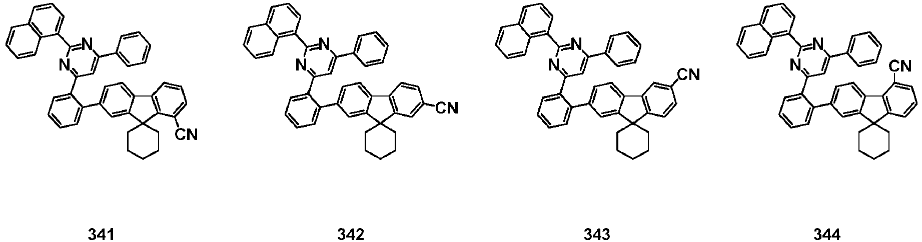

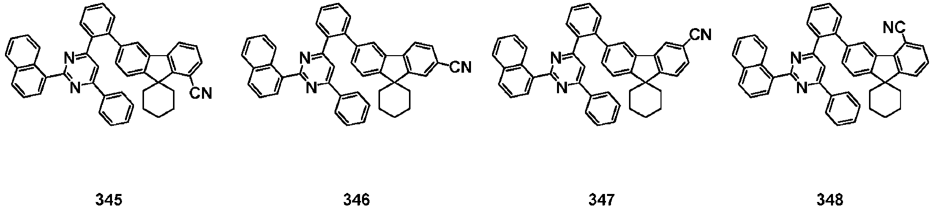

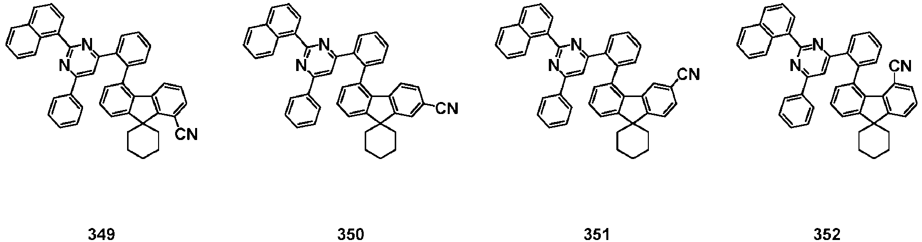

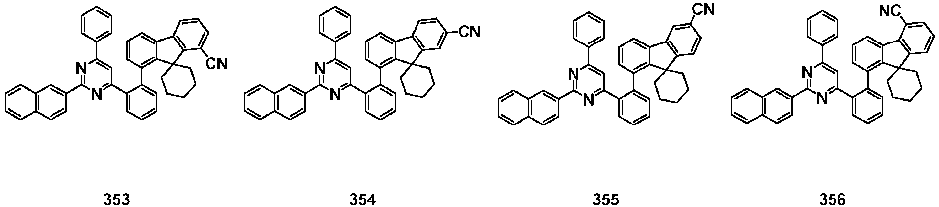

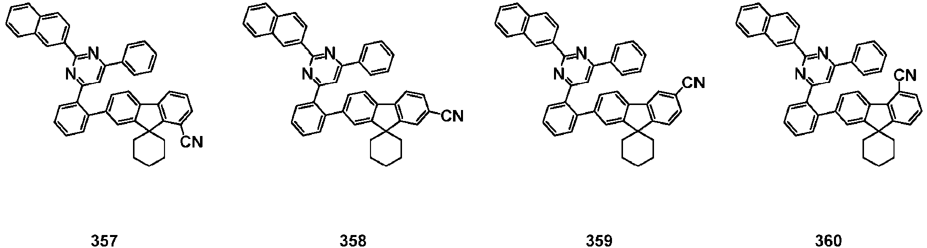

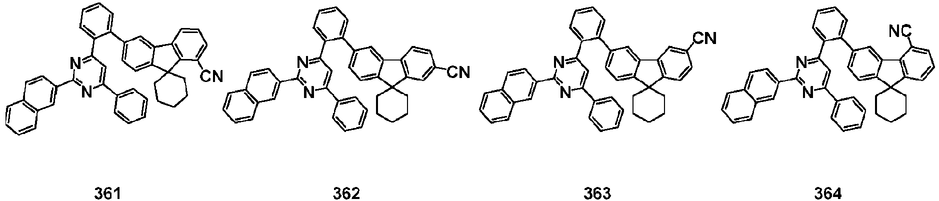

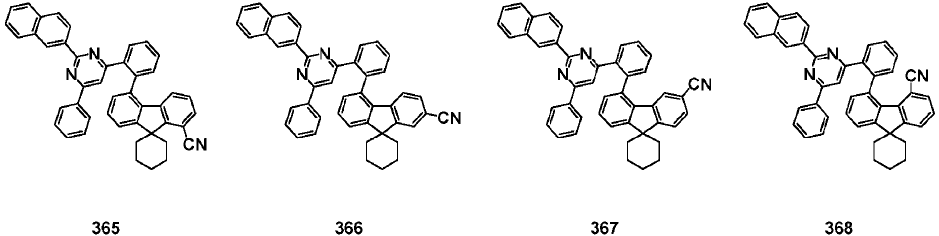

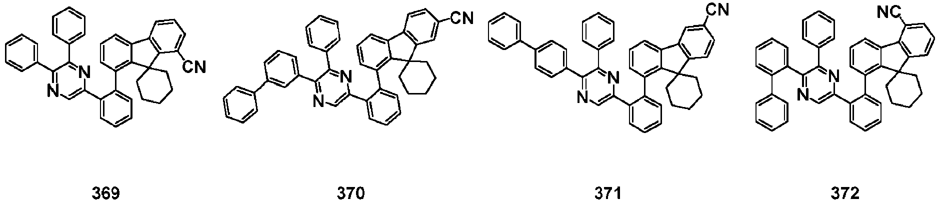

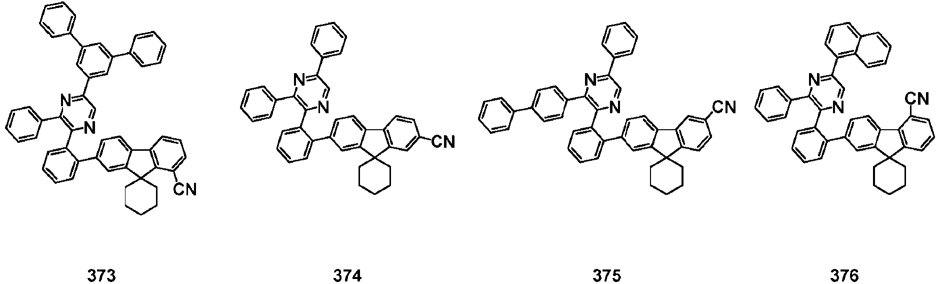

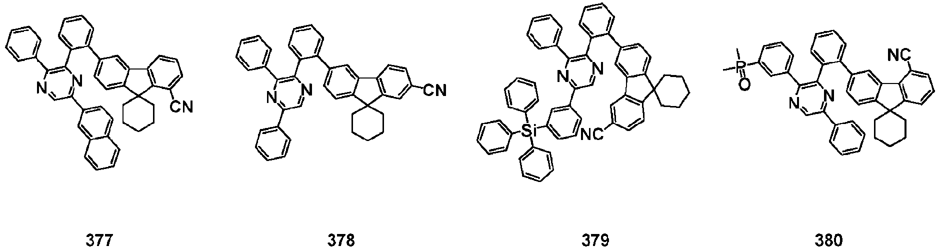

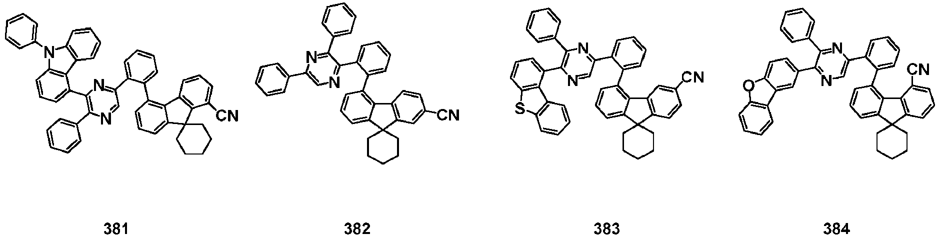

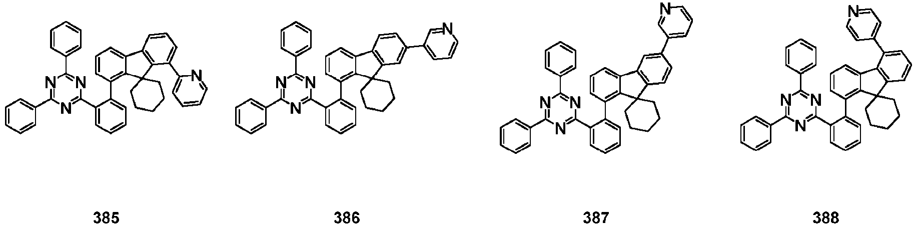

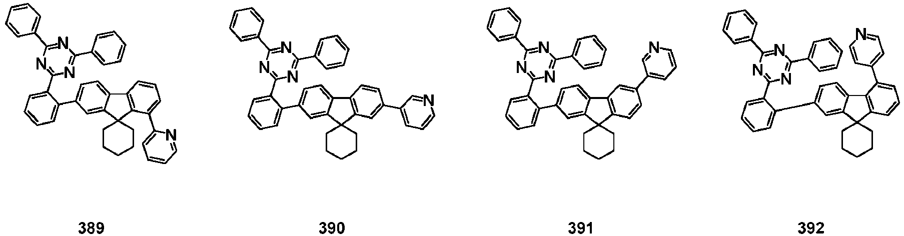

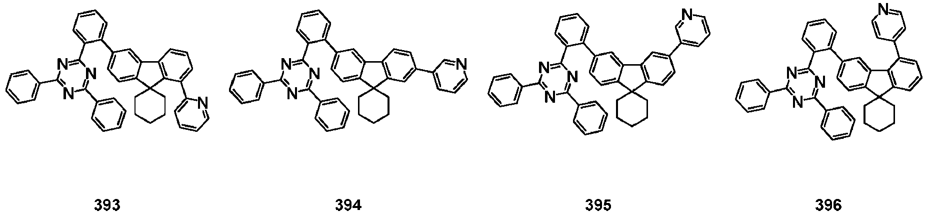

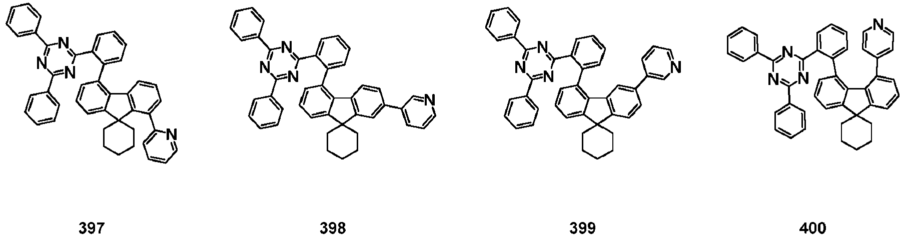

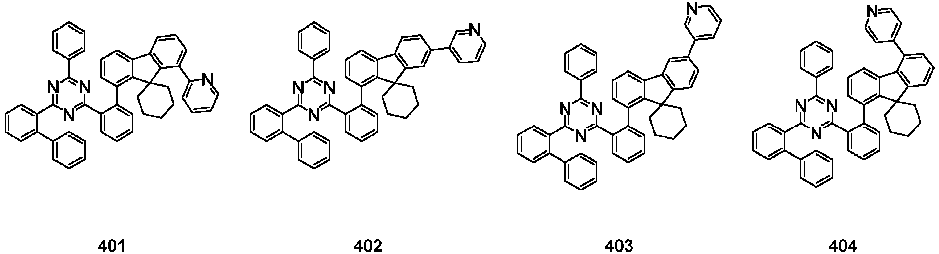

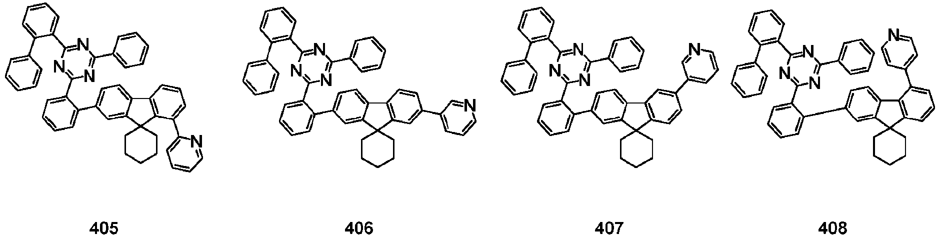

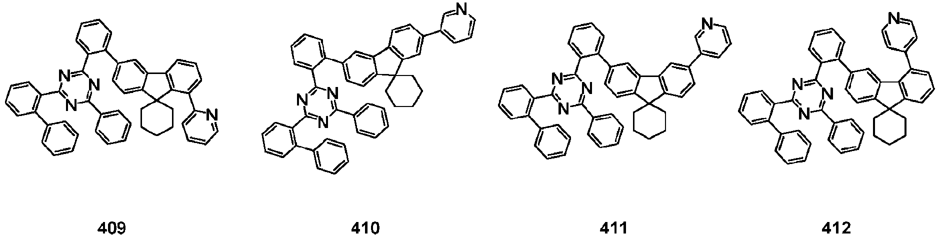

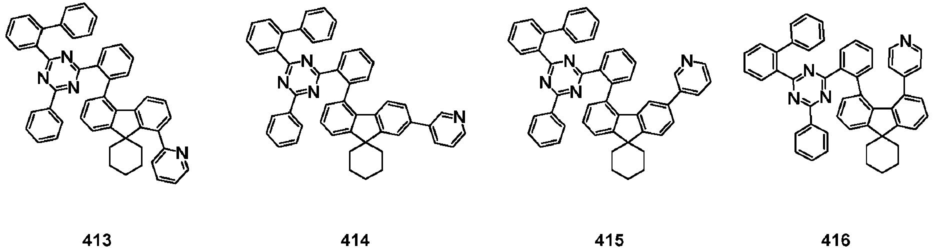

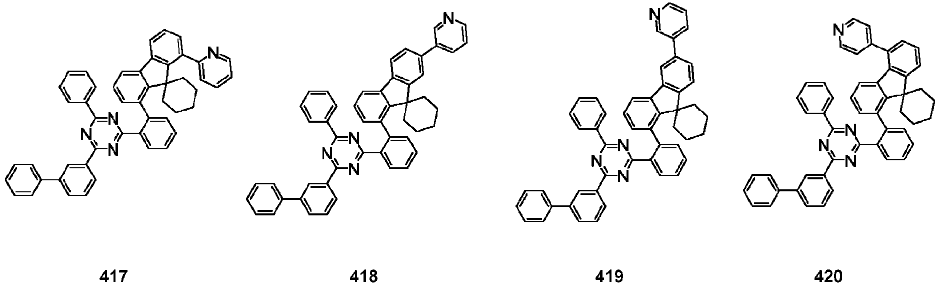

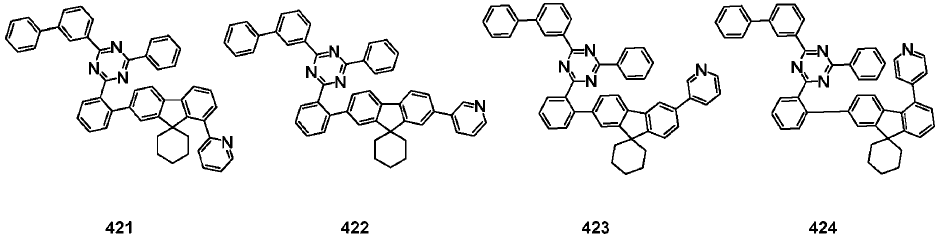

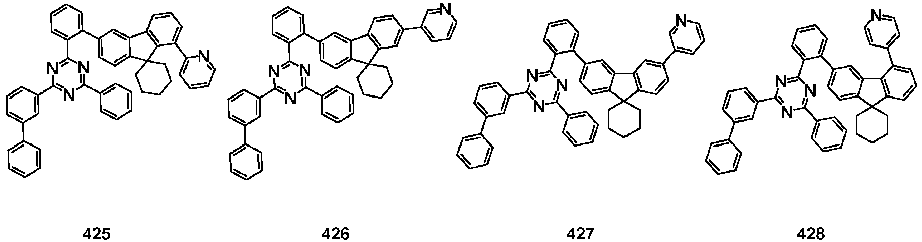

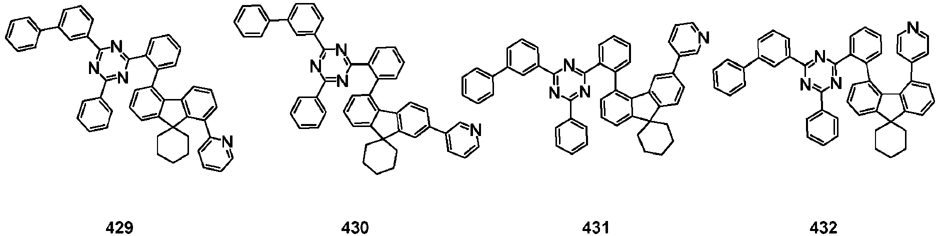

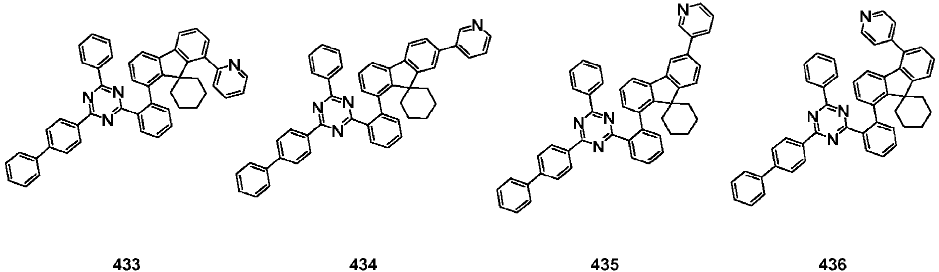

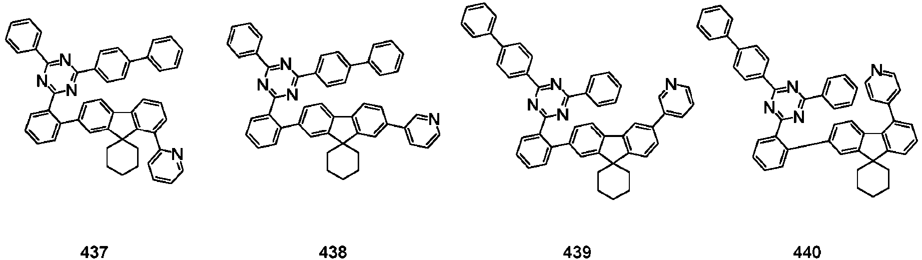

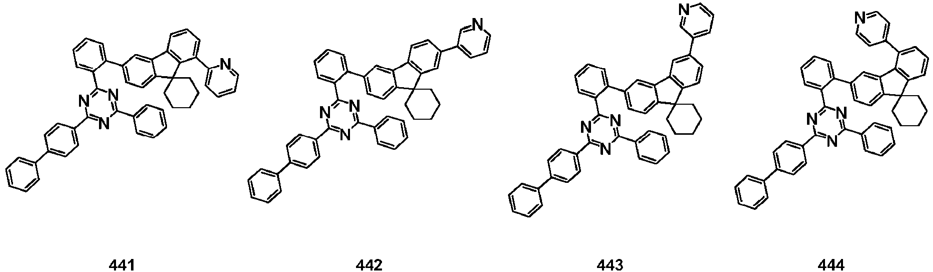

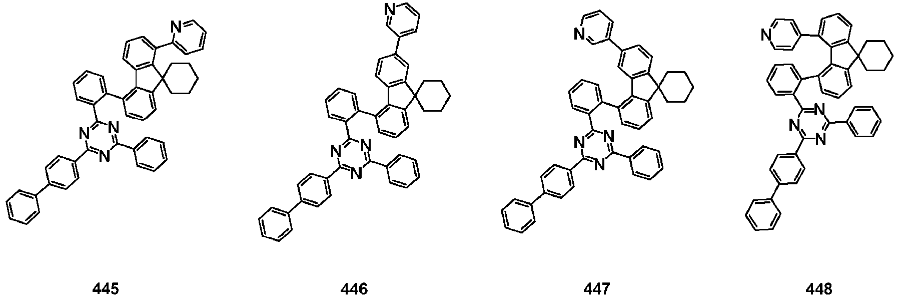

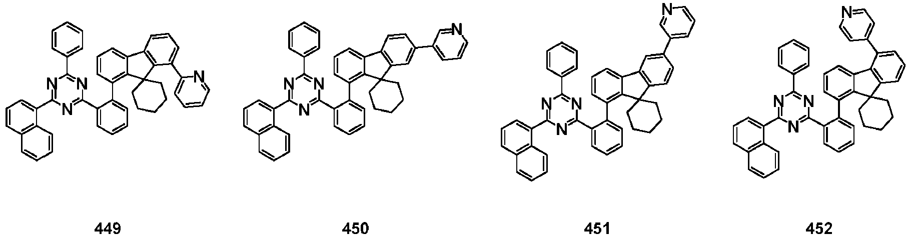

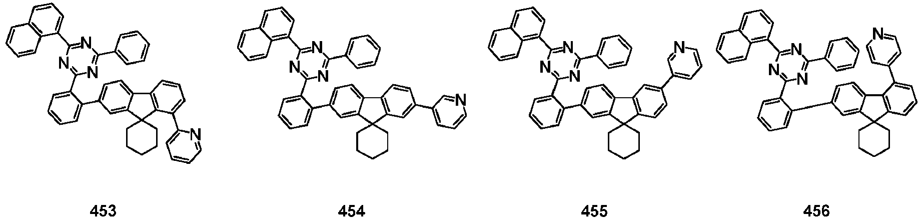

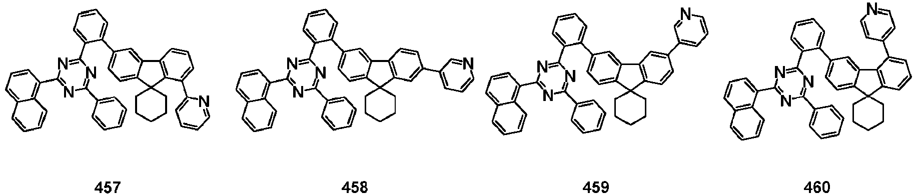

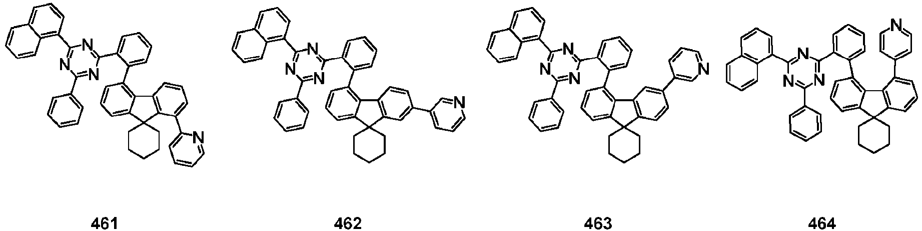

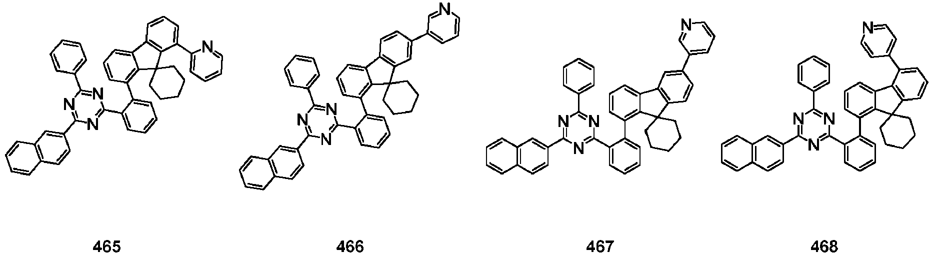

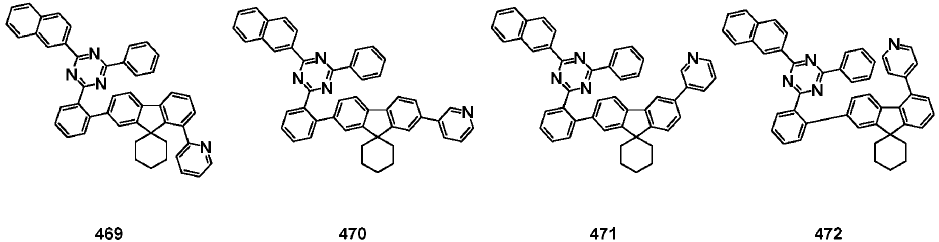

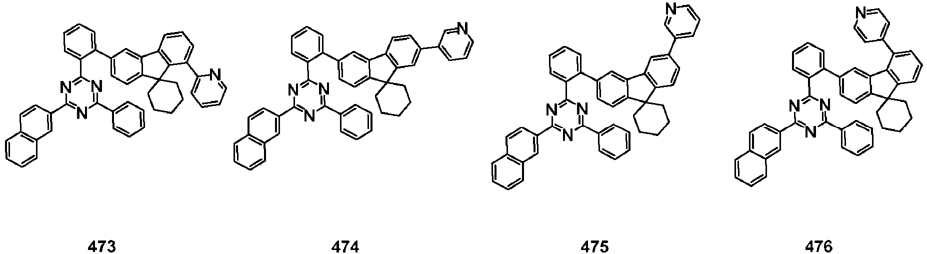

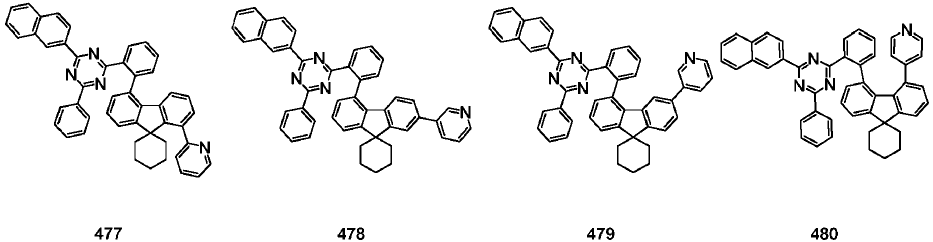

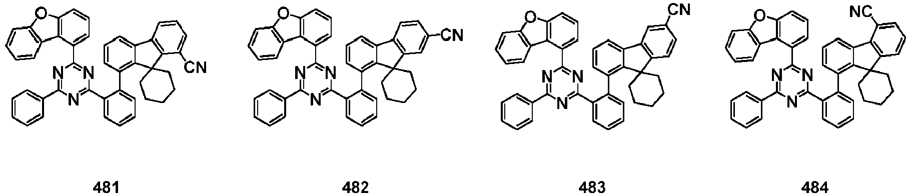

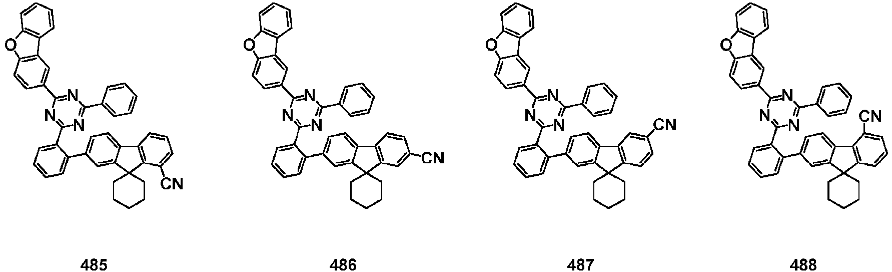

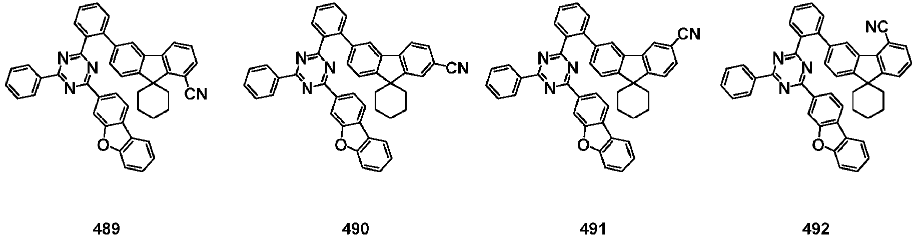

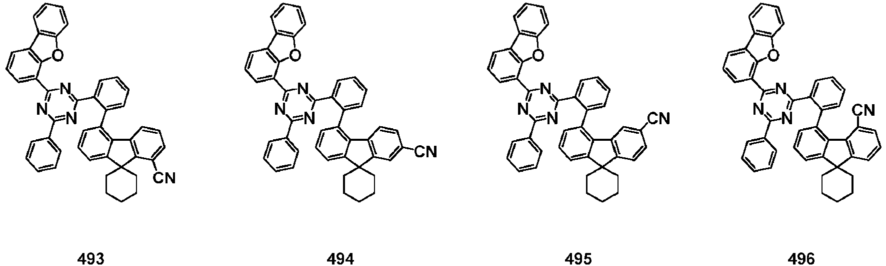

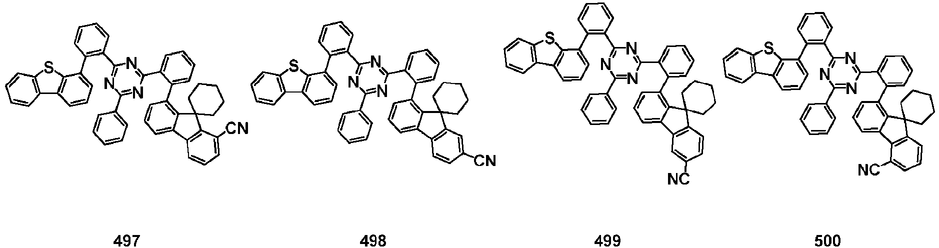

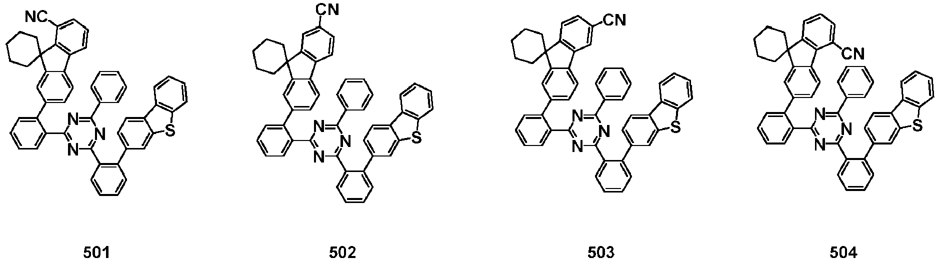

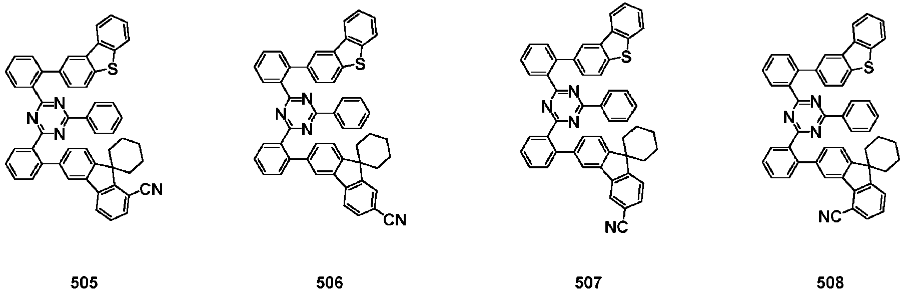

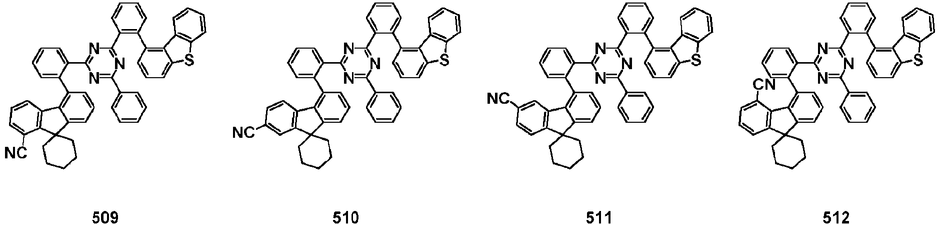

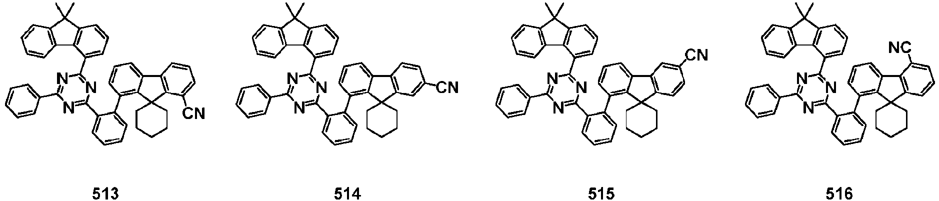

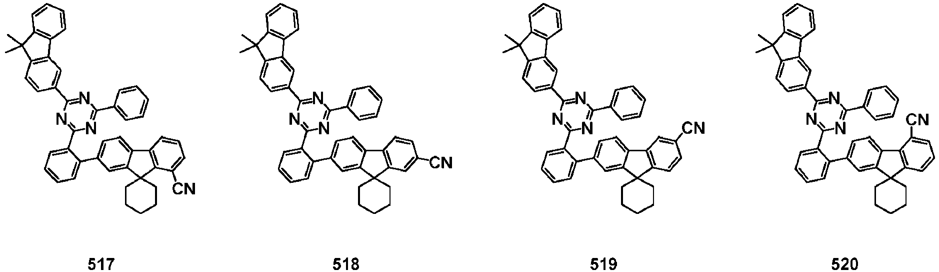

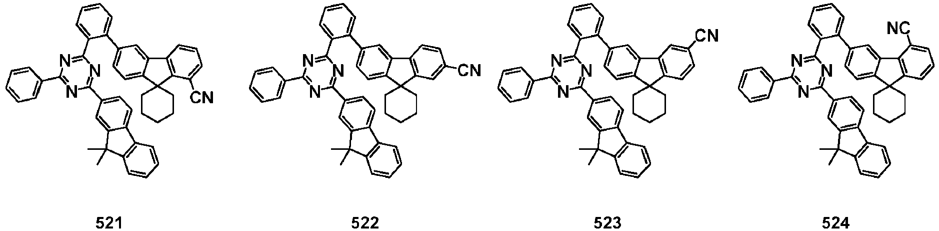

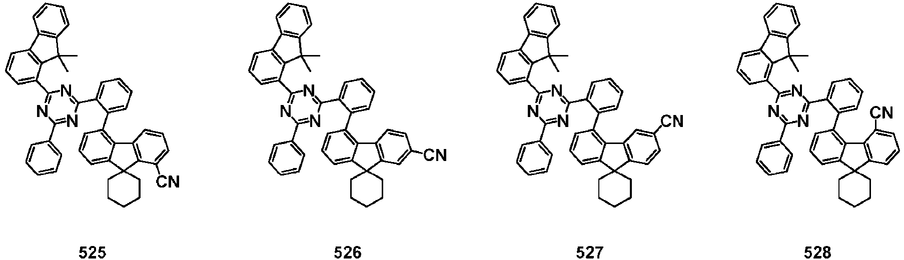

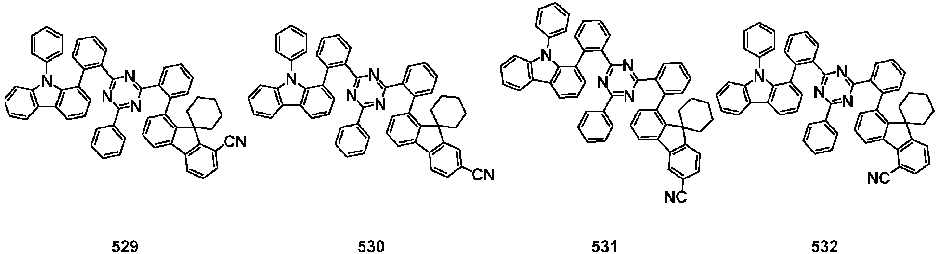

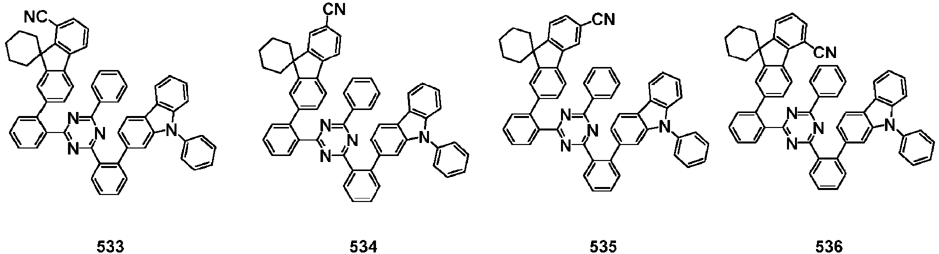

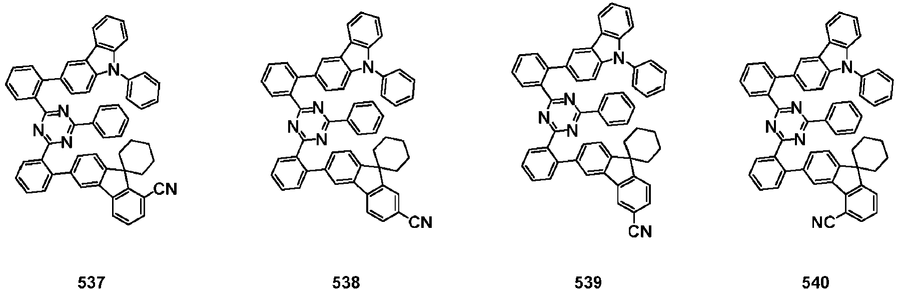

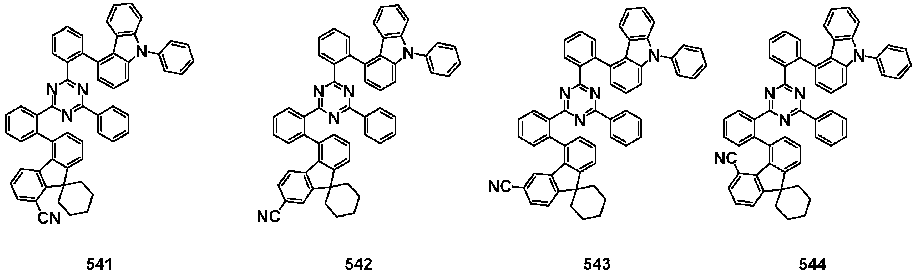

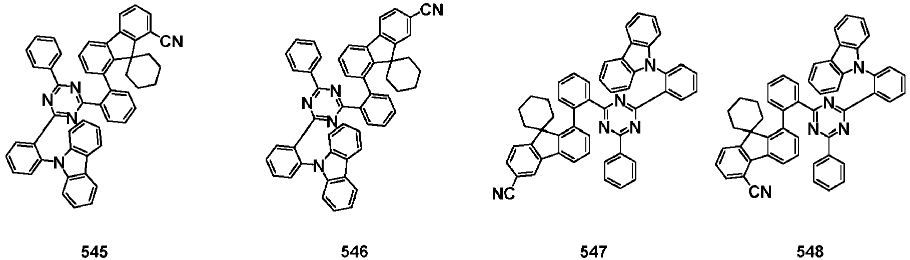

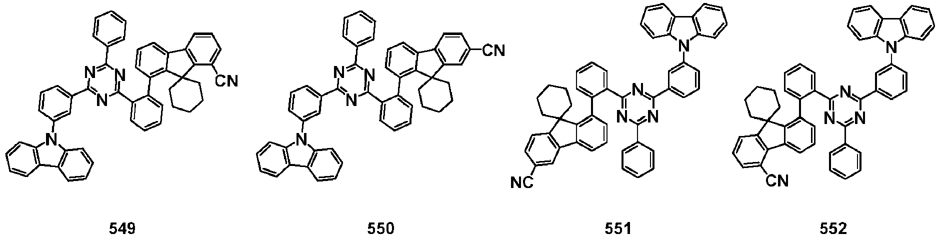

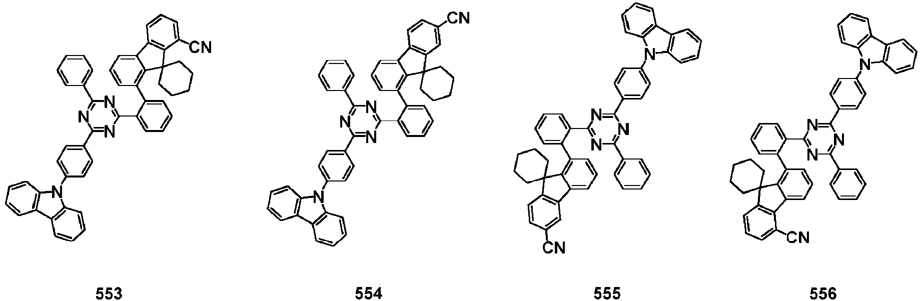

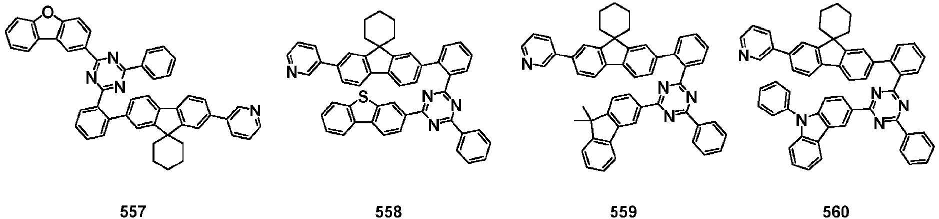

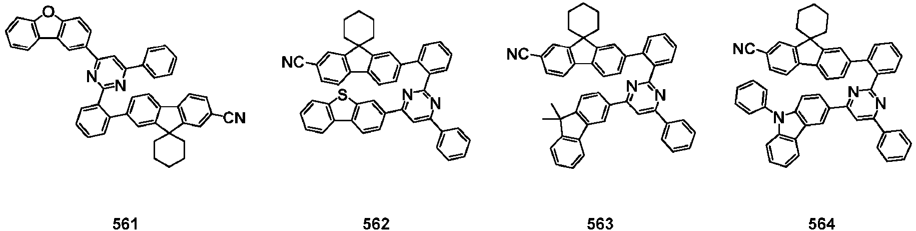

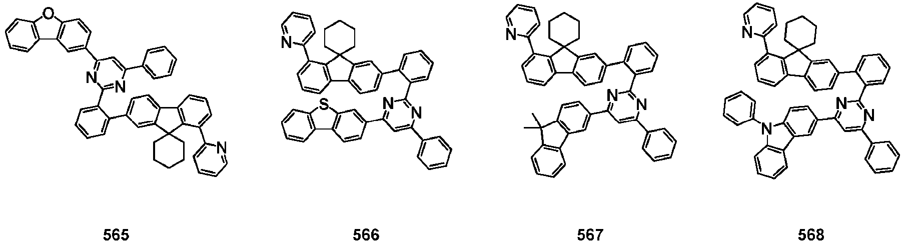

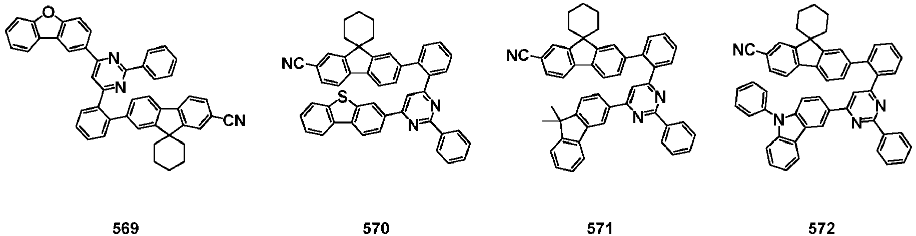

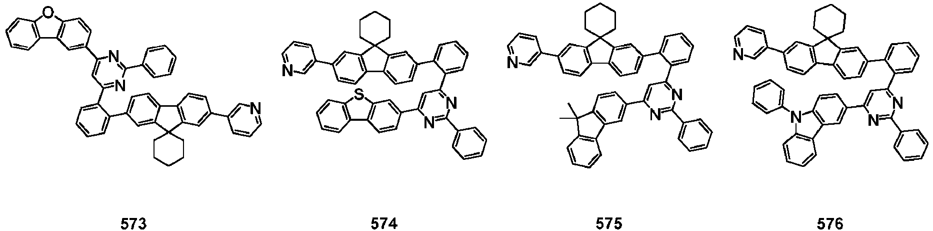

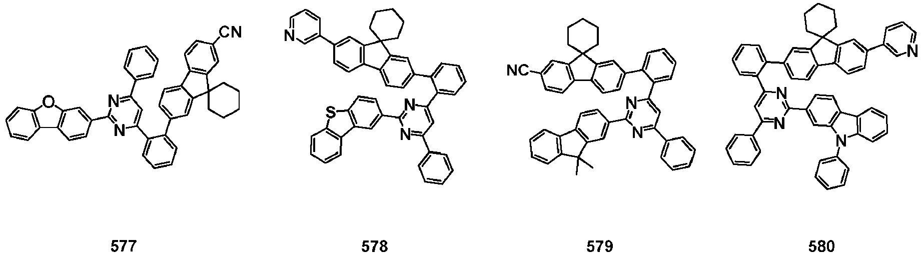

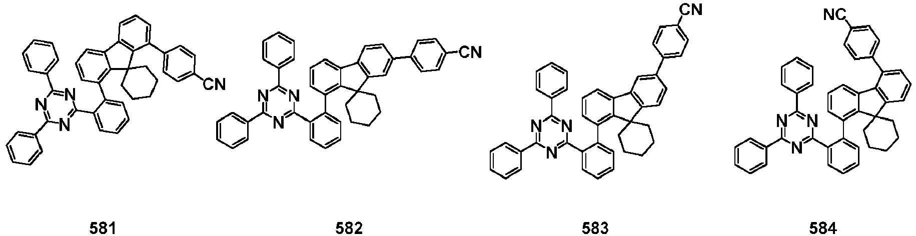

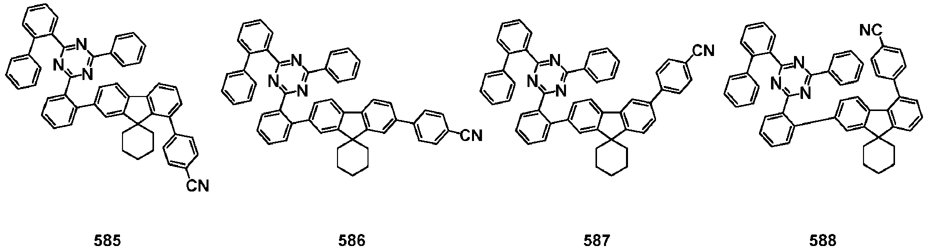

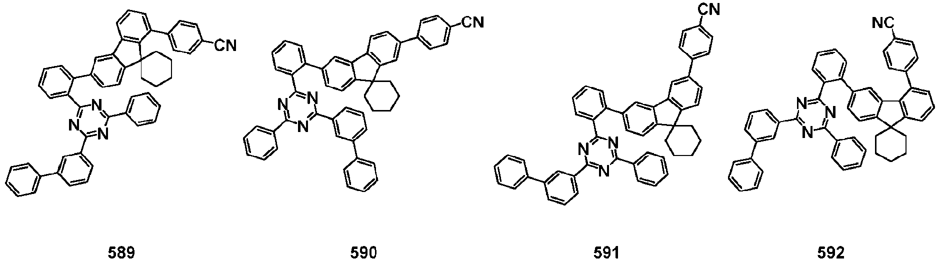

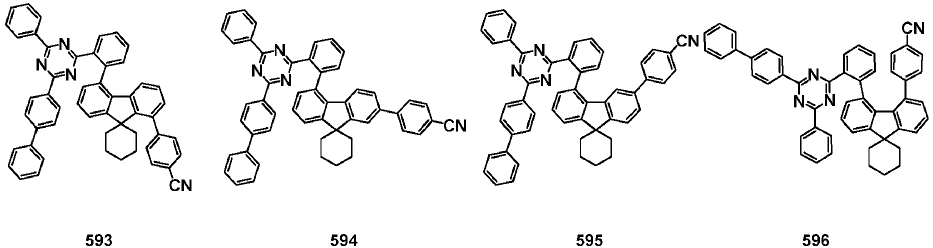

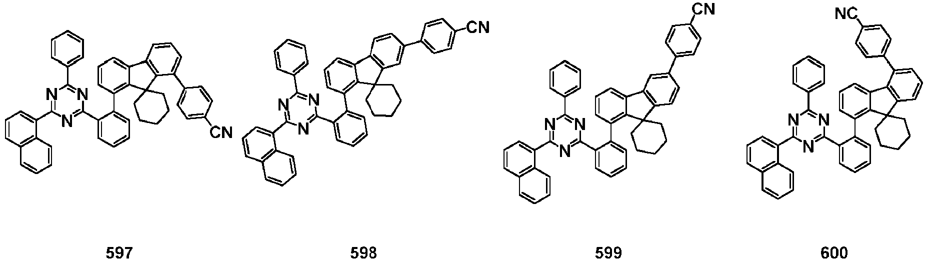

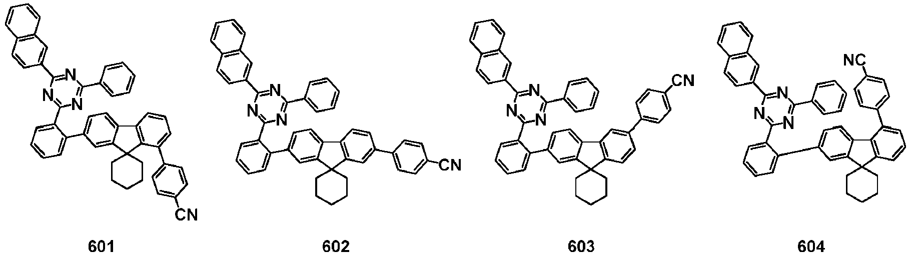

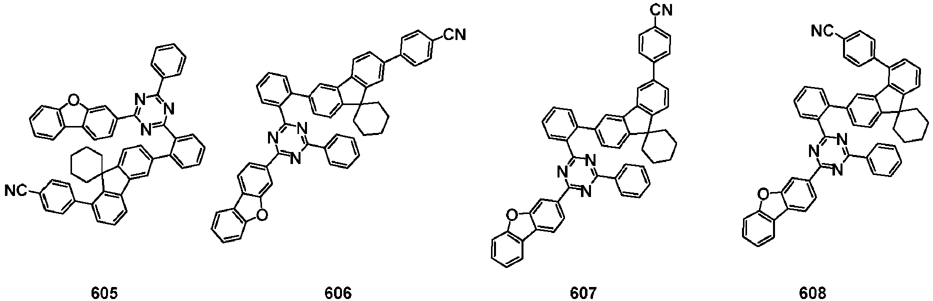

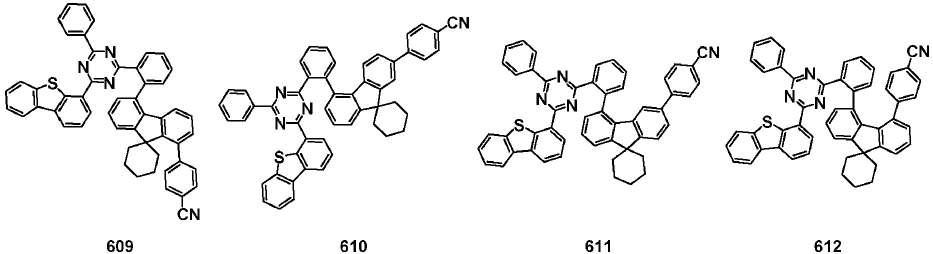

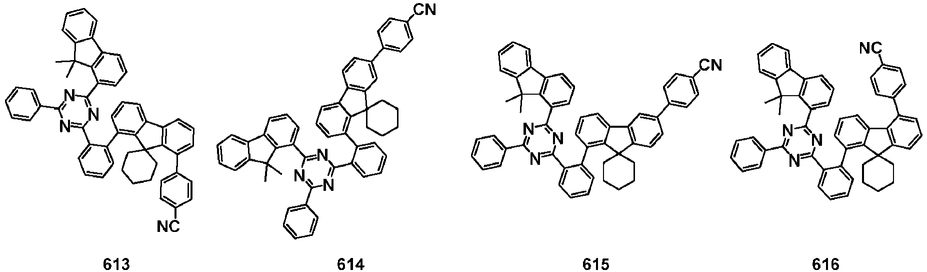

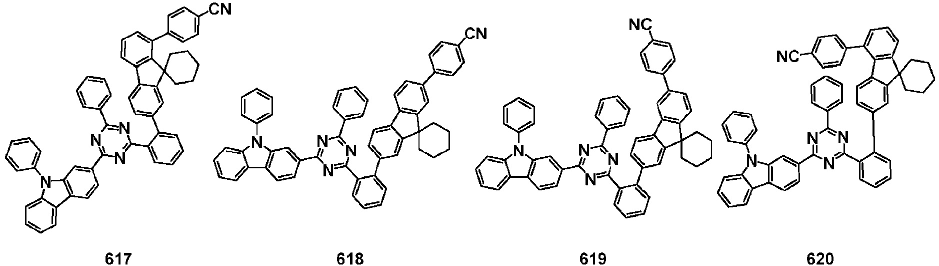

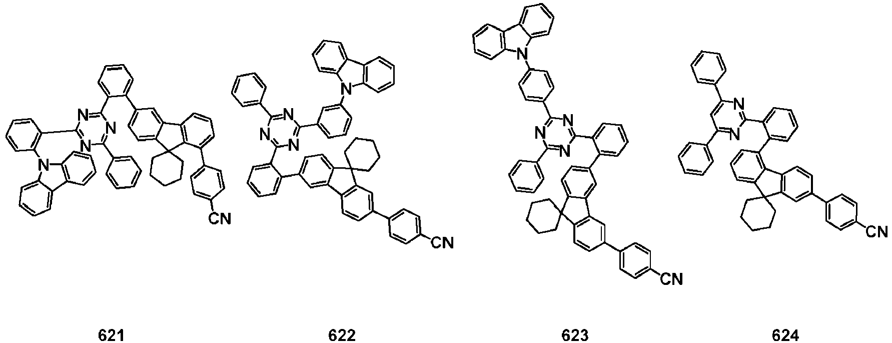

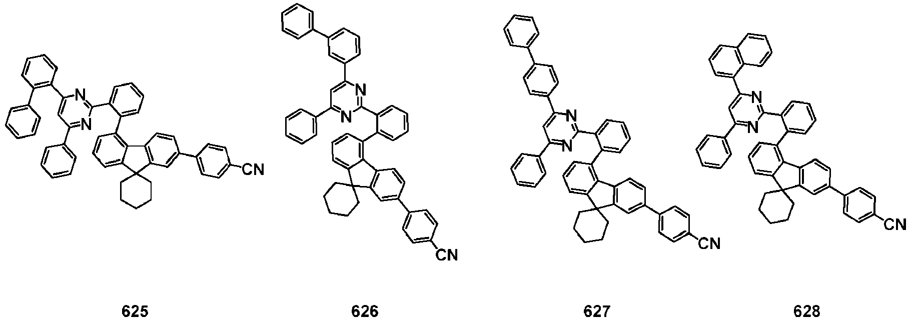

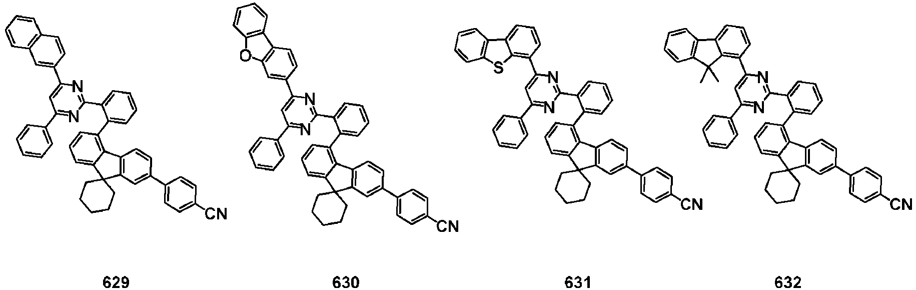

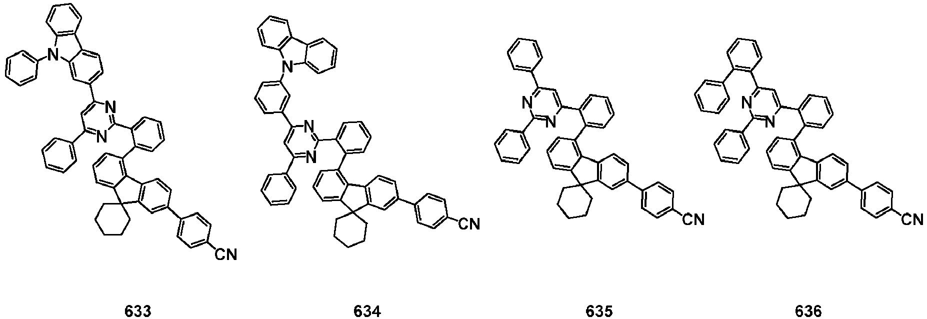

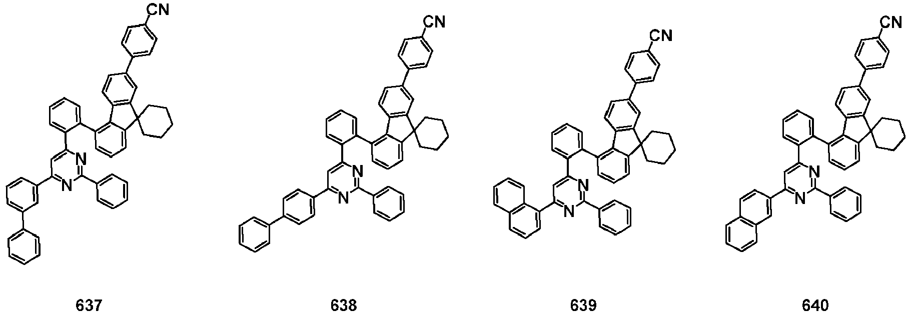

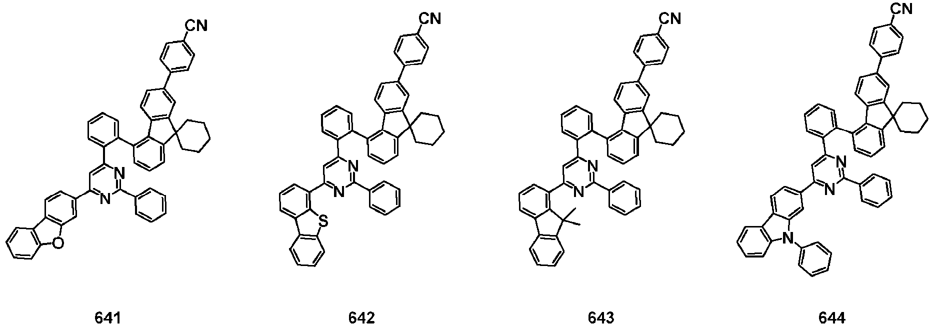

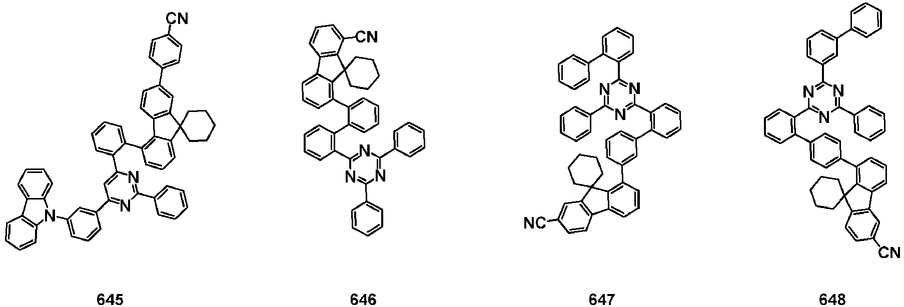

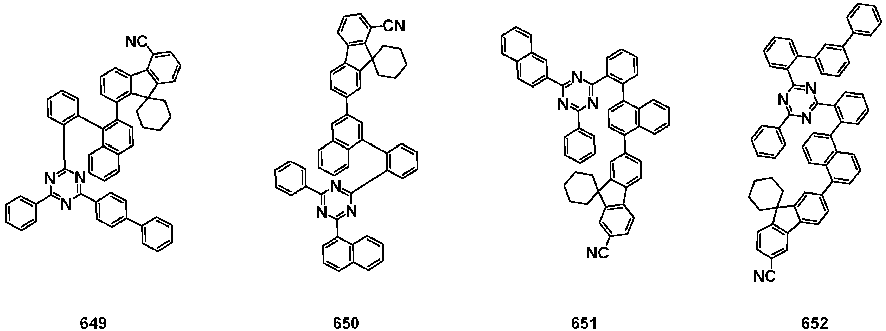

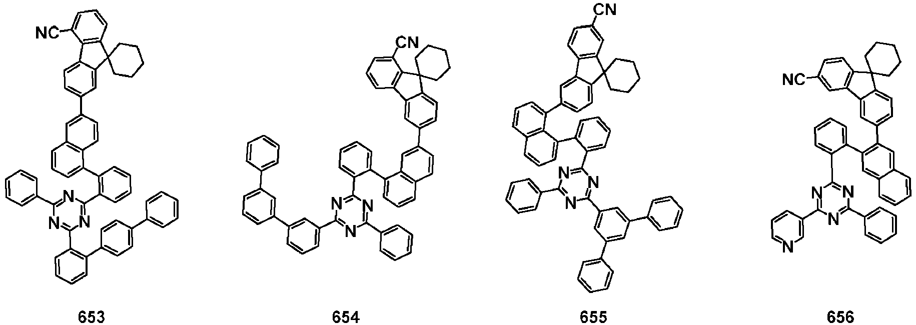

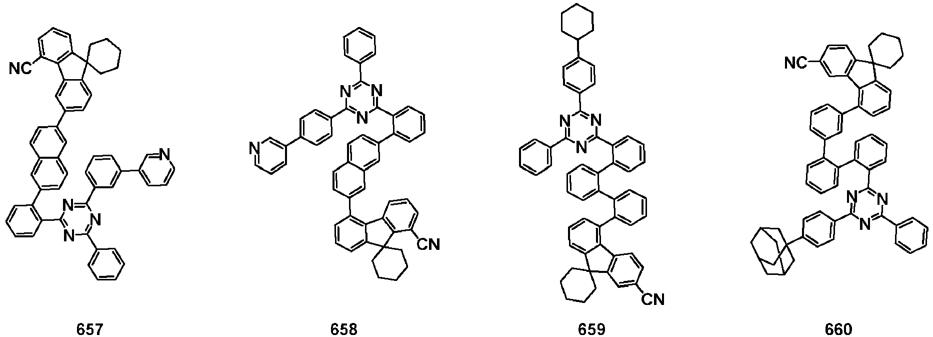

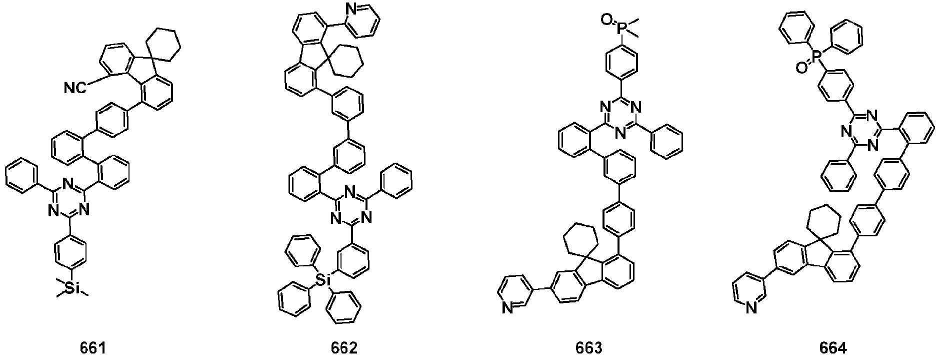

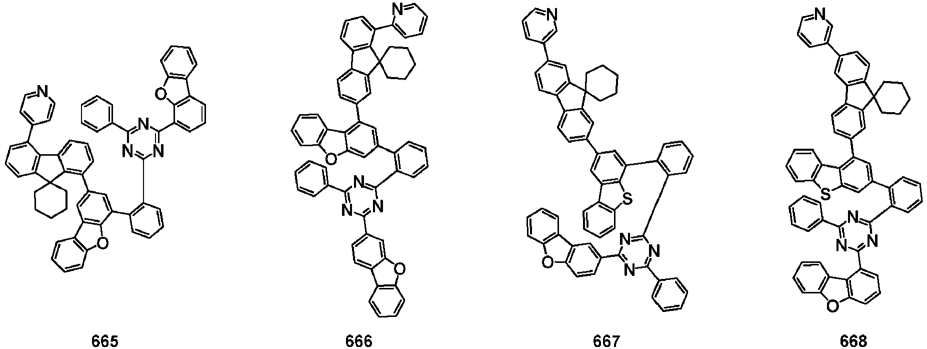

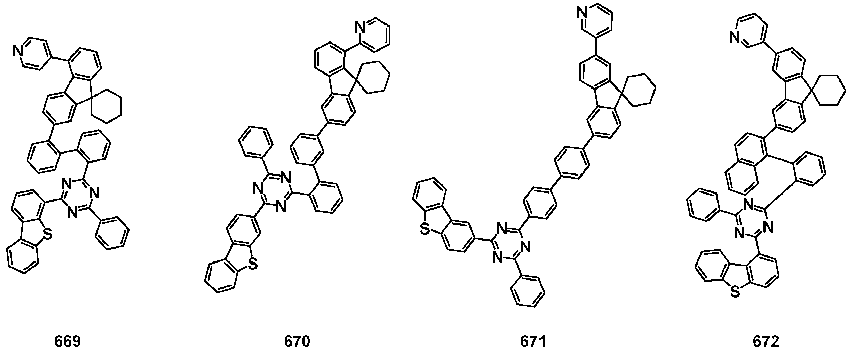

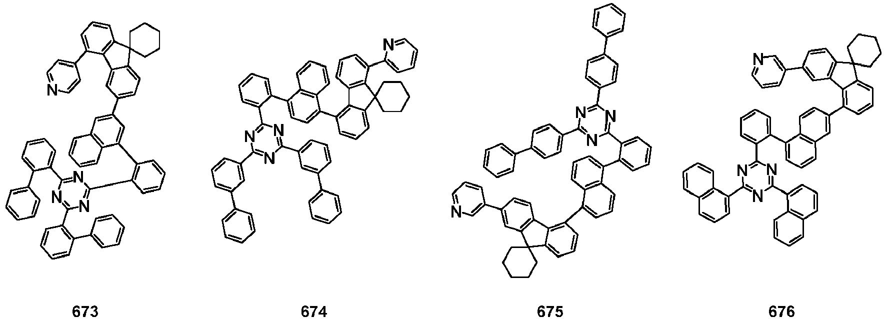

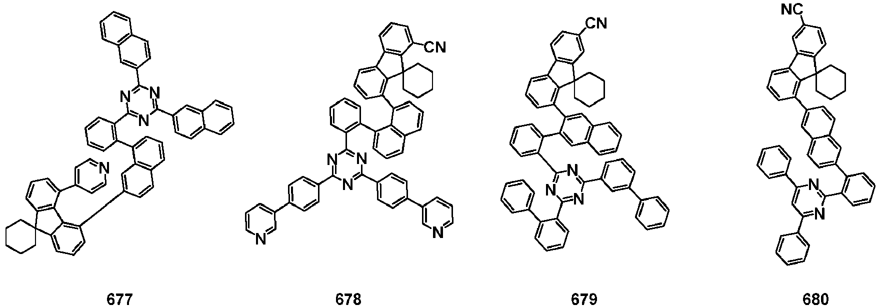

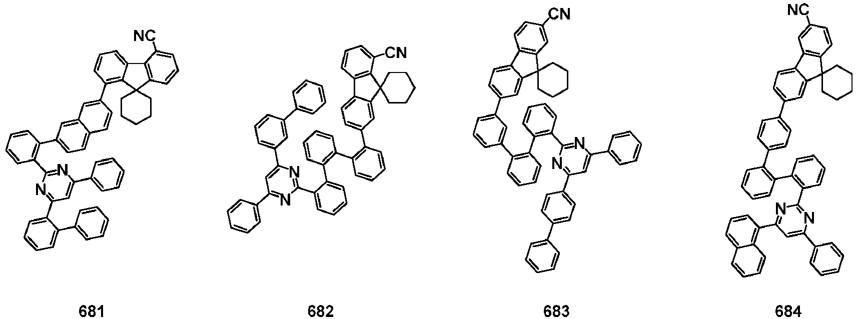

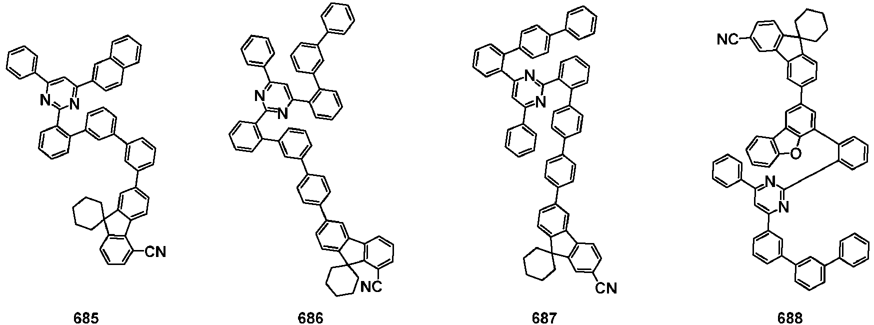

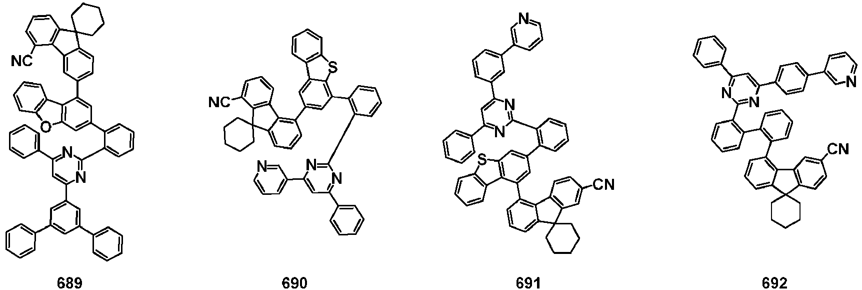

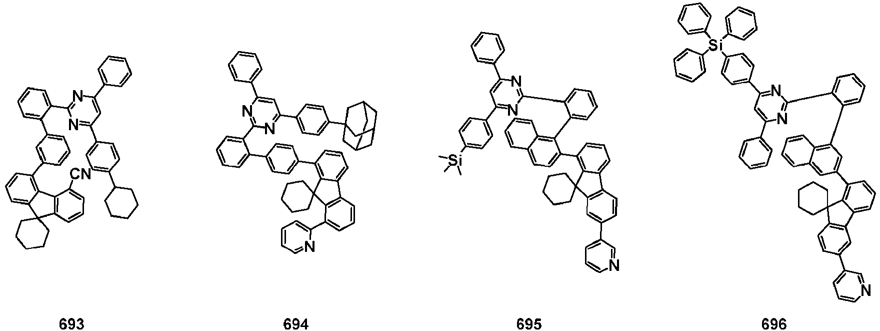

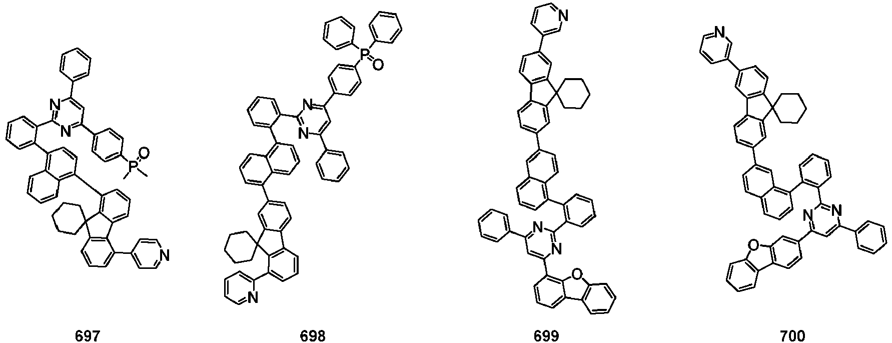

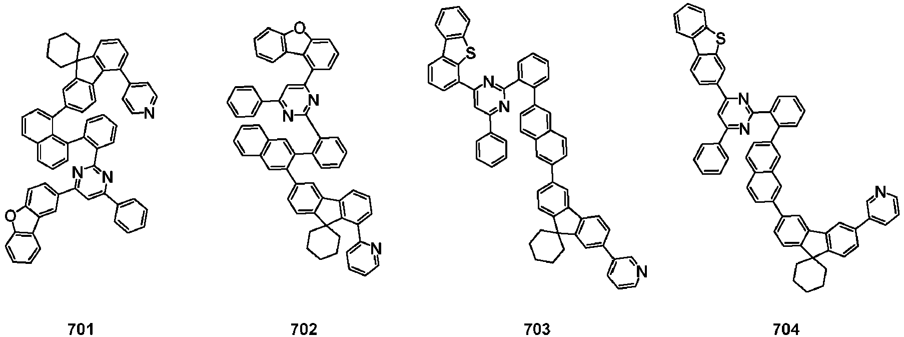

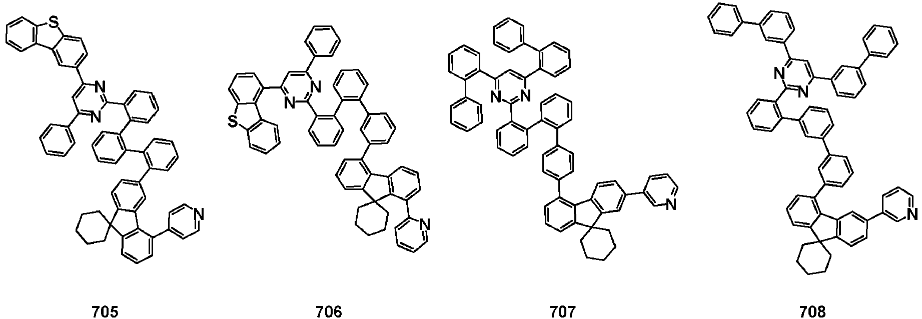

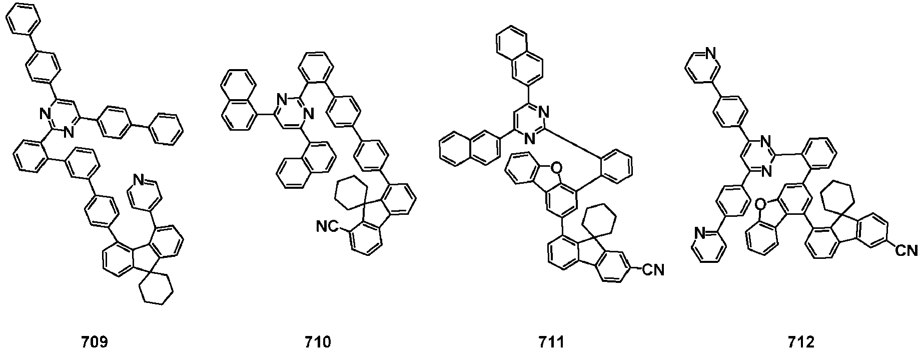

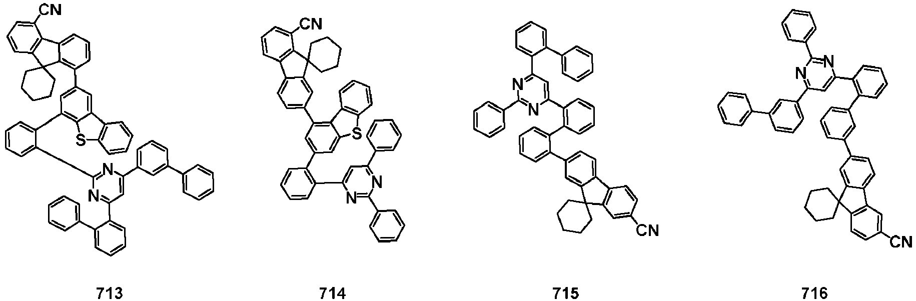

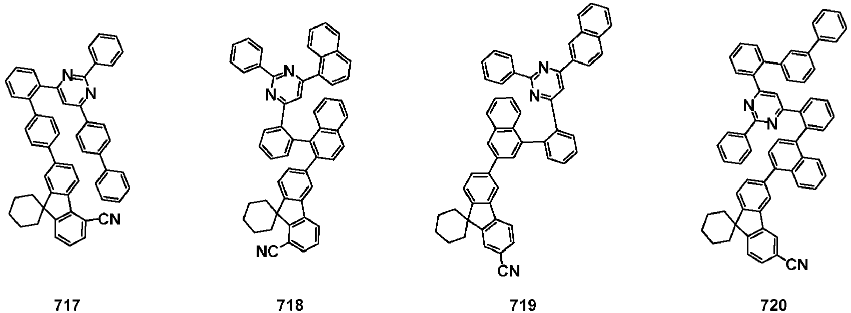

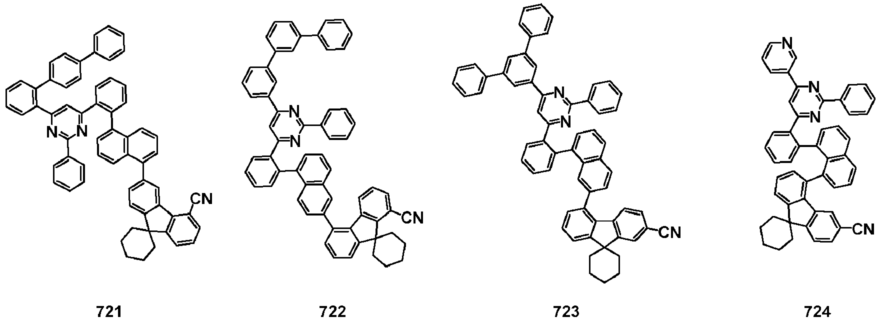

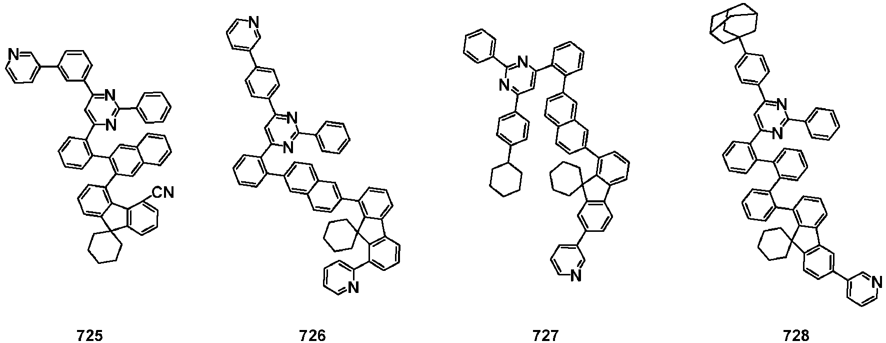

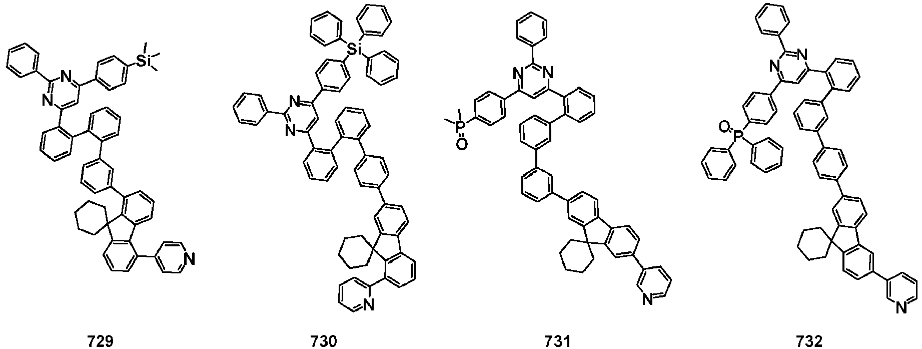

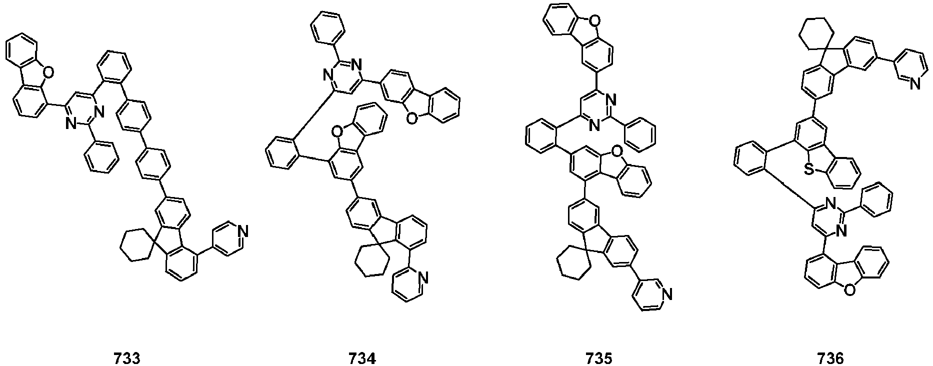

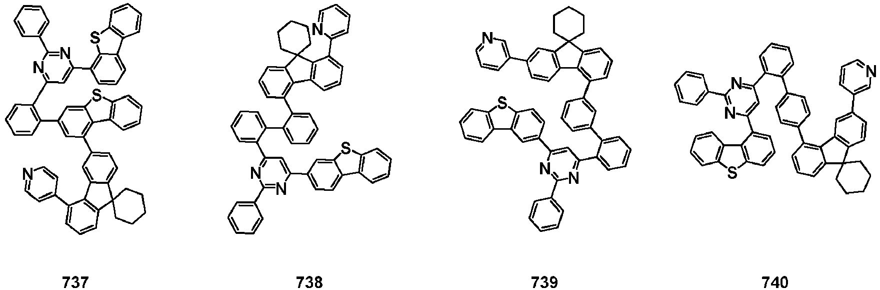

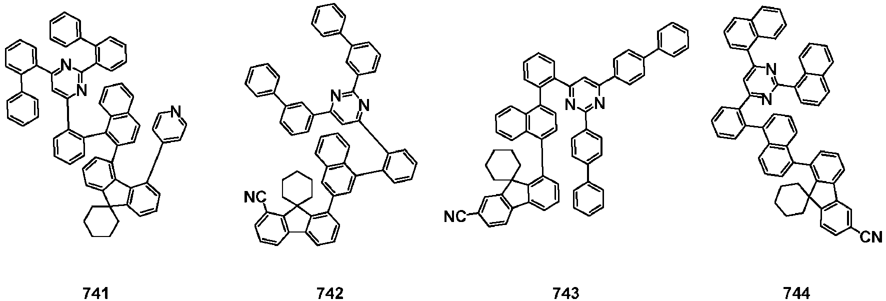

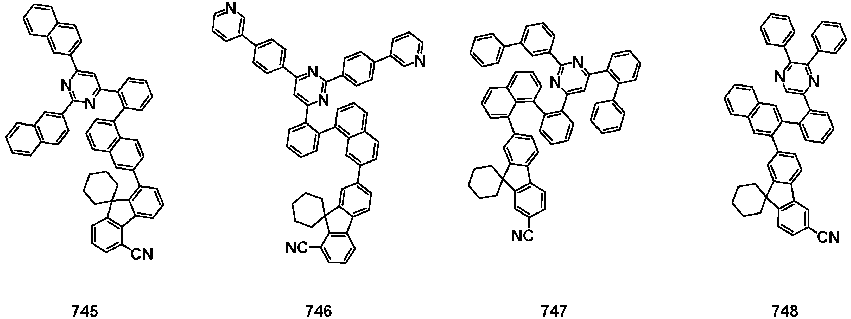

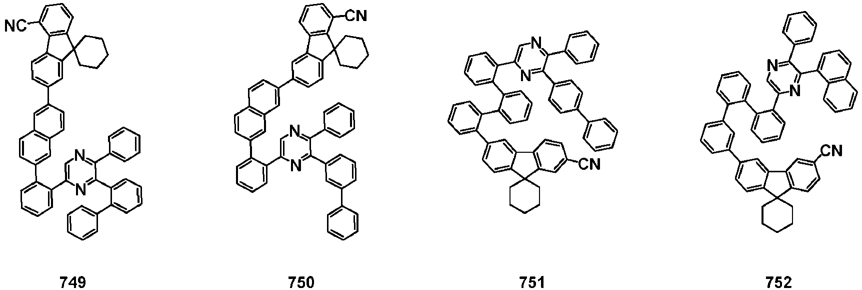

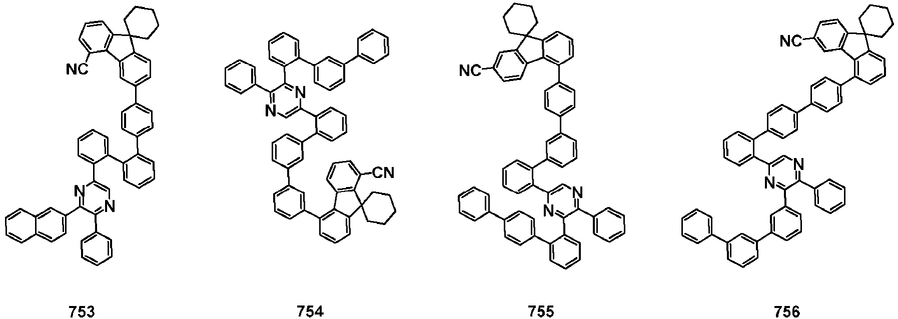

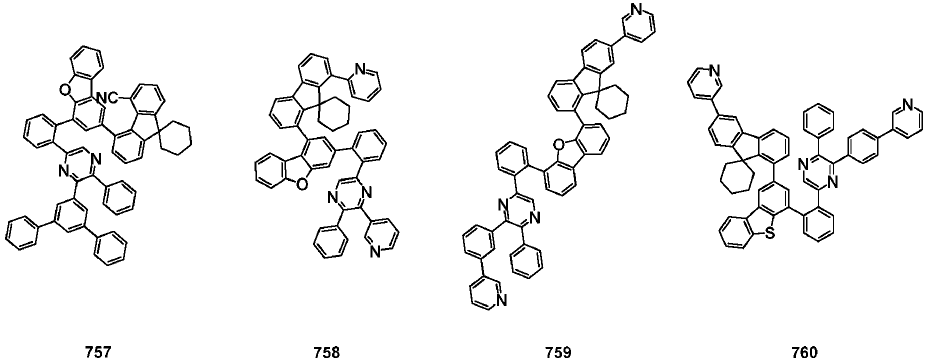

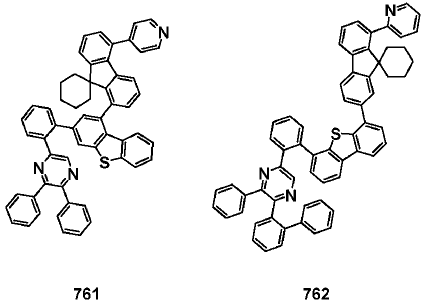

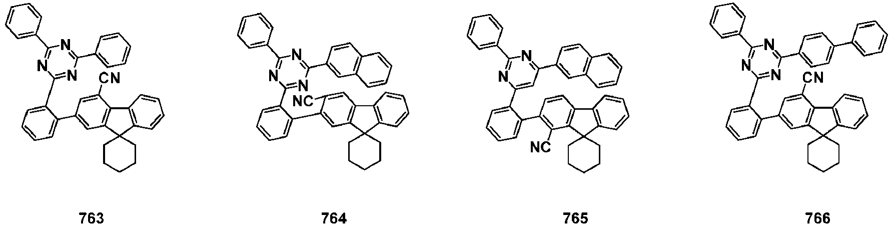

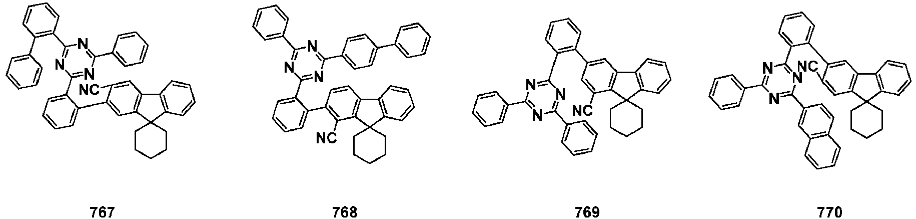

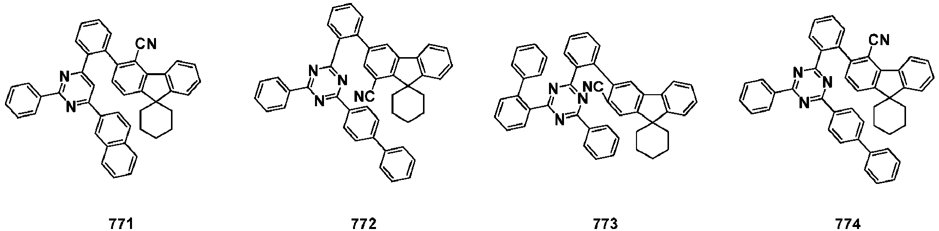

7. Dispositif électroluminescent organique comprenant le composé électroluminescent organique selon l'une quelconque des revendications 1 à 6.

8. Dispositif électroluminescent organique selon la revendication 7, comprenant :
une anode ;
une cathode ;
une couche électroluminescente disposée entre la cathode et l'anode ; et
une région de transport d'électrons disposée entre la cathode et la couche électroluminescente,
dans lequel la région de transport d'électrons comprend le composé électroluminescent organique.

9. Dispositif électroluminescent organique selon la revendication 8, dans lequel la région de transport d'électrons comprend au moins l'une parmi une couche de transport d'électrons et une couche auxiliaire de transport d'électrons, et
le composé électroluminescent organique est contenu dans au moins une couche parmi la couche de transport d'électrons et de la couche auxiliaire de transport d'électrons.

10. Utilisation du composé électroluminescent organique selon l'une quelconque des revendications 1 à 6 pour un dispositif électroluminescent organique.

11. Utilisation selon la revendication 10, dans laquelle le composé électroluminescent organique est utilisé en tant que matériau de transport d'électrons pour le dispositif électroluminescent organique.
